(19) Europäisches Patentamt
European Patent Office
Office européen des brevets

(11) EP 1 857 104 A1

(12) **EUROPEAN PATENT APPLICATION**
published in accordance with Art. 158(3) EPC

(43) Date of publication:
**21.11.2007 Bulletin 2007/47**

(21) Application number: **06728806.8**

(22) Date of filing: **02.03.2006**

(51) Int Cl.:
***A61K 31/00*** *(2006.01)* ***C07D 213/00*** *(2006.01)*
***C07D 215/00*** *(2006.01)* ***C07D 279/00*** *(2006.01)*
***C07D 311/00*** *(2006.01)* ***C07D 401/00*** *(2006.01)*
***C07D 405/00*** *(2006.01)* ***C07D 413/00*** *(2006.01)*
***C07D 417/00*** *(2006.01)* ***C07D 471/00*** *(2006.01)*

(86) International application number:
**PCT/JP2006/304539**

(87) International publication number:
**WO 2006/093339 (08.09.2006 Gazette 2006/36)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **02.03.2005 JP 2005057364**

(71) Applicant: **Sumitomo Chemical Company, Limited**
**Tokyo 104-8260 (JP)**

(72) Inventors:
- **SHIRAKI, Hiroaki**
  **Rockville, MD 20852 (US)**
- **HIGASHI, Kiyoshi**
  **5420012 (JP)**
- **TOMIGAHARA, Yoshitaka**
  **Osaka 5600013 (JP)**
- **TAKAHASHI, Junya**
  **, 6660262 (JP)**

(74) Representative: **Vossius & Partner**
**Siebertstrasse 4**
**81675 München (DE)**

(54) **USE OF CINNAMOYL COMPOUND**

(57) A composition for inhibiting the extracellular matrix gene transcription comprising a cinnamoyl compound represented by the formula(I):

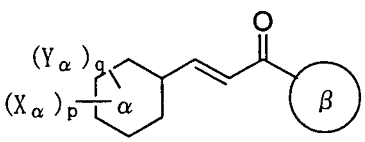

(I)

and an inert carrier, or the like.

EP 1 857 104 A1

SEQUENCE LISTING

<110> Sumitomo Chemical Company Limited

<120> Use of Cinnamoyl compounds

<130> S13494WO01

<150> JP 2005-057364
<151> 2005-03-02

<160> 5

<210> 1
<211> 32
<212> DNA
<213> Artificial Sequence

<220>
<223> Designed oligonucleotide primer to amplify collagen promoter DNA

<400> 1
ccaagctagc gaaattatct tttctttcat ag 32

<210> 2
<211> 28
<212> DNA

<213> Artificial Sequence

<220>

<223> Designed oligonucleotide primer to amplify collagen promoter DNA

<400> 2

ccaaaagctt gcagtcgtgg ccagtacc 28

<210> 3

<211> 22

<212> DNA

<213> Artificial Sequence

<220>

<223> Designed oligonucleotide primer to detect fibronectin DNA

<400> 3

tcgccatcag tagaaggtag ca 22

<210> 4

<211> 25

<212> DNA

<213> Artificial Sequence

<220>

<223> Designed oligonucleotide primer to detect fibronectin DNA

<400> 4

tatactgaac accaggttgc aagtc 25

<210> 5

<211> 30

<212> DNA

<213> Artificial Sequence

<220>

<223> Designed oligonucleotide primer to detect fibronectin DNA

<400> 5

ctcaaccttc ctgaaactgc aaactccgtc 30

## Description

Technical Field

The present invention relates to use of a cinnamoyl compound and the like.

Background Art

In diseases and disorders such as hepatic cirrhosis, chronic pancreatitis, scirrhous gastric cancer, interstitial pulmonary disease, asthma, chronic obstructive pulmonary diseases, glomerular nephritis, lupus nephritis, tubulointerstitial nephritis, IgA nephritis, renal sclerosis, diabetic nephropathy, hereditary renal disease, myocardial fibrosis, heart failure, restenosis after PTCA, atherosclerosis, marrow fibrosis, rheumatoid arthritis, hyperplasia scar after inflammation, postoperative scars or burn scars, atopic dermatitis, hypertrophic scar, hysteromyoma, prostate hypertrophy, scleroderma, Alzheimer disease, sclerotic peritonitis, diabetic retinopathy, and type I diabetes, excessive accumulation of an extracellular matrix, a representative of which is collagen or fibronectin, causes fibrosis and sclerosis of tissues, resulting in decreased functions, cicatrization and the like in the organs or tissues. Such excessive accumulation of an extracellular matrix is induced by increased production of the extracellular matrix due to a breakdown of balance between biosynthesis and degradation of the extracellular matrix. In fact, it has been observed that expression of an extracellular matrix gene such as a collagen gene (in particular, a Type I collagen gene, a Type III collagen gene, or a Type IV collagen gene), a fibronectin gene, a laminin gene, a proteoglycan gene or the like has been increased in a fibrotic tissue [J. Invest. Dermatol., 94, 365, (1990); Proc. Natl. Acad. Sci. USA, 88, 6642, (1991): J. Am. Soc. Nephrol., 15, 2637, (2004); Cardiovasc. Pathol., 13, 119, (2004); Clin. Nephrol., 44, 211, (1995); J. Hepatol., 29, 263, (1998))].

It has been also observed that the amount of TGF-β, which is a cytokine, has been increased in a fibrotic tissue [e.g. J. Invest. Dermatol., 94, 365, (1990) and Proc. Natl. Acad. Sci. USA, 88, 6642, (1991)]. It has been suggested that TGF-β has increased expression of an extracellular matrix gene and been involved in increased production of an extracellular matrix protein and, consequently, fibrosis of a tissue [e.g. J. Invest. Dermatol., 94, 365, (1990); and Lab. Invest., 63, 171, (1990)]. It has been also shown that by administering an anti-TGF-β antibody or a soluble anti-TGF-β receptor to a model animal of tissue fibrosis, improvement of tissue fibrosis has been achieved and thereby the tissue function has been also improved [e.g. Diabetes, 45, 522-530, (1996), Proc. Natl. Acad. Sci. USA, 96, 12719-12724, (1999); and Proc. Natl. Acad. Sci. USA, 97, 8015-8020, (2000)]. It has been also known that by administering a compound which suppressively acts on intracellular signal transduction via TGF-β, improvement in fibrosis of a tissue has been achieved and thereby the tissue function has been also improved [e.g. Autoimmunity, 35, 277-282, (2002); J. Hepatol., 37, 331-339, (2002); and Life Sci., 71, 1559-1606, (2002)].

On the other hand, it is believed that a cause of heart failure such as left ventricular diastolic failure or renal failure such as diabetic nephropathy or renal sclerosis is cardiac fibrosis under a hypertensive condition.

Thus, there is a need for development and provision of a drug which improves fibrosis of a tissue by decreasing expression of an extracellular matrix gene in the tissue to reduce accumulation of the extracellular matrix (i.e. an extracellular matrix accumulation-suppressing agent, a fibrosing disease-treating agent, or a heart failure-treating agent).

Disclosure of Invention

The present invention relates to compounds represented by the following formulas (I) to (VI), (I'), (II') and (V') having the ability to suppress transcription of an extracellular matrix gene.

That is, the present invention provides:

1. A composition for suppressing transcription of an extracellular matrix gene which comprises an inert carrier and a cinnamoyl compound represented by the formula (I):

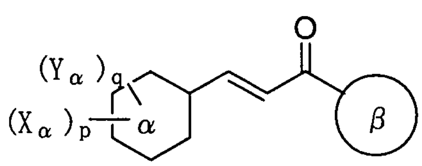

(I)

wherein,

I. α represents a benzene ring or a pyridine ring; in $(Y_\alpha)_q$, $Y_\alpha$ is a substituent on a carbon atom and represents

a group included in the following $X_0$ group or the $Y_0$ group, q represents 0, 1, 2, 3, 4 or 5, and when q is not less than 2, $Y_\alpha$s are the same or different, and when q is not less than 2, adjacent two same or different $Y_\alpha$s may together form a group included in the $Z_0$ group to be fused to the $\alpha$ ring; and in $(X_\alpha)_p$, $X_\alpha$ is a substituent on a carbon atom and represents a group which does not belong to the following $X_0$ group, $Y_0$ group and $Z_0$ group, p represents 0, 1, 2, 3, 4 or 5, and when p is not less than 2, $X_\alpha$s are the same or different and the sum of p and q is not more than 5;

(1) the $X_0$ group: a $M_a$-group, wherein $M_a$ represents a $R_b$- group (wherein $R_b$ represents a C1-C10 alkyl group optionally substituted with a halogen atom), a halogen atom, a nitro group, a cyano group, a hydroxyl group, a $R_c$-$B_a$-$R_d$-group (wherein $R_c$ represents a C1-C10 alkyl group optionally substituted with a halogen atom, $B_a$ represents an oxy group, a thio group, a sulfinyl group or a sulfonyl group, and $R_d$ represents a single bond or a C1-C10 alkylene group), an $HOR_d$- group (wherein $R_d$ is as defined above), a $R_e$-CO-$R_d$- group (wherein $R_e$ represents a hydrogen atom, or a C1-C10 alkyl group optionally substituted with a halogen atom, and $R_d$ is as defined above), a $R_e$-CO-O-$R_d$- group (wherein $R_e$ and $R_d$ are as de-fined above), a $R_e$O-CO-$R_d$- group (wherein $R_e$ and $R_d$ are as defined above), an HO-CO-CH=CH-group, a $R_eR_e$'N-$R_d$- group (wherein $R_e$ and $R_e$' are the same or different, $R_e$ is as defined above, $R_e$' has the same meaning as $R_e$ has, and $R_d$ is as defined above), a $R_e$-CO-$NR_e$'-$R_d$- group (wherein $R_e$, $R_e$' and $R_d$ are as defined above), a $R_b$O-CO-N($R_e$)-$R_d$- group (wherein $R_b$, $R_e$ and $R_d$ are as defined above), a $R_eR_e$'N-CO-$R_d$- group (wherein $R_e$, $R_e$' and $R_d$ are as defined above), a $R_eR_e$'N-CO-$NR_e$"-$R_d$- group (wherein $R_e$, $R_e$' and $R_e$" are the same or different, $R_e$ and $R_e$' are as defined above, $R_e$" has the same meaning as $R_e$ has, and $R_d$ is as defined above), a $R_eR_e$'N-C(=$NR_e$")-$NR_e$"'-$R_d$- group (wherein $R_e$, $R_e$', $R_e$" and $R_e$"' are the same or different, Re, $R_e$' and $R_e$" are as defined above, $R_e$"' has the same meaning as $R_e$ has, and $R_d$ is as defined above), a $R_b$-$SO_2$-$NR_e$-$R_d$- group (wherein $R_b$, $R_e$ and $R_d$ are as defined above), a $R_eR_e$'N-$SO_2$-$R_d$- group (wherein $R_e$, $R_e$' and $R_d$ are as defined above), a C2-C10 alkenyl group or a C2-C10 alkynyl group;

(2) the $Y_0$ group: a $M_{b0}$-$R_d$- group, wherein $M_{b0}$ represents a $M_{c0}$- group [wherein $M_{co}$ represents a $M_{do}$-$R_d$'- group {wherein $M_{do}$ represents a 6 to 10-membered aryl group optionally substituted with a $M_a$- group (wherein $M_a$ is as defined above), a 5 to 10-membered heteroaryl group optionally substituted with a $M_a$-group (wherein $M_a$ is as defined above), a 3 to 10-membered cyclic hydrocarbon or heterocyclic group optionally substituted with a $M_a$- group (wherein $M_a$ is as defined above) and optionally containing an unsaturated bond, a ($b_0$)- group represented by

($b_0$) 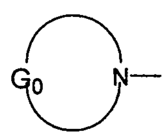

(wherein Go forms an optionally substituted, saturated or unsaturated, nonaromatic 5 to 14-membered hydrocarbon ring or heterocyclic ring), a ($c_0$)- group represented by

($c_0$) 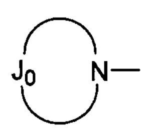

(wherein, $J_0$ forms a 5 to 7-membered aromatic ring optionally containing a nitrogen atom), a (do)- group represented by

($d_0$) 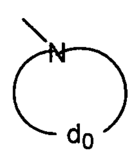

[wherein do forms a 5 to 12-membered hydrocarbon ring which is substituted with a carbonyl group or a thiocarbonyl group and further which may be optionally substituted with an oxy group, a thio group, a -$NR_1$-group {wherein $R_1$ represents a hydrogen atom, or a C1-C10 alkyl group, or a C2-C10 alkyl group substituted with a halogen atom or a $R_2$-$B_1$- group (wherein $R_2$ represents a C1-C10 alkyl group, a C3-C10 alkenyl group or a C3-C10 alkynyl group, and $B_1$ represents an oxy group, a thio group, a sulfinyl group or a sulfonyl group), a C3-C10 alkenyl group, or a C3-C10 alkynyl group}, a sulfinyl group, or a sulfonyl group], or a ($e_0$)-group represented by

($e_0$)

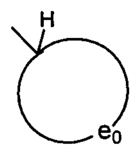

{wherein $e_0$ forms a 5 to 12-membered hydrocarbon ring optionally substituted with a carbonyl group, a thiocarbonyl group, an oxy group, a thio group, a -$NR_1$-group (wherein $R_1$ is as defined above), a sulfinyl group or a sulfonyl group}; and $R_d$' is the same as or different from $R_d$ and has the same meaning as $R_d$ has], a $M_{c0}$-$B_a$- group (wherein $M_{c0}$ and $B_a$ are as defined above), a $M_{c0}$-CO- group (wherein $M_{c0}$ is as defined above), a $M_{c0}$-CO-O-group (wherein $M_{c0}$ is as defined above), a $M_{c0}$O-CO- group (wherein $M_{c0}$ is as defined above), a $M_{c0}R_eN$- group (wherein $M_{c0}$ and $R_e$ are as defined above), a $M_{c0}$-CO-$NR_e$- group (wherein $M_{c0}$ and $R_e$ are as defined above), a $M_{c0}$O-CO-$NR_e$-group (wherein $M_{c0}$ and $R_e$ are as defined above), a $M_{c0}R_eN$-CO-group (wherein $M_{c0}$ and $R_e$ are as defined above), a $M_{c0}R_eN$-CO-$NR_e$'- group (wherein $M_{c0}$, $R_e$ and $R_e$' are as defined above), a $M_{c0}R_eN$-C(=$NR_e$')-$NR_e$"- group (wherein $M_{c0}$, Re, $R_e$' and $R_e$" are as defined above), a $M_{c0}$-$SO_2$-$NR_e$- group (wherein $M_{c0}$ and $R_e$ are as defined above) or a $M_{c0}R_eN$-$SO_2$-group (wherein $M_{c0}$ and $R_e$ are as defined above), and $R_d$ is as defined above;

(3) the $Z_0$ group: a 5- to 12-membered hydrocarbon ring or heterocyclic ring which may be substituted with a halogen atom, a C1-C10 alkoxy group, C3-C10 alkenyloxy group, a C3-C10 alkynyloxy group, a carbonyl group, a thiocarbonyl group, an oxy group, a thio group, a sulfinyl group or a sulfonyl group, and which is aromatic or nonaromatic and monocyclic or fused ring and is fused to the α ring; and

II. β represents

a group represented by formula (I-1):

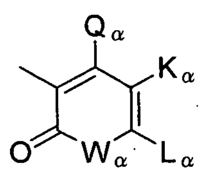

(I-1)

wherein,

(1)$Q_\alpha$ represents an optionally substituted hydroxyl group, or an optionally substituted amino group,

(2)$W_\alpha$ represents an oxygen atom or a -$NT_\alpha$- group (wherein $T_\alpha$ represents a hydrogen atom, or a substituent on the nitrogen atom),

(3)$K_\alpha$ and $L_\alpha$ form a -$V_\alpha$=$V_\alpha$'-$V_\alpha$"=$V_\alpha$"'- group (wherein $V_\alpha$, $V_\alpha$', $V_\alpha$" and $V_\alpha$"' are the same or different, and represent an optionally substituted methine group or a -N= group, and at least one of $V_\alpha$, $V_\alpha$', $V_\alpha$" and $V_\alpha$"' represents a -N= group);

a group represented by formula (I-2):

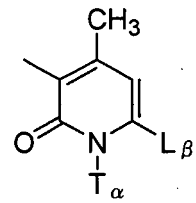

(I-2)

wherein $T_{\alpha}$ is as defined above, and $L_{\beta}$ represents a hydroxyl group or a methyl group;
a group represented by formula (I-3):

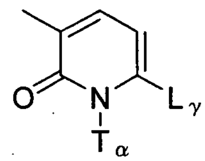

(I-3)

wherein $T_{\alpha}$ is as defined above, and $L_{\gamma}$ represents a C1-C10 alkyl group;
a group represented by formula (I-4):

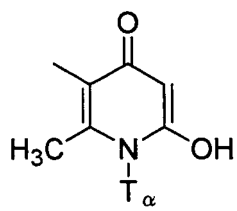

(I-4)

wherein $T_{\alpha}$ is as defined above;
a group represented by formula (I-5):

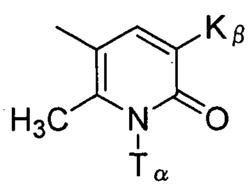

(I-5)

wherein $T_{\alpha}$ is as defined above, and $K_{\beta}$ represents a cyano group or a UOCO- group (wherein U represents a hydrogen atom or a C1-C10 alkyl group);
a group represented by formula (I-6):

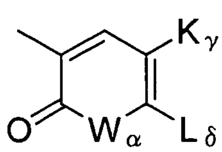

(I-6)

wherein $W_{\alpha}$ is as defined above, and $K_{\gamma}$ and $L_{\delta}$ form an optionally substituted C3-C10 alkylene group or an optionally substituted C4-C10 alkenylene group;
a group represented by formula (I-7):

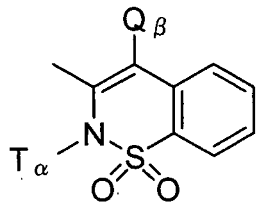

(I-7)

wherein $T_\alpha$ is as defined above, and $Q_\beta$ represents an optionally substituted hydroxyl group; or a group represented by formula (I-8):

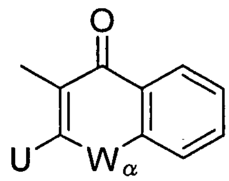

(I-8)

wherein U and $W_\alpha$ are as defined above;
the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range;

2. A cinnamoyl compound represented by the formula (II):

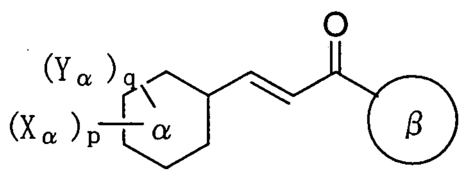

(II)

wherein,

I. $\alpha$ represents a benzene ring or a pyridine ring; in $(Y_\alpha)_q$, $Y_\alpha$ is a substituent on a carbon atom and represents a group included in the following $X_0$ group or the $Y_0$ group, q represents 0, 1, 2, 3, 4 or 5, and when q is not less than 2, $Y_\alpha$s are the same or different, and when q is not less than 2, adjacent two same or different $Y_\alpha$s may together form a group included in the $Z_0$ group to be fused to the $\alpha$ ring; and in $(X_\alpha)_p$, $X_\alpha$ is a substituent on a carbon atom and represents a group which does not belong to the following $X_0$ group, $Y_0$ group and $Z_0$ group, p represents 0, 1, 2, 3, 4 or 5, and when p is not less than 2, $X_\alpha$s are the same or different and the sum of p and q is not more than 5;

(1) the $X_0$ group: a $M_a$-group, wherein $M_a$ represents a $R_b$- group (wherein $R_b$ represents a C1-C10 alkyl group optionally substituted with a halogen atom), a halogen atom, a nitro group, a cyano group, a hydroxyl group, a $R_c$-$B_a$-$R_d$-group (wherein $R_c$ represents a C1-C10 alkyl group optionally substituted with a halogen atom, $B_a$ represents an oxy group, a thio group, a sulfinyl group or a sulfonyl group, and $R_d$ represents a single bond or a C1-C10 alkylene group), an $HOR_d$- group (wherein $R_d$ is as defined above), a $R_e$-CO-$R_d$- group (wherein $R_e$ represents a hydrogen atom, or a C1-C10 alkyl group optionally substituted with a halogen atom, and $R_d$ is as defined above), a $R_e$-CO-O-$R_d$- group (wherein $R_e$ and $R_d$ are as defined above), a $R_e$O-CO-$R_d$- group (wherein $R_e$ and $R_d$ are as defined above), an HO-CO-CH=CH-group, a $R_eR_e$'N-$R_d$- group (wherein $R_e$ and $R_e$' are the same or different, $R_e$ is as defined above, $R_e$' has the same meaning as $R_e$ has, and $R_d$ is as defined above), a $R_e$-CO-N$R_e$'-$R_d$- group (wherein $R_e$, $R_e$' and $R_d$ are as defined above), a $R_b$O-CO-N($R_e$)-$R_d$- group (wherein $R_b$, $R_e$ and $R_d$ are as defined above), a $R_eR_e$'N-CO-$R_d$- group (wherein $R_e$, $R_e$' and $R_d$ are as defined above), a $R_eR_e$'N-CO-N$R_e$"-$R_d$- group (wherein $R_e$, $R_e$'

and $R_e$" are the same or different, $R_e$ and $R_e$' are as defined above, $R_e$" has the same meaning as $R_e$ has, and $R_d$ is as defined above), a $R_eR_e$'N-C(=N$R_e$")-N$R_e$"'-$R_d$- group (wherein $R_e$, $R_e$', $R_e$" and $R_e$"' are the same or different, $R_e$, $R_e$' and $R_e$" are as defined above, $R_e$"' has the same meaning as $R_e$ has, and $R_d$ is as defined above), a $R_b$-$SO_2$-N$R_e$-$R_d$- group (wherein $R_b$, $R_e$ and $R_d$ are as defined above), a $R_eR_e$'N-$SO_2$-$R_d$- group (wherein $R_e$, $R_e$' and $R_d$ are as defined above), a C2-C10 alkenyl group or a C2-C10 alkynyl group;

(2) the $Y_0$ group: a $M_{b0}$-$R_d$- group, wherein $M_{b0}$ represents a $M_{c0}$- group [wherein $M_{co}$ represents a $M_{do}$-$R_d$'- group {wherein $M_{do}$ represents a 6 to 10-membered aryl group optionally substituted with a $M_a$- group (wherein $M_a$ is as defined above), a 5 to 10-membered heteroaryl group optionally substituted with a $M_a$-group (wherein $M_a$ is as defined above), a 3 to 10-membered cyclic hydrocarbon or heterocyclic group optionally substituted with a $M_a$- group (wherein $M_a$ is as defined above) and optionally containing an unsaturated bond, a ($b_0$)- group represented by

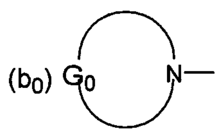

(wherein Go forms an optionally substituted, saturated or unsaturated, nonaromatic 5 to 14-membered hydrocarbon ring or heterocyclic ring), a ($c_0$)- group represented by

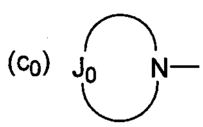

(wherein, $J_0$ forms a 5 to 7-membered aromatic ring optionally containing a nitrogen atom), a (do)- group represented by

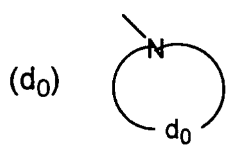

[wherein do forms a 5 to 12-membered hydrocarbon ring which is substituted with a carbonyl group or a thiocarbonyl group and further which may be optionally substituted with an oxy group, a thio group, a -N$R_1$- group {wherein $R_1$ represents a hydrogen atom, or a C1-C10 alkyl group, or a C2-C10 alkyl group substituted with a halogen atom or a $R_2$-$B_1$- group (wherein $R_2$ represents a C1-C10 alkyl group, a C3-C10 alkenyl group or a C3-C10 alkynyl group, and $B_1$ represents an oxy group, a thio group, a sulfinyl group or a sulfonyl group), a C3-C10 alkenyl group, or a C3-C10 alkynyl group}, a sulfinyl group, or a sulfonyl group], or a ($e_0$)- group represented by

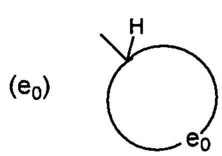

{wherein $e_0$ forms a 5 to 12-membered hydrocarbon ring optionally substituted with a carbonyl group, a thiocarbonyl group, an oxy group, a thio group, a -N$R_1$-group (wherein $R_1$ is as defined above), a sulfinyl group or a sulfonyl group}; and $R_d$' is the same as or different from $R_d$ and has the same meaning as $R_d$ has], a $M_{c0}$-$B_a$- group (wherein $M_{c0}$ and $B_a$ are as defined above), a $M_{c0}$-CO- group (wherein $M_{c0}$ is as defined

above), a $M_{c0}$-CO-O-group (wherein $M_{c0}$ is as defined above), a $M_{c0}$O-CO- group (wherein $M_{c0}$ is as defined above), a $M_{c0}R_eN$- group (wherein $M_{c0}$ and $R_e$ are as defined above), a $M_{c0}$-CO-$NR_e$- group (wherein $M_{c0}$ and $R_e$ are as defined above), a $M_{c0}$O-CO-$NR_e$-group (wherein $M_{c0}$ and $R_e$ are as defined above), a $M_{c0}R_eN$-CO-group (wherein $M_{c0}$ and $R_e$ are as defined above), a $M_{c0}R_eN$-CO-$NR_e$'- group (wherein $M_{c0}$, $R_e$ and $R_e$' are as defined above), a $M_{c0}R_eN$-C(=$NR_e$')-$NR_e$"- group (wherein $M_{c0}$, $R_e$, $R_e$' and $R_e$" are as defined above), a $M_{c0}$-$SO_2$-$NR_e$- group (wherein $M_{c0}$ and $R_e$ are as defined above) or a $M_{c0}R_eN$-$SO_2$-group (wherein $M_{c0}$ and $R_e$ are as defined above), and $R_d$ is as defined above;

(3) the $Z_0$ group: a 5- to 12-membered hydrocarbon ring or heterocyclic ring which may be substituted with a halogen atom, a C1-C10 alkoxy group, C3-C10 alkenyloxy group, a C3-C10 alkynyloxy group, a carbonyl group, a thiocarbonyl group, an oxy group, a thio group, a sulfinyl group or a sulfonyl group, and which is aromatic or nonaromatic and monocyclic or fused ring and is fused to the α ring; and

II. β represents
a group represented by formula (II-1):

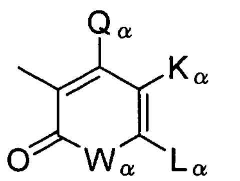

(II-1)

wherein,

(1) $Q_\alpha$ represents an optionally substituted hydroxyl group, or an optionally substituted amino group,
(2) $W_\alpha$ represents an oxygen atom or a -$NT_\alpha$- group (wherein $T_\alpha$ represents a hydrogen atom, or a substituent on the nitrogen atom),
(3) $K_\alpha$ and $L_\alpha$ form a -$V_\alpha$=$V_\alpha$'-$V_\alpha$"=$V_\alpha$"'- group (wherein $V_\alpha$, $V_\alpha$', $V_\alpha$" and $V_\alpha$"' are the same or different, and represent an optionally substituted methine group or a -N= group, and at least one of $V_\alpha$, $V_\alpha$', $V_\alpha$" and $V_\alpha$"' represents a -N= group);
a group represented by formula (II-2):

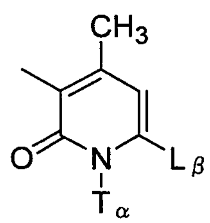

(II-2)

wherein $T_\alpha$ is as defined above, and $L_\beta$ represents a hydroxyl group or a methyl group;
a group represented by formula (II-3):

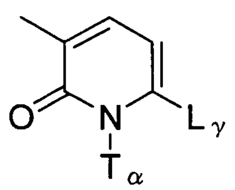

(II-3)

wherein $T_\alpha$ is as defined above, and $L_\gamma$ represents a C1-C10 alkyl group;
a group represented by formula (II-4):

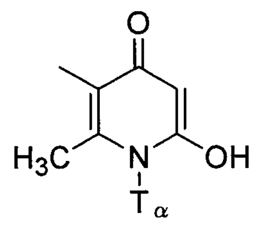

(II-4)

wherein $T_\alpha$ is as defined above;
a group represented by formula (II-5):

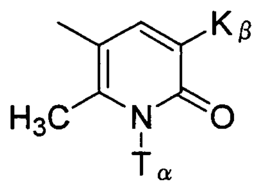

(II-5)

wherein $T_\alpha$ is as defined above, and $K_\beta$ represents a cyano group or a UOCO- group (wherein U represents a hydrogen atom or a C1-C10 alkyl group);
a group represented by formula (II-6):

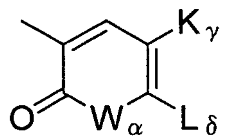

(II-6)

wherein $W_\alpha$ is as defined above, and $K_\gamma$ and $L_\delta$ form an optionally substituted C3-C10 alkylene group or an optionally substituted C4-C10 alkenylene group;
a group represented by formula (II-7):

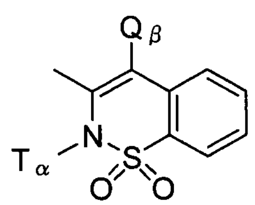

(II-7)

wherein $T_\alpha$ is as defined above, and $Q_\beta$ represents an optionally substituted hydroxyl group; or
a group represented by formula (II-8):

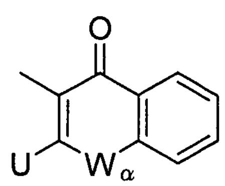

(II-8)

wherein U and $W_\alpha$ are as defined above;
the term "as defined above" used for the same symbols among plural substituents means that the plural substituents

independently represent the same meaning as that described above and, among the plural substituents, although the selection range of substituents to be selected is the same, selected substituents may be the same or different as long as they are selected within the range;

3. A cinnamoyl compound represented by the formula (III):

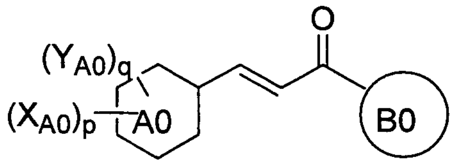

(III)

wherein

I. A0 represents a benzene ring or pyridine ring;

II. in $(X_{A0})_p$, $X_{A0}$ is a substituent on a carbon atom and represents a group included in any group of the following $A_0$ group to the No group, p represents 0, 1, 2, 3, 4 or 5, and when p is not lees than 2, $X_{A0}$s are the same or different;

(1) the $A_0$ group:

a $D_1$-$R_4$- group, wherein $D_1$ represnts a $(R_1$-$(O)_k$-$)A_1N$-$(O)_{k'}$- group [wherein $R_1$ represents a hydrogen atom, or a C1-C10 alkyl group, or a C2-C10 alkyl group substituted with a halogen atom or a $R_2$-$B_1$-group (wherein $R_2$ represents a C1-C10 alkyl group, a C3-C10 alkenyl group or a C3-C10 alkynyl group, and $B_1$ represents an oxy group, a thio group, a sulfinyl group or a sulfonyl group), or a C3-C10 alkenyl group, or a C3-C10 alkynyl group, k represents 0 or 1, $A_1$ represents a $R_3$-$(CHR_0)_m$-$(8_2$-$8_3)_{m'}$- group {wherein $R_3$ represents a hydrogen atom, or a C1-C10 alkyl group optionally substituted with a halogen atom or a $R_2$-$B_1$-group (wherein $R_2$ and $B_1$ are as defined above), or a C2-C10 alkenyl group, or a C2-C10 alkynyl group, $R_0$ represents a hydrogen atom, a C1-C10 alkyl group or a C2-C10 haloalkyl group, m represents 0 or 1, $B_2$ represents a single bond, an oxy group, a thio group or a -$N((O)_nR_1')$- group (wherein $R_1'$ is the same as or different from $R_1$, and has the same meaning as $R_1$ has, and n represents 0 ro 1), $B_3$ represents a carbonyl group, a thiocarbonyl group or a sulfonyl group, m' represents 0 or 1, and $B_3$ is not a sulfonyl group when m is 0 and $R_3$ is a hydrogen atom}, and k' represents 0 or 1], and $R_4$ represents a C1-C10 alkylene group, provided that a $R_0'R_0''N$-$R_4$- group (wherein $R_0'$ and $R_0''$ are the same as or different from $R_0$ and have the same meaning as $R_0$ has, and $R_4$ is as defined above) is excluded,

a $D_2$-$R_4$- group, wherein $D_2$ represents a cyano group, a $R_1R_1'NC(=N$-$(O)_n$-$A_1)$- group (wherein $R_1$, $R_1'$, n and $A_1$ are as defined above), an $A_1N=C(-OR_2)$- group (wherein $A_1$ and $R_2$ are as defined above) or a $NH_2$-CS- group, and $R_4$ is as defined above,

a $D_3$-$R_4$- group, wherein $D_3$ represents a nitro group or a $R_1OSO_2$- group (wherein $R_1$ is as defined above), and $R_4$ is as defined above, and

a $R_1OSO_2$- group, wherein $R_1$ is as defined above;

(2) the $B_0$ group: an $(a_0)$- group represented by

$(a_0)$ 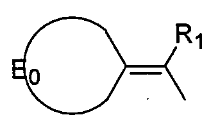

wherein $E_0$ forms an optionally substituted, saturated or unsaturated, aromatic or nonaromatic 5 to 14-membered hydrocarbon ring or heterocyclic ring, and $R_1$ is as defined above;

(3) the $C_0$ group: a C2-C10 alkenyl group substituted with a halogen atom, a $R_2$-$B_1$- group (wherein $R_2$ and $B_1$ are as defined above), a $D_4$-$R_4$- group [wherein $D_4$ represents a hydroxyl group or an $A_1$-O- group (wherein $A_1$ is as defined above), and $R_4$ is as defined above], a $D_5$- group [wherein $D_5$ represents a O=C$(R_3)$- group (wherein $R_3$ is as defined above), an $A_1$-$(O)_n$-$N=C(R_3)$- group (wherein $A_1$, n and $R_3$ are as

defined above), a $R_1$-$B_0$-CO-$R_4$-$(O)_n$-N=C($R_3$)- group {wherein $R_1$, $R_4$, n and $R_3$ are as defined above, and $B_0$ represents an oxy group, a thio group or a -N($(O)_m R_1$')-group (wherein $R_1$' and m are as defined above)}, a $D_2$-$R_4$-$(O)_n$-N=C($R_3$)- group (wherein $D_2$, $R_4$, n and $R_3$ are as defined above) or a $R_1A_1$N-N=C($R_3$)- group (wherein $R_1$, $A_1$ and $R_3$ are as defined above)], a $R_1A_1$N-O-$R_4$- group (wherein $R_1$, $A_1$ and $R_4$ are as defined above), a $R_1$($A_1$-$(O)_n$-)N- group (wherein $R_1$, $A_1$ and n are as defined above), a $D_2$- group (wherein $D_2$ is as defined above) or a $D_3$- group (wherein $D_3$ is as defined above);

(4) the $D_0$ group: a C2-C10 alkynyl group substituted with a ($b_0$)-$R_4$- group (in ($b_0$)

($b_0$) 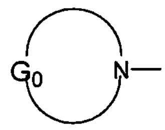 ,

Go forms an optionally substituted, saturated or unsaturated, nonaromatic 5 to 14-membered hydrocarbon ring or heterocyclic ring), a ($c_0$)-$R_4$- group (in ($c_0$)

($c_0$) 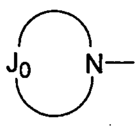 ,

$J_0$ forms an aromatic 5 to 7-membered ring optionally containing a nitrogen atom and $R_4$ is as defined above), a halogen atom, a $R_2$-$B_1$-$R_4$- group (wherein $R_2$, $B_1$ and $R_4$ are as defined above), a $D_4$-$R_4$- group (wherein $D_4$ and $R_4$ are as defined above), a $D_5$- group (wherein $D_5$ is as defined above), a $D_1$-$R_4$- group (wherein $D_1$ and $R_4$ are as defined above), a $D_2$- group (wherein $D_2$ is as defined above) or a $D_3$-$R_4$- group (wherein $D_3$ and $R_4$ are as defined above);

(5) the $E_0$ group: an $A_2$-CO-$R_5$- group, provided that $R_5$ is not a vinylene group when $A_2$ is a hydroxyl group [wherein $A_2$ represents

(i) an $A_3$-$B_4$- group
wherein $A_3$ represents a hydrogen atom, or a C1-C10 alkyl group, or a C2-C10 haloalkyl group, or a C2-C10 alkenyl group optionally substituted with a halogen atom, or a C3-C10 alkynyl group optionally substituted with a halogen atom, or a $R_{a0}$-$(R_4)_m$- group (wherein $R_{a0}$ represents an optionally substituted 5 to 7-membered aryl group or heteroaryl group, and $R_4$ and m are as defined above), or a C1-C10 alkyl group substituted with a ($b_0$)-$R_4$- group (wherein ($b_0$) and $R_4$ are as defined above), a ($c_0$)-$R_4$- group (wherein ($c_0$) and $R_4$ are as defined above), a $R_2$-$B_1$-$R_4$-group (wherein $R_2$, $B_1$ and $R_4$ are as defined above), a $D_4$-$R_4$-group (wherein $D_4$ and $R_4$ are as defined above), a $D_5$- group (wherein $D_5$ is as defined above), a $D_1$-$R_4$- group (wherein $D_1$ and $R_4$ are as defined above), a $D_2$- group (wherein $D_2$ is as defined above), a $D_3$-$R_4$- group (wherein $D_3$ and $R_4$ are as defined above) or an $A_4$-$SO_2$-$R_4$- group {wherein $A_4$ represents a ($b_0$)- group (wherein ($b_0$) is as defined above), a ($c_0$)-group (wherein ($c_0$) is as defined above) or a $R_1R_1$' N- group (wherein $R_1$ and $R_1$' are as defined above), and $R_4$ is as defined above}, and
$B_4$ represents an oxy group, a thio group or a -N($(O)_m R_1$)-group (wherein $R_1$ and m are as defined above), provided that $A_3$ is not a hydrogen atom when $B_4$ is a thio group;
(ii) a $R_1$-$B_4$-CO-$R_4$-$B_4$'- group (wherein $R_1$, $B_4$ and $R_4$ are as defined above, $B_4$' is the same as or different from $B_4$ and has the same meaning as $B_4$ has, provided that $R_2$ is not a hydrogen atom when $B_4$ is a thio group) or a $D_2$-$R_4$-$B_4$-group (wherein $D_2$, $R_4$ and $B_4$ are as defined above);
(iii) a $R_2$-$SO_2$-$NR_1$- group (wherein $R_2$ is as defined above, provided that a hydrogen atom is excluded, and $R_1$ is as defined above);
(iv) a ($b_0$)- group, wherein ($b_0$) is as defined above;
(v) a ($c_0$)- group, wherein ($c_0$) is as defined above; or
(vi) a $R_1A_1$N-$NR_1$'- group, wherein $R_1$, $A_1$ and $R_1$' are as defined above; and
$R_5$ represents a C2-C10 alkenylene group optionally substituted with a halogen atom or a C2-C10 alkynylene group];

(6) the $F_0$ group: an $A_5$-$B_5$-$R_6$- group, wherein
$A_5$ represents a C2-C10 alkyl group substituted with a $D_4$- group (wherein $D_4$ is as defined above), a $D_1$-group (wherein $D_1$ is as defined above), a $D_3$- group (wherein $D_3$ is as defined above) or an $A_4$-$SO_2$- group (wherein $A_4$ is as defined above), or a C1-C10 alkyl group substituted with a $R_2$-$B_1$- group (wherein $R_2$ and $B_1$ are as defined above), a $D_2$-group (wherein $D_2$ is as defined above), a $D_5$- group (wherein $D_5$ is as defined above) or an $A_2$-CO- group (wherein $A_2$ is as defined above), and
$B_5$ represents a $B_1$- group (wherein $B_1$ is as defined above) or a -$NA_1$- group (wherein $A_1$ is as defined above) and $R_6$ represent a single bond or a C1-C10 alkylene group;
(7) the Go group: an $A_6$-$B_5$-$R_6$- group wherein
$A_6$ represents an $(a_0)$-$R_4$- group (wherein $(a_0)$ and $R_4$ are as defined above), or a C2-C10 alkenyl group, or a C2-C10 alkynyl group, or a C2-C10 alkenyl group substituted with a halogen atom, a $R_2$-$B_1$- group (wherein $R_2$ and $B_1$ are as defined above), a $D_5$- group (wherein $D_5$ is as defined above), a $D_2$- group (wherein $D_2$ is as defined above) or an $A_2$-CO- group (wherein $A_2$ is as defined above), or a C2-C10 alkynyl group substituted with a halogen atom, a $R_2$-$B_1$-group (wherein $R_2$ and $B_1$ are as defined above), a $D_5$-group (wherein $D_5$ is as defined above), $D_2$- group (wherein $D_2$ is as defined above) or an $A_2$-CO- group (wherein $A_2$ is as defined above), or a C3-C10 alkenyl group substituted with a $(b_0)$- group (wherein $(b_0)$ is as defined above), a $(c_0)$-group (wherein $(c_0)$ is as defined above), a $D_4$- group (wherein $D_4$ is as defined above), a $D_1$- group (wherein $D_1$ is as defined above) or a $D_3$- group (wherein $D_3$ is as defined above), or a C3-C10 alkynyl group substituted with a $D_4$-group (wherein $D_4$ is as defined above), a $D_1$- group (wherein $D_1$ is as defined above) or a $D_3$- group (wherein $D_3$ is as defined above), and
$B_5$ and $R_6$ are as defined above;
(8) the $H_0$ group:

a $D_2$-N(-$(O)_n$-$A_1$)-$R_6$- group (wherein $D_2$, n, $A_1$ and $R_6$ are as defined above),
a $D_2$- group (wherein $D_2$ is as defined above, provided that a cyano group is excluded),
a $R_1$($R_1$'$(O)_n$)N-$CR_1$"=N-$R_6$- group (wherein $R_1$, $R_1$', n and $R_6$ are as defined above, $R_1$" is the same as or different from $R_1$ and has the same meaning as $R_1$ has),
a $R_1$-$(O)_n$-N=$CR_1$'-$NR_2$-$R_6$- group (wherein $R_1$, n, $R_1$', $R_2$ and $R_6$ are as defined above),
a $R_2$-$B_3$-$NR_1$-CO-$NR_1$'-$R_6$- group (wherein $R_2$, $B_3$, $R_1$, $R_1$' and $R_6$ are as defined above),
a $D_2$-CO-$NR_1$-$R_6$- group (wherein $D_2$, $R_1$ and $R_6$ are as defined above), and
an $A_2$-COCO-$NR_1$-$R_6$- group (wherein $A_2$, $R_1$ and $R_6$ are as defined above);

(9) the $I_0$ group:

an $A_7$-$B_6$-N($(O)_n$$R_1$)-$R_6$- group [wherein $A_7$ represents a C2-C10 alkenyl group optionally substituted with a halogen atom, or a C2-C10 alkynyl group, or a C3-C10 haloalkynyl group, or a $R_2$-$B_1$-$R_4$- group (wherein $R_2$, $B_1$ and $R_4$ are as defined above), or a $D_4$-$R_4$- group (wherein $D_4$ and $R_4$ are as defined above), or a $D_5$-$R_4$- group (wherein $D_5$ and $R_4$ are as defined above), or a $D_1$-$R_4$- group (wherein $D_1$ and $R_4$ are as defined above), or a $(b_0)$-$R_4$- group (wherein $(b_0)$ and $R_4$ are as defined above), or a $(c_0)$-$R_4$- group (wherein $(c_0)$ and $R_4$ are as defined above), or a $D_2$-$R_4$- group (wherein $D_2$ and $R_4$ are as defined above), or a $D_3$-$R_4$- group (wherein $D_3$ and $R_4$ are as defined above), or an $A_4$-$SO_2$-$R_4$- group (wherein $A_4$ and $R_4$ are as defined above), or an $A_2$-CO-$R_4$- group (wherein $A_2$ and $R_4$ are as defined above), $B_6$ represents a carbonyl group or a thiocarbonyl group, and n, $R_1$ and $R_6$ are as defined above],
an $A_8$-CS-N($(O)_n$$R_1$)-$R_6$- group [wherein $A_8$ represents a hydrogen atom or a C1-C10 alkyl group optionally substituted with a halogen atom, and n, $R_1$ and $R_4$ are as defined above],
an $A_7$'-$B_2$'-$B_3$-N($(O)_n$$R_1$)-$R_6$- group [wherein $A_7$' represents a C3-C10 alkenyl group optionally substituted with a halogen atom, or a C3-C10 alkynyl group optionally substituted with a halogen atom, or a $R_2$-$B_1$-$R_4$'- group (wherein $R_2$ and $B_1$ are as defined above, and $R_4$' represents a C2-C10 alkylene group), or a $D_4$-$R_4$'- group (wherein $D_4$ and $R_4$' are as defined above), or a $D_1$-$R_4$' - group (wherein $D_1$ and $R_4$' are as defined above), or a $(b_0)$-$R_4$'- group (wherein $(b_0)$ and $R_4$' are as defined above), or a $(c_0)$-$R_4$'- group (wherein $(c_0)$ and $R_4$' are as defined above), or a $D_2$-$R_4$-group (wherein $D_2$ and $R_4$ are as defined above), or a $D_3$-$R_4$'-group (wherein $D_3$ and $R_4$' are as defined above), or an $A_2$-CO-$R_4$-group (wherein $A_2$ and $R_4$ are as defined above), $B_2$' represents an oxy group, a thio group or a -N($(O)_n$, $R_1$')-group (wherein n' is the same as or different from n and has the same meaning as n has, and $R_1$' is as defined above), and $B_3$, n, $R_1$ and $R_6$ are as defined above],
an $A_8$'-$B_2$'-CS-N($(O)_n$$R_1$)-$R_6$- group [wherein $A_8$' represents a C1-C10 alkyl group or a C2-C10 haloalkyl group, $B_2$' is as defined above, and n, $R_1$ and $R_6$ are as defined above],

an $A_8$'-S-$B_3$'-N((O)$_n$$R_1$)-$R_6$- group [wherein $A_8$', n, $R_1$ and $R_6$ are as defined above, and $B_3$' represents a carbonyl group or a sulfonyl group], and
an $A_7$"-$SO_2$-N((O)$_n$$R_1$)-$R_6$- group [wherein $A_7$" represents a C2-C10 alkenyl group, or a C3-C10 alkenyl group substituted with a halogen atom, or a C3-C10 alkynyl group optionally substituted with a halogen atom, or a $R_2$-$B_1$-$R_4$'- group (wherein $R_2$, $B_1$ and $R_4$' are as defined above), or a $D_4$-$R_4$'-group (wherein $D_4$ and $R_4$' are as defined above), or a $D_5$-$R_4$-group (wherein $D_5$ and $R_4$ are as defined above), or a $D_1$-$R_4$'-group (wherein $D_1$ and $R_4$' are as defined above), or a ($b_0$)-$R_4$'- group (wherein ($b_0$) and $R_4$' are as defined above), or a ($c_0$)-$R_4$'- group (wherein ($c_0$) and $R_4$' are as defined above), or a $D_2$-$R_4$-group (wherein $D_2$ and $R_4$ are as defined above), or a $NO_2$-$R_4$- group (wherein $R_4$ is as defined above), or an $A_2$-CO-$R_4$- group (wherein $A_2$ and $R_4$ are as defined above), and n, $R_1$ and $R_6$ are as defined above];

(10) the $J_0$ group:

an $A_7$-CO- group (wherein $A_7$ is as defined above),
an $A_9$-CS- group (wherein $A_9$ represents $A_7$ or $A_8$),
an $A_9$' (O)$_m$N=C($A_9$)- group (wherein $A_9$' represents $A_7$' or $A_8$', and m and $A_9$ are as defined above),
a $D_2$-CO- group (wherein $D_2$ is as defined above),
an $A_2$-COCO- group (wherein $A_2$ is as defined above),
an $A_9$-CO-$B_1$'-$R_6$- group (wherein $A_9$ and $R_6$ are as defined above, and $B_1$' represents an oxy group or a thio group, provided that $A_9$ is not $A_8$ when $B_1$' is an oxy group),
an $A_9$-CS-$B_1$'-$R_6$- group (wherein $A_9$, $B_1$' and $R_6$ are as defined above),
an $A_7$"-$SO_2$-$B_1$'-$R_6$- group (wherein $A_7$", $B_1$' and $R_6$ are as defined above),
an $A_8$-$SO_2$-$B_1$'-$R_6$- group (wherein $A_8$, $B_1$' and $R_6$ are as defined above, provided that $A_8$ is not a hydrogen atom),
an $A_9$'-$B_2$'-$B_3$-$B_1$'-$R_6$- group (wherein $A_9$', $B_2$', $B_3$, $B_1$' and $R_6$ are as defined above), and
a C2-C10 alkenyl group substituted with a ($b_0$)- group (wherein ($b_0$) is as defined above) or a ($c_0$)- group (wherein ($c_0$) is as defined above);

(11) the $K_0$ group: an $A_{10}$-N((O)$_n$$R_1$)-CO-$R_6$- group,
wherein $A_{10}$ represents a hydrogen atom (provided that n is not 0), an $A_7$"-$SO_2$- group (wherein $A_7$" is as defined above), an $A_8$-$SO_2$- group (wherein $A_8$ is as defined above, provided that $A_8$ is not a hydrogen atom), an $A_9$'O- group (wherein $A_9$' is as defined above, (provided that n is not 1), an $A_9$'-group (wherein $A_9$' is as defined above, provided that $A_8$' is excluded when n is 0), a $R_2OCH_2$- group (wherein $R_2$ is as defined above), an $A_2$-CO-$R_4$- group (wherein $A_2$ and $R_4$ are as defined above) or an $A_2$-CO-CH($CH_2$CO-$A_2$)- group (wherein $A_2$ is as defined above), and n, $R_1$ and $R_6$ are as defined above;
(12) the $L_0$ group:

an $A_{10}$'-N((O)$_n$$R_1$)-$SO_2$-$R_6$- group [wherein $A_{10}$' represents a hydrogen atom (provided that n is not 1), an $A_9$'O- group (wherein $A_9$' is as defined above, provided that n is not 0), an $A_9$'- group (wherein $A_9$' is as defined above, provided that $A_8$' is excluded when n is 0), a $R_2$-CO- group (wherein $R_2$ is as defined above), an $A_2$-CO-$R_4$- group (wherein $A_2$ and $R_4$ are as defined above) or an $A_2$-CO-CH ($CH_2$CO-$A_2$)- group (wherein $A_2$ is as defined above), and n, $R_1$ and $R_6$ are as defined above],
an Ag"$R_1$N-$SO_2$-N((O)$_n$$R_1$')-$R_6$- group [wherein $A_9$" represents a hydrogen atom or an $A_9$'- group (wherein $A_9$' is as defined above), and $R_1$, n, $R_1$' and $R_6$ are as defined above] and
a ($b_0$)-$SO_2$-N((O)$_n$$R_1$')-$R_6$- group [wherein ($b_0$), n, $R_1$' and $R_6$ are as defined above];

(13) the $M_0$ group:

a $R_1$($R_2$S)C=N-$R_6$- group (wherein $R_1$, $R_2$ and $R_6$ are as defined above),
a $R_2$B($R_2$'B')C=N-$R_6$- group (wherein $R_2$ and $R_6$ are as defined above, $R_2$' is the same as or different from $R_2$ and has the same meaning as $R_2$ has, and B and B' are the same or different and represent an oxy group or a thio group),
a $R_1$$R_1$'N-($R_2$S)C=N-$R_6$- group (wherein $R_1$, $R_1$', $R_2$ and $R_6$ are as defined above),
a $R_1$N=C($SR_2$)-$NR_2$'-$R_6$- group (wherein $R_1$, $R_2$, $R_2$' and $R_6$ are as defined above), and
a $R_1$($R_1$'O)N-$R_6$- group (wherein $R_1$, $R_1$' and $R_6$ are as defined above);

(14) the No group: a $A_{11}$-P(=O) (O$R_1$')-$R_4$- group, wherein $A_{11}$ represents a $R_1$- group (wherein $R_1$ is as

defined above), a $R_1O$-$R_6$- group (wherein $R_1$ and $R_6$ are as defined above) or a $R_1OCO$-$CHR_0$- group (wherein $R_1$ and $R_0$ are as defined above), and $R_1$' and $R_4$ are as defined above;

III. in $(Y_{A0})_q$, $Y_{A0}$ is a substituent on a carbon atom and represents a group included in the following $X_0$ group and $Y_0$ group, q represents 0, 1, 2, 3, 4 or 5, the sum of p (wherein p is as defined above) and q is not more than 5, and when q is not less than 2, $Y_{A0}$s are the same or different, and when q is not less than 2, adjacent two same or different $Y_{A0}$s may together form a group included in the $Z_0$ group to be fused to the A0 ring;

(1) the $X_0$ group: a $M_a$-group, wherein $M_a$ represents a $R_b$- group (wherein $R_b$ represents a C1-C10 alkyl group optionally substituted with a halogen atom), a halogen atom, a nitro group, a cyano group, a hydroxyl group, a $R_c$-$B_a$-$R_d$-group (wherein $R_c$ represents a C1-C10 alkyl group optionally substituted with a halogen atom, $B_a$ represents an oxy group, a thio group, a sulfinyl group or a sulfonyl group, and $R_d$ represents a single bond or a C1-C10 alkylene group), an $HOR_d$- group (wherein $R_d$ is as defined above), a $R_e$-CO-$R_d$- group (wherein $R_e$ represents a hydrogen atom, or a C1-C10 alkyl group optionally substituted with a halogen atom, and $R_d$ is as defined above), a $R_e$-CO-O-$R_d$- group (wherein $R_e$ and $R_d$ are as defined above), a $R_eO$-CO-$R_d$- group (wherein $R_e$ and $R_d$ are as defined above), an HO-CO-CH=CH-group, a $R_eR_e$'N-$R_d$- group (wherein $R_e$ and $R_e$' are the same or different, $R_e$ is as defined above, $R_e$' has the same meaning as $R_e$ has, and $R_d$ is as defined above), a $R_e$-CO-$NR_e$'-$R_d$- group (wherein $R_e$, $R_e$' and $R_d$ are as defined above), a $R_bO$-CO-N($R_e$)-$R_d$- group (wherein $R_b$, $R_e$ and $R_d$ are as defined above), a $R_eR_e$'N-CO-$R_d$- group (wherein $R_e$, $R_e$' and $R_d$ are as defined above), a $R_eR_e$'N-CO-$NR_e$"-$R_d$- group (wherein $R_e$, $R_e$' and $R_e$" are the same or different, $R_e$ and $R_e$' are as defined above, $R_e$" has the same meaning as $R_e$ has, and $R_d$ is as defined above), a $R_eR_e$'N-C(=$NR_e$")-$NR_e$"'-$R_d$- group (wherein $R_e$, $R_e$', $R_e$" and $R_e$"' are the same or different, $R_e$, $R_e$' and $R_e$" are as defined above, $R_e$"' has the same meaning as $R_e$ has, and $R_d$ is as defined above), a $R_b$-$SO_2$-$NR_e$-$R_d$- group (wherein $R_b$, $R_e$ and $R_d$ are as defined above), a $R_eR_e$'N-$SO_2$-$R_d$- group (wherein $R_e$, $R_e$' and $R_d$ are as defined above), a C2-C10 alkenyl group or a C2-C10 alkynyl group;

(2) the $Y_0$ group: a $M_{b0}$-$R_d$- group, wherein $M_{b0}$ represents a $M_{c0}$- group [wherein $M_{co}$ represents a $M_{do}$-$R_d$'- group {wherein $M_{do}$ represents a 6 to 10-membered aryl group optionally substituted with a $M_a$- group (wherein $M_a$ is as defined above), a 5 to 10-membered heteroaryl group optionally substituted with a $M_a$-group (wherein $M_a$ is as defined above), a 3 to 10-membered cyclic hydrocarbon or heterocyclic group optionally substituted with a $M_a$- group (wherein $M_a$ is as defined above) and optionally containing an unsaturated bond, a ($b_0$)- group represented by

($b_0$) 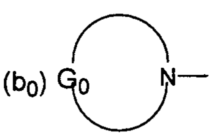

(wherein ($b_0$) is as defined above), a ($c_0$)- group represented by

($c_0$) 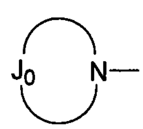

(wherein, ($c_0$) is as defined above), a ($d_0$)- group represented by

($d_0$) 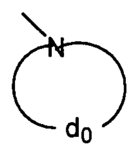

{wherein do forms a 5 to 12-membered hydrocarbon ring which is substituted with a carbonyl group or a thiocarbonyl group and further which may be optionally substituted with an oxy group, a thio group, a -$NR_1$-

group (wherein $R_1$ is as defined above), a sulfinyl group or a sulfonyl group}, or a ($e_0$)- group represented by

($e_0$) 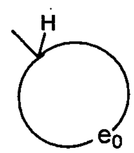

{wherein $e_0$ forms a 5 to 12-membered hydrocarbon ring optionally substituted with a carbonyl group, a thiocarbonyl group, an oxy group, a thio group, a -$NR_1$-group (wherein $R_1$ is as defined above), a sulfinyl group or a sulfonyl group}, and $R_d$' is the same as or different from $R_d$ and has the same meaning as $R_d$ has], a $M_{c0}$-$B_a$- group (wherein $M_{c0}$ and $B_a$ are as defined above), a $M_{c0}$-CO- group (wherein $M_{c0}$ is as defined above), a $M_{c0}$-CO-O- group (wherein $M_{c0}$ is as defined above), a $M_{c0}$O-CO- group (wherein $M_{c0}$ is as defined above), a $M_{c0}R_e$N- group (wherein $M_{c0}$ and $R_e$ are as defined above), a $M_{c0}$-CO-$NR_e$- group (wherein $M_{c0}$ and $R_e$ are as defined above), a $M_{c0}$O-CO-$NR_e$- group (wherein $M_{c0}$ and $R_e$ are as defined above), a $M_{c0}F_e$N-CO- group (wherein $M_{c0}$ and $R_e$ are as defined above), a $M_{c0}R_e$N-CO-$NR_e$'- group (wherein $M_{c0}$, $R_e$ and $R_e$' are as defined above), a $M_{c0}R_e$N-C(=$NR_e$')-$NR_e$"- group (wherein $M_{c0}$, $R_e$, $R_e$' and $R_e$" are as defined above), a $M_{c0}$-$SO_2$-$NR_e$- group (wherein $M_{c0}$ and $R_e$ are as defined above), or a $M_{c0}R_e$N-$SO_2$- group (wherein $M_{c0}$ and $R_e$ are as defined above), and $R_d$ is as defined above;

(3) the $Z_0$ group: a 5- to 12-membered hydrocarbon ring or heterocyclic ring which may be substituted with a halogen atom, a C1-C10 alkoxy group, C3-C10 alkenyloxy group, a C3-C10 alkynyloxy group, a carbonyl group, a thiocarbonyl group, an oxy group, a thio group, a sulfinyl group or a sulfonyl group, and which is aromatic or nonaromatic and monocyclic or fused ring and is fused to the A0 ring; and

IV. B0 is a group represented by formula (III-1):

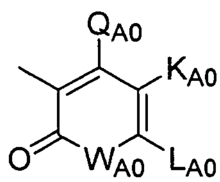 (III-1)

[wherein,

(1) $Q_{A0}$ represents a hydroxyl group, a ($b_0$)- group (wherein ($b_0$) is as defined above), an $A_9$-$B_6$-$B_c$- group [wherein $A_9$ and $B_6$ are as defined above, and $B_c$ represents an oxy group or a N-($(O)_mR_1$)- group (wherein m and $R_1$ are as defined above), provided that $B_c$ is not a sulfonyl group when $A_9$ is a hydrogen atom], an $A_7$"-$SO_2$-$B_c$- group (wherein $A_7$" and $B_c$ are as defined above), an $A_8$-$SO_2$-$B_c$- group (wherein $A_8$ and $B_c$ are as defined above, provided that $A_8$ is not a hydrogen atom), a $R_1R_1$'N-$SO_2$-$B_c$- group (wherein $R_1$, $R_1$' and $B_c$ are as defined above), a ($b_0$)-$SO_2$-$B_c$- group (wherein ($b_0$) and $B_c$ are as defined above), an $A_9$'-$B_c$- group (wherein $A_9$' and $B_c$ are as defined above), a $D_5$-$R_4$-$B_c$- group, (wherein $D_5$, $R_4$ and $B_c$ are as defined above), a $M_{c0}$-$B_3$-$B_c$- group (wherein $M_{c0}$, $B_3$ and $B_c$ are as defined above) or a $M_{c0}$-$B_c$- group (wherein $M_{c0}$ and $B_c$ are as defined above),

(2) $W_{A0}$ represents an oxygen atom or a -$NT_{A0}$- group wherein $T_{A0}$ represents a hydrogen atom, an $A_9$'- group (wherein $A_9$' is as defined above), a $D_5$-$R_4$- group (wherein $D_5$ and $R_4$ are as defined above) or a $M_{cO}$- group (wherein $M_{cO}$ is as defined above),

(3) $K_{A0}$ and $L_{A0}$ form a -$V_{A0}$=$V_{A0}$'-$V_{A0}$"=$V_{A0}$"'- group {wherein $V_{A0}$, $V_{A0}$', $V_{A0}$" and $V_{A0}$"' are the same or different, and represent a methine group optionally substituted with a $M_a$-group (wherein $M_a$ is as defined above), or a -N= group, and at least one of $V_{A0}$, $V_{A0}$', $V_A$0" and $V_{A0}$"' represents a -N= group},

a group represented by formula (III-2):

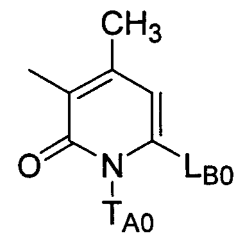

(III-2)

[wherein $T_{A0}$ is as defined above, and $L_{B0}$ represents a hydroxyl group or a methyl group],
a group represented by formula (III-3):

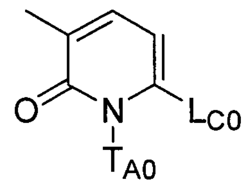

(III-3)

[wherein $T_{A0}$ is as defined above, and $L_{C0}$ represents a C1-C10 alkyl group],
a group represented by formula (III-4) :

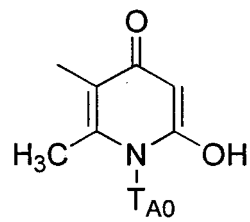

(III-4)

[wherein $T_{A0}$ is as defined above],
a group represented by formula (III-5):

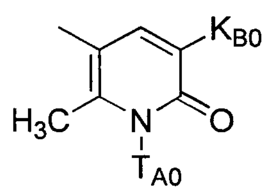

(III-5)

[wherein $T_{A0}$ is as defined above, and $K_{B0}$ represents a cyano group or a UOCO- group (wherein U represents a hydrogen atom or a C1-C10 alkyl group)],
a group represented by formula (III-6):

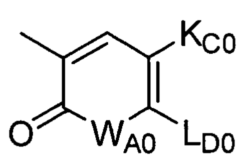

(III-6)

[wherein $W_{A0}$ is as defined above, and $K_{C0}$ and $L_{D0}$ form a C3-C10 alkylene group, or a C4-C10 alkenylyne

optionally substituted with 1 or more same or different $M_a$- groups (wherein $M_a$ is as defined above)], a group represented by formula (III-7):

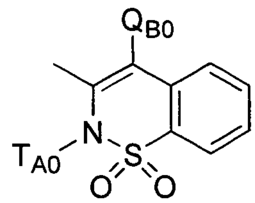

(III-7)

[wherein $T_{A0}$ is as defined above, and $Q_{B0}$ represents a hydroxyl group, a $A_9$-$B_6$-O- group (wherein $A_9$ and $B_6$ are as defined above), an $A_7$"-$SO_2$-O- group (wherein $A_7$" is as defined above), an $A_8$-$SO_2$-O- group, (wherein $A_8$ is as defined above, provided that $A_8$ is not a hydrogen), a $R_1R_1$'N-$SO_2$-O- group (wherein $R_1$ and $R_1$' are as defined above), a ($b_0$)-$SO_2$-O- group (wherein ($b_0$) is as defined above), an $A_9$'-O- group (wherein $A_9$' is as defined above), a $D_5$-$R_4$-O- group (wherein $D_5$ and $R_4$ are as defined above), a $M_{co}$-$B_3$-O- group (wherein $M_{cO}$ and $B_3$ are as defined above) or a $M_{c0}$-O- group (wherein $M_{cO}$ is as defined above)], or a group represented by formula (III-8):

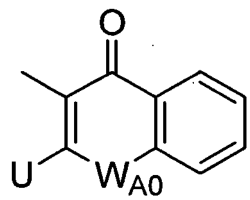

(III-8)

[wherein U and $W_{A0}$ are as defined above]; and
the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range;

4. A cinnamoyl compound represented by the formula (IV):

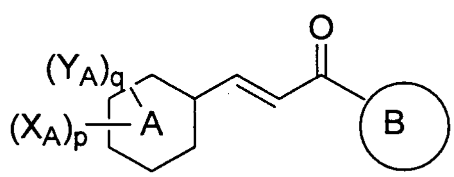

(IV)

wherein,

I. A represents a benzene ring or a pyridine ring;
II. in $(X_A)_p$, $X_A$ is a substituent on a carbon atom, and represents a group included in any group of the following A group to N group, p represents 0, 1, 2, 3, 4 or 5 and, when p is not less than 2, $X_A$s are the same or different;

(1) the A group:

a $D_1$-$R_4$- group, wherein $D_1$ represents a ($R_1$-$(O)_k$-($A_1$N-$(O)_k$'- group [wherein $R_1$ represents a hydrogen atom, or a C1-C10 alkyl group, or a C2-C10 alkyl group substituted with a halogen atom or a $R_2$-$B_1$- group (wherein $R_2$ represents a C1-C10 alkyl group, a C3-C10 alkenyl group or a C3-C10 alkynyl group, and $B_1$ represents an oxy group, a thio group, a sulfinyl group or a sulfonyl group), or a C3-C10 alkenyl group, or a C3-C10 alkynyl group, k represents 0 or 1, $A_1$ represents a $R_3$-$(CHR_0)_m$-$(B_2$-$B_3)_{m'}$-

group {wherein $R_3$ represents a hydrogen atom, or a C1-C10 alkyl group optionally substituted with a halogen atom or a $R_2$-$B_1$-group (wherein $R_2$ and $B_1$ are as defined above), or a C2-C10 alkenyl group, or a C2-C10 alkynyl group, $R_0$ represents a hydrogen atom, a C1-C10 alkyl group or a C2-C10 haloalkyl group, m represents 0 or 1, $B_2$ represents a single bond, an oxy group, a thio group or a -N((O)$_n$$R_1$')-group (wherein $R_1$' is the same as or different from $R_1$ and has the same meaning as $R_1$ has, and n represents 0 or 1), $B_3$ represents a carbonyl group, a thiocarbonyl group or a sulfonyl group, m' represents 0 or 1, and $B_3$ is not a sulfonyl group when m is 0 and $R_3$ is a hydrogen atom}, and k' represents 0 or 1], and $R_4$ represents a C1-C10 alkylene group, provided that a $R_0$'$R_0$"N-$R_4$- group (wherein $R_0$' and $R_0$" are the same as or different from $R_0$ and have the same meaning as $R_0$ has, and $R_4$ is as defined above) is excluded,

a $D_2$-$R_4$- group, wherein $D_2$ represents a cyano group, a $R_1$$R_1$'NC(=N-(O)$_n$-A1)- group (wherein $R_1$, $R_1$', n and $A_1$ are as defined above), an $A_1$N=C(-O$R_2$)- group (wherein $A_1$ and $R_2$ are as defined above) or a $NH_2$-CS- group, and $R_4$ is as defined above,

a $D_3$-$R_4$- group, wherein $D_3$ represents a nitro group or a $R_1OSO_2$- group (wherein $R_1$ is as defined above), and $R_4$ is as defined above, and

a $R_1OSO_2$- group, wherein $R_1$ is as defined above;

(2) the B group: an (a)-group represented by

(a) 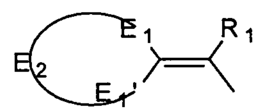

wherein $E_1$ and $E_1$' represent a methylene group optionally substituted with a C1-C10 alkyl group or a C1-C10 alkoxy group, or a carbonyl group, provided that $E_1$ and $E_1$' are not a carbonyl group at the same time, $E_2$ represents a C2-C10 alkylene group optionally substituted with an oxy group, a thio group, a sulfinyl group, a sulfonyl group or a - $NR_1$'- group (wherein $R_1$' is as defined above), or a C3-C10 alkenylene group optionally substituted with an oxy group, a thio group, a sulfinyl group, a sulfonyl group or a - $NR_1$'- group (wherein $R_1$' is as defined above), and $R_1$ is as defined above;

(3) the C group: a C2-C10 alkenyl group substituted with a halogen atom, a $R_2$-$B_1$- group (wherein $R_2$ and $B_1$ are as defined above), a $D_4$-$R_4$- group [wherein $D_4$ represents a hydroxyl group or an $A_1$-O- group (wherein $A_1$ is as defined above), and $R_4$ is as defined above], a $D_5$- group [wherein $D_5$ represents an O=C($R_3$)- group (wherein $R_3$ is as defined above), an $A_1$-(O)$_n$-N=C($R_3$)- group (wherein $A_1$, n and $R_3$ are as defined above), a $R_1$-$B_0$-CO-$R_4$-(O)$_n$-N=C($R_3$)- group {wherein $R_1$, $R_4$, n and $R_3$ are as defined above, and $B_0$ represents an oxy group, a thio group or a -N((O)$_m$$R_1$')-group (wherein $R_1$' and m are as defined above)}, a $D_2$-$R_4$-(O)$_n$-N=C($R_3$)- group (wherein $D_2$, $R_4$, n and $R_3$ are as defined above) or a $R_1A_1$N-N=C($R_3$)- group (wherein $R_1$, $A_1$ and $R_3$ are as defined above)], a $R_1A_1$N-O-$R_4$- group (wherein $R_1$, $A_1$ and $R_4$ are as defined above), a $R_1$ ($A_1$-(O)n-)N- group (wherein $R_1$, $A_1$ and n are as defined above), a $D_2$- group (wherein $D_2$ is as defined above) or a $D_3$- group (wherein $D_3$ is as defined above);

(4) the D group: a C2-C10 alkynyl group substituted with a (b)-$R_4$- group [wherein in (b)

(b) 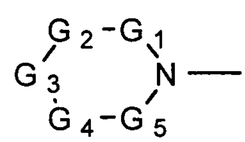 ,

$G_1$, $G_2$, $G_4$ and $G_5$ represent a methylene group which is connected with the adjacent atom via a single bond and which may be optionally substituted with a methyl group, or a methine group which is connected with the adjacent atom via a double bond and which may be optionally substituted with a methyl group, and $G_3$ represents a single bond, or a double bond, or a C1-C10 alkylene group optionally substituted with a methyl group, an oxy group, a thio group, a sulfinyl group, a sulfonyl group or a -$NR_1$- group (wherein $R_1$ is as defined above), or a C2-C10 alkenylene group optionally substituted with a methyl group, an oxy group, a thio group, a sulfinyl group, a sulfonyl group or a -$NR_1$- group (wherein $R_1$ is as defined above); and $R_4$ is as defined above], a (c)-$R_4$- group [wherein, in (c)

(c) 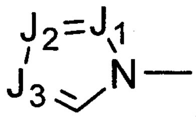 ,

$J_1$, $J_2$ and $J_3$ are the same or different, and represent a methine group optionally substituted with a methyl group, or a nitrogen atom; and $R_4$ is as defined above), a halogen atom, a $R_2$-$B_1$-$R_4$- group (wherein $R_2$, $B_1$ and $R_4$ are as defined above), a $D_4$-$R_4$- group (wherein $D_4$ and $R_4$ are as defined above), a $D_5$- group (wherein $D_5$ is as defined above), a $D_1$-$R_4$- group (wherein $D_1$ and $R_4$ are as defined above), a $D_2$- group (wherein $D_2$ is as defined above) or a $D_3$-$R_4$- group (wherein $D_3$ and $R_4$ are as defined above);
(5) the E group: an $A_2$-CO-$R_5$- group, provided that $R_5$ is not a vinylene group when $A_2$ is a hydroxyl group, [wherein $A_2$ represents

(i) an $A_3$-$B_4$- group
wherein $A_3$ represents a hydrogen atom, or a C1-C10 alkyl group, or a C2-C10 haloalkyl group, or a C2-C10 alkenyl group optionally substituted with a halogen atom, or a C3-C10 alkynyl group optionally substituted with a halogen atom, or $R_a$-$(R_4)_m$- group (wherein $R_a$ represents a phenyl group, a pyridyl group, a furyl group or a thienyl group, which may be optionally substituted with a halogen atom, a C1-C10 alkyl group, a C1-C10 alkoxy group or a nitro group, and $R_4$ and m are as defined above), or a C1-C10 alkyl group substituted with a (b)-$R_4$- group (wherein (b) and $R_4$ are as defined above), a (c)-$R_4$- group (wherein (c) and $R_4$ are as defined above), a $R_2$-$B_1$-$R_4$- group (wherein $R_2$, $B_1$ and $R_4$ are as defined above), a $D_4$-$R_4$- group (wherein $D_4$ and $R_4$ are as defined above), a $D_5$- group (wherein $D_5$ is as defined above), a $D_1$-$R_4$- group (wherein $D_1$ and $R_4$ are as defined above), a $D_2$- group (wherein $D_2$ is as defined above), a $D_3$-$R_4$- group (wherein $D_3$ and $R_4$ are as defined above) or an $A_4$-$SO_2$-$R_4$- group {wherein $A_4$ represents a (b)- group (wherein (b) is as defined above), a (c)- group (wherein (c) is as defined above) or a $R_1R_1$'N- group (wherein $R_1$ and $R_1$' are as defined above), and $R_4$ is as defined above}, and $B_4$ represents an oxy group, a thio group or a -N($(O)_mR_1$)-group (wherein $R_1$ and m are as defined above), provided that $A_3$ is not a hydrogen atom when $B_4$ is a thio group;
(ii) a $R_1$-$B_4$-CO-$R_4$-$B_4$'- group, wherein $R_1$, $B_4$ and $R_4$ are as defined above, $B_4$' is the same as or different from $B_4$ and has the same meaning as $B_4$ has, provided that $R_2$ is not a hydrogen atom when $B_4$ is a thio group, or
a $D_2$-$R_4$-$B_4$- group, wherein $D_2$, $R_4$ and $B_4$ are as defined above;
(iii) a $R_2$-$SO_2$-$NR_1$- group, wherein $R_2$ is as defined above, provided that a hydrogen atom is excluded, and $R_1$ is as defined above;
(iv) a (b)- group, wherein (b) is as defined above,
(v) a (c)- group, wherein (c) is as defined above, or
(vi) a $R_1A_1$N-$NR_1$'- group, wherein $R_1$, $A_1$ and $R_1$' are as defined above; and
$R_5$ represents a C2-C10 alkenylene group optionally substituted with a halogen atom or a C2-C10 alkynylene group];

(6) the F group: an $A_5$-$B_5$-$R_6$- group
[wherein $A_5$ represents a C2-C10 alkyl group substituted with a $D_4$- group (wherein $D_4$ is as defined above), a $D_1$-group (wherein $D_1$ is as defined above), a $D_3$- group (wherein $D_3$ is as defined above) or an $A_4$-$SO_2$- group (wherein $A_4$ is as defined above), or a C1-C10 alkyl group substituted with a $R_2$-$B_1$- group (wherein $R_2$ and $B_1$ are as defined above), a $D_2$- group (wherein $D_2$ is as defined above), a $D_5$- group (wherein $D_5$ is as defined above) or an $A_2$-CO- group (wherein $A_2$ is as defined above),
$B_5$ represents a $B_1$- group (wherein $B_1$ is as defined above) or a -$NA_1$- group (wherein $A_1$ is as defined above), and
$R_6$ represents a single bond or a C1-C10 alkylene group];
(7) the G group: an $A_6$-$B_5$-$R_6$- group
[wherein $A_6$ represents an (a)-$R_4$- group (wherein (a) and $R_4$ are as defined above), or a C2-C10 alkenyl group, or a C2-C10 alkynyl group, or a C2-C10 alkenyl group substituted with a halogen atom, a $R_2$-$B_1$-group (wherein $R_2$ and $B_1$ are as defined above), a $D_5$- group (wherein $D_5$ is as defined above), a $D_2$- group (wherein $D_2$ is as defined above) or an $A_2$-CO- group (wherein $A_2$ is as define above), or a C2-C10 alkynyl group substituted with a halogen atom, a $R_2$-$B_1$-group (wherein $R_2$ and $B_1$ are as defined above), a $D_5$-group (wherein $D_5$ is as defined above), a $D_2$- group (wherein $D_2$ is as defined above) or an $A_2$-CO- group (wherein $A_2$ is as defined above), or a C3-C10 alkenyl group substituted with a (b)- group (wherein (b) is

as defined above), a (c)-group (wherein (c) is as defined above), a $D_4$- group (wherein $D_4$ is as defined above), a $D_1$- group (wherein $D_1$ is as defined above) or a $D_3$- group (wherein $D_3$ is as defined above), or a C3-C10 alkynyl group substituted with a $D_4$-group (wherein $D_4$ is as defined above), a $D_1$- group (wherein $D_1$ is as defined above) or a $D_3$- group (wherein $D_3$ is as defined above), and $B_5$ and $R_6$ are as defined above];
(8) the H group:

a $D_2$-$_N$(-(O)$_n$-$A_1$)-$R_6$- group, wherein $D_2$, n, $A_1$ and $R_6$ are as defined above,
a $D_2$- group, wherein $D_2$ is as defined above, provided that a cyano group is excluded,
a $R_1$($R_1$'(O)$_n$)N-C$R_1$"=N-$R_6$- group, wherein $R_1$, $R_1$', n and $R_6$ are as defined above, $R_1$" is the same as or different from $R_1$ and has the same meaning as $R_1$ has,
a $R_1$-(O)$_n$-N=C$R_1$'-N$R_2$-$R_6$- group, wherein $R_1$, n, $R_1$', $R_2$ and $R_6$ are as defined above,
a $R_2$-$B_3$-N$R_1$-CO-N$R_1$'-$R_6$- group, wherein $R_2$, $B_3$, $R_1$, $R_1$' and $R_6$ are as defined above,
a $D_2$-CO-N$R_1$-$R_6$- group, wherein $D_2$, $R_1$ and $R_6$ are as defined above, or
an $A_2$-COCO-N$R_1$-$R_6$- group, wherein $A_2$, $R_1$ and $R_6$ are as defined above;

(9) the I group:

an $A_7$-$B_6$-N((O)$_n$$R_1$)-$R_6$- group [wherein $A_7$ represents a C2-C10 alkenyl group optionally substituted with a halogen atom, or a C2-C10 alkynyl group, or a C3-C10 haloalkynyl group, or a $R_2$-$B_1$-$R_4$- group (wherein $R_2$, $B_1$ and $R_4$ are as defined above), or a $D_4$-$R_4$- group (wherein $D_4$ and $R_4$ are as defined abovè), or a $D_5$-$R_4$- group (wherein $D_5$ and $R_4$ are as defined above), or a $D_1$-$R_4$- group (wherein $D_1$ and $R_4$ are as defined above), or a (b)-$R_4$- group (wherein (b) and $R_4$ are as defined above), or a (c)-$R_4$- group (wherein (c) and $R_4$ are as defined above), or a $D_2$-$R_4$- group (wherein $D_2$ and $R_4$ are as defined above), or a $D_3$-$R_4$- group (wherein $D_3$ and $R_4$ are as defined above), or an $A_4$-$SO_2$-$R_4$- group (wherein $A_4$ and $R_4$ are as defined above), or an $A_2$-CO-$R_4$- group (wherein $A_2$ and $R_4$ are as defined above), $B_6$ represents a carbonyl group or a thiocarbonyl group, and n, $R_1$ and $R_6$ are as defined above],
an $A_8$-CS-N((O)$_n$$R_1$)-$R_6$- group [wherein $A_8$ represents a hydrogen atom or a C1-C10 alkyl group optionally substituted with a halogen atom, and n, $R_1$ and $R_6$ are as defined above],
an $A_7$'-$B_2$'-$B_3$-N((O)n$R_1$)-$R_6$- group [wherein $A_7$' represents a C3-C10 alkenyl group optionally substituted with a halogen atom, or a C3-C10 alkynyl group optionally substituted with a halogen atom, or a $R_2$-$B_1$-$R_4$'- group (wherein $R_2$ and $B_1$ are as defined above, and $R_4$' represents a C2-C10 alkylene group), or a $D_4$-$R_4$'- group (wherein $D_4$ and $R_4$' are as defined above), or a $D_1$-$R_4$' - group (wherein $D_1$ and $R_4$' are as defined above), or a (b)-$R_4$'- group (wherein (b) and $R_4$' are as defined above), or a (c)-$R_4$'- group (wherein (c) and $R_4$' are as defined above), or a $D_2$-$R_4$-group (wherein $D_2$ and $R_4$ are as defined above), or a $D_3$-$R_4$'-group (wherein $D_3$ and $R_4$' are as defined above), or an $A_2$-CO-$R_4$- group (wherein $A_2$ and $R_4$ are as defined above), $B_2$' represents an oxy group, a thio group or a -N((O)$_{n'}$, $R_1$')-group (wherein n' is the same as or different from n and has the same meaning as n has, and $R_1$' is as defined above), and $B_3$, n, $R_1$ and $R_6$ are as defined above],
an $A_8$'-$B_2$'-CS-N((O)$_n$$R_1$)-$R_4$- group [wherein $A_8$' represents a C1-C10 alkyl group or a C2-C10 haloalkyl group, $B_2$' is as defined above, and n, $R_1$ and $R_6$ are as defined above],
an $A_8$'-S-$B_3$'-N((O)$_n$$R_1$)-$R_6$- group [wherein $A_8$', n, $R_1$ and $R_6$ are as defined above, and $B_3$' represents a carbonyl group or a sulfonyl group] or
an $A_7$"-$SO_2$-N((O)$_n$$R_1$)-$R_6$- group [wherein $A_7$" represents a C2-C10 alkenyl group, or a C3-C10 alkenyl group substituted with a halogen atom, or a C3-C10 alkynyl group optionally substituted with a halogen atom, or a $R_2$-$B_1$-$R_4$'- group (wherein $R_2$, $B_1$ and $R_4$' are as defined above), or a $D_4$-$R_4$'-group (wherein $D_4$ and $R_4$' are as defined above), or a $D_5$-$R_4$-group (wherein $D_5$ and $R_4$ are as defined above), or a $D_1$-$R_4$'-group (wherein $D_1$ and $R_4$' are as defined above), or a (b)-$R_4$'- group (wherein (b) and $R_4$' are as defined above), or a (c)-$R_4$'- group (wherein (c) and $R_4$' are as defined above), or a $D_2$-$R_4$- group (wherein $D_2$ and $R_4$ are as defined above), or a $NO_2$-$R_4$- group (wherein $R_4$ is as defined above), or an $A_2$-CO-$R_4$- group (wherein $A_2$ and $R_4$ are as defined above), and n, $R_1$ and $R_4$ are as defined above];

(10) the J group:

an $A_7$-CO- group (wherein $A_7$ is as defined above),
an $A_9$-CS- group (wherein $A_9$ represents $A_7$ or $A_8$),
an $A_9$'(O)$_m$N=C($A_9$)- group (wherein $A_9$' represents $A_7$' or $A_8$', and m and $A_9$ are as defined above),
a $D_2$-CO- group (wherein $D_2$ is as defined above),
an $A_2$-COCO- group (wherein $A_2$ is as defined above),

an $A_9$-CO-$B_1$'-$R_6$- group (wherein $A_9$ and $R_6$ are as defined above, and $B_1$' represents an oxy group or a thio group, provided that $A_9$ is not $A_8$ when $B_1$' is an oxy group),
an $A_9$-CS-$B_1$'-$R_6$- group (wherein $A_9$, $B_1$' and $R_6$ are as defined above),
an $A_7$"-$SO_2$-$B_1$'-$R_6$- group (wherein $A_7$", $B_1$' and $R_6$ are as defined above),
an $A_8$-$SO_2$-$B_1$'-$R_6$- group (wherein $A_8$, $B_1$' and $R_6$ are as defined above, provided that $A_8$ is not a hydrogen atom),
an $A_9$'-$B_2$'-$B_3$-$B_1$'-$R_6$- group (wherein $A_9$', $B_2$', $B_3$, $B_1$' and $R_6$ are as defined above), and
a C2-C10 alkenyl group substituted with a (b)- group (wherein (b) is as defined above) or a (c)- group (wherein (c) is as defined above);

(11) the K group: an $A_{10}$-N($(O)_nR_1$)-CO-$R_6$- group [wherein $A_{10}$ represents a hydrogen atom (provided that n is not 0), an $A_7$"-$SO_2$- group (wherein $A_7$" is as defined above), an $A_8$-$SO_2$- group (wherein $A_8$ is as defined above, provided that $A_8$ is not a hydrogen atom), an Ag'O- group (wherein $A_9$' is as defined above, provided that n is not 1), an $A_9$'-group (wherein $A_9$' is as defined above, provided that $A_8$' is excluded when n is 0), a $R_2OCH_2$- group (wherein $R_2$ is as defined above), an $A_2$-CO-$R_4$- group (wherein $A_2$ and $R_4$ are as defined above) or an $A_2$-CO-CH($CH_2$CO-$A_2$)- group (wherein $A_2$ is as defined above), and n, $R_1$ and $R_6$ are as defined above];
(12) the L group:

an $A_{10}$'-N($(O)_nR_1$)-$SO_2$-$R_6$- group [wherein $A_{10}$' represents a hydrogen atom (provided that n is not 0), an $A_9$'O- group (wherein $A_9$' is as defined above, provided that n is not 1), an $A_9$'- group (wherein $A_9$' is as defined above, provided that $A_8$' is excluded when n is 0), a $R_2$-CO- group (wherein $R_2$ is as defined above), an $A_2$-CO-$R_4$- group (wherein $A_2$ and $R_4$ are as defined above) or an $A_2$-CO-CH ($CH_2$CO-$A_2$)- group (wherein $A_2$ is as defined above), and n, $R_1$ and $R_6$ are as defined above],
an $A_9$"$R_1$N-$SO_2$-N($(O)_nR_1$')-$R_6$- group [wherein $A_9$" represents a hydrogen atom or an $A_9$'- group (wherein Ag' is as defined above), and $R_1$, n, $R_1$' and $R_6$ are as defined above], and
a (b)-$SO_2$-N($(O)_nR_1$')-$R_6$- group [wherein (b), n, $R_1$' and $R_6$ are as defined above];

(13) the M group:

a $R_1(R_2S)C$=N-$R_6$- group (wherein $R_1$, $R_2$ and $R_6$ are as defined above),
a $R_2B(R_2'B')C$=N-$R_6$- group (wherein $R_2$ and $R_6$ are as defined above, $R_2$' is the same as or different from $R_2$ and has the same meaning as $R_2$ has, and B and B' are the same or different and represent an oxy group or a thio group),
a $R_1R_1$'N-($R_2$S)C=N-$R_6$- group (wherein $R_1$, $R_1$', $R_2$ and $R_6$ are as defined above),
a $R_1$N=C($SR_2$)-$NR_2$'-$R_6$- group (wherein $R_1$, $R_2$, $R_2$' and $R_6$ are as defined above), and
a $R_1$($R_1$'O)N-$R_6$- group (wherein $R_1$, $R_1$' and $R_6$ are as defined above);

(14) the N group: an $A_{11}$-P(=O) ($OR_1$')-$R_4$- group
[wherein $A_{11}$ represents a $R_1$- group (wherein $R_1$ is as defined above), a $R_1$O-$R_6$- group (wherein $R_1$ and $R_6$ are as defined above) or a $R_1$OCO-$CHR_0$- group (wherein $R_1$ and $R_0$ are as defined above), and $R_1$' and $R_4$ are as defined above];

III. in $(Y_A)_q$, $Y_A$ is a substituent on a carbon atom and represents a group included in the following X group or Y group, q represents 0, 1, 2, 3, 4 or 5, and the sum of p (wherein p is as defined above) and q is not more than 5, and when q is not less than 2, $Y_A$s are the same or different, and when q is not less than 2, adjacent two same or different $Y_A$s may together form a group included in the Z group to be fused to the A ring;

(1) the X group: a $M_a$-group, wherein $M_a$ represents a $R_b$- group (wherein $R_b$ represents a C1-C10 alkyl group optionally substituted with a halogen atom), a halogen atom, a nitro group, a cyano group, a $R_e$-$B_a$-$R_d$- group (wherein $R_e$ represents a C1-C10 alkyl group optionally substituted with a halogen atom, $B_a$ represents an oxy group, a thio group, a sulfinyl group or a sulfonyl group, and $R_d$ represents a single bond or a C1-C10 alkylene group), an $HOR_d$- group (wherein $R_d$ is as defined above), a $R_e$-CO-$R_d$- group (wherein $R_e$ represents a hydrogen atom, or a C1-C10 alkyl group optionally substituted with a halogen atom, and $R_d$ is as defined above), a $R_e$-CO-O-$R_d$- group (wherein $R_e$ and $R_d$ are as defined above), a $R_e$O-CO-$R_d$- group (wherein $R_e$ and $R_d$ are as defined above), an HO-CO-CH=CH- group, a $R_eR_e$'N-$R_d$- group (wherein $R_e$ and $R_e$' are the same or different, $R_e$ is as defined above, $R_e$' has the same meaning as $R_e$ has, and $R_d$ is as defined above), a $R_e$-CO-$NR_e$'-$R_d$- group (wherein $R_e$, $R_e$' and $R_d$ are as defined above), a $R_b$O-

CO-N($R_e$)-$R_d$- group (wherein $R_b$, $R_e$ and $R_d$ are as defined above), a $R_eR_e$'N-CO-$R_d$-group (wherein $R_e$, $R_e$' and $R_d$ are as defined above), a $R_eR_e$'N-CO-N$R_e$"-$R_d$- group (wherein $R_e$, $R_e$' and $R_e$" are the same or different, $R_e$ and $R_e$' are as defined above, $R_e$" has the same meaning as $R_e$ has, and $R_d$ is as defined above), a $R_eR_e$'N-C(=N$R_e$")-N$R_e$"'-$R_d$- group (wherein $R_e$, Re', $R_e$" and $R_e$"' are the same or different, $R_e$, $R_e$' and $R_e$" are as defined above, $R_e$"' has the same meaning as $R_e$ has, and $R_d$ is as defined above), a $R_b$-$SO_2$-N$R_e$-$R_d$- group (wherein $R_b$, $R_e$ and $R_d$ are as defined above), a $R_eR_e$'N-$SO_2$-$R_d$- group (wherein $R_e$, $R_e$' and $R_d$ are as defined above), a C2-C10 alkenyl group or a C2-C10 alkynyl group;

(2) the Y group: a $M_b$-$R_d$- group, wherein $M_b$ represents a $M_e$-group [wherein $M_c$ represents a $M_d$-$R_d$'- group {wherein $M_d$ represents a phenyl group optionally substituted with a $M_a$- group (wherein $M_a$ is as defined above), a pyridyl group optionally substituted with a $M_a$- group (wherein $M_a$ is as defined above), a naphthyl group optionally substituted with a $M_a$- group (wherein $M_a$ is as defined above), a (b)-group (wherein (b) is as defined above), a (c)- group (wherein (c) is as defined above), a (d)- group

(d) 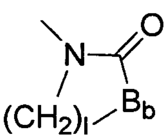

(wherein 1 is 2, 3 or 4, $B_b$ represents an oxy group or a thio group) or an (e)- group

(e) 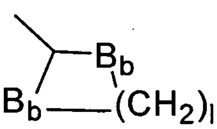

(wherein 1 and $B_b$ are as defined above)}, and $R_d$' is the same as or different from $R_d$ and has the same meaning as $R_d$ has], a $M_c$-$B_a$- group (wherein $M_c$ and $B_a$ are as defined above), a $M_c$-CO- group (wherein $M_c$ is as defined above), a $M_c$-CO-O- group (wherein $M_c$ is as defined above), a $M_c$O-CO-group (wherein $M_c$ is as defined above), a $M_cR_e$N- group (wherein $M_c$ and $R_e$ are as defined above), a $M_c$-CO-N$R_e$- group (wherein $M_c$ and $R_e$ are as defined above), a $M_c$O-CO-N$R_e$-group (wherein $M_c$ and $R_e$ are as defined above), a $M_cR_e$N-CO-group (wherein $M_c$ and $R_e$ are as defined above), a $M_cR_e$N-CO-N$R_e$'- group (wherein $M_c$, $R_e$ and $R_e$' are as defined above), a $M_cR_e$N-C(=N$R_e$')-N$R_e$"- group (wherein $M_c$, $R_e$, $R_e$', and $R_e$" are as defined above), a $M_c$-$SO_2$-N$R_e$- group (wherein $M_c$ and $R_e$ are as defined above) or a $M_cR_e$N-$SO_2$-group (wherein $M_c$ and $R_e$ are as defined above), and

$R_d$ is as defined above;

(3) the Z group: a -N=C($Y_a$)-$Y_a$'- group (wherein $Y_a$ represents a hydrogen atom, or a C1-C10 alkyl group optionally substituted with a halogen atom, or a C1-C10 alkoxy group, and $Y_a$' represents an oxy group, or a thio group, or an imino group optionally substituted with a C1-C10 alkyl group),

a -$Y_b$-$Y_b$'-$Y_b$"- group (wherein $Y_b$ and $Y_b$" are the same or different, and represent a methylene group, or an oxy group, or a thio group, or a sulfinyl group, or an imino group optionally substituted with a C1-C10 alkyl group, and $Y_b$' represents a C1-C4 alkylene group optionally substituted with a halogen atom, or a C1-C4 alkylene group optionally substituted with an oxo group), or

a -$Y_c$-O-$Y_c$'-O- group (wherein $Y_e$ and $Y_e$' are the same or different, and represent a C1-C10 alkylene group);

IV. B is
a group represented by formula (IV-1):

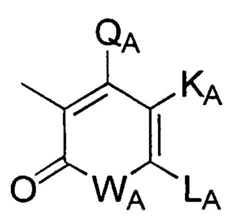 (IV-1)

wherein,

(1) $Q_A$ represents a hydroxyl group, a (b)- group (wherein (b) is as defined above), an $A_9$-$B_6$-$B_c$- group [wherein $A_9$ and $B_6$ are as defined above, and $B_c$ represents an oxy group or a -N((O)$_m$$R_1$)- group (wherein m and $R_1$ are as defined above), provided that $B_c$ is not a sulfonyl group when $A_9$ is a hydrogen atom], an $A_7$''-$SO_2$-$B_c$- group (wherein $A_7$'' and $B_c$ are as defined above), an $A_8$-$SO_2$-$B_c$- group (wherein $A_8$ and $B_c$ are as defined above, provided that $A_B$ is not a hydrogen atom), a $R_1R_1$'N-$SO_2$-$B_c$- group (wherein $R_1$, $R_1$' and $B_c$ are as defined above), a ($b_0$)-$SO_2$-O- group (wherein (b) and $B_c$ are as defined above), an $A_9$'-$B_c$- group (wherein $A_9$' and $B_c$ are as defined above), a $D_5$-$R_4$-$B_c$- group (wherein $D_{5,}$ $R_4$ and $B_c$ are as defined above), a $M_c$-$8_3$-$B_c$-group (wherein $M_c$, $B_3$ and $B_c$ are as defined above) or a $M_c$-$B_c$- group (wherein $M_c$ and $B_c$ are as defined above),
(2) $W_A$ represents an oxygen atom or a -$NT_A$- group [wherein $T_A$ represents a hydrogen atom, an $A_9$'- group (wherein $A_9$' is as defined above), a $D_5$-$R_4$- group (wherein $D_5$ and $R_4$ are as defined above) or a $M_c$- group (wherein $M_c$ is as defined above)], and
(3) $K_A$ and $L_A$ form a -$V_A$=$V_A$'-$V_A$''=$V_A$'''- group {wherein $V_A$, $V_A$', $V_A$'' and $V_A$''' are the same or different, and represent a methine group optionally substituted with a $M_a$-group (wherein $M_a$ is as defined above), or a -N= group, and at least one of $V_A$, $V_A$', $V_A$'' and $V_A$''' represents a -N= group};
a group represented by formula (IV-2):

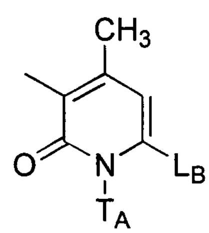

(IV-2)

wherein $T_A$ is as defined above, and $L_B$ represents a hydroxyl group or a methyl group;
a group represented by formula (IV-3):

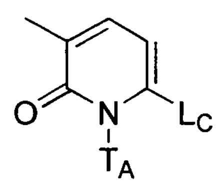

(IV-3)

wherein $T_A$ is as defined above, and $L_C$ represents a C1-C10 alkyl group;
a group represented by formula (IV-4):

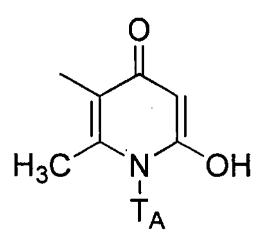

(IV-4)

wherein $T_A$ is as defined above;
a group represented by formula (IV-5):

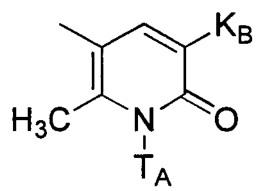

(IV-5)

wherein $T_A$ is as defined above, and $K_B$ represents a cyano group or a UOCO- group (wherein U represents a hydrogen atom or C1-C10 alkyl group);
a group represented by formula (IV-6):

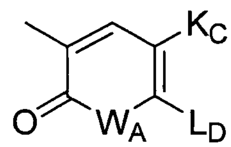

(IV-6)

wherein $W_A$ is as defined above, and $K_C$ and $L_D$ form a C3-C10 alkylene group, or a C4-C10 alkenylene group optionally substituted with 1 or more same or different $M_a$- groups (wherein $M_a$ is as defined above);
a group represented by formula (IV-7):

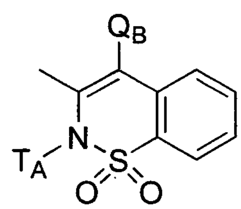

(IV-7)

wherein $T_A$ is as defined above, $Q_B$ represents a hydroxyl group, an $A_9$-$B_6$-O- group [wherein $A_9$ and $B_6$ are as defined above], an $A_7$''-$SO_2$-O- group (wherein $A_7$'' is as defined above), an $A_8$-$SO_2$-O- group (wherein $A_8$ is as defined above, provided that $A_8$ is not a hydrogen atom), a $R_1R_1$'N-$SO_2$-O-group (wherein $R_1$ and $R_1$' are as defined above), a (b)-$SO_2$-O- group (wherein (b) is as defined above), an $A_9$'-O- group (wherein $A_9$' is as defined above), a $D_5$-$R_4$-O- group (wherein $D_5$ and $R_4$ are as defined above), a $M_c$-$B_3$-O- group (wherein $M_e$ and $B_3$ are as defined above), or a $M_c$-O- group (wherein $M_c$ is as defined above); or
a group represented by formula (IV-8)

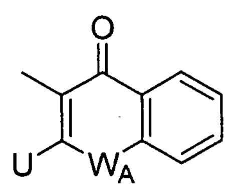

(IV-8)

wherein U and $W_A$ are as defined above; and
the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range];

5. A cinnamoyl compound represented by the formula (V):

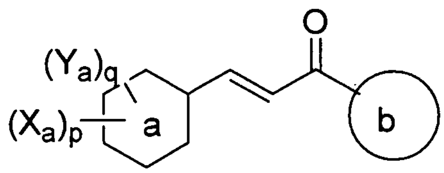

(V)

wherein,

I. a represents a benzene ring or a pyridine ring, $X_a$ is a substituent on a carbon atom and represents a C1-C10 alkyl group substituted with a cyano group, or a C1-C10 alkyl group substituted with a tetrahydropyran-4-ylidene group, or a C2-C10 alkenyl group substituted with a halogen atom or a cyano group, or a C2-C10 alkenyl group substituted with a C1-C10 alkoxycarbonyl group, or a C3-C10 alkynyl group substituted with a hydroxyl group, or an $a_0$-$r_1$-b-$r_1$'- group {wherein $a_0$ represents a methyl group substituted with a C1-C10 alkylthio group, a methyl group substituted with a C1-C10 alkylsulfinyl group, a methyl group substituted with a C1-C10 alkylsulfonyl group, a C2-C10 alkenyl group, a C2-C10 alkynyl group, a $r_2$O-CO- group (wherein $r_2$ represents a C1-C10 alkyl group or a C2-C10 alkyl group substituted with a hydroxyl group), a carboxy group, a rr'N-CO- group (wherein r and r' are the same or different, and respresent a hydrogen atom or a C1-C10 alkyl group), $a_1$-NH-CO- group (wherein $a_1$ represents a C2-C10 alkyl group substituted with a C1-C10 alkoxy group), an $a_1$'-CO-group (wherein $a_1$' represents a morpholino group), a rr'N-$CH_2$- group (wherein r and r' are as defined above), a $r_0$-$(O)_1$-CONH-$CH_2$- group (wherein $r_0$ represents a C1-C10 alkyl group, and 1 represents 0 or 1), a r-$OCH_2$-group (wherein r is as defined above), a $r_0$-CO- group (wherein $r_0$ is as defined above), a cyano group or a sulfomethyl group, $r_1$ represents a C1-C10 alkylene group, $r_1$' represents a single bond or a C1-C10 alkylene group, and b represents an oxy group, a thio group, a sulfinyl group, a sulfonyl group, or an imino group}, or an $a_2$-y-CO-NH- group (wherein $a_2$ represents a C2-C10 alkyl group substituted with a C1-C10 alkoxy group, and y represents an oxy group or an imino group), or a rO-COCO-NH- group (wherein r is as defined above), or an $a_3$-z-NH- group (wherein $a_3$ represents a C2-C10 alkenyl group, or a C1-C10 alkyl group substituted with a C1-C10 alkoxy group, a C1-C10 alkoxycarbonyl group, a carboxy group or a cyano group, and z represents a carbonyl group or a sulfonyl group), or an $a_4$-NHCO- group {wherein $a_4$ represents a C1-C10 alkoxy group, or a C3-C10 alkenyloxy group, or a $r_0$-$SO_2$- group (wherein $r_0$ is as defined above), or a C2-C10 alkyl group substituted with a hydroxyl group or a C1-C10 alkoxy group, or a C2-C10 alkyl group substituted with a $r_0r_0$'N-group (wherein $r_0$ is as defined above, and $r_0$' is the same as or different from $r_0$, and represents the same meaning as $r_0$ has), a C1-C10 alkyl group substituted with a rO-CO- group (wherein r is as defined above), a cyano group or an aminocarbonyl group, or a rO-CO-(rO-$COCH_2$)CH- group (wherein r is as defined above)}, or an $a_5$-$NHSO_2$- group (wherein $a_5$ represents a C2-C10 alkyl group substituted with a C1-C10 alkoxyl group), or a $r_0$ON=CH- group (wherein $r_0$ is as defined above), or a $r_0$NHCSNH- group (wherein $r_0$ is as defined above), or a $r_0$NHC(-S$r_0$')=N- group (wherein $r_0$ and $r_0$' are as defined above), or a $(rO)_2P(=O)CH_2$- group (wherein r is as defined above), p represents 0, 1, 2 or 3 and, and when p is not less than 2, $X_a$s are same or different,
$Y_a$ represents are halogen atom, or a C1-C10 alkyl group optionally substituted with a halogen atom, or a C1-C10 alkyl group optionally substituted with a C1-C10 alkoxy group, or a C2-C10 alkenyl group, or a C2-C10 alkynyl group, or a 2-oxo-oxazolidin-3-yl group, or a [1,3]dioxolan-2-yl group, or a C1-C10 alkoxy group substituted with a morpholino group, or an $a_0$'-b'- group (wherein $a_0$' represents a C1-C10 alkyl group optionally substituted with a halogen atom, and b' represents an oxy group, a thio group, a sulfinyl group or a sulfonyl group), or a nitro group, or a cyano group, or a rO-CO- group (wherein r is as defined above), or a $r_0r_0$'N- group (wherein $r_0$ and $r_0$' are as defined above), or a $r_0$CO-NH- group (wherein $r_0$ is as defined above), or a $r_0r_0$'NCONH-group (wherein $r_0$ and $r_0$' are as defined above), or a rr'NCO- group (wherein r and r' are as defined above), or a hydroxyl group, q represents 0, 1, 2 or 3, and when q is not less than 2, $Y_a$s are the same or different, and when q is not less than 2, adjacent $Y_a$s may be fused to the a ring to form a 4,5,6,7-tetrahydrobenzo[b]thiophene ring;
II. b is
a group represented by formula (V-1):

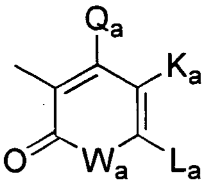

(V-1)

wherein $Q_a$ represents a $r_a$-O- group {$r_a$ represents a hydrogen atom, or a C1-C10 alkyl group, or a C3-C10 alkenyl group, or a C3-C10 alkynyl, or a C1-C10 alkyl group substituted with a $r_0r_0$'N-$CH_2$- group (wherein $r_0$ and $r_0$' are the same or different), a $rOCH_2$ group (wherein r is as defined above), a $r_0$-CO- group (wherein $r_0$ is as defined above), a C1-C10 alkoxycarbonyl group, a carboxy group, an aminocarbonyl group or a cyano group, or a $r_3$-$r_1$-group (wherein $r_3$ represents a phenyl group or a pyridyl group, and $r_1$ is as defined above)}, or a piperidino group, or a morpholino group, or a $r_4r_4$'N-group (wherein $r_4$ and $r_4$' are the same of different, and represent a hydrogen atom, or a C1-C10 alkyl group, or C3-C10 alkenyl group, or a C3-C10 alkynyl group, or a C2-C10 alkyl group substituted with a C1-C10 alkoxy group, provided that they are not a hydrogen atom at the same time); $W_a$ represents an oxygen atom or a - $NT_a$- group [wherein $T_a$ represents a $r_b$- group (wherein $r_b$ is the same as or different from $r_a$, and represents the same meaning as $r_a$ has) or a $r_3$'- group (wherein $r_3$' is the same as or different from $r_3$, and represents the same meaning as $r_3$ has)], and $K_a$ and $L_a$ form a -$V_a$=$V_a$'-$V_a$"=$V_a$"'- group (wherein $V_a$, $V_a$', $V_a$" and $V_a$"' are the same or different, and represent a methine group or a -N= group, and at least one of $V_a$, $V_a$'$V_a$" and $V_a$"' represents a -N= group);
a group represented by formula (V-2):

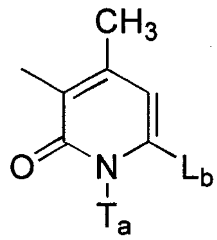

(V-2)

wherein $T_a$ is as defined above, and $L_b$ represents a hydroxyl group or a methyl group;
a group represented by formula (V-3):

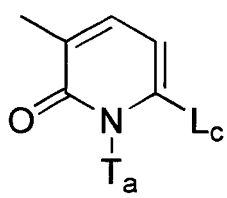

(V-3)

wherein $T_a$ is as defined above, and $L_c$ represents a C1-C10 alkyl group;
a group represented by formula (V-4):

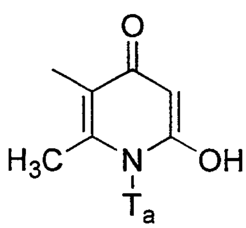

(V-4)

wherein $T_a$ is as defined above;

a group represented by formula (V-5):

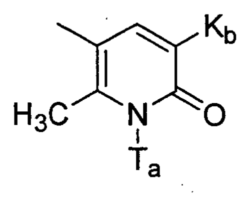

(V-5)

wherein $T_a$ is as defined above, and $K_b$ represents a cyano group or a UOCO- group (wherein U represents a hydrogen atom or C1-C10 alkyl group);
a group represented by formula (V-6):

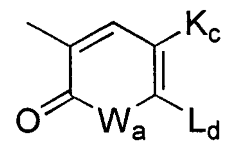

(V-6)

wherein $W_a$ is as defined above, and $K_c$ and $L_d$ form a C3-C10 alkylene group or a C4-C10 alkenylene group;
a group represented by formula (V-7):

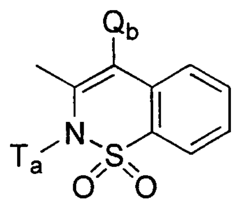

(V-7)

wherein $T_a$ is as defined above, and $Q_b$ represents a $r_a$-O-group (wherein $r_a$ is as defined above); or
a group represented by formula (V-8):

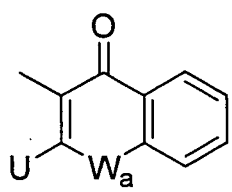

(V-8)

wherein U and $W_a$ are as defined above; and
the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range;
6. A process for producing a cinnamoyl compound represented by formula (VI'):

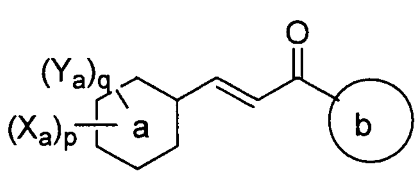

(VI')

wherein a, $X_a$, $Y_a$, p and q are defined below, and
b represents a group represented by formula (VI'-1):

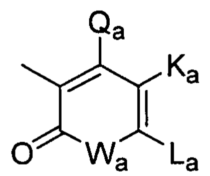

(VI'-1)

wherein $Q_a$, $W_a$, $K_a$ and $L_a$ are as defined below, a group represented by formula (VI'-2):

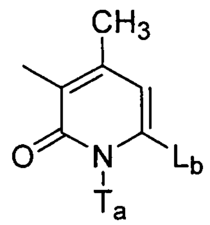

(VI'-2)

wherein $T_a$ and $L_b$ are as defined below, a group represented by formula (VI'-3):

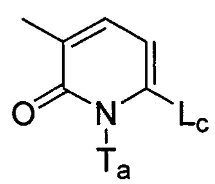

(VI'-3)

wherein $T_a$ and $L_C$ are as defined below, a group represented by formula (VI'-4):

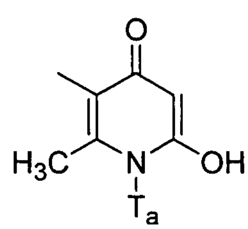

(VI'-4)

wherein $T_a$ is as defined below, a group represented by formula (VI'-5):

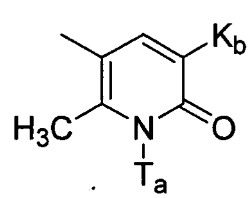

(VI'-5)

wherein $T_a$ and $K_b$ are as defined below, a group represented by formula (VI'-6):

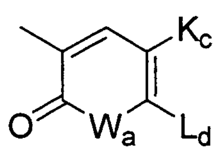

(VI'-6)

wherein $W_a$, $K_c$ and $L_d$ are as defined below, a group represented by formula (VI'-7):

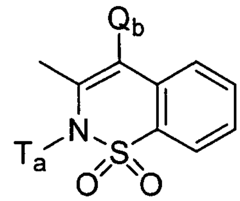

(VI'-7)

wherein $T_a$ and $Q_b$ are as defined below, or a group represented by formula (VI'-8):

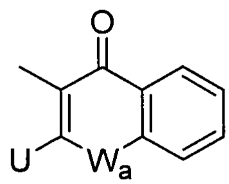

(VI'-8)

wherein U and $W_a$ are as defined below; which comprises reacting a benzaldehyde derivative represented by the formula (VI):

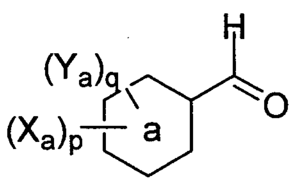

(VI)

[wherein,

I. a represents a benzene ring or a pyridine ring, $X_a$ is a substituent on a carbon atom and represents a C1-C10 alkyl group substituted with a cyano group, or a C1-C10 alkyl group substituted with a tetrahydropyran-4-ylidene group, or a C2-C10 alkenyl group substituted with a halogen atom or a cyano group, or a C2-C10 alkenyl group substituted with a C1-C10 alkoxycarbonyl group, or a C3-C10 alkynyl group substituted with a hydroxyl group, or an $a_0$-$r_1$-b-$r_1$'- group {wherein $a_0$ represents a methyl group substituted with a C1-C10 alkylthio group, a methyl group substituted with a C1-C10 alkylsulfinyl group, a methyl group substituted with a C1-C10 alkylsulfonyl group, a C2-C10 alkenyl group, a C2-C10 alkynyl group, a $r_2$O-CO- group (wherein $r_2$ represents a C1-C1 alkyl group or a C2-C10 alkyl group substituted with a hydroxyl group), a carboxy group, a rr'N-CO- group (wherein r and r' are the same or different, and respresent a hydrogen atom or a C1-C10 alkyl group), $a_1$-NH-CO- group (wherein $a_1$ represents a C2-C10 alkyl group substituted with a C1-C10 alkoxy group), an $a_1$'-CO- group (wherein $a_1$' represents a morpholino group), a rr'N-$CH_2$- group (wherein r and r' are as defined above), a $r_0$-$(O)_1$-CONH-$CH_2$- group (wherein $r_0$ represents a C1-C10 alkyl group, and 1 represents 0 or 1), a r-$OCH_2$-group (wherein r is as defined above), a $r_0$-CO- group (wherein $r_0$ is as defined above), a cyano group or a sulfomethyl group, $r_1$ represents a C1-C10 alkylene group, $r_1$' represents a single bond or a C1-C10 alkylene group, and b represents an oxy group, a thio group, a sulfinyl group, a sulfonyl group, or an imino group}, or an $a_2$-y-CO-NH- group (wherein $a_2$ represents a C2-C10 alkyl group substituted with a C1-C10 alkoxy group, and y represents an oxy group or an imino group), or a rO-COCO-NH- group (wherein r is as defined above),

or an $a_3$-z-NH- group (wherein $a_3$ represents a C2-C10 alkenyl group, or a C1-C10 alkyl group substituted with a C1-C10 alkoxy group, a C1-C10 alkoxycarbonyl group, a carboxy group or a cyano group, and z represents a carbonyl group or a sulfonyl group), or an $a_4$-NHCO- group {wherein $a_4$ represents a C1-C10 alkoxy group, or a C3-C10 alkenyloxy group, or a $r_0$-$SO_2$- group (wherein $r_0$ is as defined above), or a C2-C10 alkyl group substituted with a hydroxyl group or a C1-C10 alkoxy group, or a C2-C10 alkyl group substituted with a $r_0r_0$'N-group (wherein $r_0$ is as defined above, and $r_0$' is the same as or different from $r_0$, and represents the same meaning as $r_0$ has), or a C1-C10 alkyl group substituted with a rO-CO- group (wherein r is as defined above), a cyano group or an aminocarbonyl group, or a rO-CO-(rO-$COCH_2$)CH- group (wherein r is as defined above)}, or an $a_5$-$NHSO_2$- group (wherein $a_5$ represents a C2-C10 alkyl group substituted with a C1-C10 alkoxyl group), or a $r_0$ON=CH- group (wherein $r_0$ is as defined above), or a $r_0$NHCSNH- group (wherein $r_0$ is as defined above), or a $r_0$NHC(-S$r_0$')=N- group (wherein $r_0$ and $r_0$' are as defined above), or a $(rO)_2P(=O)CH_2$- group (wherein r is as defined above), p represents 0, 1, 2 or 3 and, and when p is not less than 2, $X_a$s are same or different,

$Y_a$ represents are halogen atom, or a C1-C10 alkyl group optionally substituted with a halogen atom, or a C1-C10 alkyl group optionally substituted with a C1-C10 alkoxy group, or a C2-C10 alkenyl group, or a C2-C10 alkynyl group, or a 2-oxo-oxazolidin-3-yl group, or a [1,3]dioxolan-2-yl group, or a C1-C10 alkoxy group substituted with a morpholino group, or an $a_0$'-b'- group (wherein $a_0$' represents a C1-C10 alkyl group optionally substituted with a halogen atom, and b' represents an oxy group, a thio group, a sulfinyl group or a sulfonyl group), or a nitro group, or a cyano group, or a rO-CO- group (wherein r is as defined above), or a $r_0r_0$'N- group (wherein $r_0$ and $r_0$' are as defined above), or a $r_0$CO-NH- group (wherein $r_0$ is as defined above), or a $r_0r_0$'NCONH-group (wherein $r_0$ and $r_0$' are as defined above), or a rr'NCO- group (wherein r and r' are as defined above), or a hydroxyl group, q represents 0, 1, 2 or 3, and when q is not less than 2, $Y_a$s are the same or different, and when q is not less than 2, adjacent $Y_a$s may be fused to the a ring to form a 4,5,6,7-tetrahydrobenzo[b]thiophene ring] with a compound represented by formula (VI-1):

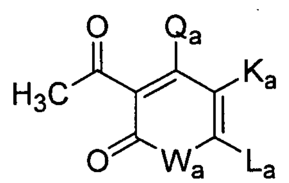

(VI-1)

[wherein

$Q_a$ represents a $r_a$-O- group {$r_a$ represents a hydrogen atom, or a C1-C10 alkyl group, or a C3-C10 alkenyl group, or a C3-C10 alkynyl, a C1-C10 alkyl group substituted with a $r_0r_0$'N-$CH_2$- group (wherein $r_0$ and $r_0$' are the same or different), a $rOCH_2$ group (wherein r is as defined above), a $r_0$-CO- group (wherein $r_0$ is as defined above), a C1-C10 alkoxycarbonyl group, a carboxy group, an aminocarbonyl group or a cyano group, or a $r_3$-$r_1$-group (wherein $r_3$ represents a phenyl group or a pyridyl group, and $r_1$ is as defined above)}, or a piperidino group, or a morpholino group, or a $r_4r_4$'N-group (wherein $r_4$ and $r_4$' are the same of different, and represent a hydrogen atom, or a C1-C1 alkyl group, or C3-C10 alkenyl group, or a C3-C10 alkynyl group, or a C2-C10 alkyl group substituted with a C1-C10 alkoxy group, provided that they are not a hydrogen atom at the same time);

$W_a$ represents an oxygen atom or a -$NT_a$- group [wherein $T_a$ represents a $r_b$- group (wherein $r_b$ is the same as or different from $r_a$, and represents the same meaning as $r_a$ has) or a $r_3$'- group (wherein $r_3$' is the same as or different from $r_3$, and represents the same meaning as $r_3$ has)], and $K_a$ and $L_a$ form a -$V_a$=$V_a$'-$V_a$''=$V_a$'''- group (wherein $V_a$, $V_a$', $V_a$'' and $V_a$''' are the same or different, and represent a methine group or a -N= group, and at least one of $V_a$, $V_a$'$V_a$'' and $V_a$''' represents a -N= group)], a compound represented by formula (VI-2):

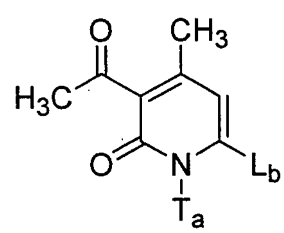

(VI-2)

[wherein $T_a$ is as defined above, and $L_b$ represents a hydroxyl group or a methyl group], a compound represented

by formula (VI-3):

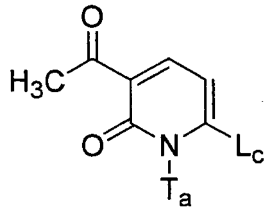

(VI-3)

[wherein $T_a$ is as defined above, and $L_c$ represents a C1-C10 alkyl group], a compound represented by formula (VI-4):

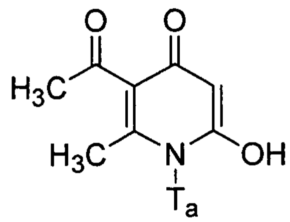

(VI-4)

[wherein $T_a$ is as defined above], a compound represented by formula (VI-5):

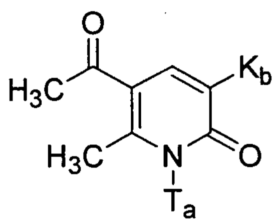

(VI-5)

[wherein $T_a$ is as defined above, and $K_b$ represents a cyano group or a UOCO- group (wherein U represents a hydrogen atom or C1-C10 alkyl group)], a compound represented by formula (VI-6):

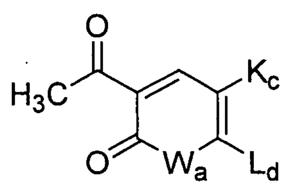

(VI-6)

[wherein $W_a$ is as defined above, and $K_c$ and $L_d$ form a C3-C10 alkylene group or a C4-C10 alkenylene group], a compound represented by formula (VI-7):

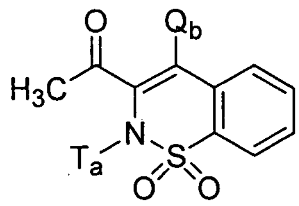

(VI-7)

[wherein $T_a$ is as defined above, and $Q_b$ represents a $r_a$-O-group (wherein $r_a$ is as defined above)], or a compound represented by formula (VI-8):

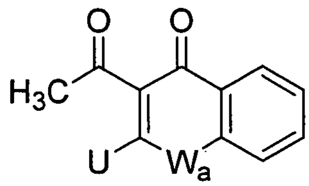

(VI-8)

[wherein U and $W_a$ are as defined above]; and
the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range;

7. A process for producing a compound represented by formula (VII-3):

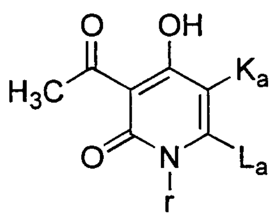

(VII-3)

[wherein r, $K_a$ and $L_a$ are as defined below] which comprises 3-oxobutyrylamidating a compound represented by formula (VII-1):

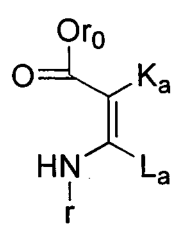

(VII-1)

[wherein r represents a hydrogen atom or a C1-C10 alkyl group, $r_0$ represents a C1-10 alkyl group, $K_a$ and $L_a$ form a $-V_a=V_a'-V_a''=V_a'''-$ group (wherein $V_a$, $V_a'$, $V_a''$ and $V_a'''$ are the same or different, represent a methine group or a -N= group, and at least one of $V_a$, $V_a'$, $V_a''$ and $V_a'''$ represents a -N= group)] to produce a compound represented by formula (VII-2):

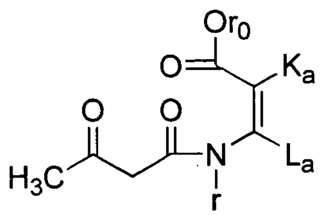

(VII-2)

[wherein r, $r_0$, $K_a$ and $L_a$ are as defined above], and then cyclizing the compound represented by the formula (VII-2);

8. A process for producing a compound represented by formula (VIII-2):

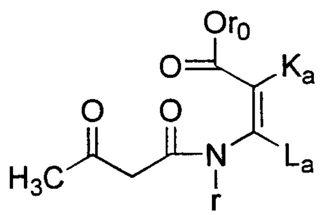

(VIII-2)

[wherein r, $r_0$, $K_a$ and $L_a$ are as defined below] which comprises 3-oxobutyrylamidating a compound represented by formula (VIII-1):

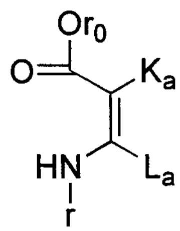

(VIII-1)

[wherein r represents a hydrogen atom or a C1-C10 alkyl group, $r_0$ represents a C1-10 alkyl group, $K_a$ and $L_a$ form a - $V_a$=$V_a$'-$V_a$"=$V_a$"'- group (wherein $V_a$, $V_a$', $V_a$" and $V_a$"' are the same or different, represent a methine group or a -N= group, and at least one of $V_a$, $V_a$', $V_a$" and $V_a$"' represents a -N= group)];

9. A compound represented by the formula (IX):

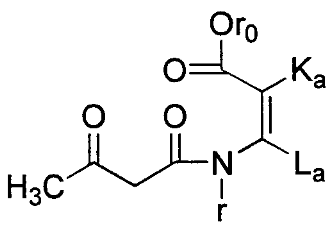

(IX)

wherein r represents a hydrogen atom or a C1-C10 alkyl group, $r_0$ represents a C1-10 alkyl group, $K_a$ and $L_a$ form a - Va=Va'-Va"=Va"'- group (wherein $V_a$, $V_a$', $V_a$" and $V_a$"' are the same or different, represent a methine group or a -N= group, and at least one of $V_a$, $V_a$', $V_a$" and $V_a$"' represents a -N= group);

10. A compound represented by the formula (X):

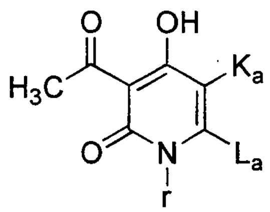

(X)

wherein r represents a hydrogen atom or a C1-C10 alkyl group, $K_a$ and $L_a$ form a $-V_a=V_a'-V_a''=V_a'''-$ group (wherein $V_a$, $V_a'$, $V_a''$ and $V_a'''$ are the same or different and represent a methine group or a -N= group, and at least one of $V_a$, $V_a'$, $V_a''$ and $V_a'''$ represents a -N= group);

11. A process for producing a compound represented by formula (XI-2) :

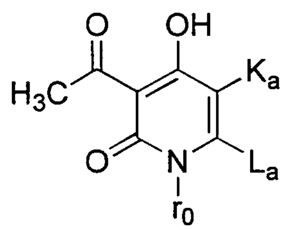

(XI-2)

[wherein $r_0$ represents a C1-C10 alkyl group, and $K_a$ and $L_a$ are as defined below] which comprises alkylating a compound representd by formula (XI-1):

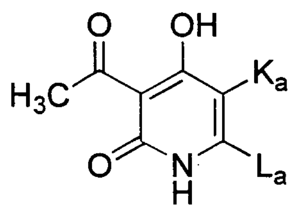

(XI-1)

[wherein $K_a$ and $L_a$ form a $-V_a=V_a'-V_a''=V_a'''-$ group (wherein $V_a$, $V_a'$, $V_a''$ and $V_a'''$ are the same or different, and represent a methine group or a -N= group, and at least one of $V_a$, $V_a'$, $V_a''$ and $V_a'''$ represents a -N= group)];

12. A composition for suppressing transcription of an extracellular matrix gene, which comprises an inert carrier and a cinnamoyl compound represented by formula (I') :

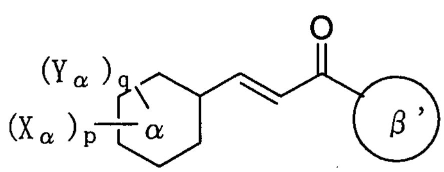

(I')

wherein,

I. α represents a benzene ring or a pyridine ring; in $(Y_\alpha)_q$, $Y_\alpha$ is a substituent on a carbon atom and represents a group included in the following $X_0$ group or the $Y_0$ group, q represents 0, 1, 2, 3, 4 or 5, and when q is not less than 2, $Y_\alpha$s are the same or different, and when q is not less than 2, adjacent two same or different $Y_\alpha$s may together form a group included in the $Z_0$ group to be fused to the α ring; and in $(X_\alpha)_p$, $X_\alpha$ is a substituent on a carbon atom and represents a group which does not belong to the following $X_0$ group, $Y_0$ group and $Z_0$ group, p represents 0, 1, 2, 3, 4 or 5, and when p is not less than 2, $X_\alpha$s are the same or different and the sum

of p and q is not more than 5;

(1) the $X_0$ group: a $M_a$-group, wherein $M_a$ represents a $R_b$- group (wherein $R_b$ represents a C1-C10 alkyl group optionally substituted with a halogen atom), a halogen atom, a nitro group, a cyano group, a hydroxyl group, a $R_c$-$B_a$-$R_d$-group (wherein $R_c$ represents a C1-C10 alkyl group optionally substituted with a halogen atom, $B_a$ represents an oxy group, a thio group, a sulfinyl group or a sulfonyl group, and $R_d$ represents a single bond or a C1-C10 alkylene group), an $HOR_d$- group (wherein $R_d$ is as defined above), a $R_e$-CO-$R_d$-group (wherein $R_e$ represents a hydrogen atom, or a C1-C10 alkyl group optionally substituted with a halogen atom, and $R_d$ is as defined above), a $R_e$-CO-O-$R_d$- group (wherein $R_e$ and $R_d$ are as defined above), a $R_e$O-CO-$R_d$- group (wherein $R_e$ and $R_d$ are as defined above), an HO-CO-CH=CH-group, a $R_eR_e$'N-$R_d$- group (wherein $R_e$ and $R_e$' are the same or different, $R_e$ is as defined above, $R_e$' has the same meaning as $R_e$ has, and $R_d$ is as defined above), a $R_e$-CO-N$R_e$'-$R_d$- group (wherein $R_e$, $R_e$' and $R_d$ are as defined above), a $R_b$O-CO-N($R_e$)-$R_d$- group (wherein $R_b$, $R_e$ and $R_d$ are as defined above), a $R_eR_e$'N-CO-$R_d$- group (wherein $R_e$, $R_e$' and $R_d$ are as defined above), a $R_eR_e$'N-CO-N$R_e$"-$R_d$- group (wherein $R_e$, $R_e$' and $R_e$" are the same or different, $R_e$ and $R_e$' are as defined above, $R_e$" has the same meaning as $R_e$ has, and $R_d$ is as defined above), a $R_eR_e$'N-C(=N$R_e$")-N$R_e$"'-$R_d$- group (wherein $R_e$, $R_e$', $R_e$" and $R_e$"' are the same or different, $R_e$, $R_e$' and $R_e$" are as defined above, $R_e$"' has the same meaning as $R_e$ has, and $R_d$ is as defined above), a $R_b$-$SO_2$-N$R_e$-$R_d$- group (wherein $R_b$, $R_e$ and $R_d$ are as defined above), a $R_eR_e$'N-$SO_2$-$R_d$- group (wherein $R_e$, $R_e$' and $R_d$ are as defined above), a C2-C10 alkenyl group or a C2-C10 alkynyl group;

(2) the $Y_0$ group: a $M_{b0}$-$R_d$- group, wherein $M_{b0}$ represents a $M_{c0}$- group
[wherein $M_{co}$ represents a $M_{do}$-$R_d$'- group {wherein $M_{do}$ represents a 6 to 10-membered aryl group optionally substituted with a $M_a$- group (wherein $M_a$ is as defined above), a 5 to 10-membered heteroaryl group optionally substituted with a $M_a$-group (wherein $M_a$ is as defined above), a 3 to 10-membered cyclic hydrocarbon or heterocyclic group optionally substituted with a $M_a$- group (wherein $M_a$ is as defined above) and optionally containing an unsaturated bond, a ($b_0$)- group represented by

($b_0$) 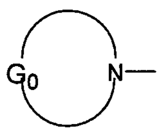

(wherein Go forms an optionally substituted, saturated or unsaturated, nonaromatic 5 to 14-membered hydrocarbon ring or heterocyclic ring), a ($c_0$)- group represented by

($c_0$) 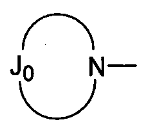

(wherein, $J_0$ forms a 5 to 7-membered aromatic ring optionally containing a nitrogen atom), a (do)- group represented by

($d_0$) 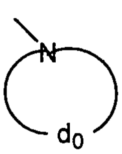

[wherein do forms a 5 to 12-membered hydrocarbon ring which is substituted with a carbonyl group or a thiocarbonyl group and further which may be optionally substituted with an oxy group, a thio group, a -$NR_1$-group {wherein $R_1$ represents a hydrogen atom, or a C1-C10 alkyl group, or a C2-C10 alkyl group substituted with a halogen atom or a $R_2$-$B_1$- group (wherein $R_2$ represents a C1-C10 alkyl group, a C3-C10 alkenyl group or a C3-C10 alkynyl group, and $B_1$ represents an oxy group, a thio group, a sulfinyl group or a sulfonyl

group), a C3-C10 alkenyl group, or a C3-C10 alkynyl group}, a sulfinyl group, or a sulfonyl group], or a ($e_0$)-group represented by

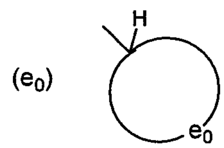

{wherein $e_0$ forms a 5 to 12-membered hydrocarbon ring optionally substituted with a carbonyl group, a thiocarbonyl group, an oxy group, a thio group, a $-NR_1$-group (wherein $R_1$ is as defined above), a sulfinyl group or a sulfonyl group}; and $R_d$' is the same as or different from $R_d$ and has the same meaning as $R_d$ has], a $M_{c0}$-Ba- group (wherein $M_{c0}$ and $B_a$ are as defined above), a $M_{c0}$-CO- group (wherein $M_{c0}$ is as defined above), a $M_{c0}$-CO-O-group (wherein $M_{c0}$ is as defined above), a $M_{c0}$O-CO- group (wherein $M_{c0}$ is as defined above), a $M_{c0}R_eN$- group (wherein $M_{c0}$ and $R_e$ are as defined above), a $M_{c0}$-CO-$NR_e$- group (wherein $M_{c0}$ and $R_e$ are as defined above), a $M_{c0}$O-CO-$NR_e$-group (wherein $M_{c0}$ and $R_e$ are as defined above), a $M_{c0}R_eN$-CO-group (wherein $M_{c0}$ and $R_e$ are as defined above), a $M_{c0}R_eN$-CO-$NR_e$'- group (wherein $M_{c0}$, $R_e$ and $R_e$' are as defined above), a $M_{c0}R_eN$-C(=$NR_e$')-$NR_e$"- group (wherein $M_{c0}$, $R_e$, $R_e$' and $R_e$" are as defined above), a $M_{c0}$-$SO_2$-$NR_e$- group (wherein $M_{c0}$ and $R_e$ are as defined above) or a $M_{c0}R_eN$-$SO_2$-group (wherein $M_{c0}$ and $R_e$ are as defined above), and $R_d$ is as defined above;

(3) the $Z_0$ group: a 5- to 12-membered hydrocarbon ring or heterocyclic ring which may be substituted with a halogen atom, a C1-C10 alkoxy group, C3-C10 alkenyloxy group, a C3-C10 alkynyloxy group, a carbonyl group, a thiocarbonyl group, an oxy group, a thio group, a sulfinyl group or a sulfonyl group, and which is aromatic or nonaromatic and monocyclic or fused ring and is fused to the α ring; and

II. β' represents
a group represented by formula (I'-1):

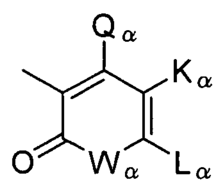

(I'-1)

wherein,

(1)$Q_\alpha$ represents an optionally substituted hydroxyl group, or an optionally substituted amino group,
(2)$W_\alpha$ represents an oxygen atom or a -$NT_\alpha$- group (wherein $T_\alpha$ represents a hydrogen atom, or a substituent on the nitrogen atom),
(3)$K_\alpha$ and $L_\alpha$ form a -$V_\alpha$=$V_\alpha$' -$V_\alpha$"=$V_\alpha$"'- group (wherein $V_\alpha$, $V_\alpha$', $V_\alpha$" and $V_\alpha$"' are the same or different, and represent an optionally substituted methine group or a -N= group, and at least one of $V_\alpha$, $V_\alpha$', $V_\alpha$" and $V_\alpha$"' represents a -N= group);
a group represented by formula (I'-2):

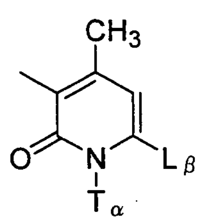

(I'-2)

wherein $T_\alpha$ is as defined above, and Lβ represents a hydroxyl group or a methyl group;
a group represented by formula (I'-3):

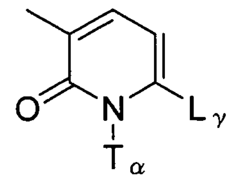

(I'-3)

wherein $T_\alpha$ is as defined above, and $L_\gamma$ represents a C1-C10 alkyl group; a group represented by formula (I'-4):

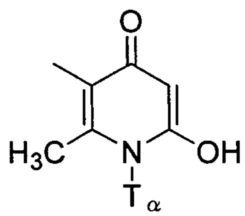

(I'-4)

wherein $T_\alpha$ is as defined above; a group represented by formula (I'-5):

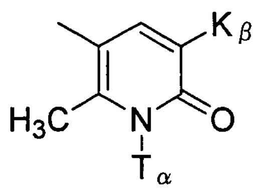

(I'-5)

wherein $T_\alpha$ is as defined above, and $K_\beta$ represents a cyano group or a UOCO- group (wherein U represents a hydrogen atom or a C1-C10 alkyl group); a group represented by formula (I'-6):

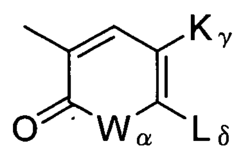

(I'-6)

wherein $W_\alpha$ is as defined above, and $K_\gamma$ and $L_\delta$ form an optionally substituted C3-C10 alkylene group or an optionally substituted C4-C10 alkenylene group; a group represented by formula (I'-7):

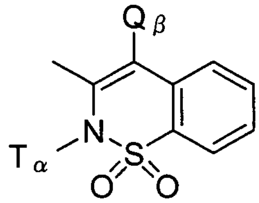

(I'-7)

wherein $T_\alpha$ is as defined above, and $Q_\beta$ represents an optionally substituted hydroxyl group; or

a group represented by formula (I'-8):

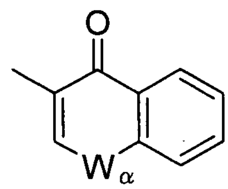

(I'-8)

wherein $W_\alpha$ is as defined above;
the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range;

13. A cinnamoyl compound represented by formula (II'):

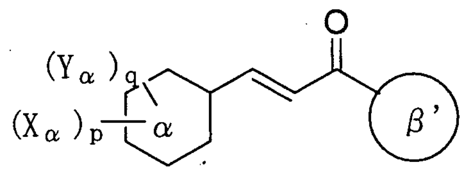

(II')

wherein,

I. $\alpha$ represents a benzene ring or a pyridine ring; in $(Y_\alpha)q$, $Y_\alpha$ is a substituent on a carbon atom and represents a group included in the following $X_0$ group or the $Y_0$ group, q represents 0, 1, 2, 3, 4 or 5, and when q is not less than 2, $Y_\alpha$s are the same or different, and when q is not less than 2, adjacent two same or different $Y_\alpha$s may together form a group included in the $Z_0$ group to be fused to the $\alpha$ ring; and in $(X_\alpha)_p$, $X_\alpha$ is a substituent on a carbon atom and represents a group which does not belong to the following $X_0$ group, $Y_0$ group and $Z_0$ group, p represents 0, 1, 2, 3, 4 or 5, and when p is not less than 2, $X_\alpha$s are the same or different and the sum of p and q is not more than 5;

(1) the $X_0$ group: a $M_a$-group, wherein $M_a$ represents a $R_b$- group (wherein $R_b$ represents a C1-C10 alkyl group optionally substituted with a halogen atom), a halogen atom, a nitro group, a cyano group, a hydroxyl group, a $R_c$-$B_a$-$R_d$-group (wherein $R_c$ represents a C1-C10 alkyl group optionally substituted with a halogen atom, $B_a$ represents an oxy group, a thio group, a sulfinyl group or a sulfonyl group, and $R_d$ represents a single bond or a C1-C10 alkylene group), an $HOR_d$- group (wherein $R_d$ is as defined above), a $R_e$-CO-$R_d$- group (wherein $R_e$ represents a hydrogen atom, or a C1-C10 alkyl group optionally substituted with a halogen atom, and $R_d$ is as defined above), a $R_e$-CO-O-$R_d$- group (wherein $R_e$ and $R_d$ are as defined above), a $R_e$O-CO-$R_d$- group (wherein $R_e$ and $R_d$ are as defined above), an HO-CO-CH=CH-group, a $R_eR_e'$N-$R_d$- group (wherein $R_e$ and $R_e'$ are the same or different, $R_e$ is as defined above, $R_e'$ has the same meaning as $R_e$ has, and $R_d$ is as defined above), a $R_e$-CO-N$R_e'$-$R_d$- group (wherein $R_e$, $R_e'$ and $R_d$ are as defined above), a $R_b$O-CO-N($R_e$)-$R_d$- group (wherein $R_b$, $R_e$ and $R_d$ are as defined above), a $R_eR_e'$N-CO-$R_d$- group (wherein $R_e$, $R_e'$ and $R_d$ are as defined above), a $R_eR_e'$N-CO-N$R_e''$-$R_d$- group (wherein $R_e$, $R_e'$ and $R_e''$ are the same or different, $R_e$ and $R_e'$ are as defined above, $R_e''$ has the same meaning as $R_e$ has, and $R_d$ is as defined above), a $R_eR_e'$N-C(=N$R_e''$)-N$R_e'''$-$R_d$- group (wherein $R_e$, $R_e'$, $R_e''$ and $R_e'''$ are the same or different, $R_e$, $R_e'$ and $R_e''$ are as defined above, $R_e'''$ has the same meaning as $R_e$ has, and $R_d$ is as defined above), a $R_b$-$SO_2$-N$R_e$-$R_d$- group (wherein $R_b$, $R_e$ and $R_d$ are as defined above), a $R_eR_e'$N-$SO_2$-$R_d$- group (wherein $R_e$, $R_e'$ and $R_d$ are as defined above), a C2-C10 alkenyl group or a C2-C10 alkynyl group;
(2) the $Y_0$ group: a $M_{b0}$-$R_d$- group, wherein $M_{b0}$ represents a $M_{c0}$- group
[wherein $M_{co}$ represents a $M_{do}$-$R_d'$- group {wherein $M_{do}$ represents a 6 to 10-membered aryl group optionally substituted with a $M_a$- group (wherein $M_a$ is as defined above), a 5 to 10-membered heteroaryl group optionally substituted with a $M_a$-group (wherein $M_a$ is as defined above), a 3 to 10-membered cyclic hydrocarbon or heterocyclic group optionally substituted with a $M_a$- group (wherein $M_a$ is as defined above) and optionally containing an unsaturated bond, a ($b_0$)- group represented by

($b_0$) 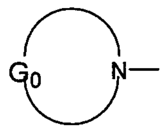

(wherein Go forms an optionally substituted, saturated or unsaturated, nonaromatic 5 to 14-membered hydrocarbon ring or heterocyclic ring), a ($c_0$)- group represented by

($c_0$) 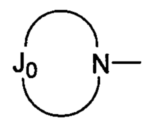

(wherein, $J_0$ forms a 5 to 7-membered aromatic ring optionally containing a nitrogen atom), a (do)- group represented by

($d_0$) 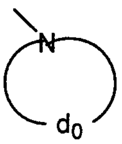

[wherein do forms a 5 to 12-membered hydrocarbon ring which is substituted with a carbonyl group or a thiocarbonyl group and further which may be optionally substituted with an oxy group, a thio group, a -$NR_1$- group {wherein $R_1$ represents a hydrogen atom, or a C1-C10 alkyl group, or a C2-C10 alkyl group substituted with a halogen atom or a $R_2$-$B_1$- group (wherein $R_2$ represents a C1-C10 alkyl group, a C3-C10 alkenyl group or a C3-C10 alkynyl group, and $B_1$ represents an oxy group, a thio group, a sulfinyl group or a sulfonyl group), a C3-C10 alkenyl group, or a C3-C10 alkynyl group}, a sulfinyl group, or a sulfonyl group], or a ($e_0$)-group represented by

($e_0$) 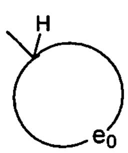

(wherein $e_0$ forms a 5 to 12-membered hydrocarbon ring optionally substituted with a carbonyl group, a thiocarbonyl group, an oxy group, a thio group, a -$NR_1$-group (wherein $R_1$ is as defined above), a sulfinyl group or a sulfonyl group}; and $R_d$' is the same as or different from $R_d$ and has the same meaning as $R_d$ has], a $M_{c0}$-$B_a$- group (wherein $M_{c0}$ and $B_a$ are as defined above), a $M_{c0}$-CO- group (wherein $M_{c0}$ is as defined above), a $M_{c0}$-CO-O-group (wherein $M_{c0}$ is as defined above), a $M_{c0}$O-CO- group (wherein $M_{c0}$ is as defined above), a $M_{c0}R_e$N- group (wherein $M_{c0}$ and $R_e$ are as defined above), a $M_{c0}$CO-$NR_e$- group (wherein $M_{c0}$ and $R_e$ are as defined above), a $M_{c0}$O-CO-$NR_e$-group (wherein $M_{c0}$ and $R_e$ are as defined above), a $M_{c0}R_e$N-CO-group (wherein $M_{c0}$ and $R_e$ are as defined above), a $M_{c0}R_e$N-CO-$NR_e$'- group (wherein $M_{c0}$, $R_e$ and $R_e$' are as defined above), a $M_{c0}R_e$N-C(=NRe')-$NR_e$"- group (wherein $M_{c0}$, $R_e$, $R_e$' and $R_e$" are as defined above), a $M_{c0}$-$SO_2$-$NR_e$- group (wherein $M_{c0}$ and $R_e$ are as defined above) or a $M_{c0}R_e$N-$SO_2$-group (wherein $M_{c0}$ and $R_e$ are as defined above), and $R_d$ is as defined above;

(3) the $Z_0$ group: a 5- to 12-membered hydrocarbon ring or heterocyclic ring which may be substituted with a halogen atom, a C1-C10 alkoxy group, C3-C10 alkenyloxy group, a C3-C10 alkynyloxy group, a carbonyl

group, a thiocarbonyl group, an oxy group, a thio group, a sulfinyl group or a sulfonyl group, and which is aromatic or nonaromatic and monocyclic or fused ring and is fused to the α ring; and

II. β' represents
a group represented by formula (II'-1):

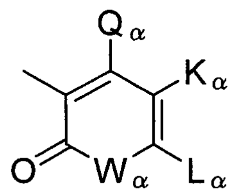

(II'-1)

wherein,

(1) $Q_\alpha$ represents an optionally substituted hydroxyl group, or an optionally substituted amino group,
(2) $W_\alpha$ represents an oxygen atom or a $-NT_\alpha-$ group (wherein $T_\alpha$ represents a hydrogen atom, or a substituent on the nitrogen atom),
(3) $K_\alpha$ and $L_\alpha$ form a $-V_\alpha=V_\alpha'-V_\alpha''=V_\alpha'''-$ group (wherein $V_\alpha$, $V_\alpha'$, $V_\alpha''$ and $V_\alpha'''$ are the same or different, and represent an optionally substituted methine group or a -N= group, and at least one of $V_\alpha$, $V_\alpha'$, $V_\alpha''$ and $V_\alpha'''$ represents a -N= group);
a group represented by formula (II'-2):

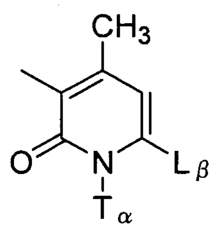

(II'-2)

wherein $T_\alpha$ is as defined above, and $L_\beta$ represents a hydroxyl group or a methyl group;
a group represented by formula (II'-3):

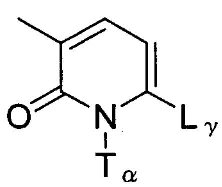

(II'-3)

wherein $T_\alpha$ is as defined above, and $L_\gamma$ represents a C1-C10 alkyl group;
a group represented by formula (II'-4):

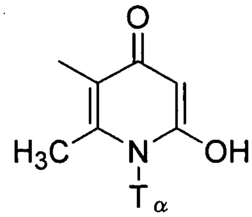

(II'-4)

wherein $T_\alpha$ is as defined above;
a group represented by formula (II'-5):

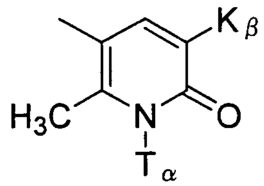

(II'-5)

wherein $T_\alpha$ is as defined above, and $K_\beta$ represents a cyano group or a UOCO- group (wherein U represents a hydrogen atom or a C1-C10 alkyl group);
a group represented by formula (II'-6):

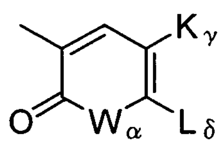

(II'-6)

wherein $W_\alpha$ is as defined above, and $K_\gamma$ and $L_\delta$ form an optionally substituted C3-C10 alkylene group or an optionally substituted C4-C10 alkenylene group;
a group represented by formula (II'-7):

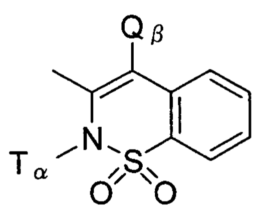

(II'-7)

wherein $T_\alpha$ is as defined above, and $Q_\beta$ represents an optionally substituted hydroxyl group; or
a group represented by formula (II'-8):

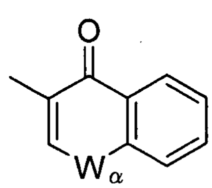

(II'-8)

wherein $W_\alpha$ is as defined above;
the term "as defined above" used for the same symbols among plural substituents means that the plural

substituents independently represent the same meaning as that described above and, among the plural substituents, although the selection range of substituents to be selected is the same, selected substituents may be the same or different as long as they are selected within the range;

14. A cinnamoyl compound represented by formula (V'):

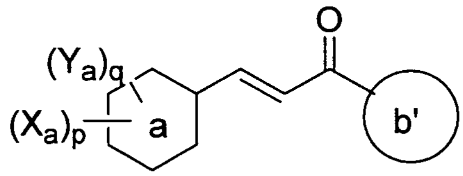
(V')

[wherein,

I. a represents a benzene ring or a pyridine ring, $X_a$ is a substituent on a carbon atom and represents a C1-C10 alkyl group substituted with a cyano group, or a C1-C10 alkyl group substituted with a tetrahydropyran-4-ylidene group, or a C2-C10 alkenyl group substituted with a halogen atom or a cyano group, or a C2-C10 alkenyl group substituted with a C1-C10 alkoxycarbonyl group, or a C3-C10 alkynyl group substituted with a hydroxyl group, or an $a_0$-$r_1$-b-$r_1$'- group {wherein $a_0$ represents a methyl group substituted with a C1-C10 alkylthio group, a methyl group substituted with a C1-C10 alkylsulfinyl group, a methyl group substituted with a C1-C10 alkylsulfonyl group, a C2-C10 alkenyl group, a C2-C10 alkynyl group, a $r_2$O-CO- group (wherein $r_2$ represents a C1-C10 alkyl group or a C2-C10 alkyl group substituted with a hydroxyl group), a carboxy group, a rr'N-CO- group (wherein r and r' are the same or different, and respresent a hydrogen atom or a C1-C10 alkyl group), $a_1$-NH-CO- group (wherein $a_1$ represents a C2-C10 alkyl group substituted with a C1-C10 alkoxy group), an $a_1$'-CO- group (wherein $a_1$' represents a morpholino group), a rr'N-$CH_2$- group (wherein r and r' are as defined above), a $r_0$-$(O)_1$-CONH-$CH_2$- group (wherein $r_0$ represents a C1-C10 alkyl group, and 1 represents 0 or 1), a r-$OCH_2$-group (wherein r is as defined above), a $r_0$-CO- group (wherein $r_0$ is as defined above), a cyano group or a sulfomethyl group, $r_1$ represents a C1-C10 alkylene group, $r_1$' represents a single bond or a C1-C10 alkylene group, and b represents an oxy group, a thio group, a sulfinyl group, a sulfonyl group, or an imino group}, or an $a_2$-y-CO-NH- group (wherein $a_2$ represents a C2-C10 alkyl group substituted with a C1-C10 alkoxy group, and y represents an oxy group or an imino group), or a rO-COCO-NH- group (wherein r is as defined above), or an $a_3$-z-NH- group (wherein $a_3$ represents a C2-C10 alkenyl group, or a C1-C10 alkyl group substituted with a C1-C10 alkoxy group, a C1-C10 alkoxycarbonyl group, a carboxy group or a cyano group, and z represents a carbonyl group or a sulfonyl group), or an $a_4$-NHCO- group {wherein $a_4$ represents a C1-C10 alkoxy group, or a C3-C10 alkenyloxy group, or a $r_0$-$SO_2$- group (wherein $r_0$ is as defined above), or a C2-C10 alkyl group substituted with a hydroxyl group or a C1-C10 alkoxy group, or a C2-C10 alkyl group substituted with a $r_0r_0$'N-group (wherein $r_0$ is as defined above, and $r_0$' is the same as or different from $r_0$, and represents the same meaning as $r_0$ has), or a C1-C10 alkyl group substituted with a rO-CO- group (wherein r is as defined above), a cyano group or an aminocarbonyl group, or a rO-CO-(rO-$COCH_2$)CH- group (wherein r is as defined above)}, or an $a_5$-$NHSO_2$- group (wherein $a_5$ represents a C2-C10 alkyl group substituted with a C1-C10 alkoxyl group), or a $r_0$ON=CH- group (wherein $r_0$ is as defined above), or a $r_0$NHCSNH- group (wherein $r_0$ is as defined above), or a $r_0$NHC(-S$r_0$')=N- group (wherein $r_0$ and $r_0$' are as defined above), or a $(rO)_2P(=O)CH_2$- group (wherein r is as defined above), p represents 0, 1, 2 or 3 and, and when p is not less than 2, $X_a$s are same or different,
$Y_a$ represents are halogen atom, or a Cl-C10 alkyl group optionally substituted with a halogen atom, or a C1-C10 alkyl group optionally substituted with a C1-C10 alkoxy group, or a C2-C10 alkenyl group, or a C2-C10 alkynyl group, or a 2-oxo-oxazolidin-3-yl group, or a [1,3]dioxolan-2-yl group, or a C1-C10 alkoxy group substituted with a morpholino group, or an $a_0$'-b'- group (wherein $a_0$' represents a C1-C10 alkyl group optionally substituted with a halogen atom, and b' represents an oxy group, a thio group, a sulfinyl group or a sulfonyl group), or a nitro group, or a cyano group, or a rO-CO- group (wherein r is as defined above), or a $r_0r_0$'N- group (wherein $r_0$ and $r_0$' are as defined above), or a $r_0$CO-NH- group (wherein $r_0$ is as defined above), or a $r_0r_0$'NCONH-group (wherein $r_0$ and $r_0$' are as defined above), or a rr'NCO- group (wherein r and r' are as defined above), or a hydroxyl group, q represents 0, 1, 2 or 3, and when q is not less than 2, $Y_a$s are the same or different, and when q is not less than 2, adjacent $Y_a$s may be fused to the a ring to form a 4,5,6,7-tetrahydrobenzo[b]thiophene ring;
II. b is
a group represented by formula (V'-1):

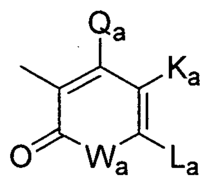

(V' -1)

wherein $Q_a$ represents a $r_a$-O- group {$r_a$ represents a hydrogen atom, or a C1-C10 alkyl group, or a C3-C10 alkenyl group, or a C3-C10 alkynyl, or a C1-C10 alkyl group substituted with a $r_0r_0$'N-$CH_2$- group (wherein $r_0$ and $r_0$' are the same or different), a $rOCH_2$ group (wherein r is as defined above), a $r_0$-CO- group (wherein $r_0$ is as defined above), a C1-C10 alkoxycarbonyl group, a carboxy group, an aminocarbonyl group or a cyano group, or a $r_3$-$r_1$- group (wherein $r_3$ represents a phenyl group or a pyridyl group, and $r_1$ is as defined above)}, or a piperidino group, or a morpholino group, or a $r_4r_4$'N-group (wherein $r_4$ and $r_4$' are the same of different, and represent a hydrogen atom, or a C1-C10 alkyl group, or C3-C10 alkenyl group, or a C3-C10 alkynyl group, or a C2-C10 alkyl group substituted with a C1-C10 alkoxy group, provided that they are not a hydrogen atom at the same time); $W_a$ represents an oxygen atom or a - $NT_a$- group [wherein $T_a$ represents a $r_b$- group (wherein $r_b$ is the same as or different from $r_a$, and represents the same meaning as $r_a$ has) or a $r_3$'- group (wherein $r_3$' is the same as or different from $r_3$, and represents the same meaning as $r_3$ has)], and $K_a$ and $L_a$ form a -$V_a$=$V_a$'-$V_a$"=$V_a$"'- group (wherein $V_a$, $V_a$', $V_a$" and $V_a$"' are the same or different, and represent a methine group or a -N= group, and at least one of $V_a$, $V_a$' $V_a$" and $V_a$"' represents a -N= group);
a group represented by formula (V'-2):

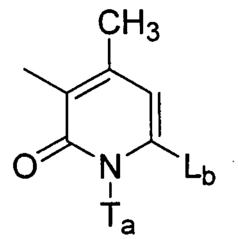

(V' -2)

wherein $T_a$ is as defined above, and $L_b$ represents a hydroxyl group or a methyl group;
a group represented by formula (V'-3):

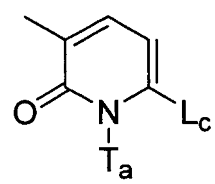

(V' -3)

wherein $T_a$ is as defined above, and $L_c$ represents a C1-C10 alkyl group;
a group represented by formula (V'-4):

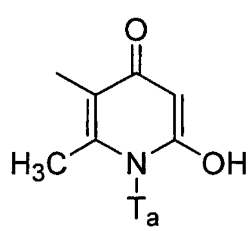

(V' -4)

wherein $T_a$ is as defined above;

a group represented by formula (V'-5):

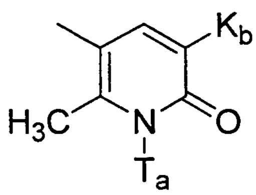

(V' -5)

wherein $T_a$ is as defined above, and $K_b$ represents a cyano group or a UOCO- group (wherein U represents a hydrogen atom or C1-C10 alkyl group);
a group represented by formula (V'-6):

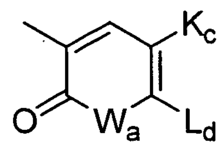

(V' -6)

wherein $W_a$ is as defined above, and $K_c$ and $L_d$ form a C3-C10 alkylene group or a C4-C10 alkenylene group;
a group represented by formula (V'-7):

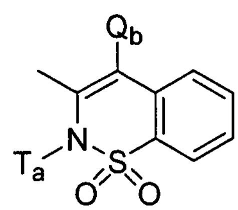

(V' -7)

wherein $T_a$ is as defined above, and $Q_b$ represents a $r_a$-O-group (wherein $r_a$ is as defined above); or
a group represented by formula (V'-8):

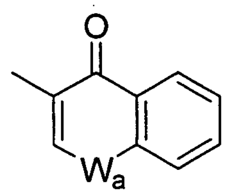

(V' -8)

wherein $W_a$ is as defined above; and
the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range;

15. A composition for suppressing transcription of an extracellular matrix gene, which comprises the compound according to any one of the above items 2 to 5, 13 and 14, and an inert carrier;
16. A composition for suppressing transcription of an extracellular matrix gene, which comprises the compound according to the above item 5 or 14, and an inert carrier;
17. Use of a cinnamoyl compound contained in the composition according to the above item 1 or 12 as an active ingredient, as an active ingredient for suppressing transcription of an extracellular matrix gene;
18. Use of the compound according to any one of the above items 2 to 5, 13 and 14, as an active ingredient for suppressing transcription of an extracellular matrix gene;

19. Use of the compound according to the above item 5 or 14, as an active ingredient for suppressing transcription of an extracellular matrix gene;
20. Use of a cinnamoyl compound contained in the composition according to the above item 1 or 12 as an active ingredient, as an active ingredient for decreasing expression of an extracellular matrix gene to induce a reduction in accumulation of an extracellular matrix and thereby improving tissue fibrosis;
21. Use of the compound according to any one of the above items 2 to 5, 13 and 14, as an active ingredient for decreasing expression of an extracellular matrix gene to induce a reduction in accumulation of an extracellular matrix and thereby improving tissue fibrosis;
22. A method for improving tissue fibrosis, which comprises administering an effective amount of a cinnamoyl compound contained in the composition according to the above item 1 or 12 as an active ingredient to a mammalian patient in need thereof;
23. A method for improving tissue fibrosis, which comprises administering an effective amount of the compound according to any one of the above items 2 to 5, 13 and 14 to a mammalian patient in need thereof;
24. An agent for treating chronic renal failure, which comprises the compound according to any one of the above items 2 to 5, 13 and 14 and an inert carrier;
25. Use of a cinnamoyl compound contained in the composition according to the above item 1 or 12 as an active ingredient, or the compound according to any one of the above items 2 to 5, 13 and 14, as an active ingredient for treating chronic renal failure;
26. A method for treating chronic renal failure, which comprises administering an effective amount of a cinnamoyl compound contained in the composition according to the above item 1 or 12 as an active ingredient, or the compound according to any one of the above items 2 to 5, 13 and 14 to a mammalian patient in need thereof;
27. An agent for treating heart failure, which comprises a cinnamoyl compound contained in the composition according to the above item 1 or 12 as an active ingredient, or the compound according to any one of the above items 2 to 5, 13 and 14, and an inert carrier;
28. Use of a cinnamoyl compound contained in the composition according to the above item 1 or 12 as an active ingredient, or the compound according to any one of the above items 2 to 5, 13 and 14, as an active ingredient for treating heart failure;
29. A method for treating heart failure, which comprises administering an effective amount of a cinnamoyl compound contained in the composition according to the above item 1 or 12 as an active ingredient, or the composition according to any one of the above items 2 to 5, 13 and 14 to a mammalian patient in need thereof;
30. A composition for suppressing the activity of TGF-β, which comprises a cinnamoyl compound contained in the composition according to the above item 1 or 12 as an active ingredient, and an inert carrier;
31. A composition for suppressing the activity of TGF-β, which comprises the compound according to any one of the above items 2 to 5, 13 and 14, and an inert carrier;
32. Use of a cinnamoyl compound contained in the composition according to the above item 1 or 12 as an active ingredient, as an active ingredient for suppressing the activity of TGF-β;
33. Use of the compound according to any one of the above items 2 to 5, 13 and 14, as an active ingredient for suppressing the activity of TGF-β;
34. A composition for hair growth which comprises a cinnamoyl compound contained in the composition according to the above item 1 or 12 as an active ingredient, or the compound according to any one of the above items 2 to 5, 13 and 14, and an inert carrier;
35. Use of a cinnamoyl compound contained in the composition according to the above item 1 or 12 as an active ingredient, or the compound according to any one of the above items 2 to 5, 13 and 14, as an active ingredient for inhibiting a promoting effect of TGF-β on transition to a hair regression phase to induce extension of a hair growth phase and thereby providing a hair-growing effect;
36. A method for growing hair, which comprises administering an effective amount of a cinnamoyl compound contained in the composition according to the above item 1 or 12 as an active ingredient, or the compound according to any one of the above items 2 to 5, 13 and 14, to a mammalian patient in need thereof; and the like.

Best Mode for Carrying Out the Invention

The present invention will be explained in detail below.

In the present invention, a saturated hydrocarbon group in an alkyl group, a haloalkyl group, an alkoxy group, an alkoxycarbonyl group, an alkylthio group, an alkylsulfinyl group, an alkylsulfonyl group and an alkylene group may be branched, or a part or all of carbon atoms thereof may form a ring. An unsaturated hydrocarbon group in an alkenyl group, an alkenyloxy group, an alkynyl group, an alkynyloxy group, an alkenylene group and an alkynylene group may be branched, or a part or all of carbon atoms thereof may form a ring, and the number of unsaturated bonds thereof may be singular or plural.

In the present invention, examples of an alkyl group include a methyl group, an ethyl group, an isopropyl group, a cyclohexyl group, a cyclopropylmethyl group and the like. Examples of a haloalkyl group include a 2,2,2-trifluoroethyl group and the like. Examples of an alkoxy group include a methoxy group, an ethoxy group, a cyclopentyloxy group, a 2-cyclohexylethoxy group and the like. Examples of an alkylthio group include a methylthio group and the like. Examples of an alkylsulfinyl group include a methylsulfinyl group and the like. Examples of an alkylsulfonyl group include a methylsulfonyl group and the like. Examples of an alkylene group include a methylene group, an ethylethylene group, a 1,4-cyclohexylene group and the like. Examples of an alkenyl group include a vinyl group, a 2-propenyl group, a 3-methyl-2-butenyl group, a 1,3-butadienyl group, a 3-cyclohexenyl group and the like. Examples of an alkynyl group include an ethynyl group, a 2-propynyl group, a 2-penten-4-ynyl group and the like. Examples of an alkenylene group include a vinylene group, a propenylene group, a 1,3-butadienylene group and the like. Examples of an alkynylene group include an ethynylene group, a propynylene group and the like.

In the present invention, examples of a halogen atom include a fluorine atom, a chlorine atom, a bromine atom and an iodine atom.

In the present invention, a pyridyl group includes a 2-pyridyl group, a 3-pyridyl group and a 4-pyridyl group. A furyl group includes a 2-furyl group and a 3-furyl group. A thienyl group includes a 2-thienyl group and a 3-thienyl group. A naphthyl group includes a 1-naphthyl group and a 2-naphthyl group.

In cinnamoyl compounds represented by the formulas (I), (II), (III), (IV), (V), (VI), (VI'), (I'), (II') and (V') (hereinafter, referred to as the compounds (I), (II), (III), (IV), (V), (VI), (VI'), (I'), (II') and (V'), respectively, in some cases), when the $\alpha$ ring, the A0 ring, the A ring or the a ring is a pyridine ring, an N-oxide thereof is also included.

The compounds (I) to (V), (I'), (II') or (V') (hereinafter referred to as present compound in some cases) includes a pharmaceutically acceptable salt thereof. The pharmaceutically acceptable salt includes a salt of the compound (I) to (V), (I') (II') or (V') with an inorganic acid, an organic acid, an inorganic base, or an organic base. Examples of a salt with an inorganic acid include hydrochloride, hydrobromide and the like. Examples of a salt with an organic acid include acetate, benzoate and the like. Examples of a salt with an inorganic base include a potassium salt, a sodium salt and the like. Examples of a salt with an organic base include a pyridine salt, a morpholine salt and the like.

$X_{A0}$, $Y_{A0}$, $G_{A0}$, $K_{A0}$, $L_{A0}$ and $T_{A0}$ in the compound (III) are independently represented by a group represented by $D_1$, $D_2$, $D_3$, $D_4$, $D_5$, $R_0$, $R_0'$, $R_0''$, $R_1$, $R_1'$, $R_1''$, $R_2$, $R_2'$, $R_3$, $R_4$, $R_4'$, $R_5$, $R_6$, $A_1$, $A_2$, $A_3$, $A_4$, $A_5$, $A_6$, $A_7$, $A_7'$, $A_7''$, $A_8$, $A_8'$, $A_9$, $A_9'$, $A_9''$, $A_{10}$, $A_{10}'$, $A_{11}$, B, B', $B_0$, $B_1$, $B_1'$, $B_2$, $B_2'$, $B_3$, $B_3'$, $B_4$, $B_4'$, $B_5$, $B_6$, ($a_0$), ($b_0$), ($c_0$), ($d_0$), ($e_0$), $M_a$, $M_a'$, $M_a''$, $M_a'''$, $M_a''''$, $M_{b0}$, $Mc_0$, $Md_0$, $Ra_0$, $R_b$, $R_c$, $R_d$, $R_d'$, $R_e$, $R_e'$, $R_e''$, $R_e'''$, $B_a$, $B_b$, $B_c$, $Y_a$, $Y_a'$, $Y_b$, $Y_b'$, $Y_b''$, $Y_c$ and $Y_c'$, and an integer represented by k, k', 1, m, m', n and n'.

$X_A$, $Y_A$, $Q_A$, $K_A$, $L_A$ and $T_A$ in the compound (IV) are independently represented by a group represented by $D_1$, $D_2$, $D_3$, $D_4$, $D_5$, $R_0$, $R_0'$, $R_0''$, $R_1$, $R_1'$, $R_1''$, $R_2$, $R_2'$, $R_3$, $R_4$, $R_4'$, $R_5$, $R_6$, $A_1$, $A_2$, $A_3$, $A_4$, $A_5$, $A_6$, $A_7$, $A_7'$, $A_7''$, $A_8$, $A_8'$, $A_9$, $A_9'$, $A_9''$, $A_{10}$, $A_{10}'$, $A_{11}$, B, B', $B_0$, $B_1$, $B_1'$, $B_2$, $B_2'$, $B_3$, $B_3'$, $B_4$, $B_4'$, $B_5$, $B_6$, (a), (b), (c), (d), (e), $M_a$, $M_a'$, $M_a''$, $M_a'''$, $M_a''''$, $M_b$, $M_c$, $M_d$, $R_a$, $R_b$, $R_c$, $R_d$, $R_d'$, $R_e$, $R_e'$, $R_e''$, $R_e'''$, $B_a$, $B_b$, $B_c$, $Y_a$, $Y_a'$, $Y_b$, $Y_b'$, $Y_b''$, $Y_c$ and $Y_c'$, and an integer represented by k, k', 1, m, m', n and n'.

$X_a$, $Y_a$, $q_a$ and $t_a$ in compounds (V) and (V') are independently represented by a group represented by $a_0$, $a_0'$, $a_1$, $a_1'$, $a_2$, $a_3$, $a_4$, $a_5$, b, b', r, r', $r_0$, $r_0'$, $r_1$, $r_1'$, $r_2$, $r_3$, $r_3'$, $r_4$, $r_4'$, $r_a$, $r_b$, y and z, and an integer represented by 1.

The "6- to 10-membered aryl group", in the $Y_0$ group of substituents which may be selected as $Y_\alpha$ of the compounds (I), (II), (I') and (II'), represents a group forming a monocyclic or fused aromatic hydrocarbon ring, and examples thereof include a phenyl group, a 1-naphthyl group, a 2-naphtyl group, a 6-indanyl group and the like. The "5- to 10-memberd heteroaryl group" represents a group forming a monocyclic or fused aromatic heterocycle, and examples thereof include a 2-furyl group, a 3-furyl group, a 2-thienyl group, a 3-thienyl group, a 2-pyridyl group, a 3-pyridyl group, a 4-pyridyl group, a 2-quinolyl group, and the like. The "3- to 10-memberd cyclic hydrocarbon or heterocyclic group optionally containing an unsaturated bond" includes a monocyclic or fused ring, and examples thereof include a 2-cyclohexenyl group, a 2-morpholinyl group, a 4-piperidyl group and the like. The "3- to 10-memberd cyclic hydrocarbon or heterocyclic group optionally containing an unsaturated bond" may be substituted with 1 or more same or different $M_a$-groups as describe above.

The "group which is fused to the $\alpha$ ring", in the $Z_0$ group of substituents which may be selected as $Y_\alpha$ of the compounds (I), (II), (I') and (II'), may have 1 or more same or different atoms or groups selected from a halogen atom, a C1-C10 alkoxy group, a C3-C10 alkenyloxy group, a C3-C10 alkynyloxy group, a carbonyl group, a thiocarbonyl group, an oxy group, a thio group, a sulfinyl group and a sulfonyl group.

In $R_{a0}$ of the $E_o$ group of substituents which may be selected as $X_{A0}$ of the compound (III), the "optionally substituted 5- to 7-membered aryl group or heteroaryl group" represents a group forming a monocyclic or fused aromatic hydrocarbon ring or a group forming a monocyclic or fused aromatic heterocycle, and examples thereof include a phenyl group, a 1-naphthyl group, a 2-naphthyl group, a 6-indanyl group, a 2-furyl group, a 3-furyl group, a 2-thienyl group, a 3-thienyl group, a 2-pyridyl group, a 3-pyridyl group, a 4-pyridyl group, a 2-quinolyl group and the like. The "optionally substituted 5- to 7-membered aryl group or heteroaryl group" may be substituted with 1 or more same or different $M_a$-groups as described above.

In (do) of the $Y_0$ group of substituents which may be selected as $Y_\alpha$ or $Y_{A0}$ of the compounds (I), (II), (III), (I') and (II'), the phrase "forming a 5 to 12-membered hydrocarbon ring which is substituted with a carbonyl group or a thiocarbonyl group and further which may be optionally substituted with an oxy group, a thio group, a -$NR_1$- group (wherein $R_1$ is as defined above), a sulfinyl group or a sulfonyl group" means forming a 5- to 12-membered hydrocarbon ring in which one or more of carbon atoms are substituted with a carbonyl group or a thiocarbonyl group, and further, one or more of carbon atoms may be substituted with one or more same or different groups selected from an oxy group, a thio group, a -$NR_1$- group (wherein $R_1$ is as defined above), a sulfinyl group and a sulfonyl group.

In ($e_0$) of the $Y_0$ group of substituents which may be selected as $Y_\alpha$ or $Y_{A0}$ of the compounds (I), (II), (III), (I') and (II'), the phrase "forming a 5 to 12-membered hydrocarbon ring optionally substituted with a carbonyl group, a thiocarbonyl group, an oxy group, a thio group, a -$NR_1$- group (wherein $R_1$ is as defined above), a sulfinyl group or a sulfonyl group" means forming a 5- to 12-membered hydrocarbon ring in which one or more of carbon atoms are optionally substituted with one or more same or different groups selected from a carbonyl group, a thiocarbonyl group, an oxy group, a thio group, a -$NR_1$-group (wherein $R_1$ is as defined above), a sulfinyl group and a sulfonyl group.

In (a) of the B group of substituents which may be selected as $X_A$ of the compound (IV), the "C2-C10 alkylene group optionally substituted with an oxy group, a thio group, a sulfinyl group, a sulfonyl group or a -$NR_1'$- group (wherein $R_1'$ is as defined above)" means a C2-C10 alkylene group in which one or more of carbon atoms may be substituted with one or more same or different groups selected from an oxy group, a thio group, a sulfinyl group, a sulfonyl group and a -$NR_1'$- group (wherein $R_1'$ is as defined above), and the "C3-C10 alkenylene group optionally substituted with an oxy group, a thio group, a sulfinyl group, a sulfonyl group or a -$NR_1'$- group (wherein $R_1'$ is as defined above)" means a C3-C10 alkenylene group in which one or more of carbon atoms may be substituted with oen or more same or different groups selected from an oxy group, a thio group, a sulfinyl group, a sulfonyl group and a -$NR_1'$-group (wherein $R_1'$ is as defined above).

In (b) of the D group of substituents which may be selected as $X_A$ of the compound (IV), the "C1-C10 alkylene group optionally substituted with a methyl group, an oxy group, a thio group, a sulfinyl group, a sulfonyl group or a -$NR_1$- group (wherein $R_1$ is as defined above)" means a C2-C10 alkylene group in which one or more of carbon atoms may be substituted with a methyl group, or one or more of carbon atoms may be substituted with one or more same or different groups selected from an oxy group, a thio group, a sulfinyl group, a sulfonyl group and a -$NR_1$- group (wherein $R_1$ is as defined above), and the "C2-C10 alkenylene group optionally substituted with a methyl group, an oxy group, a thio group, a sulfinyl group, a sulfonyl group or a -$NR_1$- group (wherein $R_1$ is as defined above)" means a C2-C10 alkenylene group in which one or more of carbon atoms may be substituted with a methyl group, or one or more carbon atoms may be substituted with one or more same or different groups selected from an oxy group, a thio group, a sulfinyl group, a sulfonyl group and a -$NR_1$- group (wherein $R_1$ is as defined above).

Examples of groups belonging to the $X_0$ group, the $Y_0$ group and the $Z_0$ group which may be selected as $Y_\alpha$ of the compounds (I), (II), (I') and (II') are shown in the following Table 24, Table 25 and Table 26, respectively.

Examples of groups belonging to the $A_0$ group, the $B_0$ group, the $C_0$ group, the $D_0$ group, the $E_0$ group, the $F_0$ group, the Go group, the $H_0$ group, the $I_0$ group, the $J_0$ group, the $K_0$ group, the $L_0$ group, the $M_0$ group and the No group which may be selected as $X_{A0}$ of the compound (III) are shown in the following Table 1, Table 2, Table 3, Table 4, Tables 5 to 8, Tables 9 to 12, Tables 13 to 16, Table 17, Table 18, Table 19, Table 20, Table 21, Table 22 and Table 23, respectively. Examples of groups belonging to the $X_0$ group, the $Y_0$ group and the $Z_0$ group which may be selected as $Y_{A0}$ of the compound (III) are shown in the following Table 24, Table 25 and Table 26. Examples of groups which may be selected as $Q_{A0}$ and $T_{A0}$ of the compound (III) are shown in the following Table 27 to Table 28 and Table 29, respectively.

Examples of groups belonging to the A group, the B group, the C group, the D group, the E group, the F group, the G group, the H group, the I group, the J group, the K group, the L group, the M group and the N group which may be selected as $X_A$ of the compound (IV) are shown in the following Table 1, Table 2, Table 3, Table 4, Tables 5 to 8, Tables 9 to 12, Tables 13 to 16, Table 17, Table 18, Table 19, Table 20, Table 21, Table 22 and Table 23, respectively. Examples of groups belonging to the X group, the Y group and the Z group which may be selected as $Y_A$ of the compound (IV) are shown in the following Table 24, Table 25 and Table 26, respectively. Examples of groups which may be selected as $Q_A$ and $T_A$ of the compound (IV) are shown in the following Table 27 to Table 28 and Table 29, respectively.

Examples of the aforementioned groups belonging to the $A_0$ group to No group and the A group to N group are shown in the following Table 1 to Table 23. When said groups have geometrical isomers, all of the geometrical isomers are included, and when said groups have tautomers, all of the tautomers are included.

Examples of groups belonging to the $A_0$ group and the A group are shown in Table 1.

Table 1

| No. | Group |
|---|---|
| A-1 | $-CH_2ONH_2$ |
| A-2 | $-CH_2ON(CH_3)_2$ |
| A-3 | $-CH_2ONHCOCH_3$ |
| A-4 | $-CH_2NHOCH_2CH=CH_2$ |
| A-5 | $-CH_2CN$ |
| A-6 | $-CH_2CH_2CN$ |
| A-7 | $-CH_2CH_2C(=NH)NH_2$ |
| A-8 | $-CH_2CH_2C(=NCH2C=CH)N(CH_3)_2$ |
| A-9 | $-CH_2C(=NH)NHCOCH_3$ |
| A-10 | $-CH_2C(=NOCOCH_3)-NH_2$ |
| A-11 | $-CH_2C(=NCOCH_3)-OCH_3$ |
| A-12 | $-CH_2CSNH_2$ |
| A-13 | $-CH_2NO_2$ |
| A-14 | $-CH_2SO_3H$ |
| A-15 | $-SO_3H$ |

Examples of groups belonging to the $B_0$ group and the B group are shown in Table 2.

Table 2

| No. | Group | No. | Group |
|---|---|---|---|
| B-1 | 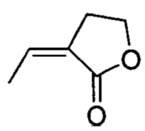 | B-4 | 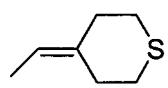 |
| B-2 | 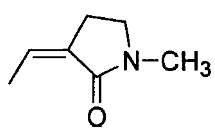 | B-5 | 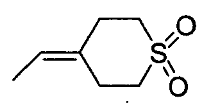 |
| B-3 | 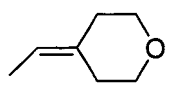 | B-6 | 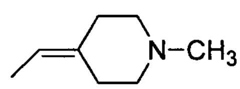 |

Examples of groups belonging to the $C_0$ group and the C group are shown in Table 3.

Table 3

| No. | Group |
|---|---|
| C-1 | $-CH=CF_2$ |
| C-2 | $-CH=CHOCH_3$ |
| C-3 | $-CH=CHSCH_3$ |
| C-4 | $-CH=CHSOCH_3$ |
| C-5 | $-CH=CHSO_2CH_3$ |

(continued)

| No. | Group |
|---|---|
| C-6 | $-CH=CHCH_2OH$ |
| C-7 | $-CH=CHCH_2OCOCH_3$ |
| C-8 | $-CH=CHCHO$ |
| C-9 | $-CH=CHCH=NCH_2CH=CH_2$ |
| C-10 | $-CH=CHCH=NOH$ |
| C-11 | $-CH=CHCH=NOCH_2COOCH_3$ |
| C-12 | $-CH=CHCH=NOCH_2CN$ |
| C-13 | $-CH=CHCH=NN(CH_3)_2$ |
| C-14 | $-CH=CHCH=NNHCOCH_3$ |
| C-15 | $-CH=CHCOCH_3$ |
| C-16 | $-CH=C(CH_3)COCH_3$ |
| C-17 | $-CH=CHCOCF_3$ |
| C-18 | $-CH=CHCH_2ON(CH_3)_2$ |
| C-19 | $-CH=CHCH_2ON(SO_2CH_3)CH_3$ |
| C-20 | $-CH=CHCH_2N(CH_2CH=CH_2)_2$ |
| C-21 | $-CH=CHCH_2N(OH)CH_3$ |
| C-22 | $-CH=CHNHCOCH_3$ |
| C-23 | $-CH=CHCN$ |
| C-24 | $-CH=CHC(=NH)N(CH_3)_2$ |
| C-25 | $-CH=CHC(=NH)NHOCH_3$ |
| C-26 | $-CH=CHCSNH_2$ |
| C-27 | $-CH=CHNO_2$ |
| C-28 | $-CH=CHSO_3H$ |

Examples of groups belonging to the $D_0$ group and the D group are shown in Table 4.

Table 4

| No. | Group |
|---|---|
| D-1 | 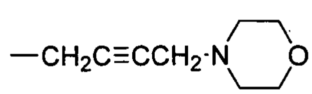 |
| D-2 | 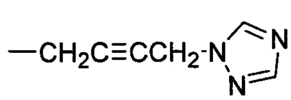 |
| D-3 | $-C{\equiv}CI$ |
| D-4 | $-C{\equiv}CCH_2SCH_3$ |
| D-5 | $-C{\equiv}CC(CH_3)_2OH$ |
| D-6 | $-C{\equiv}CCH_2OCOOCH_3$ |
| D-7 | $-C{\equiv}CCH=NCH_3$ |

(continued)

| No. | Group |
|---|---|
| D-8 | $-C{\equiv}CCH{=}NOCH_3$ |
| D-9 | $-C{\equiv}CCH{=}NN(CH_3)_2$ |
| D-10 | $-C{\equiv}CCH_2ON(CH_3)_2$ |
| D-11 | $-C{\equiv}CCH_2N(CH_3)_2$ |
| D-12 | $-C{\equiv}CCH_2CH_2NO_2$ |

Examples of groups belonging to the $E_0$ group and the E group are shown in Table 5 to Table 8.

Table 5

| No. | Group |
|---|---|
| E-1 | $-CH{=}CHCOOCH_3$ |
| E-2 | $-CH{=}CHCOOC_2H_5$ |
| E-3 | $-CH{=}CHCOOCH_2CH_2Cl$ |
| E-4 | $-CH{=}CHCOOCH_2CF_3$ |
| E-5 | $-CH{=}CHCOOCH_2CH{=}CH_2$ |
| E-6 | $-CH{=}CHCOOCH_2C{=}CH$ |
| E-7 | 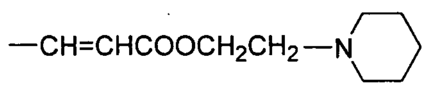 |
| E-8 | 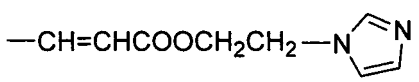 |
| E-9 | $-CH{=}CHCOOCH_2CH_2OCH_3$ |
| E-10 | $-CH{=}CHCOOCH_2CH_2SCH_3$ |
| E-11 | $-CH{=}CHCOOCH_2CH_2SOCH_3$ |
| E-12 | $-CH{=}CHCOOCH_2CH_2SO_2CH_3$ |
| E-13 | $-CH{=}CHCOOCH_2CH_2OH$ |
| E-14 | $-CH{=}CHCOOCH_2CH_2OSO_2N(CH_3)_2$ |
| E-15 | $-CH{=}CHCOOCH_2CH_2COCH_3$ |
| E-17 | $-CH{=}CHCOOCH_2CH_2ON(CH_3)_2$ |
| E-18 | $-CH{=}CHCOOCH_2CH_2N(CH_3)_2$ |
| E-19 | $-CH{=}CHCOOCH_2CH_2N(OC_ZH_5)C_2H_5$ |
| E-20 | $-CH{=}CHCOOCH_2CH_2NHCOCH_3$ |
| E-21 | $-CH{=}CHCOOCH_2CH_2N\ (CH_3)\ COCH_3$ |
| E-22 | $-CH{=}CHCOOCH_2CH_2NHCOOCH_2CH_2OCH_3$ |
| E-23 | $-CH{=}CHCOOCH_2CH_2NHCOSCH_2CH{=}CH_2$ |
| E-24 | $-CH{=}CHCOOCH_2CH_2NHCONHC_2H_5$ |
| E-25 | $-CH{=}CHCOOCH_2CH_2NHCON(CH_3)_2$ |

Table 6

| No. | Group |
|---|---|
| E-26 | $-CH{=}CHCOOCH_2CH_2NHCON\ (OCH_3)\ CH_3$ |
| E-27 | $-CH{=}CHCOOCH_2CH_2NHCSNHCH_2CH_2Cl$ |
| E-28 | $-CH{=}CHCOOCH_2CH_2NHSO_2N(CH_3)_2$ |
| E-29 | $-CH{=}CHCOOCH_2CH_2CN$ |
| E-30 | $-CH{=}CHCOOCH_2CH_2NO_2$ |
| E-31 | $-CH{=}CHCOOCH_2CH_2SO_3H$ |
| E-32 | 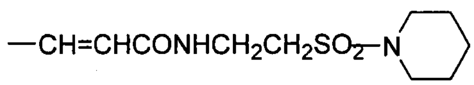 |
| E-33 | $-CH{=}CHCONHCH_2CH_2SO_2N\ (CH_3)_2$ |
| E-34 | $-CH{=}CHCOSCH_3$ |
| E-35 | $-CH{=}CHCON(CH_3)CH_2C{=}CH$ |
| E-36 | $-CH{=}CHCON(OCH_3)CH_3$ |
| E-37 | $-CH{=}CHCONHOCH_3$ |
| E-38 | $-CH{=}CHCONHOCH_2CH{=}CH_2$ |
| E-39 | $-CH{=}CHCOOCH_2COOCH_3$ |
| E-40 | $-CH{=}CHCOSCH_2COOCH_3$ |
| E-41 | $-CH{=}CHCONHCH_2COOCH_3$ |
| E-42 | $-CH{=}CHCONHCH_2CON(CH_3)_2$ |
| E-43 | $-CH{=}CHCONHCH_2CN$ |
| E-44 | $-CH{=}CHCONHCH_2C({=}NH)N(CH_3)CH_2CH{=}CH_2$ |
| E-45 | $-CH{=}CHCONHCH_2C({=}NH)NHOH$ |

Table 7

| No. | Group |
|---|---|
| E-46 | $-CH{=}CHCONHSO_2CH_3$ |
| E-47 | 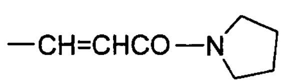 |
| E-48 | 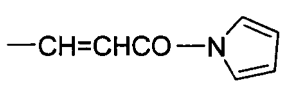 |
| E-49 | 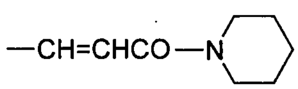 |
| E-50 | 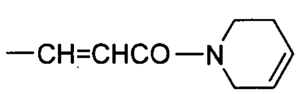 |

(continued)

| No. | Group |
|---|---|
| E-51 | 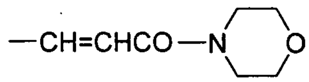 |
| E-52 | 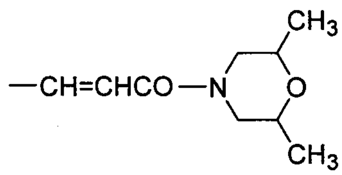 |
| E-53 | 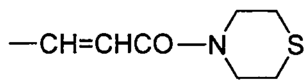 |
| E-54 | 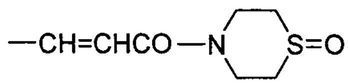 |
| E-55 | 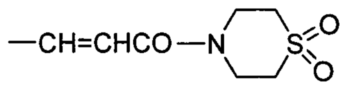 |

Table 8

| No. | Group |
|---|---|
| E-56 | 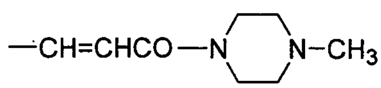 |
| E-57 | 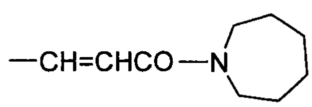 |
| E-58 | 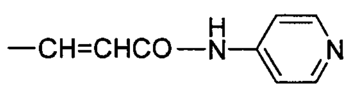 |
| E-59 | $-CH=CHCONHN(CH_3)_2$ |
| E-60 | $-CH=CHCONHNHCOOC_2H_5$ |
| E-61 | $-CH=CHCONHNHCSNH(c)C_6H_{11}$ |
| E-62 | $-CH=CFCOOCH_3$ |

Examples of groups belonging to the $F_0$ group and the F group are shown in Table 8 to Table 12.

Table 9

| No. | Group |
|---|---|
| F-1 | $-OCH_2CH_2OH$ |
| F-2 | $-OCH_2CH_2CH_2OH$ |

(continued)

| No. | Group |
|---|---|
| F-3 | $-CH_2OCH_2CH_2OH$ |
| F-4 | $-OCH_2CH_2OCON\ (CH_3)_2$ |
| F-5 | $-OCH_2CH_2ONH_2$ |
| F-6 | $-OCH_2CH_2N(CH_3)_2$ |
| F-7 | $-OCH_2CH_2CH_2N(CH_3)_2$ |
| F-8 | $-OCH_2CH_2N(OCH_3)CH_3$ |
| F-9 | $-OCH_2CH_2NH_2$ |
| F-10 | $-OCH_2CH_2NHCOCH_3$ |
| F-11 | $-OCH_2CH_2N(CH_3)COCH_3$ |
| F-12 | $-OCH_2CH_2NHCOO(t)C_4H_9$ |
| F-13 | $-OCH_2CH_2NHCOSCH_2CH=CH_2$ |
| F-14 | $-OCH_2CH_2NHCONHC_2H_5$ |
| F-15 | $-OCH_2CH_2NHCON(CH_3)_2$ |
| F-16 | $-OCH_2CH_2NHCON\ (OCH_3)CH_3$ |
| F-17 | $-OCH_2CH_2NHCSNHCH_2CH_2Cl$ |
| F-18 | $-OCH_2CH_2NO_2$ |
| F-19 | $-OCH_2CH_2SO_3H$ |
| F-20 | $-OCH_2CH_2CH_2SO_3H$ |
| F-21 | $-OCH_2CH_2CH_2CH_2SO_3H$ |
| F-22 | $-OCH_2CH_2NHSO_2N\ (CH_3)_2$ |
| F-23 | 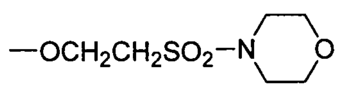 |
| F-24 | $-OCH_2CH_2OCH_3$ |
| F-25 | $-OCH_2CH_2SCH_3$ |

Table 10

| No. | Group |
|---|---|
| F-26 | $-OCH_2CH_2SOCH_3$ |
| F-27 | $-OCH_2CH_2SO_2CH_3$ |
| F-28 | $-OCH_2CN$ |
| F-29 | $-OCH_2C(=NH)NH_2$ |
| F-30 | $-OCH_2CSNH_2$ |
| F-31 | $-OCH_2COCH_3$ |
| F-32 | $-OCH_2COCF_3$ |
| F-33 | $-OCH_2CHO$ |
| F-34 | $-OCH_2CH=NOCH_2C=CH$ |
| F-35 | $-OCH_2CH=NN(CH_3)_2$ |

(continued)

| No. | Group |
|---|---|
| F-36 | $-OCH_2COOH$ |
| F-37 | $-OCH_2COOCH_3$ |
| F-38 | $-OCH_2COOCH_2CH_2Cl$ |
| F-39 | $-OCH_2COOCH_2CH{=}CH_2$ |
| F-40 | $-OCH_2COOCH_2C{=}CH$ |
| F-41 | 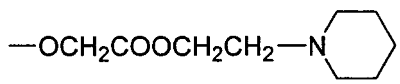 |
| F-42 | 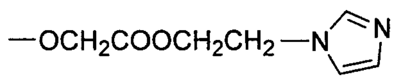 |
| F-43 | $-OCH_2COOCH_2CH_2OCH_3$ |
| F-44 | $-OCH_2COOCH_2CH_2SCH_3$ |
| F-45 | $-OCH_2COOCH_2CH_2SOCH_3$ |
| F-46 | $-OCH_2COOCH_2CH_2SO_2CH_3$ |
| F-47 | $-OCH_2COOCH_2CH_2OH$ |
| F-48 | $-OCH_2COO(CH_2)_9OH$ |
| F-49 | $-OCH_2COOCH_2CH_2OSO_2N(CH_3)_2$ |
| F-50 | $-OCH_2COOCH_2CH_2COCH_3$ |

Table 11

| No. | Group |
|---|---|
| F-51 | $-OCH_2COOCH_2CH_2ON(CH_3)_2$ |
| F-52 | $-OCH_2COOCH_2CH_2N(CH_3)_2$ |
| F-53 | $-OCH_2COOCH_2CH_2N(OC_2H_5)C_2H_5$ |
| F-54 | $-OCH_2COOCH_2CH_2NHCOCH_3$ |
| F-55 | $-OCH_2COOCH_2CH_2N(CH_3)COCH_3$ |
| F-56 | $-OCH_2COOCH_2CH_2NHCOOCH_2CH_2OCH_3$ |
| F-57 | $-OCH_2COOCH_2CH_2NHCOSCH_2CH{=}CH_2$ |
| F-58 | $-OCH_2COOCH_2CH_2NHCONHC_2H_5$ |
| F-59 | $-OCH_2COOCH_2CH_2NHCON(CH_3)_2$ |
| F-60 | $-OCH_2COOCH_2CH_2NHCON(OCH_3)CH_3$ |
| F-61 | $-OCH_2COOCH_2CH_2NHCSNHCH_2CH_2Cl$ |
| F-62 | $-OCH_2COOCH_2CH_2NHSO_2N(CH_3)_2$ |
| F-63 | $-OCH_2COOCH_2CH_2CN$ |
| F-64 | $-OCH_2COOCH_2CH_2NO_2$ |
| F-65 | $-OCH_2COOCH_2CH_2SO_3H$ |

(continued)

| No. | Group |
|---|---|
| F-66 | 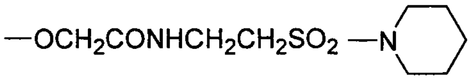 |
| F-67 | $-OCH_2CONHCH_2CH_2SO_2N\ (CH_3)_2$ |
| F-68 | $-OCH_2COSCH_3$ |
| F-69 | $-OCH_2CONH_2$ |
| F-70 | $-OCH_2CONHCH_3$ |
| F-71 | $-OCH_2CON(CH_3)_2$ |
| F-72 | $-OCH_2CON(CH_3)CH_2C{\equiv}CH$ |
| F-73 | $-OCH_2CON\ (OCH_3)\ CH_3$ |
| F-74 | $-OCH_2CONHOCH_3$ |
| F-75 | $-OCH_2CONHOCH_2CH{=}CH_2$ |

Table 12

| No. | Group |
|---|---|
| F-76 | $-OCH_2COOCH_2COOCH_3$ |
| F-77 | $-OCH_2COSCH_2COOCH_3$ |
| F-78 | $-OCH_2CONHCH_2COOCH_3$ |
| F-79 | $-OCH_2CONHCH_2CON(CH_3)_2$ |
| F-80 | $-OCH_2CONHCH_2CN$ |
| F-81 | $-OCH_2CONHCH_2C(=NH)NH_2$ |
| F-82 | $-OCH_2CONHSO_2CH_3$ |
| F-83 | 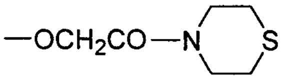 |
| F-84 | $-OCH_2CONHN\ (CH_3)\ _2$ |
| F-85 | $-OCH_2CONHNHCOOC_2H_5$ |
| F-86 | $-OCH_2CONHNHCSNH\ (c)\ C_6H_{11}$ |
| F-87 | $-SCH_2CN$ |
| F-88 | $-CH_2SCH_2COOCH_3$ |
| F-89 | $-CH_2SOCH_2COOCH_3$ |
| F-90 | $-CH_2SO_2CH_2COOCH_3$ |
| F-91 | $-NHCH_2COOCH_3$ |
| F-92 | $-NHCH_2CH_2N(CH_3)_2$ |
| F-93 | $-N\ (COCH_3)\ CH_2CH_2OH$ |
| F-94 | $-CH_2OCH_2COOCH_3$ |
| F-95 | $-CH_2SCH_2COOH$ |
| F-96 | $-CH_2SOCH_2COOH$ |
| F-97 | $-CH_2SO_2CH_2COOH$ |

Examples of groups belonging to the Go group and the G group are shown in Table 13 to Table 16.

Table 13

| No. | Group | No. | Group |
|---|---|---|---|
| G-1 | 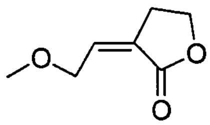 | G-4 | 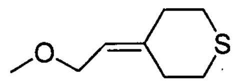 |
| G-2 | 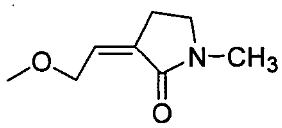 | G-5 | 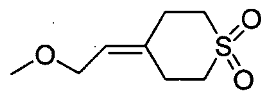 |
| G-3 | 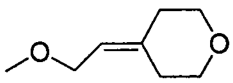 | G-6 | 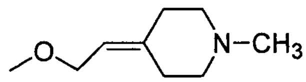 |
| No. | Group | | |
| G-7 | $-OCH_2CH=CH_2$ | | |
| G-8 | $-OCH_2C \equiv CH$ | | |
| G-9 | $-OCH_2CH=CHCl$ | | |
| G-10 | $-SCH=CHOCH_3$ | | |
| G-11 | $-SO_2CH=CHOCH_3$ | | |
| G-12 | $-OCH=CHCOCH_3$ | | |
| G-13 | -OCH=CHCHO | | |
| G-14 | $-OCH=CHCH=NCH_2CH=CH_2$ | | |
| G-15 | $-OCH=CHCH=NOCH_3$ | | |
| G-16 | $-OCH=CHCH=NN(CH_3)_2$ | | |
| G-17 | -OCH=CHCN | | |
| G-18 | $-OCH=CHC(=NH)NH_2$ | | |
| G-19 | -OCH=CHCOOH | | |
| G-20 | $-OCH_2C \equiv CCOOH$ | | |

Table 14

| No. | Group |
|---|---|
| G-21 | $-OCH=CHCOOCH_3$ |
| G-22 | $-OCH=CHCOOCH_2CH_2Cl$ |
| G-23 | $-OCH=CHCOOCH_2CH=CH_2$ |
| G-24 | $-OCH=CHCOOCH_2C \equiv CH$ |
| G-25 | 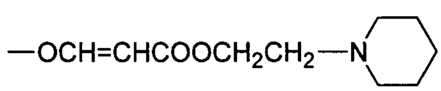 |
| G-26 | 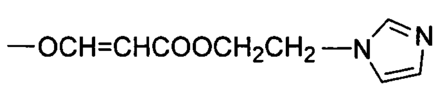 |

(continued)

| No. | Group |
|---|---|
| G-27 | $-OCH{=}CHCOOCH_2CH_2OCH_3$ |
| G-28 | $-OCH{=}CHCOOCH_2CH_2SCH_3$ |
| G-29 | $-OCH{=}CHCOOCH_2CH_2SOCH_3$ |
| G-30 | $-OCH{=}CHCOOCH_2CH_2SO_2CH_3$ |
| G-31 | $-OCH{=}CHCOOCH_2CH_2OH$ |
| G-32 | $-OCH{=}CHCOOCH_2CH_2OSO_2N(CH_3)_2$ |
| G-33 | $-OCH{=}CHCOOCH_2CH_2COCH_3$ |
| G-34 | $-OCH{=}CHCOOCH_2CH_2ON(CH_3)_2$ |
| G-35 | $-OCH{=}CHCOOCH_2CH_2N\ (CH_3)_2$ |
| G-36 | $-OCH{=}CHCOOCH_2CH_2N\ (OC_2H_5)C_2H_5$ |
| G-37 | $-OCH{=}CHCOOCH_2CH_2NHCOCH_3$ |
| G-38 | $-OCH{=}CHCOOCH_2CH_2N\ (CH_3)\ COCH_3$ |
| G-39 | $-OCH{=}CHCOOCH_2CH_2NHCOOCH_2CH_2OCH_3$ |
| G-40 | $-OCH{=}CHCOOCH_2CH_2NHCOSCH_2CH{=}CH_2$ |
| G-41 | $-OCH{=}CHCOOCH_2CH_2NHCONHC_2H_5$ |
| G-42 | $-OCH{=}CHCOOCH_2CH_2NHCON\ (CH_3)_2$ |
| G-43 | $-OCH{=}CHCOOCH_2CH_2NHCON\ (OCH_3)\ CH_3$ |
| G-44 | $-OCH{=}CHCOOCH_2CH_2NHCSNHCH_2CH_2Cl$ |
| G-45 | $-OCH{=}CHCOOCH_2CH_2NHSO_2N\ (CH_3)_2$ |
| G-46 | $-OCH{=}CHCOOCH_2CH_2C\ ({=}NH)\ NH_2$ |
| G-47 | $-OCH{=}CHCOOCH_2CH_2NO_2$ |
| G-48 | $-OCH{=}CHCOOCH_2CH_2SO_3H$ |

Table 15

| No. | Group |
|---|---|
| G-49 | 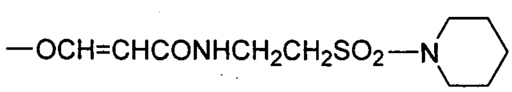 |
| G-50 | $-OCH{=}CHCONHCH_2CH_2SO_2N(CH_3)_2$ |
| G-51 | $-OCH{=}CHCOSCH_3$ |
| G-52 | $-OCH{=}CHCON(CH_3)CH_2C{\equiv}CH$ |
| G-53 | $-OCH{=}CHCON(OCH_3)CH_3$ |
| G-54 | $-OCH{=}CHCONHOCH_3$ |
| G-55 | $-OCH{=}CHCONHOCH_2CH{=}CH_2$ |
| G-56 | $-OCH{=}CHCONHCH_2COOCH_3$ |
| G-57 | $-OCH{=}CHCONHCH_2CON\ (CH_3)_2$ |
| G-58 | $-OCH{=}CHCONHSO_2CH_3$ |

(continued)

| No. | Group |
|---|---|
| G-59 | 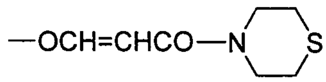 |
| G-60 | $-OCH=CHCONHN(CH_3)_2$ |
| G-61 | $-OCH=CHCONHNHCOOC_2H_5$ |
| G-62 | $-OCH=CHCONHNHCSNH(c)C_6H_{11}$ |
| G-63 | 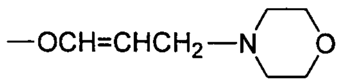 |
| G-64 | 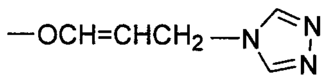 |
| G-65 | $-OCH=CHCH_2OCH_3$ |
| G-66 | $-OCH=CHCH_2SCH_3$ |
| G-67 | $-OCH=CHCH_2SOCH_3$ |
| G-68 | $-OCH=CHCH_2SO_2CH_3$ |
| G-69 | $-OCH=CHCH_2OH$ |
| G-70 | $-OCH=CHCH_2OCOCH$ |

Table 16

| No. | Group |
|---|---|
| G-71 | $-OCH_2C=CCH_2OH$ |
| G-72 | $-OCH=CHCH_2ON(CH_3)_2$ |
| G-73 | $-OCH=CHCH_2N(CH_3)_2$ |
| G-74 | $-OCH=CHCH_2N(CH_2CH=CH_2)_2$ |
| G-75 | $-OCH=CHCH_2N(OH)CH_3$ |
| G-76 | $-OCH=CHCH_2NO_2$ |
| G-77 | $-OCH=CHCH_2SO_3H$ |
| G-78 | $-SCH_2CH=CH_2$ |
| G-79 | $-SOCH_2CH=CH_2$ |
| G-80 | $-SO_2CH_2CH=CH_2$ |
| G-81 | $-SCH=CHCOOH$ |
| G-82 | $-CH_2NHCH=CHCOOH$ |
| G-83 | $-CH_2OCH_2CH=CH_2$ |
| G-84 | $-CH_2OCH=CHCOOH$ |

Examples of groups belonging to the $H_0$ group and the H group are shown in Table 17.

Table 17

| No. | Group |
|---|---|
| H-1 | $-CH_2NHCN$ |
| H-2 | $-N(COCH_3)CN$ |
| H-3 | -NHC(=NH)NHOH |
| H-4 | $-NHC(=NH)N(CH_2CH=CH_2)CH_3$ |
| H-5 | $-C(=NH)NHCH_2CH=CH_2$ |
| H-6 | $-N=CHN(CH_3)_2$ |
| H-7 | $-N(CH_3)C(CH_3)=NOCH_2C≡CH$ |
| H-8 | $-NHCONHCOCH_3$ |
| H-9 | $-NHCONHSO_2CH_3$ |
| H-10 | -NHCOCN |
| H-11 | $-NHCOCOOCH_3$ |
| H-12 | -NHCOCOOH |

Examples of groups belonging to the $I_0$ group and the I group are shown in Table 18.

Table 18

| No. | Group |
|---|---|
| I-1 | $-NHCOCH=CH_2$ |
| I-2 | $-NHCSCH=CH_2$ |
| 1-3 | $-NHCOCF=CH_2$ |
| 1-4 | -NHCOC≡CH |
| 1-5 | $-NHCOCH_2OCH_3$ |
| I-6 | $-NHCOCH_2SCH_3$ |
| I-7 | $-NHCOCH_2COCH_3$ |
| I-8 | $-NHCOCH_2OH$ |
| I-9 | $-NHCOCH_2ONH_2$ |
| I-10 | $-NHCOCH_2N(CH_3)CH_2C≡CH$ |
| I-11 | $-NHCOCH_2NHCOCH_3$ |
| I-12 | $-NHCOCH_2COOCH_3$ |
| I-13 | $-NHCOCH_2CN$ |
| I-14 | $-NHCOCH_2NO_2$ |
| I-15 | $-NHCOCH_2SO_3H$ |
| I-16 | $-NHCOCH_2SO_2N(CH_3)_2$ |
| I-17 | $-NHCSCH_3$ |
| I-18 | $-NHCSCH_2N(CH_3)_2$ |
| I-19 | $-NHCOOCH_2CH_2OCH_3$ |
| I-20 | $-NHCOOCH_2CN$ |
| I-21 | $-NHCOOCH_2CH_2NO_2$ |
| I-22 | $-NHCOOCH_2CH_2NHCOCH_3$ |

(continued)

| No. | Group |
|---|---|
| I-23 | $-NH(CS)OCH_3$ |
| I-24 | $-NH(CO)SCH_3$ |
| I-24 | $-NHCONHCH_2CH_2OCH_3$ |
| I-25 | $-NHCSNHCH_3$ |
| I-26 | $-NHSO_2CH=CH_2$ |
| I-27 | $-NHSO_2CH_2CH=CH_2$ |
| I-28 | $-NHSO_2CH_2C=CH$ |
| I-29 | $-NHSO_2CH_2COCH_3$ |
| 1-30 | $-NHSO_2CH_2CN$ |
| I-31 | $-NHSO_2CH_2NO_2$ |
| I-32 | $-NHSO_2CH_2COOH$ |
| I-33 | $-NHSO_2CH_2COOCH_3$ |

Examples of groups belonging to the $J_0$ group and the J group are shown in Table 19.

Table 19

| No. | Group |
|---|---|
| J-1 | $-COCH=CH_2$ |
| J-2 | $-COC\equiv CH$ |
| J-3 | $-COC\equiv CCF_3$ |
| J-4 | $-COCH_2SCH_3$ |
| J-5 | $-COCH_2OH$ |
| J-6 | $-COCH_2N(CH_3)_2$ |
| J-7 | $-CSCH_3$ |
| J-8 | $-CSCF_3$ |
| J-9 | $-CH=NCH_3$ |
| J-10 | $-CH=NOCH_3$ |
| J-11 | -COCN |
| J-12 | -COC(=NH)NH2 |
| J-13 | $-COCOOCH_3$ |
| J-14 | $-CH_2OCON(CH_3)_2$ |

Examples of groups belonging to the $K_0$ group and the K group are shown in Table 20.

Table 20

| No. | Group |
|---|---|
| K-1 | $-CONHSO_2CH_3$ |
| K-2 | -CONHOH |
| K-3 | $-CONHOCH_3$ |
| K-4 | $-CONHOCH_2CH=CH_2$ |

(continued)

| No. | Group |
|---|---|
| K-5 | $-CONHCH_2CH_2OH$ |
| K-6 | $-CONHCH_2CH_2OCH_3$ |
| K-7 | $-CONHCH_2OCH_3$ |
| K-8 | $-CONHCH_2CH=CH_2$ |
| K-9 | $-CONHCH_2C \equiv CH$ |
| K-10 | $-CONHCH_2CN$ |
| K-11 | $-CONHCH_2COOH$ |
| K-12 | $-CONHCH_2COOCH_3$ |
| K-13 | $-CONHCH_2CONH_2$ |
| K-14 | $-CONHCH_2CONHCH_3$ |
| K-15 | $-CONHCH_2CONH(CH_3)_2$ |
| K-16 | $-CONHCH(CH_2COOH)COOH$ |
| K-17 | $-CONHCH(CH_2COOCH_3)COOCH_3$ |
| K-18 | $-CONHCH_2CH_2N(CH3)_2$ |

Examples of groups belonging to the $L_0$ group and the L group are shown in Table 21.

Table 21

| No. | Group |
|---|---|
| L-1 | $-SO_2NHOH$ |
| L-2 | $-SO_2NHOCH_3$ |
| L-3 | $-SO_2NHOCH_2CH=CH_2$ |
| L-4 | $-SO_2NHCH_2CH_2OCH_3$ |
| L-5 | $-SO_2NHCH_2CH=CH_2$ |
| L-6 | $-SO_2NHCH_2C \equiv CH$ |
| L-7 | $-SO_2NHCH_2CN$ |
| L-8 | $-SO_2NHCOCH_3$ |
| L-9 | $-SO_2NHCH_2COOH$ |
| L-10 | $-SO_2NHCH_2COOCH_3$ |
| L-11 | $-SO_2NHCH_2CONH_2$ |
| L-12 | $-SO_2NHCH_2CONHCH_3$ |
| L-13 | $-SO_2NHCH_2CON(CH_3)_2$ |
| L-14 | $-SO_2NHCH(CH_2COOH)COOH$ |
| L-15 | $-NHSO_2N(CH_3)_2$ |

Examples of groups belonging to the $M_0$ group and the M group are shown in Table 22.

Table 22

| No. | Group |
|---|---|
| M-1 | $-N=C(-SCH_3)CH_3$ |

(continued)

| No. | Group |
|---|---|
| M-2 | $-N=C(-OCH_3)OCH_3$ |
| M-3 | $-N=C(-SCH_3)OCH_3$ |
| M-4 | $-N=C(-SCH_3)SCH_3$ |
| M-5 | $-N=C(-SCH_3)NHCH_3$ |
| M-6 | $-N(CH_3)C(-SCH_3)=NCH_3$ |
| M-7 | $-N(CH_3)OCH_2CH=CH_2$ |
| M-8 | $-N(CH_2CH=CH_2)OCH_2CH=CH_2$ |

Examples of groups belonging to the No group and the N group are shown in Table 23.

Table 23

| No. | Group |
|---|---|
| N-1 | $-CH_2P(=O)\ (OH)_2$ |
| N-2 | $-CH_2P(=O)\ (OCH_3)_2$ |
| N-3 | $-CH_2P(=O)\ (OCH_3)-CH_3$ |
| N-4 | $-CH_2P(=O)\ (OCH_3)-CH(OH)CH_3$ |
| N-5 | $-CH_2P(=O)\ (OCH_3)-CH_2CH_2OH$ |
| N-6 | $-CH_2P(=O)\ (OCH_3)-CH_2COOCH_3$ |

Examples of the aforementioned groups belonging to the $X_0$ group to the $Z_0$ group and the X group to the Z group are shown in the following Table 24 to Table 26. When said groups have geometrical isomers, all of the geometrical isomers are included, and when said groups have tautomers, all of the tautomers are included.

Examples of groups belonging to the $X_0$ group and the X group are shown in Table 24.

Table 24

| No. | Group | No. | Group |
|---|---|---|---|
| X-1 | $-CH_3$ | X-18 | $-OCF_2CHF_2$ |
| X-2 | $-C_2H_5$ | X-19 | $-SCF_3$ |
| X-3 | $-CF_3$ | X-20 | $-CH_2OCH_3$ |
| X-4 | $-CH=CHCH_3$ | X-21 | $-COCH_3$ |
| X-5 | $-CH_2CH=CH_2$ | X-22 | $-OCOCH_3$ |
| X-6 | $-C\equiv CH$ | X-23 | -COOH |
| X-7 | -F | X-24 | $-COOCH_3$ |
| X-8 | -Cl | X-25 | -CH=CHCOOH |
| X-9 | -Br | X-26 | $-N\ (CH_3)\ _2$ |
| X-10 | $-NO_2$ | X-27 | $-NHCOCH_3$ |
| X-11 | -CN | X-28 | $-NHCOOCH_3$ |
| X-12 | $-OCH_3$ | X-29 | $-CONH_2$ |
| X-13 | $-SCH_3$ | X-30 | $-CON(CH_3)_2$ |
| X-14 | $-SOC_4H_9$ | X-31 | $-NHCON(CH_3)_2$ |
| X-15 | $-SO_2C_4H_9$ | X-32 | $-NHC(=NH)NH_2$ |

(continued)

| No. | Group | No. | Group |
|---|---|---|---|
| X-16 | $-OCHF_2$ | X-33 | $-NHSO_2CF_3$ |
| X-17 | $-OCF_3$ | X-34 | $-SO_2N(CH_3)_2$ |

Examples of groups belonging to the $Y_0$ group and the Y group are shown in Table 25.

Table 25

| No. | Group | No. | Group |
|---|---|---|---|
| Y-1 | 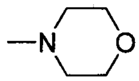 | Y-6 | 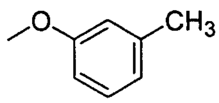 |
| Y-2 | 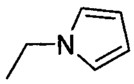 | Y-7 | 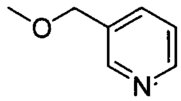 |
| Y-3 | 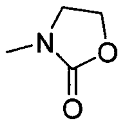 | Y-8 | 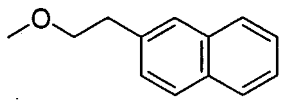 |
| Y-4 | 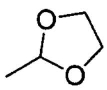 | Y-9 | 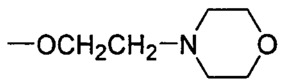 |
| Y-5 | 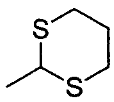 | Y-10 | 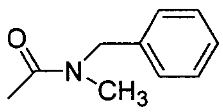 |

Examples of the α ring, the A0 ring or the A ring fused to the $Z_0$ group or the Z group are shown in Table 26.

Table 26

| No. | a ring, A0 ring or A ring fused to $Z_0$ group or Z group | No. | α ring, A0 ring or A ring fused to $Z_0$ group or Z group |
|---|---|---|---|
| Z-1 | 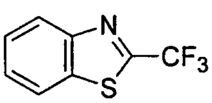 | Z-6 | 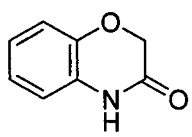 |
| Z-2 | 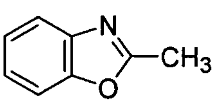 | Z-7 | 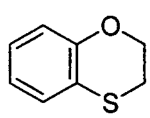 |
| Z-3 | 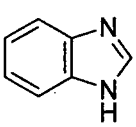 | Z-8 | 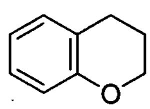 |

(continued)

| No. | a ring, A0 ring or A ring fused to $Z_0$ group or Z group | No. | $\alpha$ ring, A0 ring or A ring fused to $Z_0$ group or Z group |
|---|---|---|---|
| Z-4 | 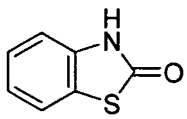 | Z-9 | 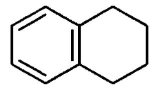 |
| Z-5 | 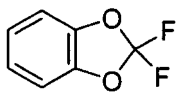 | Z-10 | 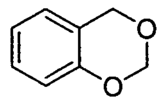 |

Examples of $Q_{A0}$ and $Q_A$ are shown in Table 27 to Table 28.

Table 27

| No. | Group |
|---|---|
| Q-1 | -OH |
| Q-2 | 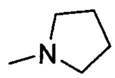 |
| Q-3 | 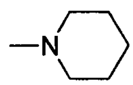 |
| Q-4 | 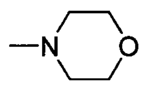 |
| Q-5 | $-OCOCH_3$ |
| Q-6 | $-OSO_2N(CH_3)_2$ |
| Q-7 | $-NHCH_2CH=CH_2$ |
| Q-8 | $-NHCH_2C\equiv CH$ |
| Q-9 | $-NHCH_2CH_2OCH_3$ |
| Q-10 | $-OCH_3$ |
| Q-11 | $-OCH_2CH_2(C)C_6H_{11}$ |
| Q-12 | $-OCH_2CH=CH_2$ |
| Q-13 | $-OCH_2C\equiv CH$ |
| Q-14 | $-OCH_2COOH$ |
| Q-15 | $-OCH_2COOCH_3$ |
| Q-16 | $-OCH_2CONH_2$ |
| Q-17 | $-OCH_2CN$ |
| Q-18 | $-OCH_2CH_2OH$ |
| Q-19 | $-OCH_2CH_2OCH_3$ |
| Q-20 | $-OCH_2CH_2N\ (CH_3)_2$ |

Table 28

| No. | Group |
|---|---|
| Q-21 | $-OCH_2COCH_3$ |
| Q-22 | $-OCOC_6H_5$ |
| Q-23 | $-OCH_2C_6H_5$ |
| Q-24 | 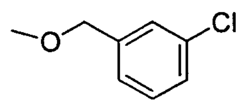 |
| Q-25 | 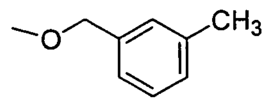 |
| Q-26 | 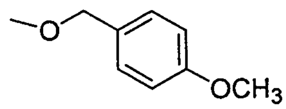 |

Examples of $T_{A0}$ and $T_A$ are shown in Table 29.

Table 29

| No. | Group |
|---|---|
| T-1 | -H |
| T-2 | $-CH_3$ |
| T-3 | $-CH_2CH_2(c)C_6H_{11}$ |
| T-4 | $-CH_2CH=CH_2$ |
| T-5 | $-CH_2C=CH$ |
| T-6 | $-CH_2C_6H_5$ |
| T-7 | $-CH_2COOH$ |
| T-8 | $-CH_2COOCH_3$ |
| T-9 | $-CH_2CONH_2$ |
| T-10 | $-CH_2CN$ |
| T-11 | $-CH_2CH_2OH$ |
| T-12 | $-CH_2CH_2OCH_3$ |
| T-13 | $-CH_2CH_2N(CH_3)_2$ |
| T-14 | $-CH_2COCH_3$ |
| T-15 | $-CH_2CF_3$ |
| T-16 | -Ph |
| T-17 | 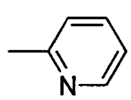 |

An example of the compound (II) includes a cinnamoyl compound represented by the formula (II"):

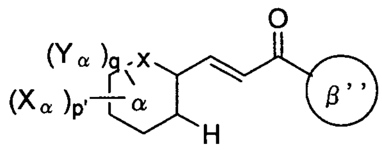

(II'')

wherein $\alpha$, $X_\alpha$, $Y_\alpha$ and q are as defined above, x represent a methine group or a nitrogen atom, p' represents 1, 2 or 3, and when p' is not less than 2, $X_\alpha$s are same or different, and the sum of p' and q is not more than 3, and $\beta$" represents a group represented by formula (II"-1):

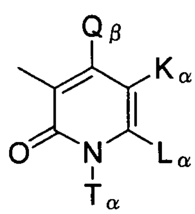

(II''-1)

(wherein $Q_\beta$, $T_\alpha$, $K_\alpha$ and $L_\alpha$ are as defined above), a group represented by the formula (II"-7) :

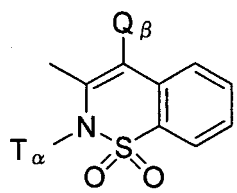

(II'' -7)

(wherein $Q_\beta$ and $T_\alpha$ are as define above), or a group represented by the formula (II"-8):

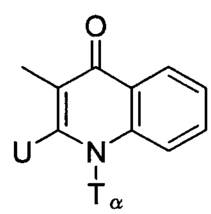

(II'' -8)

(wherein U and $T_\alpha$ are as defined above). In the cinnamoyl compound (II"), when x is a methine group, the methine group has no substituent. In a specific example of the cinnamoyl compound (II"), $\beta$" is a group represented by the formula (II"-1), (II"-7) or (II"-8), wherein the group (II"-1) is a group represented by the formula (II"'-1):

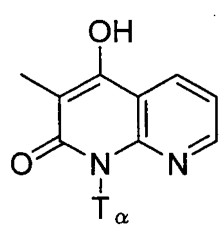

(II'''-1)

(wherein $T_\alpha$ is as defined above) and $T_\alpha$ is a hydrogen atom or a C1-C10 alkyl group, or $Q_\beta$ of the group (II"-7) is a hydroxyl group and $T_\alpha$ of the group (II"-7) is a C1-C10 alkyl group, or $T_\alpha$ of the group (II"-8) is a hydrogen atom or a C1-C10 alkyl group and U of the group (II"-8) is a hydrogen atom. In a more specific example of the cinnamoyl group

(II"), β" is a group represented by the formula (II"'-1) and $T_\alpha$ is a hydrogen atom or a C1-C10 alkyl group.

An example of the compound (III) includes a cinnamoyl compound represented by the formula (III'):

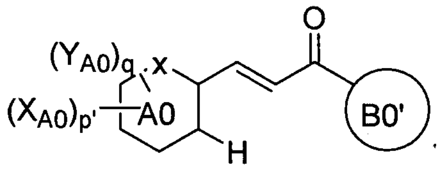

(III')

wherein A0, $X_{A0}$, $Y_{A0}$, p', q and x are as defined above, and when p' is not less than 2, $X_{A0}$s are the same or different, and the sum of p' and q is not more than 3, and B0' represents a group represented by the formula (III'-1):

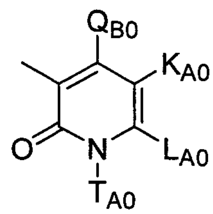

(III'-1)

(wherein $Q_{B0}$, $T_{A0}$, $K_{A0}$ and $L_{A0}$ are as defined above) or a group represented by the formula (III'-7):

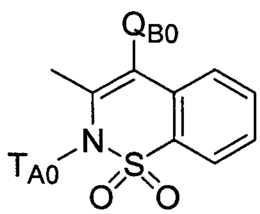

(III'-7)

(wherein $Q_{B0}$ and $T_{A0}$ are as defined above) or a group represented by the formula (III'-8):

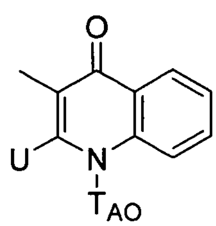

(III'-8)

(wherein U and $T_{A0}$ are as defined above). In the cinnamoyl compound (III'), when x is a methine group, the methine group has no substituent. In a specific example of the cinnamoyl compound (III'), B0' is a group represented by the formula (III'-1), (III'-7) or (III'-8), wherein the group (III'-1) is a group represented by the formula (III"-1) :

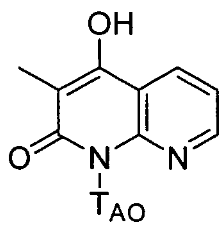

(III''-1)

(wherein $T_{A0}$ is as defined above) and $T_{A0}$ is a hydrogen atom or C1-C10 alkyl group, or $Q_{B0}$ of the group (III'-7) is a hydroxyl group and $T_{A0}$ of the group (III'-7) is a C1-C10 alkyl group, or $T_{A0}$ of the group (III'-8) is a hydrogen atom or a C1-C10 alkyl group and U of the group (III'-8) is a hydrogen atom. In a more specific example of the cinnamoyl compound (III'), B0' is the group (III"-1) and $T_{A0}$ is a hydrogen atom or a C1-C10 alkyl group.

An example of the compound (IV) includes a cinnamoyl compound represented by the formula (IV'):

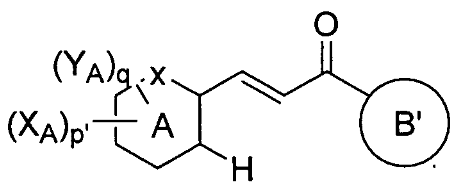

(IV')

wherein A, $X_A$, $Y_A$, p', q and x are as defined above, and when p' is not less than 2, $X_A$s are the same or different, and the sum of p' and q is not more than 3, and B' represents a group represented by the formula (IV'-1):

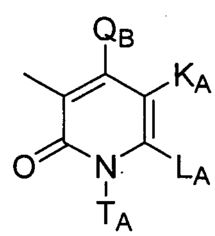

(IV'-1)

(wherein $Q_B$, $T_A$, $K_A$ and $L_A$ are as defined above) or a group represented by the formula (IV'-7):

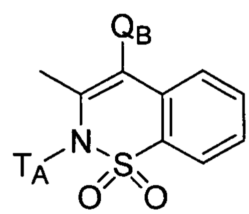

(IV'-7)

(wherein $Q_B$ and $T_A$ are as define above) or a group represented by the formula (IV'-8):

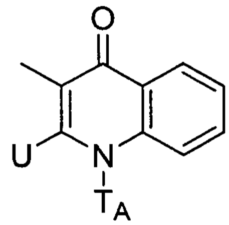

(IV' -8)

(wherein U and $T_A$ are as defined above). In the cinnamoyl compound (IV'), when x is a methine group, the methine group has no substituent. In a specific example of the cinnamoly compound (IV'), B' is a group represented by the formula (IV'-1), (IV'-7) or (IV'-8), wherein the group (IV'-1) is a group represented by the formula (IV"-1):

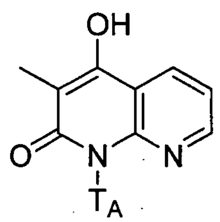

(IV' ' -1)

(wherein $T_A$ is as defined above) and $T_A$ is a hydrogen atom or a C1-C10 alkyl group, or $Q_B$ of the group (IV'-7) is a hydroxyl group and $T_A$ of the group (IV'-7) is a C1-C10 alkyl group, or $T_A$ of the group (IV'-8) is a hydrogen atom or a C1-C10 alkyl group and U of the group (IV'-8) is a hydrogen atom. In a more specific example of the cinnamoyl compound (IV'), B' is the group (IV"-1) and $T_A$ is a hydrogen atom or a C1-C10 alkyl group.

An example of the compounds (V) includes a cinnamoyl compound represented by the formula (V"):

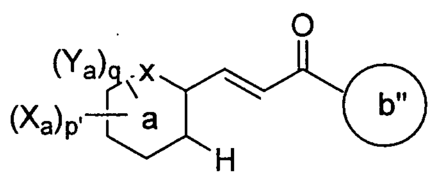

(V' ' )

wherein a, $X_a$, $Y_a$, p', q and x are as defined above, and when p' is not less than 2, $X_a$s are the same or different, and the sum of p' and q is not more than 3, and b" represents a group represented by the formula (V"-1):

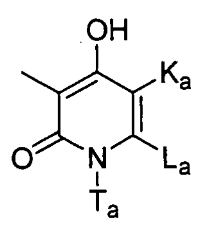

(V' ' -1)

(wherein $K_a$, $L_a$ and $T_a$ are as defined above) or a group represented by the formula (V"-7):

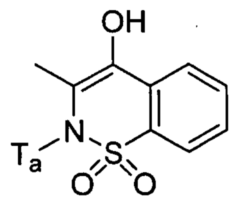

(V''-7)

(wherein $T_a$ is as defined above) or a group represented by the formula (V"-8):

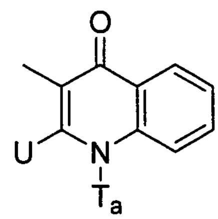

(V''-8)

(wherein U and $T_a$ are defined above). In the cinnamoyl compound (V"), when x is a methine group, the methine group has no substituent. In a specific example of the cinnamoyl compound (V"), b" is a group represented by the formula (V"-1), (V"-7) or (V"-8), wherein the group (V"-1) is a group represented by the formula (V"'-1):

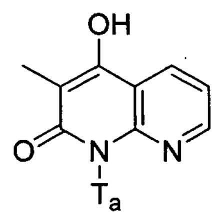

(V'''-1)

(wherein $T_a$ is as defined above) and Ta is a hydrogen atom or a C1-C10 alkyl group, or $T_a$ of the group (V"-7) is a C1-C10 alkyl group, or $T_a$ of the group (V"-8) is a hydrogen atom or C1-C10 alkyl group and U of the group (V"-8) is hydrogen atom. In a more specific example of the cinnamoyl compound (V"), b" is the group (V"'-1) and $T_A$ is a hydrogen atom or a C1-C10 alkyl group.

A further specific example of the compound (V) includes a cinnamoyl compound represented by the formula (V"'):

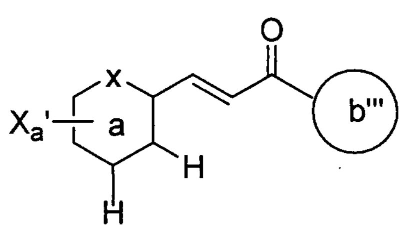

(V''' )

wherein a and x are as defined above, $X_a$' represents a $CH_3OCH_2CONH$- group, a $CH_3OCOCH_2NHCO$- group, a $HOCOCH_2NHCO$-group, a $CH_3OCH_2CH_2NHCO$- group, a $CH_3OCOCH_2O$- group, a $HOCOCH_2O$- group, a $HOCH_2CH_2O$- group, a $CH_3OCH_2CH_2OCONH$- group or a $(CH_3)_2NCH_2CH_2O$- group, and b"' represents a group represented by the formula (V""-1):

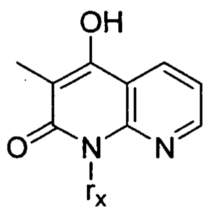

(V''''-1)

(wherein $r_x$ represents a hydrogen atom or a methyl group) or a group represented by the formula (V'''-7):

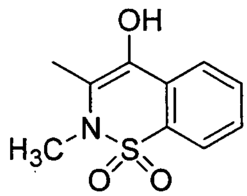

(V'''-7)

or a group represented by the formula (V'''-8):

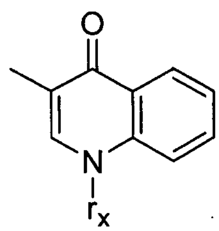

(V'''-8)

(wherein $r_x$ is as defined above). In the cinnamoyl compound (V'''), when x is a methine group, the methine group has no substituent. In a still more specific example of the cinnamoyl compound (V'''), b''' is a group represented by the formula (V''''-1).

Although WO 97/35565, JP09227547, WO 00/20371, JP2002371078, WO 01/79187 and WO 92/18483 disclose compounds having a certain conceptional skeleton, they do not describe the effect of suppressing transcription of an extracellular matrix gene in a tissue, consequently, the effect of suppressing the accumulation of an extracellular matrix.

The compound (I) or (II) can be produced, for example, by reacting a compound represented by the formula (α) (wherein α, $X_α$, $Y_α$, p and q are as defined above) and a compound represented by the formula (β) (wherein is as defined above).

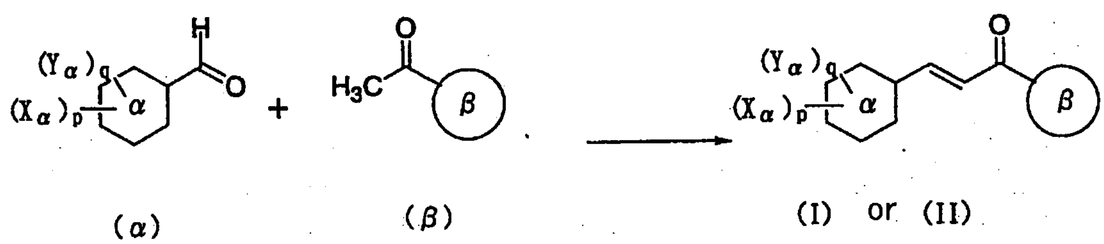

(α) (β) (I) or (II)

In the compound (β), when β is a group represented by the above formula (I-2), (I-3), (I-6) or (I-8), the compound (I) or (II) in which β is the group (1-2), (1-3), (1-6) or (1-8) can be produced, for example, according to the references (Liebigs Annalen der Chemie, (1979), D(6), 769, J. Heterocyclic Chem.(1981), 18(3), 603, Polish J. Chem. (1982), 56(2), 419, Synthetic Communication (1986), 16(10), 1195, Ukrainskii Khimicheskii Zhurnal (1982), 48(7), 772).

In the compound (β), when β is a group represented by the above formula (I-1), (I-4), (I-5) or (I-7), the compound (I) or (II) can be produced by reacting the compound (α) and the compound (β) in which β is the above group (1-1), (I-4), (I-5) or (I-7). In the reaction, the reaction temperature is usually from room temperature to the reflux temperature of a solvent, and the reaction time is usually from instant to about 24 hours. The reaction is usually performed in the presence of a base. Examples of the base used include organic basis such as piperidine, pyridine, triethylamine, N,N-dimethylaniline, tributylamine, morpholine, N-methylmorpholine and the like, and inorganic bases such as sodium hydroxide, potassium hydroxide, potassium carbonate and the like. In the reaction, usually, 0.5 to 2 mol of the compound (β) and 0.1 to 1.0 mol of the base are used per 1 mol of the compound (α). In the reaction, a solvent is not necessarily required, but the reaction is usually performed in the presence of a solvent. Examples of a solvent useable in the reaction include lower alcohols such as methanol, ethanol and the like, aliphatic hydrocarbons such as hexane, petroleum ether and the like, aromatic hydrocarbons such as benzene, toluene and the like, halogenated hydrocarbons such as chloroform, dichloroethane and the like, ethers such as dietyl ether, dioxane, tetrahydrofuran and the like, ketones such as acetone, methyl ethyl ketone and the like, esters such as ethyl acetate, diethyl carbonate and the like, nitriles such as acetonitrile, isobutyronitrile and the like, amides such as formamide, N,N-dimethylformamide and the like, sulfur compounds such as dimethyl sulfoxide and the like, and their mixtures. After completion of the reaction, a reaction solution is extracted with an organic solvent, washed with water, and subjected to a conventional post-treatment such as concentration of an organic layer under reduced pressure or the like, and then, if necessary, purified by chromatography, recrystallization or the like to obtain the compound (β).

The compound (III) can be produced, for example, by reacting a compound represented by the formula (A0) (wherein A, $X_{A0}$, $Y_{A0}$, p and q are as defined above) and a compound represented by the formula (B0) (wherein B0 is as defined above).

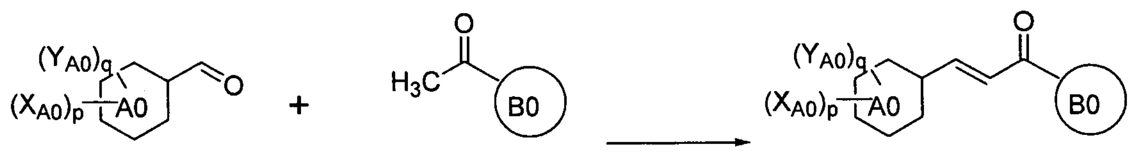

(A0) (B0)

The compound (IV) can be produced, for example, by reacting a compound represented by the formula (A) (wherein A, $X_A$, $Y_A$, p and q are as defined above) and a compound represented by the formula (B) (wherein B is as defined above).

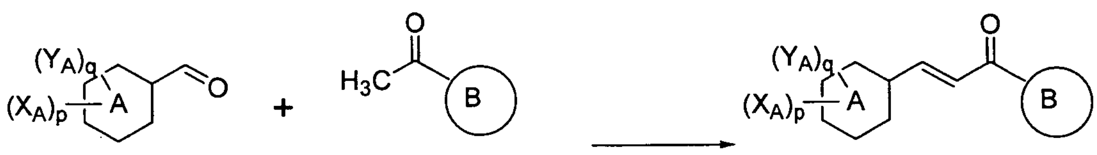

(A) (B) (IV)

The compound (V) can be produced, for example, by reacting a compound represented by the formula (a) (wherein a, $X_a$, $Y_a$, p and q are as defined above) and a compound represented by the formula (b) (wherein b is as defined above.

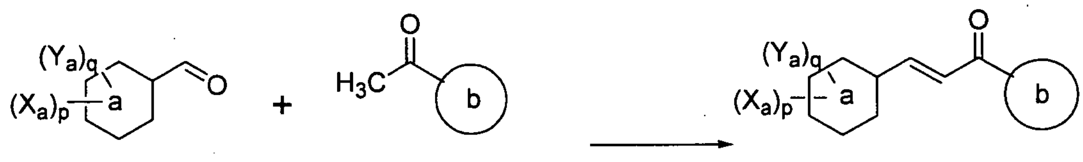

(a) (b) . (V)

A part of compounds represented by the formula (a) are described, for example, in EP330645, and are known, and can be produced by the method described in WO 2005/028439.

A part of compounds represented by the formula (b) are described, for example, in J. Organic Chem. (1965) 30, 2241, and are known. However the compound represented by the formula (VII-3) which is a part of compounds represented by the formula (VI-1) has never been reported, and is a novel substance.

The compound (VII-3) can be produced, for example, by subjecting the compound represented by the formula (VII-2) to a cyclization reaction. In the cyclization reaction, the reaction temperature is usually from room temperature to the reflux temperature of a solvent, and the reaction time is usually from instant to about 24 hours. The cyclization reaction is usually performed in the presence of a base, and examples of the base used include organic bases such as sodium methoxide, sodium ethoxide, n-butyllithium, pyridine, triethylamine, N,N-dimethylaniline, tributylamine, N-methylmorpholine and the like, and inorganic bases such as sodium hydride, sodium hydroxide, potassium hydroxide, potassium carbonate and the like. In the reaction, usually, 0.8 to 2 mol of the base is used per 1 mol of the compound (VII-2). In the reaction, a solvent is not necessarily required, but the reaction is usually performed in the presence of a solvent. Examples of a solvent useable in the reaction include lower alcohols such as methanol, ethanol and the like, aliphatic hydrocarbons such as hexane, petroleum ether and the like, aromatic hydrocarbons such as benzene, toluene and the like, halogenated hydrocarbons such as chloroform, dichloroethane and the like, ethers such as dietyl ether, dioxane, tetrahydrofuran and the like, ketones such as acetone, methyl ethyl ketone and the like, esters such as ethyl acetate, diethyl carbonate and the like, nitriles such as acetonitrile, isobutyronitrile and the like, amides such as formamide, N,N-dimethylformamide and the like, sulfur compounds such as dimethyl sulfoxide and the like, and their mixtures. After completion of the reaction, a reaction solution is extracted with an organic solvent, washed with water, and subjected to a conventional post-treatment such as concentration of an organic layer under reduced pressure or the like, and then, if necessary, purified by chromatography, recrystallization or the like to obtain the compound (VII-3).

The compound (VII-2) has never been reported, and is a novel substance. The compound (VII-2) can be produced by 3-oxobutyrylamidating the compound represented by the formula (VII-1). In the reaction, the reaction temperature is usually from room temperature to the reflux temperature of a solvent, and the reaction time is usually from instant to about 24 hours. The reaction is performed in the presence of a 3-oxobutyrylamidating agent, and examples of the 3-oxobutyrylamidating agent include diketene, 2,2,6-trimethyl-4H-1,3-dioxin-4-one, methyl acetoacetate, t-butyl acetoacetate and the like. In the reaction, usually, 0.8 to 7 mol of the 3-oxobutyrylamidating agent is used per 1 mol of the compound (VII-1). In the reaction, a solvent is not necessarily required, but the reaction is usually performed in the presence of a solvent. Examples of a solvent usable in the reaction include lower alcohols such as methanol, ethanol and the like, aliphatic hydrocarbons such as hexane, petroleum ether and the like, aromatic hydrocarbons such as benzene, toluene, xylene and the like, halogenated hydrocarbons such as chloroform, dichloroethane and the like, ethers such as diethyl ether, dioxane, tetrahydrofuran and the like, ketones such as acetone, methyl ethyl ketone and the like, esters such as ethyl acetate, diethyl carbonate and the like, nitriles such as acetonitrile, isobutyronitrile and the like, amides such as formamide, N,N-dimethylformamide and the like, sulfur compounds such as dimethyl sulfoxide and the like, and their mixtures. After completion of the reaction, a reaction solution is subjected to a post-treatment and purification in the same manner as in the case of the cyclization reaction of the compound (VII-2) to obtain the compound (VII-2).

The compound (VII-1) is described, for example, in Tetrahederon (1997), 53(47), 16061, and is known.

The compound represented by the formula (XI-2) which is a part of the compound (VII-3) can be produced by alkylating the compound represented by the formula (XI-1) which is a part of the compound (VII-3).

The reaction is performed by, for example, reacting the compound (XI-1) and an alkylating agent in the presence of a base.

The reaction of the compound (XI-1) and an alkylating agent in the presence of a base does not necessarily need a solvent, but is usually performed in a solvent. Examples of a solvent used in the reaction include acid amides such as N,N-dimethylformamide, N,N-dimethylacetamide and the like, sulfoxides such as dimethyl sulfoxide and the like, phosphoric acid amide compound such as hexamethylphosphoramide and the like, and ketones such as acetone, methyl ethyl ketone and the like.

Examples of a base used in the reaction include alkali metal hydrides such as sodium hydride, potassium hydride and the like, alkali metal carbonates such as potassium carbonate and the like, and silver oxide and the like.

Examples of an alkylating agent include alkylsulfonic acid esters such as methyl methanesulfonate and the like, arylsulfonic acid esters such as methyl p-toluenesulfonate, ethyl p-toluenesulfonate and the like, sulfuric acid esters such as dimethyl sulfate and the like, and halides such as methyl iodide, ethyl bromide and the like.

In the reaction, usually, 1 to 2 mol of the base and 1 to 2 mol of the alkylating agent are used per 1 mol of the compound (XI-1).

The reaction temperature is usually in a range of 0˚C to 100˚C, and the reaction time is usually in a range of instant to 24 hours.

After completion of the reaction, a reaction solution is subjected to a post-treatment and purification in the same manner as in the case of the cyclization reaction of the compound (VII-2) to obtain the compound (XI-2).

Table 30 shows novel compounds (VII-3) represented by the compound numbers (1A-1) to (1A-8).

Table 30

| Compound No. | 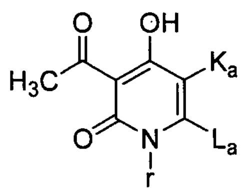 Compound (VII-3) | Compound No. | 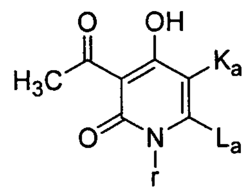 Compoundn (VII-3) |
|---|---|---|---|
| (1A-1) | 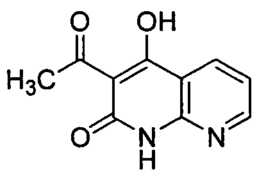 | (1A-5) | 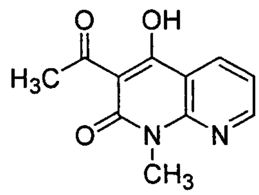 |
| (1A-2) | 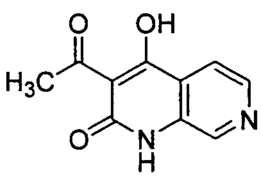 | (1A-6) | 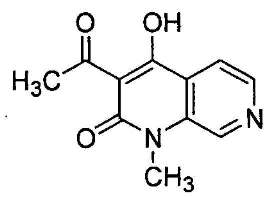 |
| (1A-3) | 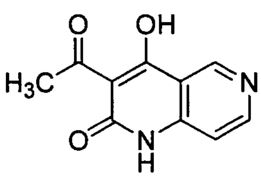 | (1A-7) | 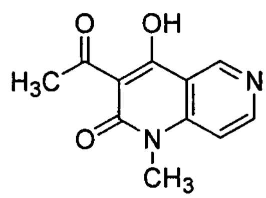 |
| (1A-4) | 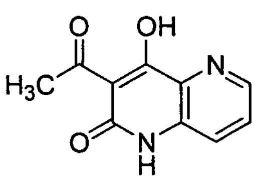 | (1A-8) | 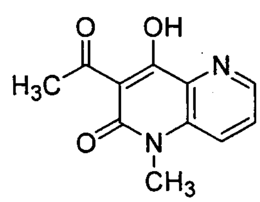 |

Table 31 shows novel compounds (VII-2) represented by the compound numbers (1B-1) to (1B-8).

Table 31

| Compound No. | compound (VII-2) | Compound No. | compound (VII-2) |
|---|---|---|---|
| | 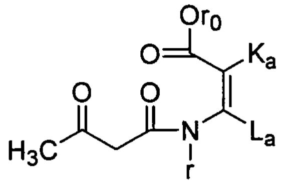 | | 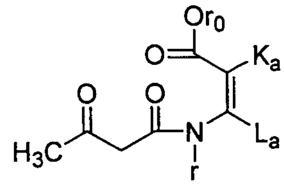 |
| (1B-1) | 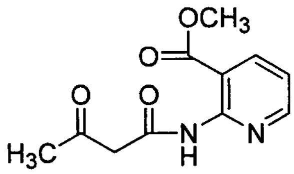 | (1B-5) | 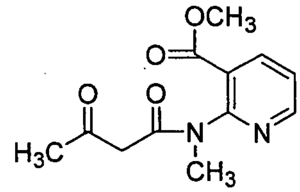 |
| (1B-2) | 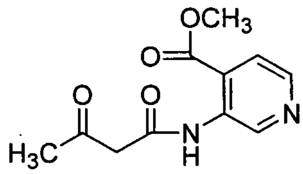 | (1B-6) | 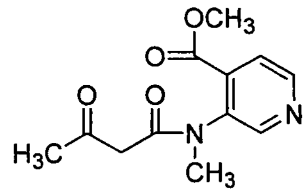 |
| (1B-3) | 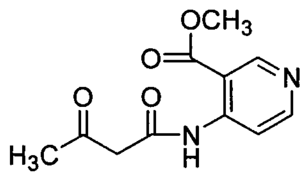 | (1B-7) | 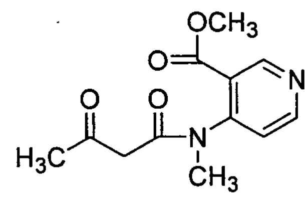 |
| (1B-4) | 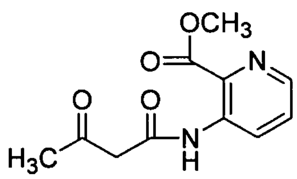 | (1B-8) | 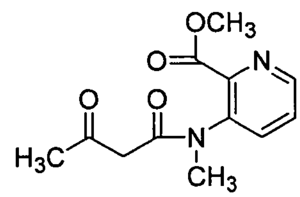 |

Among the compounds (V), examples of a compound represented by the formula (Va):

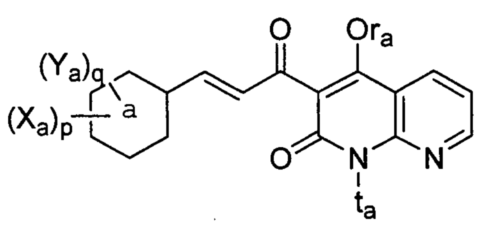

(Va)

(wherein a, $X_a$, $Y_a$, p, q, $r_a$ and $t_a$ are as defined above), which are represented by the compound numbers (1a) to (129a), are shown in Table 32 to Table 37. (Table 32 to Table 37)

In Table 32 to Table 37, in the compound numbers (1a) to (98a), (100a) to (104a) and (106a) to (123a), a represents a benzene ring.

Table 32

| Compound No. | $X_a$ and $Y_a$ | $r_a$ | $t_a$ |
|---|---|---|---|
| (1a) | 3-CH=CHCN | -H | -H |
| (2a) | $3\text{-}OCH_2CH_2SCH_3$ | -H | -H |
| (3a) | $3\text{-}OCH_2CH{=}CH_2$ | -H | -H |
| (4a) | $2\text{-}OCH_2C{\equiv}CH$ | -H | -H |
| (5a) | $3\text{-}OCH_2C{\equiv}CH$ | -H | -H |
| (6a) | $4\text{-}OCH_2C{\equiv}CH$ | -H | -H |
| (7a) | $3\text{-}OCH_2COOCH_3$ | -H | -H |
| (8a) | $3\text{-}OCH_3,4\text{-}OCH_2COOCH_3$ | -H | -H |
| (9a) | $3\text{-}OCH_2COOH$ | -H | -H |
| (10a) | $3\text{-}OCH_2CN$ | -H | -H |
| (11a) | $3\text{-}OCH_2CN$ | -H | $-CH_3$ |
| (12a) | $3\text{-}OCH_2CN$ | $-CH_3$ | $-CH_3$ |
| (13a) | $4\text{-}OCH_2CN$ | -H | -H |
| (14a) | $3\text{-}CH_3,4\text{-}OCH_2CN$ | -H | -H |
| (15a) | $3\text{-}NO_2,4\text{-}OCH_2CN$ | -H | -H |
| (16a) | $3\text{-}F,4\text{-}OCH_2CN,5\text{-}OCH_3$ | -H | -H |
| (17a) | $3\text{-}NHCOCH{=}CH_2$ | -H | -H |
| (18a) | $3\text{-}NHCOCH_2OCH_3$ | -H | -H |
| (19a) | $3\text{-}NHCOCH_2OCH_3$ | -H | $-CH_3$ |
| (20a) | $4\text{-}NHCOCH_2OCH_3$ | -H | $-CH_3$ |
| (21a) | $3\text{-}NHCOOCH_2CH_2OCH_3$ | -H | -H |
| (22a) | $3\text{-}NHCONHCH_2CH_2OCH_3$ | -H | -H |
| (23a) | $3\text{-}NHSO_2CH_2COOCH_3$ | -H | -H |
| (24a) | $3\text{-}NHSO_2CH_2COOH$ | -H | -H |
| (25a) | $3\text{-}NHCOCH_2CN$ | -H | -H |
| (26a) | $3\text{-}CONHSO_2CH_3$ | -H | -H |
| (27a) | $3\text{-}CONHCH_2CH_2OH$ | -H | -H |
| (28a) | $3\text{-}CONHCH_2COOCH_3$ | -H | -H |
| (29a) | $3\text{-}CONHCH_2COOCH_3$ | -H | $-CH_3$ |
| (30a) | $4\text{-}CONHCH_2COOCH_3$ | -H | -H |

Table 33

| Compound No. | $X_a$ and $Y_a$ | $r_a$ | $t_a$ |
|---|---|---|---|
| (31a) | $3\text{-}CONHCH_2CH_2OCH_3$ | -H | -H |
| (32a) | $3\text{-}CONHCH_2CH_2OCH_3$ | -H | $-CH_3$ |
| (33a) | $4\text{-}CONHCH_2CH_2OCH_3$ | -H | -H |
| (34a) | $3\text{-}CONHCH_2COOH$ | -H | -H |
| (35a) | $3\text{-}CONHCH_2CN$ | -H | -H |

(continued)

| Compound No. | $X_a$ and $Y_a$ | $r_a$ | $t_a$ |
|---|---|---|---|
| (36a) | 3-$OCH_2COOCH_3$ | -H | $-CH_3$ |
| (37a) | 3-$OCH_2COOH$ | -H | $-CH_3$ |
| (38a) | 3-$OCH_2CON(CH_3)_2$ | -H | $-CH_3$ |
| (39a) | 3-$OCH_2CH_2CH_2N(CH_3)_2$ | -H | -H |
| (40a) | 3-$OCH_2CH_2OH$ | -H | $-CH_3$ |
| (41a) | 3-$OCH_2CH_2OH$ | $-CH_3$ | $-CH_3$ |
| (42a) | 3-$NHCOOCH_2CH_2OCH_3$ | -H | $-CH_3$ |
| (43a) | 3-$CH{=}CF_2$ | -H | -H |
| (44a) | 3-$CH_2CH_2CN$ | -H | -H |
| (45a) | 3-$OCH_2CH_2SCH_3$ | -H | $-CH_3$ |
| (46a) | 3-$OCH_2CH_2SOCH_3$ | -H | $-CH_3$ |
| (47a) | 3-$OCH_2CH_2SO_2CH_3$ | -H | $-CH_3$ |
| (48a) | 3-$OCH_2CH_2OH$ | -H | -H |
| (49a) | 3-$CH_2OCH_2CH_2OH$ | -H | $-CH_3$ |
| (50a) | 3-$OCH_2CH_2CH_2OH$ | -H | $-CH_3$ |
| (51a) | 3-$OCH_2CH_2OCH_3$ | -H | $-CH_3$ |
| (52a) | 3-$OCH_2CH_2NH_2$ | -H | $-CH_3$ |
| (53a) | 3-$OCH_2CH_2NHCOCH_3$ | -H | $-CH_3$ |
| (54a) | 3-$OCH_2CH_2NHCOOC(CH_3)_3$ | -H | $-CH_3$ |
| (55a) | 3-$OCH_2CH_2N\ (CH_3)_2$ | -H | -H |
| (56a) | 3-$OCH_2CH_2N(CH_3)_2$ | -H | $-CH_3$ |
| (57a) | 3-$OCH_2CH_2CH_2N(CH_3)_2$ | -H | $-CH_3$ |
| (58a) | 3-$OCH_2CH_2SO_3H$ | -H | $-CH_3$ |
| (59a) | 3-$OCH_2CH_2CH_2SO_3Na$ | -Na | -H |
| (60a) | 3-$OCH_2COOCH_3$ | $-CH_3$ | $-CH_3$ |

Table 34

| Compound No. | $X_a$ and $Y_a$ | $r_a$ | $t_a$ |
|---|---|---|---|
| (61a) | 3-$OCH_2COO(CH_2)_9$-OH | -H | $-CH_3$ |
| (62a) | 4-$OCH_2COOCH_3$ | -H | -H |
| (63a) | 3-$OCH_2COOH$•pyridine | -H | -H |
| (64a) | 3-$OCH_2COOH$ | $-CH_3$ | $-CH_3$ |
| (65a) | 4-$OCH_2COOH$ | -H | -H |
| (66a) | 3-$OCH_2CONH_2$ | -H | -H |
| (67a) | 3-$OCH_2CONH_2$ | -H | $-CH_3$ |
| (68a) | 3-$OCH_2CONH_2$ | $-CH_3$ | $-CH_3$ |
| (69a) | 3-$OCH_2CON(CH_3)_2$ | -H | -H |
| (70a) | 3-$OCH_2CON(CH_3)_2$ | $-CH_3$ | $-CH_3$ |

(continued)

| Compound No. | $X_a$ and $Y_a$ | $r_a$ | $t_a$ |
|---|---|---|---|
| (71a) | 3-BR,4-$OCH_2COOCH_3$ | -H | -H |
| (72a) | 3-$CH_3$,4-$OCH_2COOCH_3$ | -H | -H |
| (73a) | 3-$CH_3$,4-$OCH_2COOCH_3$ | -H | -$CH_3$ |
| (74a) | 3-$NHCOCH_3$,4-$OCH_2CN$ | -H | -H |
| (75a) | 3-$OCH_2COCH_3$ | -H | -$CH_3$ |
| (76a) | 3-$CH_2SCH_2COOCH_3$ | -H | -$CH_3$ |
| (77a) | 3-$CH_2SOCH_2COOCH_3$ | -H | -$CH_3$ |
| (78a) | 3-$CH_2SO_2CH_2COOCH_3$ | -H | -$CH_3$ |
| (79a) | 3-$NHCOCH_2OCH_3$ | -$CH_3$ | -$CH_3$ |
| (80a) | 3-$NHCOOCH_2CH_2OCH_3$ | -$CH_3$ | -$CH_3$ |
| (81a) | 3-$NHSO_2CH_2CH$=$CH_2$ | -H | -$CH_3$ |
| (82a) | 3-$NHCH_2CH_2N(CH3)_2$ | -H | -H |
| (83a) | 3-$CONHCH_2COOCH_3$ | -$CH_3$ | -$CH_3$ |
| (84a) | 4-$CONHCH_2COOCH_3$ | -H | -$CH_3$ |
| (85a) | 4-$CONHCH_2COOCH_3$ | -CH | $_3$-$CH_3$ |
| (86a) | 3-$CONHCH_2COOH$ | -H | -$CH_3$ |
| (87a) | 3-$CONHCH_2COOH$ | -$CH_3$ | -$CH_3$ |
| (88a) | 4 -$CONHCH_2COOH$ | -H | -H |
| (89a) | 4-$CONHCH_2COOH$ | -H | -$CH_3$ |
| (90a) | 4-$CONHCH_2COOH$ | -$CH_3$ | -$CH_3$ |

Table 35

| Compound No. | $X_a$ and $Y_a$ | ra | $t_a$ |
|---|---|---|---|
| (91a) | 3-$CONHCH_2CONH_2$ | -H | -H |
| (92a) | 3-$CONHCH_2CONH_2$ | -H | -$CH_3$ |
| (93a) | 3-$CONHCH_2CONH_2$ | -$CH_3$ | -$CH_3$ |
| (94a) | 3-$CONHCH(CO_2CH_3)$ -$CH_2CO_2CH_3$ | -H | -H |
| (95a) | 3-$CONHCH(CO_2H)$ -$CH_2CO_2H$ | -H | -H |
| (96a) | 3-$CONHCH_2CH_2OH$ | -H | -$CH_3$ |
| (97a) | 3-$CONHCH_2CH_2OH$ | -$CH_3$ | -$CH_3$ |
| (98a) | 3-$CONHCH_2CH_2OCH_3$ | -$CH_3$ | -$CH_3$ |
| Compound No. | Compound (Va) | | |
| (99a) | 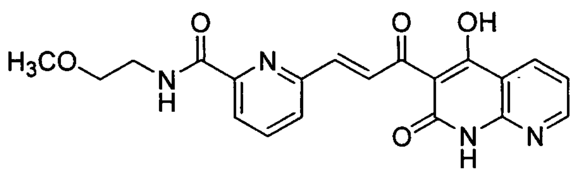 | | |
| Compound No. | $X_a$ and $Y_a$ | $r_a$ | $t_a$ |
| (100a) | 3-$CONHSO_2CH_3$ | -H | -$CH_3$ |
| (101a) | 3-$SO_2NHCH_2CH_2OCH_3$ | -H | -H |
| (102a) | 3-$SO_2NHCH_2CH_2OCH_3$ | -H | -$CH_3$ |
| (103a) | 3-$CONHOCH_3$ | -H | -$CH_3$ |
| (104a) | 3-$CONHOCH_2CH=CH_2$ | -H | -$CH_3$ |
| Compound No. | compound (Va) | | |
| (105a) | 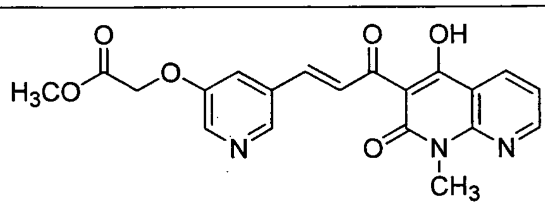 | | |
| Compound No. | $X_a$ and $Y_a$ | $r_a$ | $t_a$ |
| (106a) | 3-$CH_2CH_2CN$ | -H | -$CH_3$ |
| (107a) | 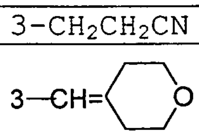 | -H | -$CH_3$ |

Table 36

| Compound No. | $X_a$ and $Y_a$ | $r_a$ | $t_a$ |
|---|---|---|---|
| (108a) | 3-CH=CHCN | -H | $-CH_3$ |
| (109a) | $3-C\equiv CC(CH_3)_2OH$ | -H | $-CH_3$ |
| (110a) | $3-CH=CHCOOCH_3$ | -H | $-CH_3$ |
| (111a) | $3-OCH_2CH=CH_2$ | -H | $-CH_3$ |
| (112a) | $3-NHCOCOOCH_3$ | -H | $-CH_3$ |
| (113a) | $3-CH=NOCH_3$ | -H | $-CH_3$ |
| (114a) | 3-NHCSNHCH3 | -H | $-CH_3$ |
| (115a) | $3-N=C(-SCH_3)NHCH_3$ | -H | $-CH_3$ |
| (116a) | $3-CH_2P(=O)\ (OCH_3)\ _2$ | -H | $-CH_3$ |
| (117a) | $3-CH_2SOCH_2COOH$ | -H | $-CH_3$ |
| (118a) | $3-CH_2SO_2CH_2COOH$ | -H | $-CH_3$ |
| (119a) | 3-NHCOCOOH | -H | $-CH_3$ |
| (120a) | $3-OCH_2CH_2NH_2$•HCl | -H | $-CH_3$ |
| (121a) | $3-CH_2P(=O)(OH)_2$ | -H | $-CH_3$ |
| (122a) | $3-CH2COO(Ca)_{0.5}$ | $-(Ca)_{0.5}$ | $-CH_3$ |
| (123a) | 3-CH2COONa | -Na | $-CH_3$ |

Table 37

| Compound No. | $X_a$ and $Y_a$ | $r_a$ | $t_a$ |
|---|---|---|---|
| Compound No. | compound (Va) | | |
| (124a) | 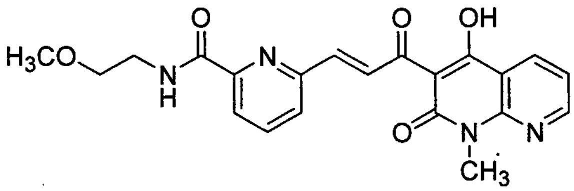 | | |
| (125a) | 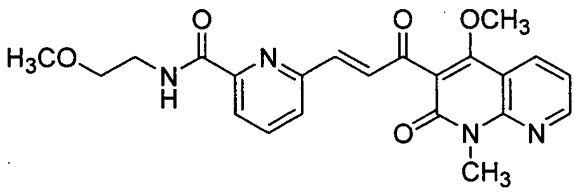 | | |
| (126a) | 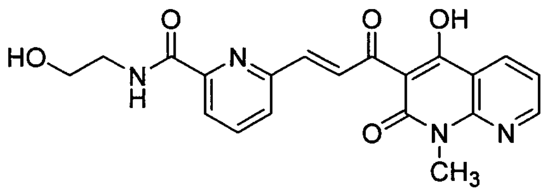 | | |
| (127a) | 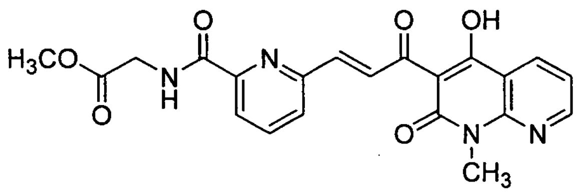 | | |
| (128a) | 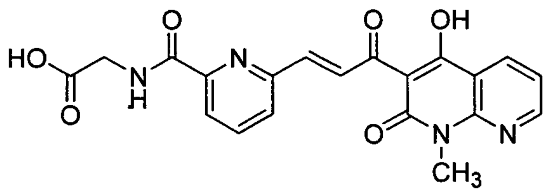 | | |
| (129a) | 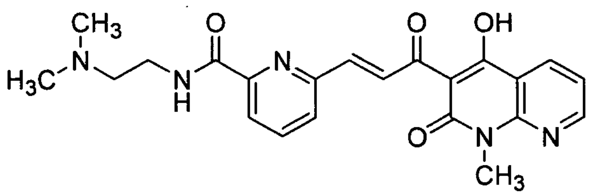 | | |

Among the compounds (V), examples of a compound represented by the formula (Vb):

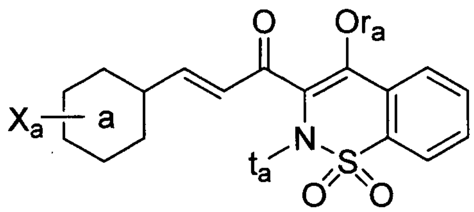

(Vb)

(wherein a, $X_a$, $r_a$ and $t_a$ are as defined above), which are represented by the compound numbers (1b) to (17b), are shown in Table 38 to Table 39.

(Table 38 to Table 39)

In Table 38, in the compound numbers (1b) to (16b), a represents a benzene ring.

Table 38

| Compound No. | $X_a$ | $r_a$ | $t_a$ |
|---|---|---|---|
| (1b) | $-OCH_2COOCH_3$ | -H | $-CH_3$ |
| (2b) | $-OCH_2COOH$ | -H | $-CH_3$ |
| (3b) | $-NHCOOCH_2CH_2OCH_3$ | -H | $-CH_3$ |
| (4b) | $-NHCONHCH_2CH_2OCH_3$ | -H | $-CH_3$ |
| (5b) | $-NHCOCH_2OCH_3$ | -H | $-CH_3$ |
| (6b) | $-NHCOCH_2OCH_3$ | $-CH_3$ | $-CH_3$ |
| (7b) | $-CONHCH_2CH_2OH$ | -H | -H |
| (8b) | $-CONHCH_2CH_2OH$ | -H | $-CH_3$ |
| (9b) | $-CONHCH_2CH_2OCH_3$ | -H | -H |
| (10b) | $-CONHCH_2CH_2OCH_3$ | -H | $-CH_3$ |
| (11b) | $-CONHCH_2CH_2OCH_3$ | $-CH_3$ | $-CH_3$ |
| (12b) | $-CONHCH_2CH_2N(CH_3)_2$ | -H | $-CH_3$ |
| (13b) | $-CONHCH_2COOCH_3$ | -H | $-CH_3$ |
| (14b) | $-CONHCH_2COOH$ | -H | $-CH_3$ |
| (15b) | $-CONHCH_2CONH_2$ | -H | $-CH_3$ |
| (16b) | $-OCH_2CH_2OH$ | -H | $-CH_3$ |

Table 39

| | | | |
|---|---|---|---|
| Compound (Vb)<br>Compound No. (17b) | 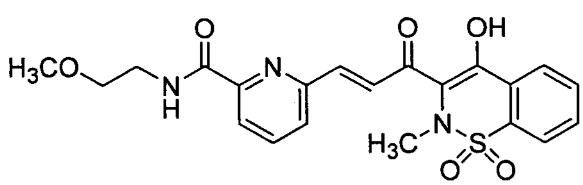 | | |

Among the compounds (V), examples of the compound (Vc) represented by the compound numbers (1c) to (26c) are shown in Tables 40 to 49.

(Tables 40 to 49)

Table 40

| Compound No. | Compound (Vc) | r |
|---|---|---|
| (1c) | 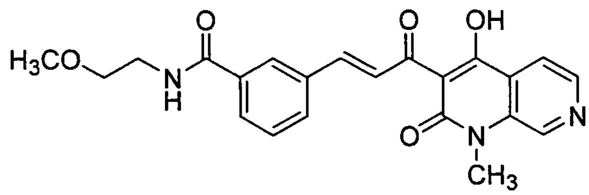 | |
| (2c) | 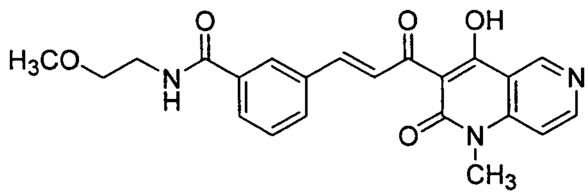 | |
| (3c) | 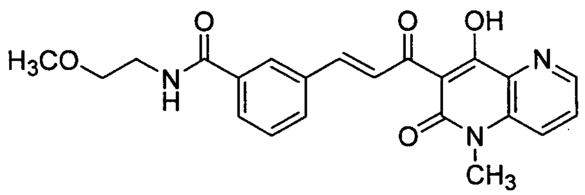 | |
| (4c) | 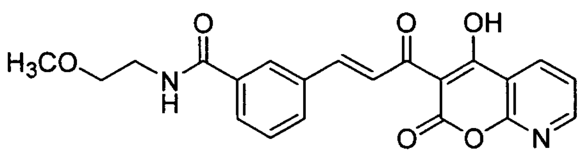 | |
| (5c) | 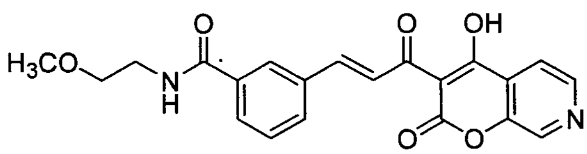 | |
| (6c) | 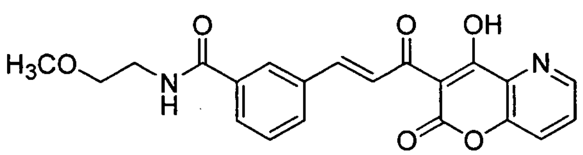 | |
| (7c) | 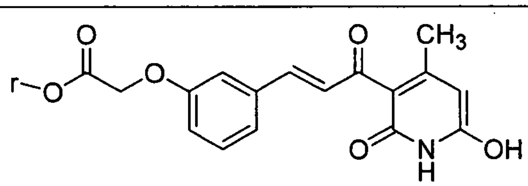 | $-CH_3$ |
| (8c) | | -H |

Table 41

| Compound No. | Compound (Vc) | r |
|---|---|---|
| (9c) | 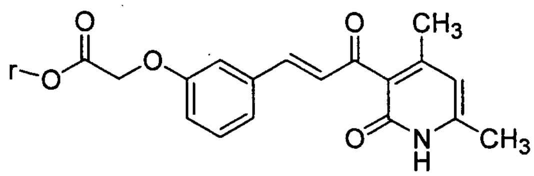 | $-CH_3$ |
| (10c) | | -H |
| (11c) | 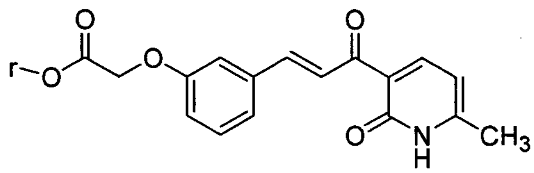 | $-CH_3$ |
| (12c) | | -H |
| (13c) | 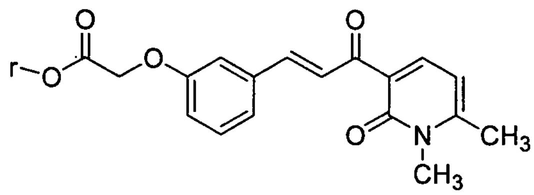 | $-CH_3$ |
| (14c) | | -H |
| (15c) | 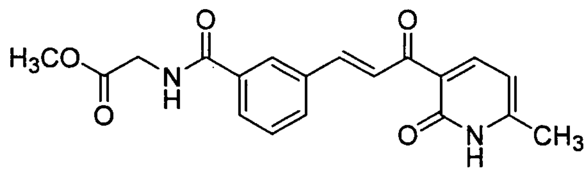 | |
| (16c) | 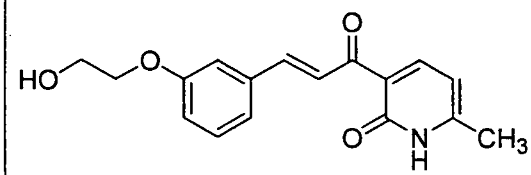 | |

Table 42

| Compound No. | Compound (Vc) | r |
|---|---|---|
| (17c) | 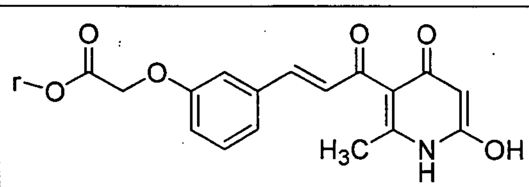 | $-CH_3$ |
| (18c) | | -H |
| (19c) | 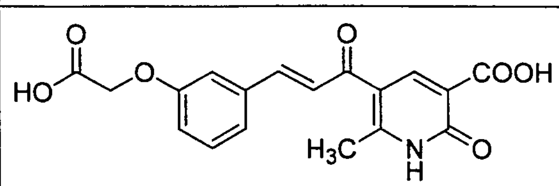 | |

Table 43

| | | |
|---|---|---|
| compound (Vc) compound No. (20c) | 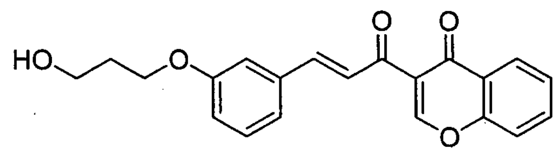 | |

Table 44

| | | |
|---|---|---|
| compound (Vc) compound No. (21c) | 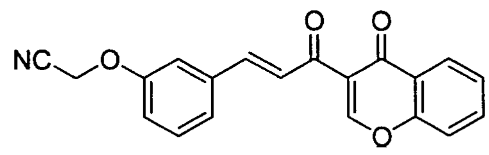 | |

Table 45

| | | |
|---|---|---|
| compound (Vc) compound No. (22c) | 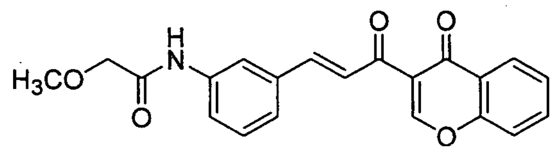 | |

Table 46

| | | |
|---|---|---|
| Compound (Vc) Compound No. (23c) | 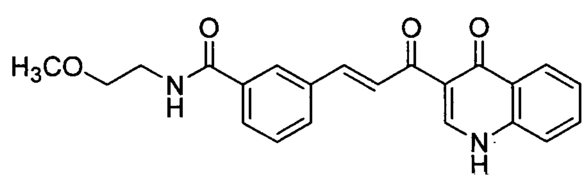 | |

Table 47

| | | |
|---|---|---|
| Compound (Vc) Compound No. (24c) | 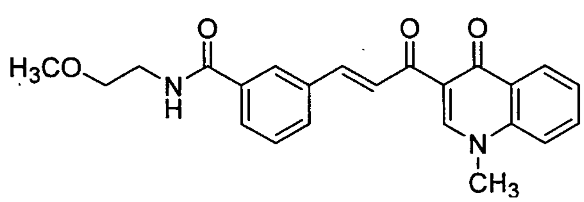 | |

Table 48

| | | |
|---|---|---|
| Compound (Vc) Compound No. (25c) | 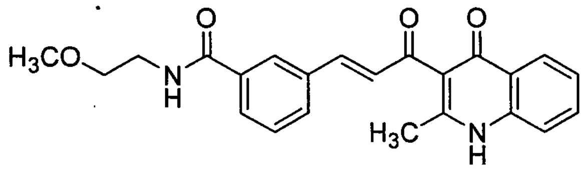 | |

Table 49

| | | |
|---|---|---|
| Compound (Vc) Compound No. (26c) | 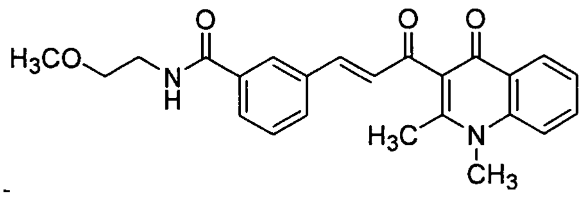 | |

Among the compounds (V), Examples of the compound (Vd) represented by the compound numbers (1d) to (40d) are shown in Table 50 to Table 51.

(Table 50 to Table 51)

Compound (Vd)

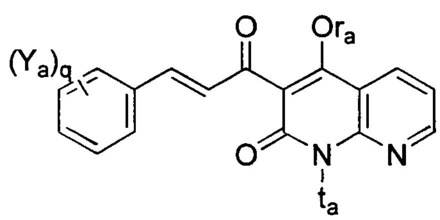

(Vd)

Table 50

| Compound No. | $(Y_a)_q$ | $r_a$ | $t_a$ |
|---|---|---|---|
| (1d) | H | H | H |
| (2d) | H | H | $CH_3$ |
| (3d) | H | $CH_3$ | $CH_3$ |
| (4d) | 3-Cl | H | H |
| (5d) | 3-$CH_3$ | H | H |
| (6d) | 4-$CF_3$ | H | H |
| (7d) | 3-$CH_2OCH_3$ | H | H |
| (8d) | 3-$CH=CHCH_3$ | H | H |
| (9d) | 3-$C\equiv CH$ | H | H |
| (10d) | 3-$OC_2H_5$ | H | H |
| (11d) | 4-$SCH_3$ | H | H |

(continued)

| Compound No. | $(Y_a)_q$ | $r_a$ | $t_a$ |
|---|---|---|---|
| (12d) | $4\text{-}S(O)CH_3$ | H | H |
| (13d) | $4\text{-}S(O)_2CH_3$ | H | H |
| (14d) | $4\text{-}NO_2$ | H | H |
| (15d) | 3-CN | H | H |
| (16d) | 4-COOH | H | H |
| (17d) | $4\text{-}COOCH_3$ | H | H |
| (18d) | $4\text{-}N(CH_3)_2$ | H | H |
| (19d) | $3\text{-}NHCOCH_3$ | H | H |
| (20d) | $3\text{-}NHCON(CH_3)_2$ | H | H |
| (21d) | $3\text{-}CONH_2$ | H | H |
| (22d) | $3\text{-}CON(CH_3)_2$ | H | H |
| (23d) | $3\text{-}OCHF_2$ | H | H |
| (24d) | $4\text{-}OCF_3$ | H | H |
| (25d) | $4\text{-}OCF_2CHF_2$ | H | H |
| (26d) | $2\text{-}SCF_3$ | H | H |
| (27d) | $3,4\text{-}Cl_2$ | H | H |
| (28d) | $2,4\text{-}(OCH_3)_2$ | H | H |
| (29d) | $3\text{-}CH_3$, $4\text{-}OCH_3$ | H | H |
| (30d) | $3\text{-}OC_2H_5$, 4-OH | H | H |

Table 51

| Compound No. | $(Y_a)_q$ | $r_a$ | $t_a$ |
|---|---|---|---|
| (31d) | $3\text{-}CF_3$, 4-Cl | H | H |
| (32d) | 3-Cl, $4\text{-}OCF_3$ | H | H |
| (33d) | 3-F, $4,5\text{-}(OCH_3)_2$ | H | H |
| (34d) | $3\text{-}COOCH_3$ | H | $CH_3$ |
| Compound No. | 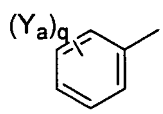 | $r_a$ | $t_a$ |
| (35d) | 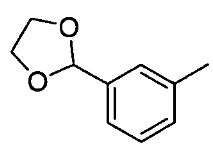 | H | $CH_3$ |
| (36d) | 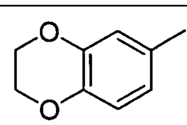 | H | $CH_3$ |

(continued)

| Compound No. | $(Y_a)_q$ | $r_a$ | $t_a$ |
|---|---|---|---|
| (37d), | 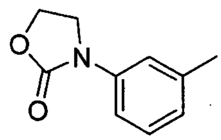 | H | H |
| (38d) | 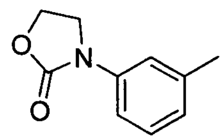 | H | $CH_3$ |
| (39d) | 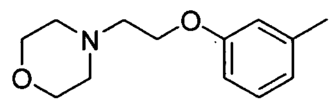 | H | H |
| (40d) | 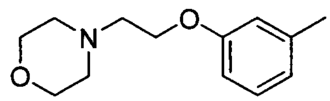 | H | $CH_3$ |

Among the compounds (V), examples of the compound (Ve) represented by the compound numbers (1e) to (3e) are shown in Table 52.

(Table 52)

Table 52

| Compound No. | Compound (Ve) |
|---|---|
| (1e) | 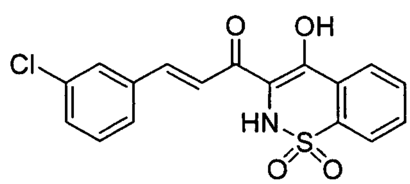 |
| (2e) | 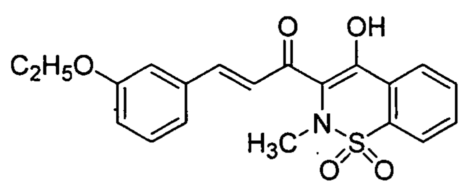 |
| (3e) | 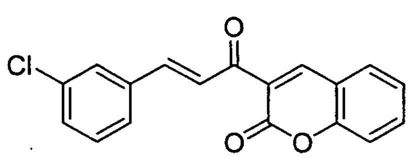 |

Among the compounds (V), examples of the compound (Vf) represented by the compound numbers (1f) to (15f) are shown in Table 53.

(Table 53)

Compound (Vf)

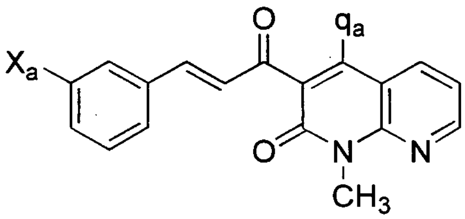

(Vf)

Table 53

| Compound No. | $X_a$ | $q_a$ |
|---|---|---|
| (1f) | $-OCH_2COOCH_3$ | $-OCH_2CH{=}CH_2$ |
| (2f) | $-OCH_2COOCH_3$ | $-OCH_2C{=}CH$ |
| (3f) | $-OCH_2COOCH_3$ | $-OCH_2COOCH_3$ |
| (4f) | $-OCH_2COOH$ | $-OCH_2COOH$ |
| (5f) | $-OCH_2CONH_2$ | $-OCH_2CONH_2$ |
| (6f) | $-OCH_2COOCH_3$ | $-OCH_2CN$ |
| (7f) | $-OCH_2COOH$ | $-OCH_2CH_2OH$ |
| (8f) | $-OCH_2COOCH_3$ | $-OCH_2Ph$ |
| (9f) | $-OCH_2COOH$ | $-OCH_2Ph$ |
| (10f) | $-OCH_2COOCH_3$ | $-OCH_2CH_2N(CH_3)_2$ |
| (11f) | $-OCH_2CH_2CH_2OH$ | 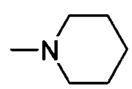 |
| (12f) | 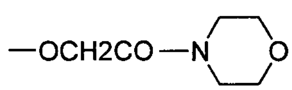 | 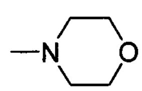 |
| (13f) | $-OCH_2COOCH_3$ | $-NHCH_2C{=}CH$ |
| (14f) | $-OCH_2CONHCH_2CH_2OCH_3$ | $-NHCH_2CH_2OCH_3$ |
| (15f) | $-OCH_2COOH$ | $-NHCH_2C{=}CH$ |

Among the compounds (V), examples of the compound (Vg) represented by the compound numbers (1g) to (11g) are shown in Table 54.

(Table 54)

Compound (Vg)

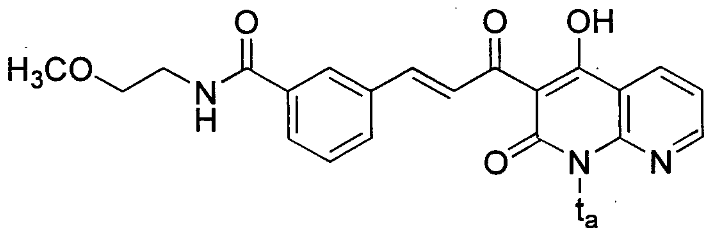

(Vg)

Table 54

| Compound No. | $t_a$ |
|---|---|
| (1g) | $-CH_2CH=CH_2$ |
| (2g) | $-CH_2C\equiv CH$ |
| (3g) | $-CH_2COOCH_3$ |
| (4g) | $-CH_2COOH$ |
| (5g) | $-CH_2CONH_2$ |
| (6g) | $-CH_2CN$ |
| (7g) | $-CH_2COCH_3$ |
| (8g) | $-CH_2CH_2OCH_3$ |
| (9g) | $-CH_2Ph$ |
| (10g) | -Ph |
| (11g) | 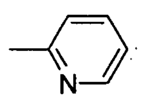 |

The compounds (I) to (V), (I'), (II') and (V') have the ability to suppress transcription of an extracellular matrix gene such as a Type I collagen gene or a fibronectin gene. The ability is important in improving tissue fibrosis because it decreases expression of an extracellular matrix gene such as a Type I collagen gene or a fibronectin gene to induce a reduction in accumulation of an extracellular matrix such as collagen or fibronectin. Therefore, the compounds (I) to (V), (I'), (II') and (V') can be utilized as an active ingredient of a composition (medicament, cosmetic, food additive etc.) which can improve tissue fibrosis by decreasing expression of an extracellular matrix gene such as a Type I collagen gene or a fibronectin gene to induce a reduction in accumulation of an extracellular matrix such as collagen or fibronectin.

A disease to which the transcription-suppressing composition of the present invention or the fibrosis-improving composition of the present invention can be applied includes, for example, a disease in which excessive accumulation of an extracellular matrix such as collagen or fibronectin causes fibrosis and then sclerosis of tissues, resulting in decreased function, cicatrization and the like in the tissues such as organs (i.e. fibrosing disease etc.). Specifically, examples of the disease include cirrhosis, chronic pancreatitis, scirrhous gastric cancer, interstitial pulmonary disease, asthma, chronic obstructive pulmonary diseases, glomerular nephritis, lupus nephritis, tubulointerstitial nephritis, IgA nephritis, renal sclerosis, diabetic nephritis, hereditary renal disease, myocardial fibrosis, heart failure, restenosis after PTCA, arterial sclerosis, marrow fibrosis, rheumatoid arthritis, hyperplasia scar after inflammation, postoperative scars or burn scars, atopic dermatitis, hypertrophic scar, hysteromyoma, prostate hypertrophy, scleroderma, Alzheimer disease, sclerotic peritonitis, diabetic retinopathy, I type diabetes, and the like. Incidentally, as an example of cirrhosis, it has been already known that C type or B type hepatitis virus induces chronic inflammation and then increased production of TGF-β, and thereby, hepatic fibrosis (particularly, accumulation of type I and type III collagen) is induced to cause cirrhosis (e.g. see Clin. Liver Dis., 7, 195-210(2003)). As an example of interstitial pulmonary disease, it has been thought that pneumonia caused by mites, viruses, tubercle bacilli or the like induces increased production of TGF-β and then pulmonary fibrosis, and thereby interstitial pulmonary disease is caused. For chronic renal failure such as diabetic nephropathy and IgA nephropathy, it has been already suggested that diabetic nephropathy is caused by increased level of TGF-β in renal glomeruli due to hyperglycemia and thereby induction of renal fibrosis (particularly accumulation

of Type I and Type IV collagen), and IgA nephropathy is caused by induction of nephritis due to accumulation of IgA in renal glomeruli followed by increased level of TGF-β, and thereby induction of renal fibrosis (particularly accumulation of Type I and Type IV collagen) (e.g. see Am. J. Physiol. Renal Phsiol., 278, F830-F838(2000), Kidney Int. 64. 149-159 (2003)). A db/db mouse, a diabetic nephropathy model animal, develops hyperglycemia by overeating because it has a mutation in a leptin receptor for suppressing ingestion, and then spontaneously develops diabetes. In the db/db mouse, the blood glucose concentration is about 4 times higher than a normal mouse, and fibrosis of renal glomeruli and increased level of TGF-β are found (e.g. see Am. J. Pathol., 158, 1653-1663(2001)). An anti-Thy-1 rat, an IgA nephropathy model animal, is produced by administering an anti-Thy-1 antibody to a normal rat to artificially cause renal fibrosis. It has been shown that renal fibrosis is suppressed by administering an anti-TGF-β receptor antibody to the model animal (e.g. see Kidney Int., 60, 1745-1755(2001)). Although the cause of scleroderma is unknown, it has been found that skin fibrosis is improved by administering a TGF-β inhibitor to a Tsk mouse, which is a model animal therefor (e.g. see J. Invest. Dermatol., 118.461-470(2001)). Thus, a compound which suppresses the activity of TGF-β can be utilized as an active ingredient of a composition (medicament, cosmetic, food additive etc.) for inhibiting the collagen synthesis-promoting activity of TGF-β to suppress tissue fibrosis and thereby providing a fibrosing disease therapeutic effect. On the other hand, it is believed that a cause of heart failure such as left ventricular diastolic failure is cardiac fibrosis under a hypertensive condition. Thus, a compound which suppresses the activity of TGF-β can be utilized as an active ingredient of a composition (medicament etc.) for inhibiting the fibronectin synthesis-promoting activity of TGF-β to suppress tissue fibrosis and thereby providing a heart failure therapeutic effect.

Such transcription-suppressing composition or fibrosis-improving composition of the present invention comprises the compound (I) to (V), (I'), (II') and (V') and an inert carrier. Such composition usually comprises 0.01% by weight to 99.99% by weight of the compound (I) to (V), (I'), (II') and (V') and 99.99% by weight to 0.01% by weight of an inert carrier. The inert carrier is a pharmaceutically acceptable carrier or excipient. The transcription-suppressing composition and fibrosis-improving composition of the present invention may further comprise pharmaceutical additives, cosmetic additives, food additives and the like.

The compound (I) to (V), (I'), (II') and (V') also inhibits the ability of TGF-β to promote transcription of a Type I collagen gene or a fibronectin gene, as shown in Examples 3 to 5 below. That is, the compound (I) to (V), (I'), (II') and (V') is a TGF-β antagonist having the ability to suppress the activity of TGF-β. Therefore, the compound (I) to (V), (I'), (II') and (V') can be also utilized as an active ingredient of a composition for suppressing the activity of TGF-β.

Such TGF-β suppressing composition of the present invention comprises the compound (I) to (V), (I'), (II') and (V') and an inert carrier. Such composition usually comprises 0.01% by weight to 99.99% by weight of the compound (I) to (V), (I'), (II') and (V') and 99.99% by weight to 0.01% by weight of an inert carrier. The inert carrier is a pharmaceutically acceptable carrier or excipient. The TGF-β suppressing composition and hair-growing composition of the present invention may further comprise pharmaceutical additives, cosmetic additives, food additives and the like.

A pharmaceutically acceptable carrier, excipient, pharmaceutical additive, food additive, cosmetic additive, a medicament additive, and the like contained in the above-described composition can be appropriately selected depending on the specific use thereof. In addition, the composition may be in a form of various solids, liquids and the like depending on the specific use thereof.

For example, when the compound (I) to (V), (I'), (II') and (V') is used as an active ingredient of a medicament, specific examples of the medicament include oral preparations such as powders, fine granules, granules, tablets, syrups, capsules, suspensions, emulsions, extracts and pills; and parenteral preparations such as injections, transdermal absorbing agents such as external liquids and ointments, suppositories and local preparations.

Oral preparations can be prepared using carriers or excipients, and pharmaceutical additives such as binders, disintegrants, surfactants, lubricants, glidants, diluents, preservatives, coloring agents, flavors, stabilizers, humectants, antiseptics, antioxidants and the like, for example, gelatin, sodium alginate, starch, corn starch, white sugar, lactose, glucose, mannit, carboxymethylcellulose, dextrin, polyvinylpyrrolidone, crystalline cellulose, soybean lecithin, sucrose, fatty acid ester, talc, magnesium stearate, polyethylene glycol, magnesium silicate, anhydrous silicic acid and the like, according to a conventional method.

A dose of the oral preparation varies depending on the age, sex and weight of a mammal to be administered, the severity of disease, the kind and dosage form of the composition of the present invention, and the like. Usually, in the case of oral administration, about 1 mg to about 2 g per day, preferably about 5 mg to about 1 g per day of the active ingredient may be administered to an adult human. The daily dose may be also administered at one time or in several divided doses.

Among parenteral preparations, an injection can be prepared using such as a water-soluble solvent such as physiological saline or sterilized water Ringer solution, a water-insoluble solvent such as vegetable oil or fatty acid ester, an isotonic agent such as glucose or sodium chloride, pharmaceutical additives such as a solubilizer, a stabilizer, an antiseptic, a suspending agent and an emulsifying agent, and the like, according to a conventional method. A transdermal absorbing agent such as external liquid or a gel-like ointment, a suppository for rectal administration and the like can be also prepared according to a conventional method. For administering such parenteral preparations, they may be

administered by injection (subcutaneously, intravenously etc.), transdermally, or rectally. The local agent can be produced, for example, by incorporating the compound (I) to (V), (I'), (II') and (V') into a pellet of a sustained-release polymer such as an ethylene vinyl acetate polymer. The pellet may be surgically transplanted into a tissue to be treated.

A dose of the parenteral preparation varies depending on the age, sex and weight of a mammal to be administered, the severity of disease, the kind and dosage form of the composition of the present invention, and the like. Usually, in the case of administration by injection, about 0.1 mg to about 500 mg of the active ingredient may be administered to an adult human. The daily dose may be also administered at one time or in several divided doses.

When the compound (I) to (V), (I'), (II') and (V') is used by adding to cosmetics, examples of specific forms of cosmetics with the compound added thereto include liquid, emulsion, cream, lotion, ointment, gel, aerosol, mousse and the like. Lotion can be prepared using cosmetic additives such as a suspending agent, an emulsifier, a preservative and the like, according to a conventional method.

A dose of the cosmetic varies depending on the age, sex and weight of a mammal to be administered, the severity of disease, the kind and dosage form of the composition of the present invention, and the like. Usually, about 0.01 mg to about 50 mg of the active ingredient may be administered to an adult human. The daily dose may be also administered at one time or in several divided doses.

When the compound (I) to (V), (I'), (II') and (V') is used as a food additive, examples of specific forms of a food with the addictive added thereto include powder, a tablet, a beverage, an edible gel or a mixed liquid of the gel and syrup, for example, general beverage and food and luxury food and beverage such as seasonings, Japanese confectionaries, western confectionaries, ice confectionaries, beverage, spreads, pastes, pickles, bottled or canned products, processed domestic animal meats, processed fish meats or marine product, processed dairy or egg products, processed vegetables, processed fruits, processed cereals and the like. Alternatively, the present compound can be also added to feeds or provenders for rearing animals such as livestocks, poultry, honey bee, silkworm, fish and the like.

A dose of the food varies depending on the age, sex and weight of a mammal to be administered, the severity of disease, the kind and dosage form of the composition of the present invention, and the like. Usually, about 0.1 mg to about 500 mg of the active ingredient may be administered to an adult human. The daily dose may be also administered at one time or in several divided doses.

Examples

The following Examples further illustrate the present invention.

Example 1

Synthesis of the compound (VII-3) represented by the compound numbers (1A-1) and (1A-5) shown in Table 30, and the compound (VII-2) represented by the compound number (1B-1) shown in Table 31 is described in Examples 1-1 to 1-3.

Examples 1-1

Synthesis of compound (VII-3) [Compound No. (1A-1)]

To a solution of 1.49 g of methyl 2-(3-oxobutyrylamino)nicotinate in 35 mL of methanol was added 0.68 g of sodium methylate, and the mixture was heated under reflux for 30 minutes. Insolubles were filtered, washed with 40 mL of methanol and then dissolved in 150 mL of water, and 30 ml of an aqueous saturated ammonium chloride solution was added thereto. Insolubles were filtered, and the filtered crystal was washed with 60 ml of water, 40 ml of tetrahydrofuran and then 20 ml of hexane to obtain 1.00 g of 3-acetyl-4-hydroxy-1H-[1,8]naphthyridin-2-one [Compound No. (1A-1)] as a white solid.

$^1$H-NMR(270MHz,DMSO-$d_6$) $\delta$ (ppm): 2.40 (s, 3H), 7.01 (dd, 1H, J=4.9, 7.6Hz), 8.17 (dd, 1H, J=1.6, 7.6Hz), 8.34 (dd, 1H, J=1.6, 4.9Hz).

Examples 1-2

Synthesis of compound (VII-3) [Compound No. (1A-5)]

To a mixture of 0.41 g of 3-acetyl-4-hydroxy-1H-[1,8]naphthyridin-2-one in 10 ml of dimethylformamide was added 0.12 g of sodium hydride (60% oily) at room temperature. After the mixture was stirred at room temperature for 1 hour, 0.4 ml of methyl iodide was added thereto, followed by stirring at room temperature overnight. Insolubes were filtered and washed with 10 ml of dimethylformamide, and 2 ml of an aqueous saturated ammonium chloride solution

was added to the filtrate. The filtrate was concentrated under reduced pressure, and to the resulting residue was added 10 ml of tetrahydrofuran. Insolubles were filtered and washed with 15 ml of tetrahydrofuran, and the filtrate was concentrated under reduced pressure. The residue was subjected to silica gel column chromatography to obtain 0.06 g 3-acetyl-4-hydroxy-1-methyl-1H-[1,8]naphthyridin-2-one [Compound No. (1A-5)] as a white solid.
$^1$H-NMR (270MHz,DMSO-$d_6$) δ (ppm): 2.74 (s, 3H), 3.64 (s, 3H), 7.39 (dd, 1H, J=4.6, 7.8Hz), 8.48 (dd, 1H, J=1.9, 7.8Hz), 8.80 (dd, 1H, J=1.9, 4.6Hz), 16.86 (broad s, 1H).

Examples 1-3

Synthesis of compound (VII-2) [Compound No. (1B-1)]

To a solution of 1.52 g of methyl 2-aminonicotinate in 3 ml of xylene was added 1.3 ml of 2,2,6-trimethyl-4H-1,3-dioxin-4-one, and the mixture was heated at 120°C for 30 minutes. The reaction solution was subjected to silica gel column chromatography to obtain 1.15 g of methyl 2-(3-oxobutyrylamino)nicotinate [Compound No. (1B-1)] as a white solid.
$^1$H-NMR (270MHz,DMSO-$d_6$) δ (ppm): 2.22 (s, 3H), 3.68 (s, 2H), 3.74 (s, 3H), 7.27~7.32 (m, 1H), 8.10~8.20 (m, 1H), 8.49~8.51 (m, 1H)

Example 2

Synthesis of the compound (Va) represented by the compound numbers (19a), (20a), (29a), (31a), (32a), (36a), (37a), (40a), (42a), (56a), (86a) and (124a) shown in Tables 32 to 37, the compound (Vb) represented by the compound numbers (1b), (2b), (5b), (9b), (10b), (13b), (14b), (16b) and (17b) shown in Table 38 to Table 39, the compound (Vc) represented by the compound numbers (9c) to (25c) shown in Tables 40 to 49, and the compound (Ve) represented by the compound number (3e) shwon in Table 52 is described in Examples 2-1 to 2-39.

Examples 2-1

Synthesis of compound (Va) [Compound No. (19a)]

To a solution of 97 mg of 3-(methoxyacetylamino)benzaldehyde in 3 ml of ethanol were added 109 mg of 3-acetyl-4-hydroxy-1-methyl-1H-[1,8]naphthyridin-2-one and 14 mg of piperidine, and the mixture was heated under reflux for 2 hours and 15 minutes. After cooling, the residue was filtered, and the filtered crystal was washed with 3 ml of ethyl acetate to obtain 68 mg of 4-hydroxy-1-methyl-3-[3-[3-(methoxyacetylamino)phenyl]acryloyl]-1H-[1,8]naphthyridin-2-one [Compound No. (19a)] as light brown powder.
$^1$H-NMR(300MHz,DMSO-$d_6$) δ (ppm): 3.47 (s, 3H), 3.66 (s, 3H), 4.04 (s, 2H), 7.35~7.44 (m, 1H), 7.44~7.48 (2H), 7.80~7.86 (m, 1H), 7.91 (d, 1H, J=15.9Hz), 8.11 (s, 1H), 8.49 (dd, 1H, J=1.8, 7.8Hz), 8.57 (d, 1H, J=15.9Hz), 8.80 (dd, 1H, J=1.5, 4.5Hz), 10.05 (broad, 1H)

Examples 2-2

Synthesis of compound (Va) [Compound No. (20a)]

According to the same manner as that of Example 2-1 except that 0.09 g of 4-(methoxyacetylamino)benzaldehyde was used in place of 3-(methoxyacetylamino)benzaldehyde, 0.15 g of 4-hydroxy-1-methyl-3-[3-[4-(methoxyacetylamino)phenyl]acryloyl]-1H-[1,8]naphthyridin-, 2-one [Compound No. (20a)] was obtained as light brown powder.
$^1$H-NMR(270MHz, DMSO-$d_6$) δ (ppm): 3.39 (s, 3H), 3.66 (s, 3H), 4.04 (s, 2H), 7.39 (dd, 1H, J=5.4, 8.1Hz), 7.74 (d, 2H, J=8.1Hz), 7.83 (d, 2H, J=8.1Hz), 7.94 (d, 1H, J=16.2Hz), 8.49 (d, 1H, J=8.1Hz), 8.53 (d, 1H, J=13.5Hz), 8.79 (d, 1H, J=5.4Hz), 10.08 (s, 1H), 18.03 (broad, 1H)

Examples 2-3

Synthesis of compound (Va) [Compound No. (29a)]

According to the same manner as that of Example 2-1 except that 0.20 g of 3-[[(methoxycarbonylmethyl)amino]carbonyl]benzaldehyde was used in place of 3-(methoxyacetylamino)benzaldehyde and 6 ml of methanol was used in place of ethanol, 0.19 g of 4-hydroxy-1-methyl-3-[3-[3-[[(methoxycarbonylmethyl)amino]carbonyl]phenyl]acryloyl]-1H-[1,8]naphthyridin-2-one [Compound No. (29a)] was obtained as a light brown crystal.

$^1$H-NMR(270MHz,DMSO-$d_6$) δ (ppm): 3.67 (s, 3H), 3.68 (s, 3H), 4.06 (d, 2H, J=5.7Hz), 7.40 (dd, 1H, J=4.9, 7.8Hz), 7.62 (t, 1H, J=7.8Hz), 7.94 (d, 1H, J=7.6Hz), 7.97 (d, 1H, J=15.4Hz), 7.99 (d, 1H, J=7.8Hz), 8.26 (s, 1H), 8.50 (dd, 1H, J=1.6, 7.6Hz), 8.60.(d, 1H, J=15.9Hz), 8.81 (dd, 1H, J=1.6, 4.6Hz), 9.16 (t, 1H, J=5.7Hz)

Examples 2-4

Synthesis of compound (Va) [Compound No. (31a)]

To a solution of 0.25 g of 3-[(2-methoxyethyl)aminocarbonyl]benzaldehyde in 5 ml of ethanol were added 0.20 g of 3-acetyl-4-hydroxy-1H-[1,8]naphthyridin-2-one and 0.03 ml of piperidine, and the mixture was heated under reflux for 30 minutes. Further, 5 ml of ethanol and 4 ml of dimethylformamide were added thereto, and the mixture was heated under reflux for 2 hours and 45 minutes. Then 15 ml of ethanol was added thereto and insolubles were filtered and recrystallized from 3 ml of dimethylformamide. The resulting crystal was washed with 10 ml of ethanol to obtain 0.07 g of 4-hydroxy-3-[3-[3-[(2-methoxyethyl)aminocarbonyl]phenyl]acryloyl]-1H-[1,8]naphthyridin-2-one [Compound No. (31a)] as a light yellow crystal.
$^1$H-NMR(270MHz,DMSO-$d_6$) δ (ppm): 3.29 (s, 3H), 3.40~3.60 (m, 4H), 7.30~7.40 (m, 1H), 7.59 (t, 1H, J=7.3Hz), 7.90 (d, 1H, J=7.8Hz), 7.93 (d, 1H, J=8.4Hz), 7.99 (d, 1H, J=16.5Hz), 8.23 (s, 1H), 8.42 (d, 1H, J=7.8Hz), 8.60~8.75(3H)

Examples 2-5

Synthesis of compound (Va) [Compound No. (32a)]

To a solution of 0.10 g of 3-[(2-methoxyethyl)aminocarbonyl]benzaldehyde in 3 ml of ethanol were added 0.06 g of 3-acetyl-4-hydroxy-1-methyl-1H-[1,8] naphthyridin-2-one and 0.01 ml of piperidine, and the mixture was heated under reflux for 1 hour and 30 minutes. After concentration under reduced pressure, 3 ml of ethyl acetate was added to the residue. The mixture was filtered, and the filtered crystal was washed with 6 ml of ethyl acetate to obtain 0.07 g of 4-hydroxy-1-methyl-3-[3-[3-[(2-methoxyethyl)aminocarbonyl]phenyl]acryloyl]-1H-[1,8]naphthyridin-2-one [Compound No. (32a)] as a light yellow crystal.
$^1$H-NMR(270MHz,DMSO-$d_6$) δ (ppm): 3.29 (s, 3H), 3.40-3.60 (m, 4H), 3.68 (s, 3H), 7.35~7.45 (m, 1H), 7.59 (t, 1H, J=7.8Hz), 7.85~8.05 (3H), 8.24 (s, 1H), 8.51 (d, 1H, J=7.8Hz), 8.58 (d, 1H, J=15.1Hz), 8.71 (broad, 1H), 8.81 (d, 1H, J=4.1Hz)

Examples 2-6

Synthesis of compound (Va) [Compound NO. (36a)]

According to the same manner as that of Example 2-1 except that 0.18 g of 3-[(methoxycarbonyl)methoxy] benzaldehyde was used in place of 3-(methoxyacetylamino)benzaldehyde and 6 ml of methanol was used in place of ethanol, 0.23 g of 4-hydroxy-1-methyl-3-[3-[3-[(methoxycarbonyl)methoxy]phenyl]acryloyl]-1H-[1,8]naphthyridin-2-one [Compound No. (36a)] was obtained as a light yellow crystal.
$^1$H-NMR (270MHz, DMSO-$d_6$) δ (ppm): 3.67 (s, 3H), 3.74 (s, 3H), 4.89 (s, 2H), 7.07~7.11 (1H), 7.31 (s, 1H), 7.36~7.46 (3H), 7.93 (d, 1H, J=16.5Hz), 8.50 (dd, 1H, J=1.9, 7.6Hz), 8.52~8.58 (1H), 8.82 (d, 1H, J=3.8Hz)

Examples 2-7

Synthesis of compound (Va) [Compound No. (37a)]

To a solution of 0.15 g of 4-hydroxy-1-methyl-3-[3-[3-[(methoxycarbonyl)methoxy]phenyl]acryloyl]-1H-[1,8] naphthyridin-2-one in 4.6 ml of methanol was added 4.6 ml of a 1N aqueous sodium hydroxide solution. The mixture was stirred at room temperature for 2 hours and 15 minutes. The solvent was distilled off under reduced pressure and then acidified with 10% hydrochloric acid. Precipitated crystals were filtered, washed with ethyl acetate and hexane, and then dried to obtain 0.13 g of 4-hydroxy-1-methyl-3-[3-[3-(carboxymethoxy)phenyl]acryloyl]-1H-[1,8]naphthyridin-2-one [Compound No. (37a)] as a tan crystal.
$^1$H-NMR(270MHz,DMSO-$d_6$) δ (ppm): 3.63 (s, 3H), 4.76 (s, 2H), 7.04 (d, 1H, J=7.6Hz), 7.28 (s, 1H), 7.34~7.44 (3H), 7.85 (d, 1H, J=15.9Hz), 8.44~8.50 (1H), 8.48 (dd, 1H, J=2.2, 7.8Hz), 8.76~8.78 (1H)

Examples 2-8

Synthesis of compound (Va) [Compound No. (40a)]

According to the same manner as that of Example 2-1 except that 0.23 g of 3-(2-hydoxyethoxy)benzaldehyde was used in place of 3-(methoxyacetylamino)benzaldehyde, 0.19 g of 4-hydroxy-1-methyl-3-[3-[3-(2-hydroxyethoxy) phenyl]acryloyl]-1H-[1,8]naphthyridin-2-one [Compound No. (40a)] was obtained as a yellow crystal.
$^1$H-NMR(270MHz,DMSO-$d_6$) δ (ppm): 3.32 (s, 3H), 3.72~3.78 (2H), 4.05 (t, 2H, J=5.4Hz), 7.08 (d, 1H, J=8.1Hz), 7.29 (s, 1H), 7.32~7.43 (3H), 7.91 (d, 1H, J=16.2Hz), 8.48 (dd, 1H, J=2.7, 8.1Hz), 8.54 (d, 1H, J=16.2Hz), 8.79 (d, 1H)

Examples 2-9

Synthesis of compound (Va) [Compound No. (42a)]

According to the same manner as that of Example 2-1 except that 0.10 g of 3-[(2-methoxyethoxy)carbonylamino] benzaldehyde was used in place of 3-(methoxyacetylamino)benzaldehyde, 0.11 g of 4-hydroxy-1-methyl-3-[3-[3-[(2-methoxyethoxy)carbonylamino]phenyl]acryloyl]-1H-[1,8]naphthyridin-2-one [Compound No. (42a)] was obtained as a tan crystal.
$^1$H-NMR(300MHz,DMSO-$d_6$) δ (ppm): 3.30 (s, 3H), 3.59 (t, 2H, J=4.5Hz), 3.65 (s, 3H), 4.24 (t, 2H, J=4.5Hz), 7.33~7.44 (3H), 7.57 (d, 1H, J=7.5Hz), 7.89 (d, 1H, J=14.7Hz), 7.94 (s, 1H), 8.45~8.50 (1H), 8.56 (d, 1H, J=15.0Hz), 8.77~8.83 (1H), 9.97 (s, 1H)

Examples 2-10

Synthesis of compound (Va) [Compound No. (56a)]

According to the same manner as that of Example 2-1 except that 0.26 g of 3-(2-dimethylaminoethoxy)benzaldehyde was used in place of 3-(methoxyacetylamino)benzaldehyde, 0.22 g of 4-hydroxy-1-methyl-3-[3-[3-(2-dimethylaminoethoxy)phenyl]acryloyl]-1H-[1,8]naphthyridin-2-one [Compound No. (56a)] was obtained as a tan crystal. $^1$H-NMR(270 MHz, DMSO-$d_6$) δ (ppm): 2.53 (s, 6H), 3.05 (t, 2H, J=5.4Hz), 3.58 (s, 3H), 4.23 (t, 2H, J=5.4Hz), 7.02 (d, 1H, J=10.8Hz), 7.23 (d, 1H, J=8.1Hz), 7.24 (s, 1H), 7.27 (d, 1H, J=8.1Hz), 7.36 (t, 1H, J=8.1Hz), 7.60 (d, 1H, J=16.2Hz), 8.10 (d, 1H, J=16.2Hz), 8.39 (dd, 1H, J=2.7, 5.4Hz), 8.63 (d, 1H)

Examples 2-11

Synthesis of compound (Va) [Compound No. (86a)]

According to the same manner as that of Example 2-7 except that 0.11 g of 4-hydroxy-1-methyl-3-[3-[3-[[(methoxycarbonylmethyl)amino]carbonyl]phenyl]acryloyl]-1H-[1,8]naphthyridin-2-one was used in place of 4-hydoroxy-1-methyl-3-[3-[(methoxycarbonyl)methoxy]phenyl]acryloyl-1H-[1,8]naphthyridin-2-one, 0.12 g of 4-hydroxy-1-methyl-3-[3-[3-[[(carboxymethyl)amino]carbonyl]phenyl]acryloyl]-1H-[1,8]naphthyridin-2-one [Compound No. (86a)] was obtained as a yellow crystal.
$^1$H-NMR(270MHz,DMSO-$d_6$) δ (ppm): 3.67 (s, 3H), 3.97 (d, 1H, J=5.9Hz), 7.35~7.45 (1H), 7.62 (t, 1H, J=7.8Hz), 7.80~8.10 (3H), 8.27 (s, 1H), 8.40~8.55 (1H), 8.60 (d, 1H, J=14.6Hz), 8.80~8.90 (1H), 9.07 (1H)

Examples 2-12

Synthesis of compound (Va) [Compound No. (124a)]

According to the same manner as that of Example 2-1 except that 0.10 g of 6-formyl-2-[(2-methoxyethyl) aminocarbonyl]pyridine was used in place of 3-(methoxyacetylamino)benzaldehyde, 0.05 g of 4-hydroxy-1-methyl-3-[3-[6-(2-methoxyethyl)aminocarbonyl-2-pyridinyl]acryloyl]-1H-[1,8]naphthyridin-2-one [Compound No. (124a)] was obtained as a light yellow crystal.
$^1$H-NMR(300MHz,DMSO-$d_6$) δ (ppm): 3.34 (s, 3H), 3.53~3.54 (m, 1H), 3.66 (s, 3H), 7.34~7.42 (1H), 7.90 (d, 1H, J=15.9Hz), 8.02 (dt, 1H, J=1.5, 7.2Hz), 8.06~8.13 (2H), 8.42 (dd, 1H, J=2.1, 8.1Hz), 8.67 (s, 1H), 8.76~8.81 (1H), 8.80 (dd, 1H, J=1.8, 4.8Hz)

Examples 2-13

Synthesis of compound (Vb) [Compound No. (1b)]

To a solution of 1.53 g of 3-[(methoxycarbonyl)methoxy]benzaldehyde in 20 ml of methanol were added 2.00 g of 3-acetyl-4-hydroxy-2-methyl-2H-1,2-benzothiazine-1,1-dioxide and 202 mg of piperidine, and the mixture was heated under reflux for 9 hours and 10 minutes. After cooling, insolubles were filtered. The resulting crystal was washed with 10 ml of methanol and then dried to obtain 2.35 g of 4-hydroxy-3-[3-[3-[(methoxycarbonyl)methoxy]phenyl]acryloyl]-2-methyl-2H-1,2-benzothiazine-1,1-dioxide [Compound No. (1b)] as a light yellow crystal.
$^1$H-NMR(270MHz,DMSO-$d_6$) $\delta$ (ppm): 2.97 (s, 3H), 3.73 (s, 3H), 4.91 (s, 2H), 7.08~7.10 (m, 1H), 7.38~7.54 (m, 4H), 7.89 (d, 1H, J=13.5Hz), 7.95~8.00 (m, 3H), 8.16~8.20 (m, 1H)

Examples 2-14

Synthesis of compound (Vb) [Compound No. (2b)]

To a solution of 300 mg of 4-hydroxy-3-[3-[3-(methoxycarbonylmethoxy)phenyl]acryloyl]-2-methyl-2H-1,2-benzothiazine-1,1-dixoide in 8 ml of methanol was added 8 ml of a 1N aqueous sodium hydroxide. The mixture was stirred at room temperature for 50 minutes. The solvent was distilled off under reduced pressure and then acidified with 2N hydrochloric acid. Precipitated crystals were filtered, washed with water and hexane and then dried to obtain 292 mg of 4-hydroxy-3-[3-[3-(carboxymethyl)phenyl]acryloyl]-2-methyl-2H-1,2-benzothiazine-1,1-dioxide [Compound No. (2b)] as a yellow crystal.
$^1$H-NMR(270MHz,DMSO-$d_6$) $\delta$ (ppm): 2.97 (s, 3H), 4.79 (s, 2H), 7.06~7.09 (m, 1H), 7.38~7.53 (m, 4H), 7.69 (d, 1H, J=15.9Hz), 7.97~7.98 (m, 3H), 8.15~8.18 (m, 1H)

Examples 2-15

Synthesis of compound (Vb) [Compound No. (5b)]

According to the same manner as that of Example 2-13 except that 763 mg of 3-(methoxyacetylamino)benzaldehyde was used in place of 3-[(methoxycarbonyl)methoxy]benzaldehyde and 10 ml of ethanol was used in place of methanol, 1.28 g of 4-hydroxy-3-[3-[3-(methoxyacetylamino)phenyl]acryloyl]-2-methyl-2H-1,2-benzothiazine-1,1-dioxide [Compound No. (5b)] was obtained as a yellow crystal.
$^1$H-NMR(270MHz,DMSO-$d_6$) $\delta$ (ppm): 2.97 (s, 3H), 3.41 (s, 3H), 4.04 (s, 2H), 7.34 (d, 1H, J=16.2Hz), 7.44 (t, 1H, J=8.1Hz), 7.58 (d, 1H, J=8.1Hz), 7.86 (d, 1H, J=16.2Hz), 7.89~7.98 (m, 4H), 8.08 (s, 1H), 8.16 (m, 1H), 9.93 (s, 1H)

Examples 2-16

Synthesis of compound (Vb) [Compound No. (9b)]

To a solution of 3-[(2-methoxyethyl)aminocarbonyl]benzaldehyde in 30 ml of ethanol were added 1.50 g of 3-acetyl-4-hydroxy-2H-1,2-benzothiazine-1,1-dioxide and 163 μl of piperidine, and the mixture was heated under reflux for 6 hours and 45 minutes. After cooling, 50 ml of 10% hydrochloric acid was added thereto, and the mixture was extracted with chloroform. An organic layer was washed with an aqueous saturated sodium chloride solution, dried over anhydrous sodium sulfate, and then concentrated under reduced pressure. Ethanol was added to the residue to induce crystallization and thereby 23 mg of of 3-[3-[3-[(2-methoxyethyl)aminocarbonyl]phenyl]acryloyl]-4-hydroxy-2H-1,2-benzothiazine-1,1-dioxide [Compound No. (9b)] was obtained as a light yellow crystal.
$^1$H-NMR (270MHz,DMSO-$d_6$) $\delta$ (ppm): 3.29 (s, 3H), 3.45~3.55 (m, 4H), 7.55 (d, 1H, J=15.9Hz), 7.58 (t, 1H, J=7.8Hz), 7.85~7.95 (6H), 8.15~8.20 (1H), 8.29 (s, 1H), 8.74 (1H), 10.30 (broad, 1H), 15.69 (broad, 1H)

Examples 2-17

Synthesis of compound (Vb) [Compound No. (10b)]

According to the same manner as that of Example 2-16 except that 1.11 g of 3-acetyl-4-hydroxy-2-methyl-2H-1,2-benzothiazine-1,1-dioxide was used in place of 3-acetyl-4-hydroxy-2H-1,2-benzothiazine-1,1-dioxide, 1.06 g of 4-hydroxy-3-[3-[3-[(2-methoxyethyl)aminocarbonyl]phenyl]acryloyl]-2-methyl-2H-1,2-benzothiazine-1,1-dioxide [Compound No. (10b)] was obtained as a yellow crystal.

$^1$H-NMR(270MHz,DMSO-$d_6$) 5 (ppm): 2.98 (s, 3H), 3.29 (s, 3H), 3.45-3.55 (m, 4H), 7.48 (d, 1H, J=16.2Hz), 7.59 (t, 1H, J=7.8Hz), 7.90-8.20 (5H), 8.28 (s, 1H), 8.70 (broad s, 1H), 15.73 (broad, 1H)

Examples 2-18

Synthesis of compound (Vb) [Compound No. (13b)]

According to the same manner as that of Example 2-13 except that 1.75 g of 3-[[(methoxycarbonylmethyl) amino]carbonyl]benzaldehyde was used in place of 3-[(methoxycarbonyl)methoxy]benzaldehyde, 2.38 g of 4-hydroxy-3-[3-[3-[[(methoxycarbonylmethyl)amino]carbonyl]phenyl]acryloyl]-2-methyl-2H-1,2-benzothiazine-1,1-dioxide [Compound No. (13b)] was obtained as a yellow crystal.
$^1$H-NMR(270MHz,DMSO-$d_6$) δ (ppm): 2.98 (s, 3H), 3.68 (s, 3H), 4.08 (d, 2H, J=5.7Hz), 7.59 (d, 1H, J=16.2Hz), 7.62 (t, 1H, J=7.6Hz), 7.93~7.99 (m, 5H), 8.09 (d, 1H, J=7.6Hz), 8.16~8.19 (m, 1H), 8.33 (s, 1H), 9.16 (t, 1H, J=5.7Hz)

Examples 2-19

Synthesis of compound (Vb) [Compound No. (14b)]

According to the same manner as that of Example 2-14 except that 300 mg of 4-hydroxy-3-[3-[3-[[(methoxycarbonylmethyl).amino]carbonyl]phenyl]acryloyl]-2-methyl-2H-1,2-benzothiazine-1,1-dioxide was used in place of 4-hydroxy-3-[3-[3-[(methoxycarbonyl)methoxy]phenyl]acryloyl]-2-methyl-2H-1,2-benzothiazine-1,1-dioxide, 263 mg of 4-hydroxy-3-[3-[3-[[(carboxymethyl)amino]carbonyl]phenyl]acryloyl]-2-methyl-2H-1,2-benzothiazine-1,1-dioxide [Compound No. (14b)] was obtained as a yellow crystal.
$^1$H-NMR(300MHz,DMSO-$d_6$) δ (ppm): 2.98 (s, 3H), 3.99 (d, 2H, J=5.7Hz), 7.50 (d, 1H, J=16.2Hz), 7.62 (t, 1H, J=7.8Hz), 7.94~7.98 (m, 5H), 8.09 (d, 1H, J=7.2Hz), 8.16~8.18 (m, 1H), 8.33 (s, 1H), 9.06 (t, 1H, J=5.7Hz)

Examples 2-20

Synthesis of compound (Vb) [Compound No. (16b)]

According to the same manner as that of Example 2-13 except that 656 mg of 3-(2-hydroxyethoxy)benzaldehyde was used in place of 3-[(methoxycarbonyl)methoxy]benzaldehyde and 10 ml of ethanol was used in place of methanol, 547 mg of 4-hyrdoxy-3-[3-[3-(2-hydroxyethoxy)phenyl]acryloyl]-2-methyl-2H-1,2-benzothiazine-1,1-dioxide [Compound No. (16b)] was obtained as a yellow crystal.
$^1$H-NMR(270MHz,$CDCl_3$) δ (ppm): 2.05 (broad s, 1H), 3.00 (s, 3H), 4.03 (s, 2H), 4.17 (t, 1H, J=5.4Hz), 7.03 (d, 1H, J=8.1Hz), 7.29 (s, 1H), 7.33 (d, 1H, J=16.2Hz), 7.37 (t, 1H, J=8.1Hz), 7.75~7.83 (m, 2H), 7.84 (d, 1H, J=16.2Hz), 7.87~7.94 (m, 1H), 8.15~8.20 (m, 1H)

Examples 2-21

Synthesis of compound (Vb) [Compound No. (17b)]

According to the same manner as that of Example 2-13 except that 1.64 g of 6-formyl-2-[(2-methoxyethyl) aminocarbonyl]pyridine was used in place of 3-[(methoxycarbonyl)methoxy]benzaldehyde, 2.70 g of 4-hydroxy-3-[3-[6-(2-methoxyethyl)aminocarbonyl-2-pyridinyl]acryloyl]-2-methyl-2H-1,2-benzothiazine-1,1-dioxide [Compound No. (17b)] was obtained as a yellow crystal.
$^1$H-NMR(270MHz,$CDCl_3$) δ (ppm): 3.05 (s, 3H), 3.62 (s, 3H), 3.66 (t, 2H, J=5.4Hz), 3.73 (t, 2H, J=5.4Hz), 7.69 (dd, 1H, J=2.7, 8.1Hz), 7.75~7.96 (m, 6H), 8.17~8.22 (m, 1H), 8.24 (d, 1H, J=8.1Hz), 8.37 (broad s, 1H)

Examples 2-22

Synthesis of compound (Vc) [Compound No. (9c)]

In 105 ml of pyridine were dissolved 10.50 g of 3-[(methoxycarbonyl)methoxy]benzaldehyde, 2.97 g of 3-acetyl-4,6-dimethyl-2(1H)-pyridinone and 2.38 g of piperidine, and the solution was heated under reflux for 14 hours. The solvent was distilled off under reduced pressure and the residue was subjected to silica gel column chromatography and then recrystallized from tetrahydrofuran to obtain 364 mg of 3-[3-[3-[(methoxycarbonyl)methoxy]phenyl]-1-oxo-2-propenyl]-4,6-dimethyl-2(1H)-pyridinone [Compound No. (9c)] as a light yellow crystal.

$^1$H-NMR(270MHz,DMSO-$d_6$) δ (ppm): 2.08 (s, 3H), 2.18 (s, 3H), 3.70 (s, 3H), 4.86 (s, 2H), 6.01 (s, 1H), 6.99 (d, 1H, J=7.6Hz), 7.15 (d, 1H, J=15.9Hz), 7.28~7.35 (3H), 7.38 (d, 1H, J=15.9Hz), 11.83 (broad, 1H)

Examples 2-23

Synthesis of compound (Vc) [Compound No. (10c)]

To a solution of 0.40 g of 3-[3-[3-[(methoxycarbonyl)methoxy]phenyl]-1-oxo-2-propenyl]-4,6-dimethyl-2(1H)-pyridinone in 10 ml of methanol was added 5 ml of a 1N aqueous sodium hydroxide solution. The mixture was stirred at room temperature for 30 minutes, and 2 ml of 10% hydrochloric acid was added thereto. Precipitated crystals were filtered and washed with a mixed solvent of methanol and water to obtain 0.27 g of 3-[3-[3-(carboxymethoxy)phenyl]-1-oxo-2-propenyl]-4,6-dimethyl-2(1H)-pyridinone [Compound No. (10c)] as light yellow powder.
$^1$H-NMR(270MHz,DMSO-$d_6$) δ (ppm): 2.08 (s, 3H), 2.20 (s, 3H), 3.55 (broad, 1H), 4.74 (s, 2H), 6.01 (s, 1H), 6.97 (d, 1H, J=8.4Hz), 7.15 (d, 1H, J=15.9Hz), 7.25~7.35 (m, 3H), 7.38 (d, 1H, J=16.2Hz), 11.86 (broad, 1H)

Examples 2-24

Synthesis of compound (Vc) [Compound No. (11c)]

According to the same manner as that of Example 2-22 except that 740 mg of 3-acetyl-6-methyl-2(1H)pyridinone was used in place of 3-acetyl-4,6-dimethyl-2-(1H)-pyridinone, 3-[3-[3-[(methoxycarbonyl)methoxy]phenyl]-1-oxo-2-propenyl]-6-methyl-2(1H)-pyridinone [Compound No. (11c)] was obtained as a light yellow crystal.
$^1$H-NMR(270MHz,DMSO-$d_6$) δ (ppm): 2.27 (s, 3H), 3.72 (s, 3H), 4.86 (s, 2H), 6.24 (d, 1H, J=7.6Hz), 6.98~7.05 (1H), 7.23 (s, 1H), 7.28~7.40 (2H), 7.58 (d, 1H, J=15.9Hz), 8.07 (1H), 8.08 (d, 1H, J=16.5Hz), 12.28 (broad, 1H)

Examples 2-25

Synthesis of compound (Vc) [Compound No. (12c)]

In 30 ml of ethanol were dissolved 1.04 g of 3-[(methoxycarbonyl)methoxy]benzaldehyde and 0.74 g of 3-acetyl-6-methyl-2(1H)-pyridinone, and 10 ml of a 1N aqueous sodium hydroxide solution was added thereto. The mixture was stirred at room temperature for 5 hours and 30 minutes, and 10% hydrochloric acid was added thereto. Precipitated crystals were filtered and washed with water and tetrahydrofuran to obtain 3-[3-[3-(carboxymethoxy)phenyl]-1-oxo-2-propenyl]-6-methyl-2(1H)pyridinone [Compound No. (12c)].
$^1$H-NMR(270MHz,DMSO-$d_6$) δ (ppm): 2.29 (s, 3H), 4.74 (s, 2H), 6.24 (d, 1H, J=7.6Hz), 6.99 (d, 1H, J=7.0Hz), 7.22 (s, 1H), 7.25~7.40 (2H), 7.58 (d, 1H, J=15.9Hz), 8.07 (1H), 8.08 (d, 1H, J=16.5Hz), 12.29 (broad, 1H), 13.03 (broad, 1H)

Examples 2-26

Synthesis of compound (Vc) [Compound No. (13c)]

To a mixture of 0.53 g of 3-[3-[3-[(methoxycarbonyl)methoxy]phenyl]-1-oxo-2-propenyl]-6-methyl-2(1H)-pyridinone and 15 ml of hexamethylphosphoramide was added 71 mg of sodium hydride (60% oily). After the mixture was stirred at room temperature for 1 hour, 0.15 ml of methyl iodide was added thereto. The mixture was stirred at room temperature for 4 hours. The reaction solution was poured into water, followed by extraction with ethyl acetate. The extract was dried over anhydrous sodium sulfate, and then concentrated under reduced pressure. The resulting residue was washed with a mixed solvent of hexane and t-butyl methyl ether to obtain 0.51 g of 3-[3-[3-[(methoxycarbonyl)methoxy]phenyl]-1-oxo-2-propenyl]-1,6-dimethyl-2(1H)-pyridinone [Compound No. (13c)] as a yellow crystal.
$^1$H-NMR(270MHz,DMSO-$d_6$) δ (ppm): 2.49 (s, 3H), 3.53 (s, 3H), 3.72 (s, 3H), 4.86 (s, 2H), 6.38 (d, 1H), 6.99 (d, 1H), 7.24 (s, 1H), 7.29~7.37 (2H), 7.57 (d, 1H), 7.99 (1H), 8.00 (d, 1H)

Examples 2-27

Synthesis of compound (Vc) [Compound No. (14c)]

According to the same manner as that of Example 2-23 except that 0.46 g of 3-[3-[3-[(methoxycarbonyl)methoxy]phenyl]-1-oxo-2-propenyl]-1,6-dimethyl-2(1H)-pyridinone was used in place of 3-[3-[3-[(methoxycarbonyl)methoxy]phenyl]-1-oxo-2-propenyl]-4,6-dimethyl-2(1H)-pyridinone, 3-[3-[3-(carboxymethoxy)phenyl]-1-oxo-2-propenyl]-1,6-methyl-2(1H)-pyridinone [Compound No. (14c)] was obtained.
$^1$H-NMR(270MHz,DMSO-$d_6$) δ (ppm): 2.49 (s, 3H), 3.53 (s, 3H), 4.73 (s, 2H), 6.38 (d, 1H, J=7.6Hz), 6.99 (d, 1H, J=7.6Hz), 7.22 (s, 1H), 7.25~7.40 (2H), 7.57 (d, 1H, J=15.9Hz), 7.97 (d, 1H, J=7.3Hz), 7.99 (d, 1H, J=15.9Hz)

Examples 2-28

Synthesis of compound (Vc) [Compound No. (15c)]

According to the same manner as that of Example 2-24 except that 0.47 g of 3-[[(methoxycarbonylmethyl) amino]carbonyl]benzaldehyde was used in place of 3-[(methoxycarbonyl)methoxy]benzaldehyde, 0.19 g of 3-[3-[3-[[(methoxycarbonylmethyl)amino]carbonyl]phenyl]-1-oxo-2-propenyl]-6-methyl-2(1H)-pyridinone [Compound No. (15c)] was obtained as a light yellow crystal.
$^1$H-NMR(300MHz,DMSO-$d_6$) δ (ppm): 2.30 (s, 3H), 3.70 (s, 3H), 4.35 (2H), 6.25 (d, 1H), 7.48~7.70 (3H), 7.70~8.00 (2H), 8.00~8.22 (3H), 9.11 (1H), 11.93 (1H)

Examples 2-29

Synthesis of compound (Vc) [Compound No. (16c)]

To 50 ml of ethanol were added 5.76 g of 3-(2-hydroxyethoxy)benzaldehyde and 1.94 g of 3-acetyl-6-methyl-2(1H)-pyridinone, and 25 ml of a 1N aqueous sodium hydroxide solution and 15 ml of water were added thereto. The mixture was heated under reflux for 2 hours, extracted with chloroform, and then dried over anhydrous sodium sulfate. The solvent was distilled off under reduced pressure, and the residue was subjected to silica gel column chromatography to obtain 0.66 g of 3-[3-[3-(2-hydorxyethoxy)phenyl]-1-oxo-2-propenyl]-6-methyl-2(1H)-pyridinone [Compound No. (16c)] as a yellow crystal.
$^1$H-NMR(270MHz,DMSO-$d_6$) δ (ppm): 2.29 (s, 3H), 3.70~3.76 (m, 1H), 4.03 (t, 1H, J=4.6Hz), 4.90 (t, 1H, J=5.7Hz), 6.24 (d, 1H, J=7.6Hz), 6.90~7.40 (4H), 7.68 (d, 1H, J=15.9Hz), 8.07 (d, 1H, J=7.3Hz), 8.08 (d, 1H, J=15.6Hz), 12.27 (broad, 1H)

Examples 2-30

Synthesis of compound (Vc) [Compound No. (17c)]

In 7 ml of chloroform were dissolved 0.85 g of 3-acetyl-6-hydroxy-2-methyl-1H-pyridin-4-one, 0.99 g of 3-[(methoxycarbonyl)methoxy]benzaldehyde and 0.35 g of piperidine. The solution was heated under reflux for 1 hour and 30 minutes while water was removed with a Soxhlet's extractor filled with molecular sieves, and then concentrated under reduced pressure. The residue was subjected to silica gel column chromatography, and then recrystallized from toluene to obtain 317 mg of 6-hydroxy-2-methyl-3-[3-[3-[(methoxycarbonyl)methoxy]phenyl]acryloyl]-1H-pyridin-4-one [Compound No. (17c)] as a light yellow crystal.
$^1$H-NMR (270MHz,$CDCl_3$) δ (ppm): 2.02 (s, 3H), 3.82 (s, 3H), 4.66 (s, 2H), 5.19 (broad, 1H), 5.24 (s, 1H), 6.65 (d, 1H, J=15.9Hz), 6.85~6.88 (1H), 7.05~7.35 (3H), 7.45 (d, 1H, J=15.9Hz), 10.22 (broad, 1H)

Examples 2-31

Synthesis of compound (Vc) [Compound No. (18c)]

According to the same manner as that of Example 2-23 except that 6-hydroxy-2-methyl-3-[3-[3-[(methoxycarbonyl)methoxy]phenyl]acryloyl]-1H-pyridin-4-one was used in place of 3-[3-[3-[(methoxycarbonyl)methoxy]phenyl]-1-oxo-2-propenyl]-4,6-dimethyl-2(1H)-pyridinone, 110 mg of 6-hydroxy-2-methyl-3-[3-[3-(carboxymethoxy)phenyl]acryloyl]-1H-pyridin-4-one [Compound No. (18c)] was obtained as a light yellow crystal.
$^1$H-NMR(270 MHz, $CDCl_3$) δ (ppm): 2.02 (s, 3H), 4.62 (s, 2H), 5.21 (s, 1H), 5.99 (broad, 1H), 6.64 (d, 1H, J=15.9Hz), 6.87~6.91 (1H), 7.09 (1H), 7.13~7.36 (2H), 7.42 (d, 1H, J=15.7Hz), 10.20 (broad, 1H)

Examples 2-32

Synthesis of compound (Vc) [Compound No. (19c)]

Four grams of potassium hydroxide, 100 g of ethanol and 60 g of water were mixed to prepare a solution. To 22 g of the solution were added 0.36 g of 3-(cyanomethoxy)benzaldehyde and 0.35 g of 5-acetyl-6-methyl-2-oxo-1,2-dihydropyridine-3-carbonitrile. The mixture was stirred at room temperature for 1 hour, heated under reflux for 3 hours, and then concentrated under reduced pressure. To the residue was added 50 ml of water, followed by extraction with 50 ml of chloroform. An aqueous layer was acidified with 10 ml of 10% hydrochloric acid, and then filtered. The filtered crystal was washed with ethyl acetate to obtain 0.38 g of 5-[3-(3-carboxymethoxyphenyl)acryloyl]-6-methyl-2-oxo-1,2-dihydropyridine-3-carboxylic acid [Compound No. (19c)] as a yellow crystal.
$^1$H-NMR(270MHz,DMSO-$d_6$) δ (ppm): 2.59 (s, 3H), 4.75 (s, 2H), 6.95~7.05 (1H), 7.25~7.50 (3H), 7.50~7.80 (3H), 8.85 (s, 1H)

Examples 2-33

Synthesis of compound (Vc) [Compound No. (20c)]

In 5 ml of chloroform were dissolved 0.53 g of 3-acetylchromen-4-one, 0.50 g of 3-(3-hydroxypropoxy)benzaldehyde and 0.32 ml of piperidine, and the solution was heated under reflux for 4 hours and 40 minutes while water was removed with a Soxhlet's extractor filled with molecular sieves. After 40 ml of water was added thereto, the mixture was extracted with 80 ml of chloroform. An organic layer was washed with an aqueous saturated sodium chloride solution, dried over anhydrous sodium sulfate, and then concentrated under reduced pressure. The residue was subjected to silica gel column chromatography to obtain 107 mg of 3-[3-[3-(3-hydoxypropoxy)phenyl]acryloyl]chromen-4-one [Compound No. (20c)] as a light yellow crystal.
$^1$H-NMR(400MHz,$CDCl_3$) δ (ppm): 1.74 (broad s, 1H), 2.04~2.10 (2H), 3.90 (q, 2H, J=4.9Hz), 4.19 (t, 2H, J=5.9Hz), 6.96 (d, 1H, J=7.3Hz), 7.23-7.33 (m, 3H), 7.50 (t, 1H, J=8.1Hz), 7.54 (d, 1H, J=8.3Hz), 7.74 (dt, 1H, J=1.7, 7.8Hz), 7.80 (d, 1H, J=15.6Hz), 8.06 (d, 1H, J=15.6Hz), 8.33 (dd, 1H, J=1.7, 8.1Hz), 8.70 (s, 1H)

Examples 2-34

Synthesis of compound (Vc) [Compound No. (21c)]

According to the same manner as that of Example 2-30 except that 753 mg of 3-acetylchromen-4-one was used in place of 3-acetyl-6-hydroxy-2-methyl-1H-pyridin-4-one and 645 mg of 3-(cyanomethoxy)benzaldehyde was used in place of 3-[(methoxycarbonyl)methoxy]benzaldehyde, 67.1 mg of 3-[3-[3-(cyanomethoxy)phenyl]acryloyl]chromen-4-one [Compound No. (21c)] was obtained as a yellow crystal.
$^1$H-NMR (400MHz, $CDCl_3$) δ (ppm): 4.82 (s, 2H), 7.02-7.04 (1H), 7.30-7.75 (7H), 7.78 (d, 1H, J=15.9Hz), 8.07 (d, 1H, J=15.9Hz), 8.31 (1H)

Examples 2-35

Synthesis of compound (Vc) [Compound No. (22c)]

According to the same manner as that of Example 2-34 except that 188 mg of 3-(methoxyacetylamino)benzaldehyde was used in place of 3-(cyanomethoxy)benzaldehyde, 118 mg of 3-[3-[3-(methoxyacetylamino)phenyl]acryloyl]]chromen-4-one [Compound No. (22c)] was obtained as a yellow crystal. $^1$H-NMR (400 MHz, $CDCl_3$) δ (ppm): 3.54 (s, 3H), 4.04 (s, 2H), 7.35-7.60 (m, 5H), 7.70~7.85 (m, 4H), 8.06 (d, 1H, J=15.6Hz), 8.34 (1H), 8.770 (1H)

Examples 2-36

Synthesis of compound (Vc) [Compound No. (23c)]

To a solution of 115 mg of 3-[(2-methoxyethyl)aminocarbonyl]benzaldehyde in 3 ml of ethanol were added 104 mg of 3-acetyl-4(1H)-quinolinone and 13.2 mg of piperidine, and the mixture was heated under reflux for 15 hours and 55 minutes. After cooling, the reaction solution was concentrated. The residue was washed with tetrahydrofuran to obtain 13 mg of 3-[3-[3-[(2-methoxyethyl)aminocarbonyl]phenyl]acryloyl]-4(1H)-quinolinone [Compound No. (23c)] as a light brown crystal.
$^1$H-NMR (300MHz, DMSO-$d_6$) δ (ppm): 3.29 (s, 3H), 3.40~3.50 (m, 4H), 7.49 (dt, 1H, J=0.9, 7.8Hz), 7.55 (t, 1H, J=7.8Hz), 7.69 (d, 1H, J=6.3Hz), 7.69 (d, 1H, J=15.9Hz), 7.76 (dt, 1H, J=1.5, 8.4Hz), 7.86 (d, 1H, J=7.8Hz), 7.90 (d, 1H, J=7.8Hz), 8.20 (s, 1H), 8.28 (dd, 1H, J=0.9, 8.1Hz), 8.38 (d, 1H, J=15.9Hz), 8.59 (s, 1H), 8.69 (broad, 1H)

Examples 2-37

Synthesis of compound (Vc) [Compound No. (24c)]

To a solution of 103 mg of 3-[(2-methoxyethyl)aminocarbonyl]benzaldehyde in 10 ml of ethanol were added 104 mg of 3-acetyl-1-methyl-4(1H)-quinolinone and 90 mg of potassium hydroxide, and the mixture was stirred at room temperature overnight. Precipitated crystals were filtered, washed with water, and then dried to obtain 74 mg of 3-[3-[3-[(2-methoxyethyl)aminocarbonyl]phenyl]acryloyl]-1-methyl-4(1H)-quinolinone [Compound No. (24c)] as white powder.
$^1$H-NMR (270MHz, DMSO-$d_6$) δ (ppm): 3.27 (s, 3H), 3.43~3.48 (4H), 4.01 (s, 3H), 7.55 (t, 1H, J=7.6Hz), 7.57 (t, 1H, J=7.3Hz), 7.70 (d, 1H, J=15.7Hz), 7.80 (d, 1H, J=7.8Hz), 7.83~7.95 (3H), 8.21 (s, 1H), 8.37 (d, 1H, J=15.9Hz), 8.37 (d, 1H, J=7.8Hz), 8.68 (1H), 8.78 (s, 1H)

Examples 2-38

Synthesis of compound (Vc) [Compound No. (25c)]

According to the same manner as that of Example 2-36 except that 360 mg of 3-acetyl-2-methyl-4(1H)-quinolinone was used in place of 3-acetyl-4(1H)-quinolinone, 70 mg of 3-[3-[3-[(2-methoxyethyl)aminocarbonyl]phenyl]acryloyl]-2-methyl-4(1H)-quinolinone [Compound No. (25c)] was obtained as white powder.
$^1$H-NMR (270MHz, DMSO-$d_6$) δ (ppm): 2.45 (s, 3H), 3.26 (s, 3H), 3.45~3.55 (4H), 7.35~7.42 (2H), 7.48 (d, 1H, J=18.9Hz), 7.45~7.58 (1H), 7.56 (d, 1H, J=16.2Hz), 7.72 (t, 1H, J=5.4Hz), 7.82 (d, 1H, J=8.1Hz), 7.88 (d, 1H, J=8.1Hz), 8.10~8.15 (1H), 8.15 (s, 1H), 8.63 (broad, 1H), 12.00 (broad s, 1H)

Example 2-39

Synthesis of compound (Ve) [Compound No. (3e)]

According to the same manner as that of Example 2-30 except that 5.65 g of 3-acetylchromen-2-one was used in place of 3-acetyl-6-hydroxy-2-methyl-1-H-pyridin-4-one and 4.22 g of 3-chlorobenzaldehyde was used in place of 3-[(methoxycarbonyl)methoxy]benzaldehyde, 0.30 g of 3-[3-(3-chlorophenyl)acryloyl]chromen-2-one [Compound No. (3e)] was obtained as a light yellow crystal.
$^1$H-NMR (300MHz, $CDCl_3$) δ (ppm): 7.32~7.45 (4H), 7.52~7.58 (1H), 7.64~7.72 (3H), 7.77 (d, 1H, J=15.7Hz), 7.95 (d, 1H, J=15.7Hz), 8.60 (1H)

Example 3 (Preparation of a plasmid having a reporter gene linked to a transcription regulatory region for a Type I collagen gene)

1 x $10^8$ cells of a normal human fetal skin fibroblast (Clontech, catalogue No. CC-2509) were cultured at 37˚C overnight under 5% $CO_2$ atmosphere. After the cultured cells were washed with a sodium phosphate buffer (hereinafter, referred to as PBS) twice, 3 ml of PBS was added thereto and the cells were scraped away the wall of a vessel using a cell scraper (Nalgen, catalogue No. 179693). The scraped cells were collected by centrifugation (1,500 rpm, 4˚C, 15 min), and these were suspended in 20 ml of PBS and centrifuged again. To the resulting precipitates were added 11 ml of Solution 2 and 4.8 μl of pronase of DNA Extraction Kit (Stratagene, catalogue No. 200600). After shaken at 60˚C for 1 hour, the resulting mixture was allowed to stand in ice for 10 minutes. Then, 4 ml of Solution 3 of the kit was added to the mixture. After mixed, the mixture was allowed to stand in ice for 5 minutes and then centrifuged (3,000 rpm, 4˚C, 15 min) to recover a supernatant. To the recovered supernatant was added 2 μl of RNase per 1 ml of the supernatant and the mixture was allowed to stand at 37˚C for 15 minutes. To the mixture was added 2-fold volume of ethanol. After mixed, a white thread-like substance (genomic DNA) appeared and the substance was recovered. The recovered genomic DNA was washed with 70% ethanol and then air-dried. The air-dried genomic DNA was dissolved in 500 μl of 10 mM Tris-HCl, 1 mM EDTA (pH 8.0) (hereinafter, referred to as TE).

The resulting genomic DNA-dissolved solution (genomic DNA 1 μg corresponding amount) was mixed with each 1 μl of an oligonucleotide consisting of a base sequence represented by SEQ ID No.: 1 (SEQ ID No. 1: an oligonucleotide primer designed to amplify a collagen promoter DNA: ccaagctagc gaaattatct tttctttcat ag 32) and an oligonucleotide (10 pmol/μl) consisting of a base sequence represented by SEQ ID No. :2 (SEQ ID No. 2: an oligonucleotide primer designed to amplify a collagen promoter DNA: ccaaaagctt gcagtcgtgg ccagtacc 28), 29 μl of distilled water, 5 μl of a buffer attached to TaKaRa LA Taq (TAKARA SHUZO Co., Ltd., catalogue No. RR002A), 5 μl of a $Mg^{2+}$ solution, 5 μl of a dNTP mixture and 0.5 μl of TaKaRa LA Taq (TAKARA SHUZO Co., Ltd., catalogue No. RR002A). After the resulting mixed solution was incubated at 94˚C for 5 minutes, the mixed solution was subjected to 30 cycles,

in which one cycle consists of incubation at 94˚C for 1 minute, at 60˚C for 1 minute and then at 72˚C for 1 minute. The mixed solution was electrophoresed on a 2% agarose gel to recover about 0.5 kb of a DNA. The recovered DNA was treated with phenol/chloroform and then precipitated with ethanol to recover the DNA. The resulting DNA was dissolved in ultrapure water. To this solution were added 2.5 μl of NheI and 2.5 μl of HindIII, and then incubated at 37˚C for 3 hours. Then, the solution was electrophoresed on a 2% agarose gel to recover about 3.5 kb of a DNA. The recovered DNA was precipitated with ethanol to recover again the DNA (hereinafter, referred to as the collagen promoter DNA).

On the other hand, the vector pGL3 (Promega, catalogue No. E1751) having the nucleotide sequence encoding firefly luciferase was digested with NheI and HindIII, and then subjected to agarose gel electrophoresis as described above to recover about 5kb of a DNA. The recovered DNA was precipitated with ethanol to recover the DNA again. To the recovered DNA were added 44 μl of distilled water, 5 μl of Buffer attached to Alkaline Phosphatase (TAKARA SHUZO, catalogue No. 2120A) and 1 μl of Alkaline Phosphatase (TAKARA SHUZO, catalogue No. 2120A). The mixed solution was incubated at 65˚C for 30 minutes. Then, the mixed solution was treated with phenol/chloroform twice, and precipitated with ethanol to recover the DNA (hereinafter referred to as the Luc vector DNA). Then, after about 20 ng of the collagen promoter DNA and about 20 ng of the Luc vector DNA were mixed, the same amount of a DNA Ligation kit Ver2 enzyme solution was added and this was incubated for 1 day at 16˚C. To the mixed solution was added Escherichia coli 5Hdα (TOYOBO, catalogue No. DNA-903), this was allowed to stand in ice for 30 minutes, and then incubated at 42˚C for 45 seconds. The resulting Escherichia coli was seeded on a LB plate containing 50 μg/ml ampicillin sodium (Nacalai, catalogue No. 027-39), and this was allowed to stand at 37˚C for 1 day. A single colony appeared and the colony was cultured in 2 ml of a LB medium containing 50 μg/ml ampicillin at 37˚C for 12 hours. From the resulting culture solution, a plasmid DNA was prepared using AUTOMATIC DNA ISOLATION SYSTEM PI-50 (KURABO). The nucleotide sequence of the prepared plasmid DNA was analyzed with a DNA sequencer. As a result, it was confirmed that the plasmid (hereinafter, referred to as COL-Luc) had a nucleotide sequence comprising a nucleotide sequence encoding the amino acid sequence of firefly luciferase as a reporter gene linked downstream of the nucleotide sequence -3500 to +57 (the transcription initiation point is +1) of a transcription regulatory region for a human-derived Type I collagen α2 chain gene.

Example 4 (Measurement of the ability of a test compound to regulate transcription of a Type I collagen gene using the expression level of a report gene as an index)

1 x $10^6$ cells of a normal human fetal skin fibroblast were seeded on a 100 mm dish and cultured at 37˚C overnight under 5% $CO_2$ atmosphere in a Dulbecco's-MEM (Nissui Seiyaku, catalogue No. 05919) medium containing 10(v/v)% heat-inactivated bovine fetal serum (hereinafter, referred to as FBS; Gibco, catalogue No. 21140-079) (hereinafter, this medium is referred to as D-MEM(+)). Then, the medium was replaced with a Dulbecco's-MEM medium not containing FBS (hereinafter, this medium is referred to as D-MEM(-)).

To 300 μl of D-MEM(-) were added 5 μg of COL-Luc and 5 μg of pCMV-β-gal (Invitrogen, catalogue No. 10586-014), and the resulting mixed solution was allowed to stand at room temperature for 5 minutes (solution 1). To 300 μl of D-MEM(-) was added 20 μl of Lipofectine (Gibco, catalogue No. 18292-011), and the resulting mixed solution was allowed to stand at room temperature for 45 minutes (solution 2). Then, the solution 1 and the solution 2 were mixed. After the mixture was allowed to stand at room temperature for 10 minutes, 5.4 ml of D-MEM(-) was added to thereto, followed by mixing. The mixed solution was added to the normal human fetal skin fibroblasts, and the cells were cultured at 37˚C under 5% $CO_2$ atmosphere. After 6 hours, the culture supernatant was removed from the dish, and the cells were washed with PBS twice. To the dish was added 1 ml of PBS containing 0.25% trypsin, and the cells were scraped off the dish. To the scraped cells was added D-MEM(+), and these were mixed well. The mixture was dispensed into a 12-well plate at 1 ml per well, and the plate was incubated at 37˚C overnight under 5% $CO_2$ atmosphere. On the next day, each well was washed with D-MEM(-) twice, and this was replaced with 1 ml of a Dulbecco's-MEM medium containing 0.1% FBS (hereinafter, this medium is referred to as D-MEM (0.1%)).

To the thus cultured cells was added 10 μL of a 1 mM solution of the present compound represented by the compound number (20a), (56a), (124a), (11c) or (16c) in 10% dimethyl sulfoxide (hereinafter, referred to as DMSO) (the present compound final concentration: 10 μM, DMSO final concentration: 0.1%). As a control, only 10 μl of DMSO was added. In addition, to the cultured cells was added 10 μL of a 0.3 mM solution of the present compound represented by the compound number (19a), (29a), (31a), (32a), (36a), (40a), (42a), (1b), (10b), (13b) or (24c) in 10% DMSO (the present compound final concentration: 3 μM, DMSO final concentration: 0.1%). In addition, to the cultured cells was added 10 μL of a 0.1 mM solution of the present compound represented by the compound number (5b), (16b) or (23c) in 10% DMSO (the present compound final concentration: 1 μM, DMSO final concentration: 0.1%).

After one hour, 10 μl of a 0.5 μg/ml aqueous solution of TGF-β (Pepro Tech) or distilled water was added to the well, and the plate was further incubated at 37˚C for 40 hours under 5% $CO_2$ atmosphere. After the incubated cells were washed with PBS twice, 200 μl of a cell lysing agent (Toyo Inc., catalogue No. PD10) was added thereto and the cells were scraped. The scraped cells were recovered as a cell suspension, and the suspension was centrifuged (15,000

rpm, 4°C, 5 min) to recover a supernatant. The recovered supernatant was transferred to a 96-well plate at 50 μl per well, and then 50 μl of a Luc assay solution (20 mM Tricine (pH 7.8), 2.67 mM $MgSO_4$, 0.1 mM EDTA, 33.3 mM DTT, 270 μM Coenzyme A, 530 μM ATP, 470 μM Luciferin) was automatically dispensed into the plate using MICROLUMAT LB96P (manufactured by EG&G BERTHOLD). Luminescence in each well was measured (Delay: 1.6 second, Meas. Interval: 20 second).

On the other hand, 50 μl of the recovered supernatant or the cell lysing agent was added to 50 μl of a β-gal substrate solution (5.8 mM o-nitrophenyl-beta-D-galactopyranoside, 1 mM $MgCl_2$, 45 mM 2-mercaptoethanol) which had been dispensed into a 96-well plate in advance, and the plate was incubated at 37°C for 2 hours. Then, an absorbance in each well was measured using a microplate reader at 420 nm. Based on the value obtained, transcription activity was calculated according to the following equation.

Transcription activity = [luminescence amount (supernatant-added section) - luminescence amount (cell lysing agent-added section)] / [420 nm absorbance (supernatant-added section) - 420 nm absorbance (cell lysing agent-added section)]

Then, based on the calculated transcription activity, an inhibitory effect of a test compound on the ability of TGF-β to promote transcription of a Type I collagen gene was calculated as an inhibition percentage according to the following equation:

Inhibition percentage = [transcription activity (DMSO and TGF-β-added test section) - transcription activity (compound and TGF-β-added test section)] / [transcription activity (DMSO and TGF-β-added test section) - transcription activity (DMSO and TGF-β non-added test section)] x 100

The inhibition percentages of the present compounds represented by the compound numbers (20a), (56a), (124a), (11c) and (16c) at the present compound final concentration of 10 μM, the present compounds represented by the compound numbers (19a), (29a), (31a), (32a), (36a), (40a), (42a), (1b), (10b), (13b) and (24c) at the present compound final concentration of 3 μM, and the present compounds represented by the compound numbers (5b), (16b) and (23c) at the present compound final concentration of 1 μM were 70 or more. It was found that the present compounds can inhibit the ability of TGF-β to promote transcription of a Type I collagen gene, and then can suppress transcription of a Type I collagen gene.

Example 5 (Measurement of the ability of a test compound to suppress TGF-β using the expression level of fibronectin as an index)

5 x $10^3$ cells/well of a normal human skin fibroblast (Clonetics, catalogue No. CC-2509) were seeded on a 96-well plate (BECTON DICKINSON, catalogue No. 35-3075), and then cultured in an incubator at 37°C and 5% $CO_2$ overnight. On the next day, the medium was exchanged with 0.1 ml of a D-MEM medium (Nissui Pharmaceutical Co., Ltd., code No. 05919) containing 0.1% bovine fetal serum. After 1 hour, to the well was added the present compound represented by the compound number (31a), (10b) or (13c) at the final concentration of 10 μM. After the cells were

cultured for 1 hour, 5 ng/ml (final concentration) of TGF-β (Peprotech, catalogue No.100-21R) was added, followed by further culturing for 26 hours. After the cells were washed with a phosphate buffer twice, a total RNA was isolated using SV96 Total RNA Isolation System (Promega, catalogue No. Z3505). To 5 ml of the isolated total RNA were added 1 μl of 20 μM oligo dT and 4 μl of RNase-free distilled water, incubated at 65˚C for 5 minutes, and immediately cooled with an ice. To 10 μl of the solution were added 4 μl of 5 x buffer, 2.4 μl of $MgCl_2$, 1 μl of 10 mM dNTP, 1 μl of RNasin, 1 μl of Improm II and 0.6 μl of RNase-free distilled water (these are all available from Promega), and the mixture was subjected to a reverse transcription reaction under the condition of 25˚C for 5 minutes, 42˚C for 1 hour and 70˚C for 15 minutes.

To 5 μl of a reverse transcription reaction solution were added each 1 μl of each 20 pmol/μl of primers represented by SEQ ID No. 3 (SEQ ID No. 3: an oligonucleotide designed as a PCR primer in order to detect a DNA of a fibronectin gene: tcgccatcag tagaaggtag ca 22) and SEQ ID No. 4 (SEQ ID No. 4: an oligonucleotide designed as a PCR primer in order to detect a DNA of a fibronectin gene: tatactgaac accaggttgc aagtc 25), and 1.25 μL of a probe for detecting a DNA of a fibronectin gene represented by SEQ ID No. 5 (SEQ ID NO. 5: an oligonucleotide designed as a probe in order to detect a DNA of a fibronectin gene: ctcaaccttc ctgaaactgc aaactccgtc 30) or 1.25 μl of Human GAPDH primer probe (Applied Biosystems, catalogue No. 4310884E). Further, 12.5 μl of TaqMan Universal PCR Master Mix (Applied Biosystems, catalogue No. 4304437) was added, and the mixture was adjusted to 50 μl with sterilized water, and then mixed in a well of Optical 96-Well Reaction Plate (Applied Biosystems, catalogue No. N801-0560). As a standard, a total RNA was prepared from the cells to which only TGF-β had been added, and 250, 125, 62.5, 31.25, 15.625 or 7.8125 ng of the total RNA was subjected to a reverse transcription reaction to obtain a cDNA solution. Then, PCR was performed under the condition of 1 cycle of 50˚C for 5 minutes and 40 cycles of 95˚C for 15 seconds and 60˚C for 1 minute using Gene Amp 7900 (Applied Biosystems). For quantitation, respective standard lines for fibronectin and GAPDH were made. Then the fibronectin amount and the GAPDH amount were calculated, and the transcription amount was calculated according to the following equation. Fibronectin transcription amount = fibronectin amount/GAPDH amount

Inhibition percentage = [transcription amount (DMSO and TGF-β-added test section) - transcription amount (compound and TGF-β-added test section)]/[transcription amount (DMSO and TGF-β-added test section) - transcription amount (DMSO and TGF-β non-added test section)] x 100

The inhibition percentages of the present compounds represented by the compound numbers (31a), (10b) and (13c) were 70% or more.

It was confirmed that the present compound suppresses the transcription amount of a fibronectin gene of a skin fibroblast promoted by TGF-β.

Industrial applicability

According to the present invention, it is possible to develop and provide a composition which decreases expression of an extracellular matrix gene in a tissue to induce a reduction in accumulation of an extracellular matrix and thereby improves tissue fibrosis (i.e. an extracellular matrix accumulation-suppressing agent, a fibrosing disease-treating agent, or heart failure treating agent).

Sequence Listing Free Text

SEQ ID NO: 1

Oligonucleotide primer designed for amplifying a collagen promoter DNA

SEQ ID NO: 2

Oligonucleotide primer designed for amplifying a collagen promoter DNA

SEQ ID NO: 3

Oligonucleotide primer designed for detecting a fibronectin DNA

SEQ ID NO: 4

Oligonucleotide primer designed for detecting a fibronectin DNA

SEQ ID NO: 5

Oligonucleotide primer designed for detecting a fibronectin DNA

## Claims

1. A composition for suppressing transcription of an extracellular matrix gene which comprises an inert carrier and a cinnamoyl compound represented by the formula (I):

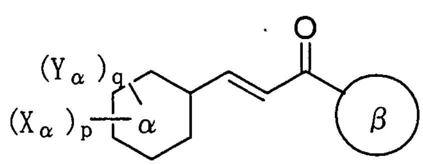

(I)

wherein,

I. α represents a benzene ring or a pyridine ring; in $(Y_\alpha)_q$, $Y_\alpha$ is a substituent on a carbon atom and represents a group included in the following $X_0$ group or the $Y_0$ group, q represents 0, 1, 2, 3, 4 or 5, and when q is not less than 2, $Y_\alpha$s are the same or different, and when q is not less than 2, adjacent two same or different $Y_\alpha$s may together form a group included in the $Z_0$ group to be fused to the α ring; and in $(X_\alpha)_p$, $X_\alpha$ is a substituent on a carbon atom and represents a group which does not belong to the following $X_0$ group, $Y_0$ group and $Z_0$ group, p represents 0, 1, 2, 3, 4 or 5, and when p is not less than 2, $X_\alpha$s are the same or different and the sum of p and q is not more than 5;

(1) the $X_0$ group: a $M_a$-group, wherein $M_a$ represents a $R_b$- group (wherein $R_b$ represents a C1-C10 alkyl group optionally substituted with a halogen atom), a halogen atom, a nitro group, a cyano group, a hydroxyl

group, a $R_c$-$B_a$-$R_d$-group (wherein $R_c$ represents a C1-C10 alkyl group optionally substituted with a halogen atom, $B_a$ represents an oxy group, a thio group, a sulfinyl group or a sulfonyl group, and $R_d$ represents a single bond or a C1-C10 alkylene group), an $HOR_d$- group (wherein $R_d$ is as defined above), a $R_e$-CO-$R_d$- group (wherein $R_e$ represents a hydrogen atom, or a C1-C10 alkyl group optionally substituted with a halogen atom, and $R_d$ is as defined above), a $R_e$-CO-O-$R_d$- group (wherein $R_e$ and $R_d$ are as defined above), a $R_e$O-CO-$R_d$- group (wherein $R_e$ and $R_d$ are as defined above), an HO-CO-CH=CH-group, a $R_eR_e$'N-$R_d$- group (wherein $R_e$ and $R_e$' are the same or different, $R_e$ is as defined above, $R_e$' has the same meaning as $R_e$ has, and $R_d$ is as defined above), a $R_e$-CO-N$R_e$'-$R_d$- group (wherein $R_e$, $R_e$' and $R_d$ are as defined above), a $R_b$O-CO-N($R_e$)-$R_d$- group (wherein $R_b$, $R_e$ and $R_d$ are as defined above), a $R_eR_e$'N-CO-$R_d$- group (wherein $R_e$, $R_e$' and $R_d$ are as defined above), a $R_eR_e$'N-CO-N$R_e$"-$R_d$- group (wherein $R_e$, $R_e$' and $R_e$" are the same or different, $R_e$ and $R_e$' are as defined above, $R_e$" has the same meaning as $R_e$ has, and $R_d$ is as defined above), a $R_eR_e$'N-C(=N$R_e$")-N$R_e$"'-$R_d$- group (wherein $R_e$, $R_e$', $R_e$" and $R_e$"' are the same or different, $R_e$, $R_e$' and $R_e$" are as defined above, $R_e$"' has the same meaning as $R_e$ has, and $R_d$ is as defined above), a $R_b$-$SO_2$-N$R_e$-$R_d$- group (wherein $R_b$, $R_e$ and $R_d$ are as defined above), a $R_eR_e$'N-$SO_2$-$R_d$- group (wherein $R_e$, $R_e$' and $R_d$ are as defined above), a C2-C10 alkenyl group or a C2-C10 alkynyl group;

(2) the $Y_0$ group: a $M_{b0}$-$R_d$- group, wherein $M_{b0}$ represents a $M_{c0}$- group

[wherein $M_{co}$ represents a $M_{do}$-$R_d$'- group {wherein $M_{do}$ represents a 6 to 10-membered aryl group optionally substituted with a $M_a$- group (wherein $M_a$ is as defined above), a 5 to 10-membered heteroaryl group optionally substituted with a $M_a$-group (wherein $M_a$ is as defined above), a 3 to 10-membered cyclic hydrocarbon or heterocyclic group optionally substituted with a $M_a$- group (wherein $M_a$ is as defined above) and optionally containing an unsaturated bond, a ($b_0$)- group represented by

($b_0$) 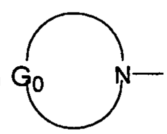

(wherein Go forms an optionally substituted, saturated or unsaturated, nonaromatic 5 to 14-membered hydrocarbon ring or heterocyclic ring), a ($c_0$)- group represented by

($c_0$) 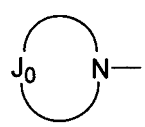

(wherein, $J_0$ forms a 5 to 7-membered aromatic ring optionally containing a nitrogen atom), a (do)- group represented by

($d_0$) 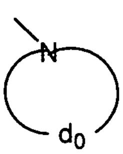

[wherein do forms a 5 to 12-membered hydrocarbon ring which is substituted with a carbonyl group or a thiocarbonyl group and further which may be optionally substituted with an oxy group, a thio group, a -$NR_1$- group {wherein $R_1$ represents a hydrogen atom, or a C1-C10 alkyl group, or a C2-C10 alkyl group substituted with a halogen atom or a $R_2$-$B_1$- group (wherein $R_2$ represents a C1-C10 alkyl group, a C3-C10 alkenyl group or a C3-C10 alkynyl group, and $B_1$ represents an oxy group, a thio group, a sulfinyl group or a sulfonyl group), a C3-C10 alkenyl group, or a C3-C10 alkynyl group}, a sulfinyl group, or a sulfonyl group], or a ($e_0$)- group represented by

($e_0$) 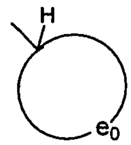

{wherein $e_0$ forms a 5 to 12-membered hydrocarbon ring optionally substituted with a carbonyl group, a thiocarbonyl group, an oxy group, a thio group, a -$NR_1$-group (wherein $R_1$ is as defined above), a sulfinyl group or a sulfonyl group}; and $R_d$' is the same as or different from $R_d$ and has the same meaning as $R_d$ has],

a $M_{c0}$-$B_a$- group (wherein $M_{c0}$ and $B_a$ are as defined above), a $M_{c0}$-CO- group (wherein $M_{c0}$ is as defined above), a $M_{c0}$-CO-O-group (wherein $M_{c0}$ is as defined above), a $M_{c0}$O-CO- group (wherein $M_{c0}$ is as defined above), a $M_{c0}R_eN$- group (wherein $M_{c0}$ and $R_e$ are as defined above), a $M_{c0}$-CO-$NR_e$- group (wherein $M_{c0}$ and $R_e$ are as defined above), a $M_{c0}$O-CO-$NR_e$-group (wherein $M_{c0}$ and $R_e$ are as defined above), a $M_{c0}R_eN$-CO-group (wherein $M_{c0}$ and $R_e$ are as defined above), a $M_{c0}R_eN$-CO-$NR_e$'- group (wherein $M_{c0}$, $R_e$ and $R_e$' are as defined above), a $M_{c0}R_eN$-C(=$NR_e$')-$NR_e$"- group (wherein $M_{c0}$, $R_e$, $R_e$' and $R_e$" are as defined above), a $M_{c0}$-$SO_2$-$NR_e$- group (wherein $M_{c0}$ and $R_e$ are as defined above) or a $M_{c0}R_eN$-$SO_2$- group (wherein $M_{c0}$ and $R_e$ are as defined above), and $R_d$ is as defined above;

(3) the $Z_0$ group: a 5- to 12-membered hydrocarbon ring or heterocyclic ring which may be substituted with a halogen atom, a C1-C10 alkoxy group, C3-C10 alkenyloxy group, a C3-C10 alkynyloxy group, a carbonyl group, a thiocarbonyl group, an oxy group, a thio group, a sulfinyl group or a sulfonyl group, and which is aromatic or nonaromatic and monocyclic or fused ring and is fused to the α ring; and

II. β represents
a group represented by formula (I-1):

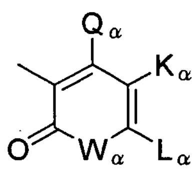 (I-1)

wherein,

(1)$Q_\alpha$ represents an optionally substituted hydroxyl group, or an optionally substituted amino group,
(2)$W_\alpha$ represents an oxygen atom or a -$NT_\alpha$- group (wherein $T_\alpha$ represents a hydrogen atom, or a substituent on the nitrogen atom),
(3)$K_\alpha$ and $L_\alpha$ form a -$V_\alpha$=$V_\alpha$'-$V_\alpha$"=$V_\alpha$"'- group (wherein $V_\alpha$, $V_\alpha$', $V_\alpha$" and $V_\alpha$"' are the same or different, and represent an optionally substituted methine group or a -N= group, and at least one of $V_\alpha$, $V_\alpha$', $V_\alpha$" and $V_\alpha$"' represents a -N= group);
a group represented by formula (I-2):

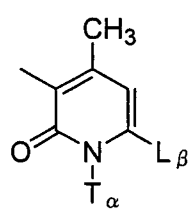 (I-2)

wherein $T_\alpha$ is as defined above, and $L_\beta$ represents a hydroxyl group or a methyl group;
a group represented by formula (I-3):

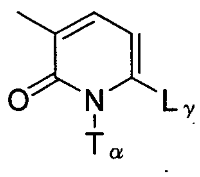

(I-3)

wherein $T_\alpha$ is as defined above, and $L_\gamma$ represents a C1-C10 alkyl group;
a group represented by formula (I-4):

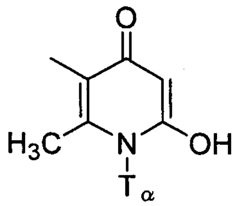

(I-4)

wherein $T_\alpha$ is as defined above;
a group represented by formula (I-5):

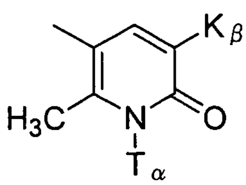

(I-5)

wherein $T_\alpha$ is as defined above, and $K_\beta$ represents a cyano group or a UOCO- group (wherein U represents a hydrogen atom or a C1-C10 alkyl group);
a group represented by formula (I-6):

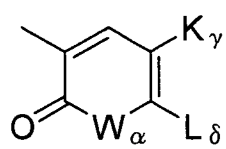

(I-6)

wherein $W_\alpha$ is as defined above, and $K_\gamma$ and $L_\delta$ form an optionally substituted C3-C10 alkylene group or an optionally substituted C4-C10 alkenylene group;
a group represented by formula (I-7):

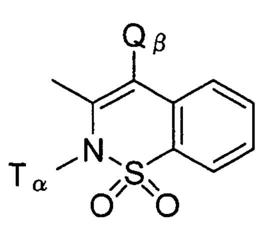

(I-7)

wherein $T_\alpha$ is as defined above, and $Q_\beta$ represents an optionally substituted hydroxyl group; or
a group represented by formula (1-8):

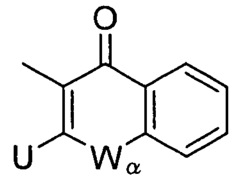

(I-8)

wherein U and $W_\alpha$ are as defined above;
the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range.

**2.** A cinnamoyl compound represented by the formula (II):

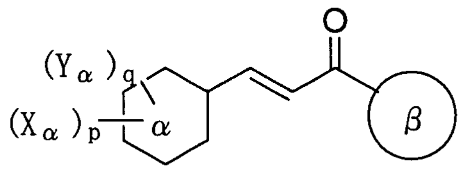

(II)

wherein,

I. $\alpha$ represents a benzene ring or a pyridine ring; in $(Y_\alpha)_q$, $Y_\alpha$ is a substituent on a carbon atom and represents a group included in the following $X_0$ group or the $Y_0$ group, q represents 0, 1, 2, 3, 4 or 5, and when q is not less than 2, $Y_\alpha$s are the same or different, and when q is not less than 2, adjacent two same or different $Y_\alpha$s may together form a group included in the $Z_0$ group to be fused to the $\alpha$ ring; and in $(X_\alpha)_p$, $X_\alpha$ is a substituent on a carbon atom and represents a group which does not belong to the following $X_0$ group, $Y_0$ group and $Z_0$ group, p represents 0, 1, 2, 3, 4 or 5, and when p is not less than 2, $X_\alpha$s are the same or different and the sum of p and q is not more than 5;

(1) the $X_0$ group: a $M_a$-group, wherein $M_a$ represents a $R_b$- group (wherein $R_b$ represents a C1-C10 alkyl group optionally substituted with a halogen atom), a halogen atom, a nitro group, a cyano group, a hydroxyl group, a $R_c$-$B_a$-$R_d$-group (wherein $R_c$ represents a C1-C10 alkyl group optionally substituted with a halogen atom, $B_a$ represents an oxy group, a thio group, a sulfinyl group or a sulfonyl group, and $R_d$ represents a single bond or a C1-C10 alkylene group), an $HOR_d$- group (wherein $R_d$ is as defined above), a $R_e$-CO-$R_d$-group (wherein $R_e$ represents a hydrogen atom, or a C1-C10 alkyl group optionally substituted with a halogen atom, and $R_d$ is as defined above), a $R_e$-CO-O-$R_d$- group (wherein $R_e$ and $R_d$ are as defined above), a $R_e$O-CO-$R_d$- group (wherein $R_e$ and $R_d$ are as defined above), an HO-CO-CH=CH-group, a $R_eR_e$'N-$R_d$- group (wherein $R_e$ and $R_e$' are the same or different, $R_e$ is as defined above, $R_e$' has the same meaning as $R_e$ has, and $R_d$ is as defined above), a $R_e$-CO-N$R_e$'-$R_d$- group (wherein $R_e$, $R_e$' and $R_d$ are as defined above), a $R_b$O-CO-N($R_e$)-$R_d$- group (wherein $R_b$, $R_e$ and $R_d$ are as defined above), a $R_eR_e$'N-CO-$R_d$- group (wherein $R_e$, $R_e$' and $R_d$ are as defined above), a $R_eR_e$'N-CO-N$R_e$"-$R_d$- group (wherein $R_e$, $R_e$' and $R_e$" are the same or different, $R_e$ and $R_e$' are as defined above, $R_e$" has the same meaning as $R_e$ has, and $R_d$ is as defined above), a $R_eR_e$'N-C(=N$R_e$")-N$R_e$"'-$R_d$- group (wherein $R_e$, $R_e$', $R_e$" and $R_e$"' are the same or different, $R_e$, $R_e$' and $R_e$" are as defined above, $R_e$"' has the same meaning as $R_e$ has, and $R_d$ is as defined above), a $R_b$-$SO_2$-N$R_e$-$R_d$- group (wherein $R_b$, $R_e$ and $R_d$ are as defined above), a $R_eR_e$'N-$SO_2$-$R_d$- group (wherein $R_e$, $R_e$' and $R_d$ are as defined above), a C2-C10 alkenyl group or a C2-C10 alkynyl group;
(2) the $Y_0$ group: a $M_{b0}$-$R_d$- group, wherein $M_{b0}$ represents a $M_{c0}$- group
[wherein $M_{co}$ represents a $M_{do}$-$R_d$' - group {wherein $M_{do}$ represents a 6 to 10-membered aryl group optionally substituted with a $M_a$- group (wherein $M_a$ is as defined above), a 5 to 10-membered heteroaryl group optionally substituted with a $M_a$-group (wherein $M_a$ is as defined above), a 3 to 10-membered cyclic hydrocarbon or heterocyclic group optionally substituted with a $M_a$- group (wherein $M_a$ is as defined above) and optionally containing an unsaturated bond, a ($b_0$)- group represented by

$(b_0)$ 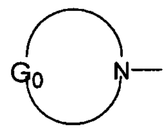

(wherein Go forms an optionally substituted, saturated or unsaturated, nonaromatic 5 to 14-membered hydrocarbon ring or heterocyclic ring), a $(c_0)$- group represented by

$(c_0)$ 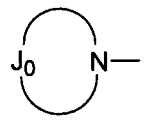

(wherein, $J_0$ forms a 5 to 7-membered aromatic ring optionally containing a nitrogen atom), a (do)- group represented by

$(d_0)$ 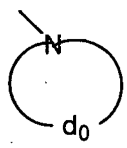

[wherein do forms a 5 to 12-membered hydrocarbon ring which is substituted with a carbonyl group or a thiocarbonyl group and further which may be optionally substituted with an oxy group, a thio group, a $-NR_1$- group {wherein $R_1$ represents a hydrogen atom, or a C1-C10 alkyl group, or a C2-C10 alkyl group substituted with a halogen atom or a $R_2$-$B_1$- group (wherein $R_2$ represents a C1-C10 alkyl group, a C3-C10 alkenyl group or a C3-C10 alkynyl group, and $B_1$ represents an oxy group, a thio group, a sulfinyl group or a sulfonyl group), a C3-C10 alkenyl group, or a C3-C10 alkynyl group}, a sulfinyl group, or a sulfonyl group], or a $(e_0)$- group represented by

$(e_0)$ 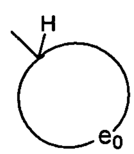

{wherein $e_0$ forms a 5 to 12-membered hydrocarbon ring optionally substituted with a carbonyl group, a thiocarbonyl group, an oxy group, a thio group, a $-NR_1$-group (wherein $R_1$ is as defined above), a sulfinyl group or a sulfonyl group}; and $R_d$' is the same as or different from $R_d$ and has the same meaning as $R_d$ has],
a $M_{c0}$-$B_a$- group (wherein $M_{c0}$ and $B_a$ are as defined above), a $M_{c0}$-CO- group (wherein $M_{c0}$ is as defined above), a $M_{c0}$-CO-O-group (wherein $M_{c0}$ is as defined above), a $M_{c0}$O-CO- group (wherein $M_{c0}$ is as defined above), a $M_{c0}R_eN$- group (wherein $M_{c0}$ and $R_e$ are as defined above), a $M_{c0}$-CO-$NR_e$- group (wherein $M_{c0}$ and $R_e$ are as defined above), a $M_{c0}$O-CO-$NR_e$-group (wherein $M_{c0}$ and $R_e$ are as defined above), a $M_{c0}R_eN$-CO-group (wherein $M_{c0}$ and $R_e$ are as defined above), a $M_{c0}R_eN$-CO-$NR_e$'- group (wherein $M_{c0}$, $R_e$ and $R_e$' are as defined above), a $M_{c0}R_eN$-C(=NRe')-$NR_e$''- group (wherein $M_{c0}$, $R_e$, $R_e$' and $R_e$'' are as defined above), a $M_{c0}$-$SO_2$-$NR_e$- group (wherein $M_{c0}$ and $R_e$ are as defined above) or a $M_{c0}R_eN$-$SO_2$- group (wherein $M_{c0}$ and $R_e$ are as defined above), and $R_d$ is as defined above;
(3) the $Z_0$ group: a 5- to 12-membered hydrocarbon ring or heterocyclic ring which may be substituted with a halogen atom, a C1-C10 alkoxy group, C3-C10 alkenyloxy group, a C3-C10 alkynyloxy group, a carbonyl group, a thiocarbonyl group, an oxy group, a thio group, a sulfinyl group or a sulfonyl group, and which is aromatic or nonaromatic and monocyclic or fused ring and is fused to the α ring; and

II. β represents
a group represented by formula (II-1):

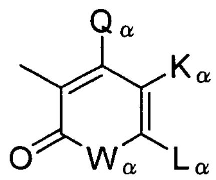

(II-1)

wherein,

(1)$Q_\alpha$ represents an optionally substituted hydroxyl group, or an optionally substituted amino group,
(2)$W_\alpha$ represents an oxygen atom or a -$NT_\alpha$- group (wherein $T_\alpha$ represents a hydrogen atom, or a substituent on the nitrogen atom),
(3)$K_\alpha$ and $L_\alpha$ form a -$V_\alpha$=$V_\alpha$'-$V_\alpha$"=$V_\alpha$"'- group (wherein $V_\alpha$, $V_\alpha$', $V_\alpha$" and $V_\alpha$"' are the same or different, and represent an optionally substituted methine group or a -N= group, and at least one of $V_\alpha$, $V_\alpha$', $V_\alpha$" and $V_\alpha$"' represents a -N= group);
a group represented by formula (II-2):

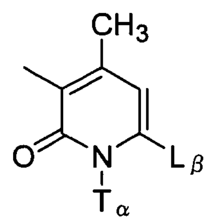

(II-2)

wherein $T_\alpha$ is as defined above, and $L_\beta$ represents a hydroxyl group or a methyl group;
a group represented by formula (II-3):

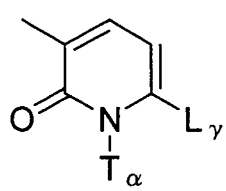

(II-3)

wherein $T_\alpha$ is as defined above, and $L_\gamma$ represents a C1-C10 alkyl group;
a group represented by formula (II-4) :

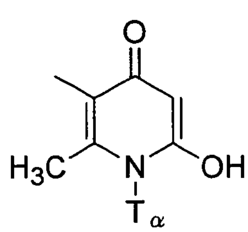

(II-4)

wherein $T_\alpha$ is as defined above;
a group represented by formula (II-5):

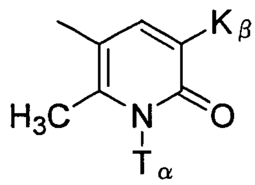

(II-5)

wherein $T_\alpha$ is as defined above, and $K_\beta$ represents a cyano group or a UOCO- group (wherein U represents a hydrogen atom or a C1-C10 alkyl group);
a group represented by formula (II-6):

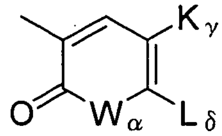

(II-6)

wherein $W_\alpha$ is as defined above, and $K_\gamma$ and $L_\delta$ form an optionally substituted C3-C10 alkylene group or an optionally substituted C4-C10 alkenylene group;
a group represented by formula (II-7):

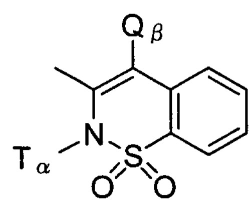

(II-7)

wherein $T_\alpha$ is as defined above, and $Q_\beta$ represents an optionally substituted hydroxyl group; or
a group represented by formula (II-8):

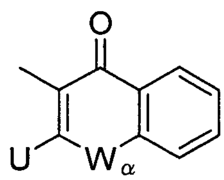

(II-8)

wherein U and $W_\alpha$ are as defined above;
the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range.

**3.** A cinnamoyl compound represented by the formula (III):

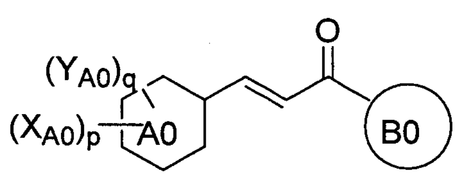

(III)

wherein

I. A0 represents a benzene ring or pyridine ring;
II. in $(X_{A0})_p$, $X_{A0}$ is a substituent on a carbon atom and represents a group included in any group of the following $A_0$ group to the No group, p represents 0, 1, 2, 3, 4 or 5, and when p is not lees than 2, $X_{A0}$s are the same or different;

(1) the $A_0$ group:

a $D_1$-$R_4$- group, wherein $D_1$ represnts a $(R_1$-$(O)_k$-$)A_1N$-$(O)_{k'}$- group [wherein $R_1$ represents a hydrogen atom, or a C1-C10 alkyl group, or a C2-C10 alkyl group substituted with a halogen atom or a $R_2$-$B_1$-group (wherein $R_2$ represents a C1-C10 alkyl group, a C3-C10 alkenyl group or a C3-C10 alkynyl group, and $B_1$ represents an oxy group, a thio group, a sulfinyl group or a sulfonyl group), or a C3-C10 alkenyl group, or a C3-C10 alkynyl group, k represents 0 or 1, $A_1$ represents a $R_3$-$(CHR_0)_m$-$(B_2$-$B_3)_{m'}$- group {wherein $R_3$ represents a hydrogen atom, or a C1-C10 alkyl group optionally substituted with a halogen atom or a $R_2$-$B_1$-group (wherein $R_2$ and $B_1$ are as defined above), or a C2-C10 alkenyl group, or a C2-C10 alkynyl group, $R_0$ represents a hydrogen atom, a C1-C10 alkyl group or a C2-C10 haloalkyl group, m represents 0 or 1, $B_2$ represents a single bond, an oxy group, a thio group or a -$N((O)_nR_1')$- group (wherein $R_1'$ is the same as or different from $R_1$, and has the same meaning as $R_1$ has, and n represents 0 ro 1), $B_3$ represents a carbonyl group, a thiocarbonyl group or a sulfonyl group, m' represents 0 or 1, and $B_3$ is not a sulfonyl group when m is 0 and $R_3$ is a hydrogen atom}, and k' represents 0 or 1], and $R_4$ represents a C1-C10 alkylene group, provided that a $R_0'R_0''N$-$R_4$- group (wherein $R_0'$ and $R_0''$ are the same as or different from $R_0$ and have the same meaning as $R_0$ has, and $R_4$ is as defined above) is excluded,
a $D_2$-$R_4$- group, wherein $D_2$ represents a cyano group, a $R_1R_1'NC(=N$-$(O)_n$-$A_1)$- group (wherein $R_1$, $R_1'$, n and $A_1$ are as defined above), an $A_1N=C(-OR_2)$- group (wherein $A_1$ and $R_2$ are as defined above) or a $NH_2$-CS- group, and $R_4$ is as defined above,
a $D_3$-$R_4$- group, wherein $D_3$ represents a nitro group or a $R_1OSO_2$- group (wherein $R_1$ is as defined above), and $R_4$ is as defined above, and
a $R_1OSO_2$- group, wherein $R_1$ is as defined above;

(2) the $B_0$ group: an $(a_0)$- group represented by

(a0) 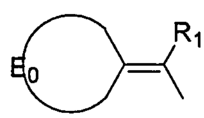

wherein $E_0$ forms an optionally substituted, saturated or unsaturated, aromatic or nonaromatic 5 to 14-membered hydrocarbon ring or heterocyclic ring, and $R_1$ is as defined above;
(3) the $C_0$ group: a C2-C10 alkenyl group substituted with a halogen atom, a $R_2$-$B_1$- group (wherein $R_2$ and $B_1$ are as defined above), a $D_4$-$R_4$- group [wherein $D_4$ represents a hydroxyl group or an $A_1$-O- group (wherein $A_1$ is as defined above), and $R_4$ is as defined above], a $D_5$- group [wherein $D_5$ represents a O=C$(R_3)$- group (wherein $R_3$ is as defined above), an $A_1$-$(O)_n$-N=C$(R_3)$- group (wherein $A_1$, n and $R_3$ are as defined above), a $R_1$-$B_0$-CO-$R_4$-$(O)_n$-N=C$(R_3)$- group {wherein $R_1$, $R_4$, n and $R_3$ are as defined above, and $B_0$ represents an oxy group, a thio group or a -$N((O)_mR_1')$-group (wherein $R_1'$ and m are as defined above)}, a $D_2$-$R_4$-$(O)_n$-N=C$(R_3)$- group (wherein $D_2$, $R_4$, n and $R_3$ are as defined above) or a $R_1A_1$N-N=C$(R_3)$- group (wherein $R_1$, $A_1$ and $R_3$ are as defined above)], a $R_1A_1$N-O-$R_4$- group (wherein $R_1$, $A_1$ and $R_4$ are as defined above), a $R_1(A_1$-$(O)_n$-$)$N- group (wherein $R_1$, $A_1$ and n are as defined above), a $D_2$- group (wherein $D_2$ is as defined above) or a $D_3$- group (wherein $D_3$ is as defined above);
(4) the $D_0$ group: a C2-C10 alkynyl group substituted with a $(b_0)$-$R_4$- group (in $(b_0)$

(b0) 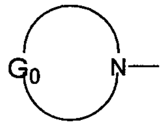
,

Go forms an optionally substituted, saturated or unsaturated, nonaromatic 5 to 14-membered hydrocarbon ring or heterocyclic ring), a $(c_0)$-$R_4$- group (in $(c_0)$

$(c_0)$ 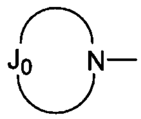 ,

$J_0$ forms an aromatic 5 to 7-membered ring optionally containing a nitrogen atom and $R_4$ is as defined above), a halogen atom, a $R_2$-$B_1$-$R_4$- group (wherein $R_2$, $B_1$ and $R_4$ are as defined above), a $D_4$-$R_4$- group (wherein $D_4$ and $R_4$ are as defined above), a $D_5$- group (wherein $D_5$ is as defined above), a $D_1$-$R_4$- group (wherein $D_1$ and $R_4$ are as defined above), a $D_2$- group (wherein $D_2$ is as defined above) or a $D_3$-$R_4$- group (wherein $D_3$ and $R_4$ are as defined above);
(5) the $E_0$ group: an $A_2$-CO-$R_5$- group, provided that $R_5$ is not a vinylene group when $A_2$ is a hydroxyl group [wherein $A_2$ represents

(i) an $A_3$-$B_4$- group
wherein $A_3$ represents a hydrogen atom, or a C1-C10 alkyl group, or a C2-C10 haloalkyl group, or a C2-C10 alkenyl group optionally substituted with a halogen atom, or a C3-C10 alkynyl group optionally substituted with a halogen atom, or a $R_{a0}$-$(R_4)_m$- group (wherein $R_{a0}$ represents an optionally substituted 5 to 7-membered aryl group or heteroaryl group, and $R_4$ and m are as defined above), or a C1-C10 alkyl group substituted with a $(b_0)$-$R_4$- group (wherein $(b_0)$ and $R_4$ are as defined above), a $(c_0)$-$R_4$- group (wherein $(c_0)$ and $R_4$ are as defined above), a $R_2$-$B_1$-$R_4$-group (wherein $R_2$, $B_1$ and $R_4$ are as defined above), a $D_4$-$R_4$-group (wherein $D_4$ and $R_4$ are as defined above), a $D_5$- group (wherein $D_5$ is as defined above), a $D_1$-$R_4$- group (wherein $D_1$ and $R_4$ are as defined above), a $D_2$- group (wherein $D_2$ is as defined above), a $D_3$-$R_4$- group (wherein $D_3$ and $R_4$ are as defined above) or an $A_4$-$SO_2$-$R_4$- group {wherein $A_4$ represents a $(b_0)$- group (wherein $(b_0)$ is as defined above), a $(c_0)$-group (wherein $(c_0)$ is as defined above) or a $R_1R_1$' N- group (wherein $R_1$ and $R_1$' are as defined above), and $R_4$ is as defined above}, and
$B_4$ represents an oxy group, a thio group or a -N($(O)_mR_1$)-group (wherein $R_1$ and m are as defined above), provided that $A_3$ is not a hydrogen atom when $B_4$ is a thio group;
(ii) a $R_1$-$B_4$-CO-$R_4$-$B_4$'- group (wherein $R_1$, $B_4$ and $R_4$ are as defined above, $B_4$' is the same as or different from $B_4$ and has the same meaning as $B_4$ has, provided that $R_2$ is not a hydrogen atom when $B_4$ is a thio group) or a $D_2$-$R_4$-$B_4$-group (wherein $D_2$, $R_4$ and $B_4$ are as defined above);
(iii) a $R_2$-$SO_2$-$NR_1$- group (wherein $R_2$ is as defined above, provided that a hydrogen atom is excluded, and $R_1$ is as defined above);
(iv) a $(b_0)$- group, wherein $(b_0)$ is as defined above;
(v) a $(c_0)$- group, wherein $(c_0)$ is as defined above; or
(vi) a $R_1A_1N$-$NR_1$'- group, wherein $R_1$, $A_1$ and $R_1$' are as defined above; and
$R_5$ represents a C2-C10 alkenylene group optionally substituted with a halogen atom or a C2-C10 alkynylene group];

(6) the $F_0$ group: an $A_5$-$B_5$-$R_6$- group, wherein
$A_5$ represents a C2-C10 alkyl group substituted with a $D_4$- group (wherein $D_4$ is as defined above), a $D_1$- group (wherein $D_1$ is as defined above), a $D_3$- group (wherein $D_3$ is as defined above) or an $A_4$-$SO_2$- group (wherein $A_4$ is as defined above), or a C1-C10 alkyl group substituted with a $R_2$-$B_1$- group (wherein $R_2$ and $B_1$ are as defined above), a $D_2$-group (wherein $D_2$ is as defined above), a $D_5$- group (wherein $D_5$ is as defined above) or an $A_2$-CO- group (wherein $A_2$ is as defined above), and
$8_5$ represents a $B_1$- group (wherein $B_1$ is as defined above) or a -$NA_1$- group (wherein $A_1$ is as defined above) and $R_6$ represent a single bond or a C1-C10 alkylene group;
(7) the Go group: an $A_6$-$B_5$-$R_6$- group wherein
$A_6$ represents an $(a_0)$-$R_4$- group (wherein $(a_0)$ and $R_4$ are as defined above), or a C2-C10 alkenyl group, or a C2-C10 alkynyl group, or a C2-C10 alkenyl group substituted with a halogen atom, a $R_2$-$B_1$- group (wherein $R_2$ and $B_1$ are as defined above), a $D_5$- group (wherein $D_5$ is as defined above), a $D_2$- group (wherein $D_2$ is as defined above) or an $A_2$-CO- group (wherein $A_2$ is as defined above), or a C2-C10 alkynyl group substituted with a halogen atom, a $R_2$-$B_1$-group (wherein $R_2$ and $B_1$ are as defined above), a $D_5$- group (wherein $D_5$ is as defined above), $D_2$- group (wherein $D_2$ is as defined above) or an $A_2$-CO- group

(wherein $A_2$ is as defined above), or a C3-C10 alkenyl group substituted with a $(b_0)$- group (wherein $(b_0)$ is as defined above), a $(c_0)$-group (wherein $(c_0)$ is as defined above), a $D_4$- group (wherein $D_4$ is as defined above), a $D_1$- group (wherein $D_1$ is as defined above) or a $D_3$- group (wherein $D_3$ is as defined above), or a C3-C10 alkynyl group substituted with a $D_4$-group (wherein $D_4$ is as defined above), a $D_1$- group (wherein $D_1$ is as defined above) or a $D_3$- group (wherein $D_3$ is as defined above), and

$8_5$ and $R_6$ are as defined above;

(8) the $H_0$ group:

a $D_2$-N(-(O)$_n$-$A_1$)-$R_6$- group (wherein $D_2$, n, $A_1$ and $R_6$ are as defined above),
a $D_2$- group (wherein $D_2$ is as defined above, provided that a cyano group is excluded),
a $R_1(R_1'(O)_n)$N-$CR_1''$=N-$R_6$- group (wherein $R_1$, $R_1'$, n and $R_6$ are as defined above, $R_1''$ is the same as or different from $R_1$ and has the same meaning as $R_1$ has),
a $R_1$-(O)$_n$-N=$CR_1'$-$NR_2$-$R_6$- group (wherein $R_1$, n, $R_1'$, $R_2$ and $R_6$ are as defined above),
a $R_2$-$B_3$-$NR_1$-CO-$NR_1'$-$R_6$- group (wherein $R_2$, $B_3$, $R_1$, $R_1'$ and $R_6$ are as defined above),
a $D_2$-CO-$NR_1$-$R_6$- group (wherein $D_2$, $R_1$ and $R_6$ are as defined above), and
an $A_2$-COCO-$NR_1$-$R_6$- group (wherein $A_2$, $R_1$ and $R_6$ are as defined above);

(9) the $I_0$ group:

an $A_7$-$B_6$-N((O)$_n$$R_1$)-$R_6$- group [wherein $A_7$ represents a C2-C10 alkenyl group optionally substituted with a halogen atom, or a C2-C10 alkynyl group, or a C3-C10 haloalkynyl group, or a $R_2$-$B_1$-$R_4$- group (wherein $R_2$, $B_1$ and $R_4$ are as defined above), or a $D_4$-$R_4$- group (wherein $D_4$ and $R_4$ are as defined above), or a $D_5$-$R_4$- group (wherein $D_5$ and $R_4$ are as defined above), or a $D_1$-$R_4$- group (wherein $D_1$ and $R_4$ are as defined above), or a $(b_0)$-$R_4$- group (wherein $(b_0)$ and $R_4$ are as defined above), or a $(c_0)$-$R_4$- group (wherein $(c_0)$ and $R_4$ are as defined above), or a $D_2$-$R_4$- group (wherein $D_2$ and $R_4$ are as defined above), or a $D_3$-$R_4$- group (wherein $D_3$ and $R_4$ are as defined above), or an $A_4$-$SO_2$-$R_4$- group (wherein $A_4$ and $R_4$ are as defined above), or an $A_2$-CO-$R_4$- group (wherein $A_2$ and $R_4$ are as defined above), $B_6$ represents a carbonyl group or a thiocarbonyl group, and n, $R_1$ and $R_6$ are as defined above],
an $A_8$-CS-N((O)$_n$$R_1$)-$R_6$- group [wherein $A_8$ represents a hydrogen atom or a C1-C10 alkyl group optionally substituted with a halogen atom, and n, $R_1$ and $R_4$ are as defined above],
an $A_7'$-$B_2'$-$B_3$-N((O)$_n$$R_1$)-$R_6$- group [wherein $A_7'$ represents a C3-C10 alkenyl group optionally substituted with a halogen atom, or a C3-C10 alkynyl group optionally substituted with a halogen atom, or a $R_2$-$B_1$-$R_4'$- group (wherein $R_2$ and $B_1$ are as defined above, and $R_4'$ represents a C2-C10 alkylene group), or a $D_4$-$R_4'$- group (wherein $D_4$ and $R_4'$ are as defined above), or a $D_1$-$R_4'$- group (wherein $D_1$ and $R_4'$ are as defined above), or a $(b_0)$-$R_4'$- group (wherein $(b_0)$ and $R_4'$ are as defined above), or a $(c_0)$-$R_4'$- group (wherein $(c_0)$ and $R_4'$ are as defined above), or a $D_2$-$R_4$-group (wherein $D_2$ and $R_4$ are as defined above), or a $D_3$-$R_4'$-group (wherein $D_3$ and $R_4'$ are as defined above), or an $A_2$-CO-$R_4$-group (wherein $A_2$ and $R_4$ are as defined above), $B_2'$ represents an oxy group, a thio group or a -N((O)$_{n'}$, $R_1'$)-group (wherein n' is the same as or different from n and has the same meaning as n has, and $R_1'$ is as defined above), and $B_3$, n, $R_1$ and $R_6$ are as defined above],
an $A_8'$-$B_2'$-CS-N((O)$_n$$R_1$)-$R_6$- group [wherein $A_8'$ represents a C1-C10 alkyl group or a C2-C10 haloalkyl group, $B_2'$ is as defined above, and n, $R_1$ and $R_6$ are as defined above],
an $A_8'$-S-$B_3'$-N((O)$_n$$R_1$)-$R_6$- group [wherein $A_8'$, n, $R_1$ and $R_6$ are as defined above, and $B_3'$ represents a carbonyl group or a sulfonyl group], and
an $A_7''$-$SO_2$-N((O)$_n$$R_1$)-$R_6$- group [wherein $A_7''$ represents a C2-C10 alkenyl group, or a C3-C10 alkenyl group substituted with a halogen atom, or a C3-C10 alkynyl group optionally substituted with a halogen atom, or a $R_2$-$B_1$-$R_4'$- group (wherein $R_2$, $B_1$ and $R_4'$ are as defined above), or a $D_4$-$R_4'$-group (wherein $D_4$ and $R_4'$ are as defined above), or a $D_5$-$R_4$-group (wherein $D_5$ and $R_4$ are as defined above), or a $D_1$-$R_4'$-group (wherein $D_1$ and $R_4'$ are as defined above), or a $(b_0)$-$R_4'$- group (wherein $(b_0)$ and $R_4'$ are as defined above), or a $(c_0)$-$R_4'$- group (wherein $(c_0)$ and $R_4'$ are as defined above), or a $D_2$-$R_4$-group (wherein $D_2$ and $R_4$ are as defined above), or a $NO_2$-$R_4$- group (wherein $R_4$ is as defined above), or an $A_2$-CO-$R_4$- group (wherein $A_2$ and $R_4$ are as defined above), and n, $R_1$ and $R_6$ are as defined above];

(10) the $J_0$ group:

an $A_7$-CO- group (wherein $A_7$ is as defined above),

an $A_9$-CS- group (wherein $A_9$ represents $A_7$ or $A_8$),
an $A_9'(O)_mN=C(A_9)$- group (wherein $A_9'$ represents $A_7'$ or $A_8'$, and m and $A_9$ are as defined above),
a $D_2$-CO- group (wherein $D_2$ is as defined above),
an $A_2$-COCO- group (wherein $A_2$ is as defined above),
an $A_9$-CO-$B_1'$-$R_6$- group (wherein $A_9$ and $R_6$ are as defined above, and $B_1'$ represents an oxy group or a thio group, provided that $A_9$ is not $A_8$ when $B_1'$ is an oxy group),
an $A_9$-CS-$B_1'$-$R_6$- group (wherein $A_9$, $B_1'$ and $R_6$ are as defined above),
an $A_7''$-$SO_2$-$B_1'$-$R_6$- group (wherein $A_7''$, $B_1'$ and $R_6$ are as defined above),
an $A_8$-$SO_2$-$B_1'$-$R_6$- group (wherein $A_8$, $B_1'$ and $R_6$ are as defined above, provided that $A_8$ is not a hydrogen atom),
an $A_9'$-$B_2'$-$B_3$-$B_1'$-$R_6$- group (wherein $A_9'$, $B_2'$, $B_3$, $B_1'$ and $R_6$ are as defined above), and
a C2-C10 alkenyl group substituted with a $(b_0)$- group (wherein $(b_0)$ is as defined above) or a $(c_0)$- group (wherein $(c_0)$ is as defined above);

(11) the $K_0$ group: an $A_{10}$-$N((O)_nR_1)$-CO-$R_6$- group,
wherein $A_{10}$ represents a hydrogen atom (provided that n is not 0), an $A_7''$-$SO_2$- group (wherein $A_7''$ is as defined above), an $A_8$-$SO_2$- group (wherein $A_8$ is as defined above, provided that $A_8$ is not a hydrogen atom), an $A_9'$O- group (wherein $A_9'$ is as defined above, (provided that n is not 1), an $A_9'$-group (wherein $A_9'$ is as defined above, provided that $A_8'$ is excluded when n is 0), a $R_2OCH_2$- group (wherein $R_2$ is as defined above), an $A_2$-CO-$R_4$- group (wherein $A_2$ and $R_4$ are as defined above) or an $A_2$-CO-$CH(CH_2CO$-$A_2)$- group (wherein $A_2$ is as defined above), and n, $R_1$ and $R_6$ are as defined above;
(12) the $L_0$ group:

an $A_{10}'$-$N((O)_nR_1)$-$SO_2$-$R_6$- group [wherein $A_{10}'$ represents a hydrogen atom (provided that n is not 1), an $A_9'$O- group (wherein $A_9'$ is as defined above, provided that n is not 0), an $A_9'$- group (wherein $A_9'$ is as defined above, provided that $A_8'$ is excluded when n is 0), a $R_2$-CO- group (wherein $R_2$ is as defined above), an $A_2$-CO-$R_4$- group (wherein $A_2$ and $R_4$ are as defined above) or an $A_2$-CO-CH $(CH_2CO$-$A_2)$- group (wherein $A_2$ is as defined above), and n, $R_1$ and $R_6$ are as defined above],
an $A_9''R_1N$-$SO_2$-$N((O)_nR_1')$ -$R_6$- group [wherein $A_9''$ represents a hydrogen atom or an $A_9'$- group (wherein $A_9'$ is as defined above), and $R_1$, n, $R_1'$ and $R_6$ are as defined above] and
a $(b_0)$-$SO_2$-$N((O)_nR_1')$-$R_6$- group [wherein $(b_0)$, n, $R_1'$ and $R_6$ are as defined above];

(13) the $M_0$ group:

a $R_1(R_2S)C=N$-$R_6$- group (wherein $R_1$, $R_2$ and $R_6$ are as defined above),
a $R_2B(R_2'B')C=N$-$R_6$- group (wherein $R_2$ and $R_6$ are as defined above, $R_2'$ is the same as or different from $R_2$ and has the same meaning as $R_2$ has, and B and B' are the same or different and represent an oxy group or a thio group),
a $R_1R_1'N$-$(R_2S)C=N$-$R_6$- group (wherein $R_1$, $R_1'$, $R_2$ and $R_6$ are as defined above),
a $R_1N=C(SR_2)$-$NR_2'$-$R_6$- group (wherein $R_1$, $R_2$, $R_2'$ and $R_6$ are as defined above), and
a $R_1(R_1'O)N$-$R_6$- group (wherein $R_1$, $R_1'$ and $R_6$ are as defined above);

(14) the No group: a $A_{11}$-$P(=O)(OR_1')$-$R_4$- group, wherein $A_{11}$ represents a $R_1$- group (wherein $R_1$ is as defined above), a $R_1O$-$R_6$- group (wherein $R_1$ and $R_6$ are as defined above) or a $R_1OCO$-$CHR_0$- group (wherein $R_1$ and $R_0$ are as defined above), and $R_1'$ and $R_4$ are as defined above;

III. in $(Y_{A0})_q$, $Y_{A0}$ is a substituent on a carbon atom and represents a group included in the following $X_0$ group and $Y_0$ group, q represents 0, 1, 2, 3, 4 or 5, the sum of p (wherein p is as defined above) and q is not more than 5, and when q is not less than 2, $Y_{A0}$S are the same or different, and when q is not less than 2, adjacent two same or different $Y_{A0}$s may together form a group included in the $Z_0$ group to be fused to the A0 ring;

(1) the $X_0$ group: a $M_a$-group, wherein $M_a$ represents a $R_b$- group (wherein $R_b$ represents a C1-C10 alkyl group optionally substituted with a halogen atom), a halogen atom, a nitro group, a cyano group, a hydroxyl group, a $R_c$-$B_a$-$R_d$-group (wherein $R_c$ represents a C1-C10 alkyl group optionally substituted with a halogen atom, $B_a$ represents an oxy group, a thio group, a sulfinyl group or a sulfonyl group, and $R_d$ represents a single bond or a C1-C10 alkylene group), an $HOR_d$- group (wherein $R_d$ is as defined above), a $R_e$-CO-$R_d$- group (wherein $R_e$ represents a hydrogen atom, or a C1-C10 alkyl group optionally substituted with a halogen atom, and $R_d$ is as defined above), a $R_e$-CO-O-$R_d$- group (wherein $R_e$ and $R_d$ are as defined

above), a $R_eO$-CO-$R_d$- group (wherein $R_e$ and $R_d$ are as defined above), an HO-CO-CH=CH-group, a $R_eR_e$'N-$R_d$- group (wherein $R_e$ and $R_e$' are the same or different, $R_e$ is as defined above, $R_e$' has the same meaning as $R_e$ has, and $R_d$ is as defined above), a $R_e$-CO-N$R_e$'-$R_d$- group (wherein $R_e$, $R_e$' and $R_d$ are as defined above), a $R_bO$-CO-N($R_e$)-$R_d$- group (wherein $R_b$, $R_e$ and $R_d$ are as defined above), a $R_eR_e$'N-CO-$R_d$- group (wherein $R_e$, $R_e$' and $R_d$ are as defined above), a $R_eR_e$'N-CO-N$R_e$"-$R_d$- group (wherein $R_e$, $R_e$' and $R_e$" are the same or different, $R_e$ and $R_e$' are as defined above, $R_e$" has the same meaning as $R_e$ has, and $R_d$ is as defined above), a $R_eR_e$'N-C(=N$R_e$")-N$R_e$"'-$R_d$- group (wherein $R_e$, $R_e$', $R_e$" and $R_e$"' are the same or different, $R_e$, $R_e$' and $R_e$" are as defined above, $R_e$"' has the same meaning as $R_e$ has, and $R_d$ is as defined above), a $R_b$-$SO_2$-N$R_e$-$R_d$- group (wherein $R_b$, $R_e$ and $R_d$ are as defined above), a $R_eR_e$'N-$SO_2$-$R_d$- group (wherein $R_e$, $R_e$' and $R_d$ are as defined above), a C2-C10 alkenyl group or a C2-C10 alkynyl group;.

(2) the $Y_0$ group: a $M_{b0}$-$R_d$- group, wherein $M_{b0}$ represents a $M_{c0}$- group [wherein $M_{co}$ represents a $M_{do}$-$R_d$'-group {wherein $M_{do}$ represents a 6 to 10-membered aryl group optionally substituted with a $M_a$- group (wherein $M_a$ is as defined above), a 5 to 10-membered heteroaryl group optionally substituted with a $M_a$-group (wherein $M_a$ is as defined above), a 3 to 10-membered cyclic hydrocarbon or heterocyclic group optionally substituted with a $M_a$- group (wherein $M_a$ is as defined above) and optionally containing an unsaturated bond, a ($b_0$)- group represented by

($b_0$) 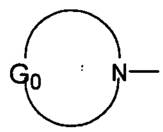

(wherein ($b_0$) is as defined above), a ($c_0$)- group represented by

($c_0$) 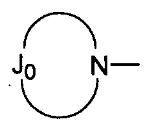

(wherein, ($c_0$) is as defined above), a (do)- group represented by

($d_0$) 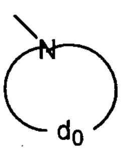

{wherein do forms a 5 to 12-membered hydrocarbon ring which is substituted with a carbonyl group or a thiocarbonyl group and further which may be optionally substituted with an oxy group, a thio group, a -N$R_1$- group (wherein $R_1$ is as defined above), a sulfinyl group or a sulfonyl group}, or a ($e_0$)- group represented by

($e_0$) 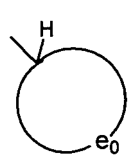

{wherein $e_0$ forms a 5 to 12-membered hydrocarbon ring optionally substituted with a carbonyl group, a thiocarbonyl group, an oxy group, a thio group, a -N$R_1$-group (wherein $R_1$ is as defined above), a sulfinyl group or a sulfonyl group}, and $R_d$' is the same as or different from $R_d$ and has the same meaning as $R_d$ has], a $M_{c0}$-$B_a$- group (wherein $M_{c0}$ and $B_a$ are as defined above), a $M_{c0}$-CO- group (wherein $M_{c0}$ is as defined above), a $M_{c0}$-CO-O- group

(wherein $M_{c0}$ is as defined above), a $M_{c0}$O-CO- group (wherein $M_{c0}$ is as defined above), a $M_{c0}R_eN$- group (wherein $M_{c0}$ and $R_e$ are as defined above), a $M_{c0}$-CO-$NR_e$- group (wherein $M_{c0}$ and $R_e$ are as defined above), a $M_{c0}$O-CO-$NR_e$- group (wherein $M_{c0}$ and $R_e$ are as defined above), a $M_{c0}R_eN$-CO- group (wherein $M_{c0}$ and $R_e$ are as defined above), a $M_{c0}R_eN$-CO-$NR_e$'- group (wherein $M_{c0}$, $R_e$ and $R_e$' are as defined above), a $M_{c0}R_eN$-C(=$NR_e$')-$NR_e$"- group (wherein $M_{c0}$, $R_e$, $R_e$' and $R_e$" are as defined above), a $M_{c0}$-$SO_2$-$NR_e$- group (wherein $M_{c0}$ and $R_e$ are as defined above), or a $M_{c0}R_eN$-$SO_2$- group (wherein $M_{c0}$ and $R_e$ are as defined above), and $R_d$ is as defined above;

(3) the $Z_0$ group: a 5- to 12-membered hydrocarbon ring or heterocyclic ring which may be substituted with a halogen atom, a C1-C10 alkoxy group, C3-C10 alkenyloxy group, a C3-C10 alkynyloxy group, a carbonyl group, a thiocarbonyl group, an oxy group, a thio group, a sulfinyl group or a sulfonyl group, and which is aromatic or nonaromatic and monocyclic or fused ring and is fused to the A0 ring; and

IV. B0 is a group represented by formula (III-1):

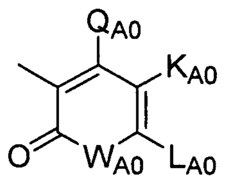

(III-1)

[wherein,

(1) $Q_{A0}$ represents a hydroxyl group, a ($b_0$)- group (wherein ($b_0$) is as defined above), an $A_9$-$B_6$-$B_c$- group [wherein $A_9$ and $B_6$ are as defined above, and $B_c$ represents an oxy group or a N-((O)$_m$$R_1$)- group (wherein m and $R_1$ are as defined above), provided that $B_c$ is not a sulfonyl group when $A_9$ is a hydrogen atom], an $A_7$"-$SO_2$-$B_c$- group (wherein $A_7$" and $B_c$ are as defined above), an $A_8$-$SO_2$-$B_c$- group (wherein $A_8$ and $B_c$ are as defined above, provided that $A_8$ is not a hydrogen atom), a $R_1R_1$'N-$SO_2$-$B_c$- group (wherein $R_1$, $R_1$' and $B_c$ are as defined above), a ($b_0$)-$SO_2$-$B_c$- group (wherein ($b_0$) and $B_c$ are as defined above), an $A_9$'-$B_c$- group (wherein $A_9$' and $B_c$ are as defined above), a $D_5$-$R_4$-$B_c$- group (wherein $D_5$, $R_4$ and $B_c$ are as defined above), a $M_{c0}$-$B_3$-$B_c$- group (wherein $M_{c0}$, $B_3$ and $B_c$ are as defined above) or a $M_{c0}$-$B_c$- group (wherein $M_{c0}$ and $B_c$ are as defined above),

(2) $W_{A0}$ represents an oxygen atom or a -$NT_{A0}$- group wherein $T_{A0}$ represents a hydrogen atom, an $A_9$'- group (wherein $A_9$' is as defined above), a $D_5$-$R_4$- group (wherein $D_5$ and $R_4$ are as defined above) or a $M_{c0}$- group (wherein $M_{c0}$ is as defined above),

(3) $K_{A0}$ and $L_{A0}$ form a -$V_{A0}$=$V_{A0}$'-$V_{A0}$"=$V_{A0}$"'- group {wherein $V_{A0}$, $V_{A0}$', $V_{A0}$" and $V_{A0}$"' are the same or different, and represent a methine group optionally substituted with a $M_a$-group (wherein $M_a$ is as defined above), or a -N= group, and at least one of $V_{A0}$, $V_{A0}$', $V_A$0" and $V_{A0}$"' represents a -N= group},

a group represented by formula (III-2):

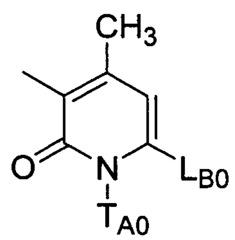

(III-2)

[wherein $T_{A0}$ is as defined above, and $L_{B0}$ represents a hydroxyl group or a methyl group],

a group represented by formula (III-3):

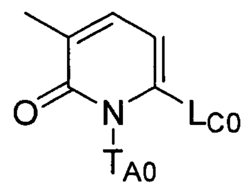

(III-3)

[wherein $T_{A0}$ is as defined above, and $L_{C0}$ represents a C1-C10 alkyl group], a group represented by formula (III-4):

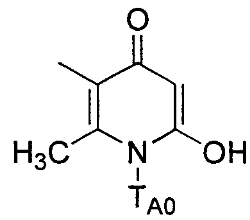

(III-4)

[wherein $T_{A0}$ is as defined above], a group represented by formula (III-5):

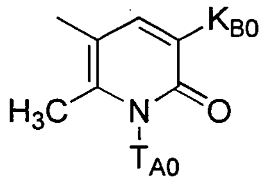

(III-5)

[wherein $T_{A0}$ is as defined above, and $K_{B0}$ represents a cyano group or a UOCO- group (wherein U represents a hydrogen atom or a C1-C10 alkyl group)], a group represented by formula (III-6):

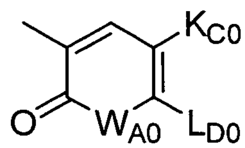

(III-6)

[wherein $W_{A0}$ is as defined above, and $K_{C0}$ and $L_{D0}$ form a C3-C10 alkylene group, or a C4-C10 alkenylyne optionally substituted with 1 or more same or different $M_a$- groups (wherein $M_a$ is as defined above)], a group represented by formula (III-7):

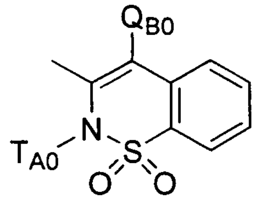

(III-7)

[wherein $T_{A0}$ is as defined above, and $Q_{B0}$ represents a hydroxyl group, a $A_9$-$B_6$-O- group (wherein $A_9$

and $B_6$ are as defined above), an $A_7$"-$SO_2$-O- group (wherein $A_7$" is as defined above), an $A_8$-$SO_2$-O-group, (wherein $A_8$ is as defined above, provided that $A_8$ is not a hydrogen), a $R_1R_1$' N-$SO_2$-O- group (wherein $R_1$ and $R_1$' are as defined above), a ($b_0$)-$SO_2$-O- group (wherein ($b_0$) is as defined above), an $A_9$'-O- group (wherein $A_9$' is as defined above), a $D_5$-$R_4$-O- group (wherein $D_5$ and $R_4$ are as defined above), a $M_{cO}$-$B_3$-O- group (wherein $M_{cO}$ and $B_3$ are as defined above) or a $M_{c0}$-O- group (wherein $M_{cO}$ is as defined above)], or
a group represented by formula (III-8):

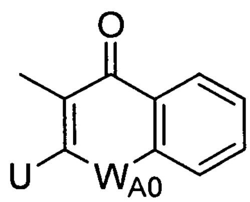

(III-8)

[wherein U and $W_{A0}$ are as defined above]; and
the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range.

**4.** A cinnamoyl compound represented by the formula (IV):

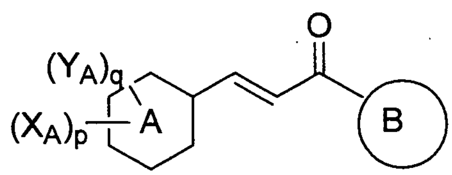

(IV)

wherein,

I. A represents a benzene ring or a pyridine ring;
II. in ($X_A$)p, $X_A$ is a substituent on a carbon atom, and represents a group included in any group of the following A group to N group, p represents 0, 1, 2, 3, 4 or 5 and, when p is not less than 2, $X_A$s are the same or different;

(1) the A group:

a $D_1$-$R_4$- group, wherein $D_1$ represents a ($R_1$-(O)$_k$-($A_1$N-(O)$_k$'- group [wherein $R_1$ represents a hydrogen atom, or a C1-C10 alkyl group, or a C2-C10 alkyl group substituted with a halogen atom or a $R_2$-$B_1$-group (wherein $R_2$ represents a C1-C10 alkyl group, a C3-C10 alkenyl group or a C3-C10 alkynyl group, and $B_1$ represents an oxy group, a thio group, a sulfinyl group or a sulfonyl group), or a C3-C10 alkenyl group, or a C3-C10 alkynyl group, k represents 0 or 1, $A_1$ represents a $R_3$-($CHR_0$)$_m$-($B_2$-$B_3$)$_{m'}$-group {wherein $R_3$ represents a hydrogen atom, or a C1-C10 alkyl group optionally substituted with a halogen atom or a $R_2$-$B_1$-group (wherein $R_2$ and $B_1$ are as defined above), or a C2-C10 alkenyl group, or a C2-C10 alkynyl group, $R_0$ represents a hydrogen atom, a C1-C10 alkyl group or a C2-C10 haloalkyl group, m represents 0 or 1, $B_2$ represents a single bond, an oxy group, a thio group or a -N((O)$_n$$R_1$')-group (wherein $R_1$' is the same as or different from $R_1$ and has the same meaning as $R_1$ has, and n represents 0 or 1), $B_3$ represents a carbonyl group, a thiocarbonyl group or a sulfonyl group, m' represents 0 or 1, and $B_3$ is not a sulfonyl group when m is 0 and $R_3$ is a hydrogen atom}, and k' represents 0 or 1], and $R_4$ represents a C1-C10 alkylene group, provided that a $R_0$'$R_0$"N-$R_4$- group (wherein $R_0$' and $R_0$" are the same as or different from $R_0$ and have the same meaning as $R_0$ has, and $R_4$ is as defined above) is excluded,
a $D_2$-$R_4$- group, wherein $D_2$ represents a cyano group, a $R_1R_1$'NC(=N-(O)$_n$-$A_1$)- group (wherein $R_1$, $R_1$', n and $A_1$ are as defined above), an $A_1$N=C(-O$R_2$)- group (wherein $A_1$ and $R_2$ are as defined above)

or a $NH_2$-CS- group, and $R_4$ is as defined above,
a $D_3$-$R_4$- group, wherein $D_3$ represents a nitro group or a $R_1OSO_2$- group (wherein $R_1$ is as defined above), and $R_4$ is as defined above, and
a $R_1OSO_2$- group, wherein $R_1$ is as defined above;

(2) the B group: an (a)-group represented by

(a) 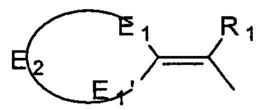

wherein $E_1$ and $E_1$' represent a methylene group optionally substituted with a C1-C10 alkyl group or a C1-C10 alkoxy group, or a carbonyl group, provided that $E_1$ and $E_1$' are not a carbonyl group at the same time, $E_2$ represents a C2-C10 alkylene group optionally substituted with an oxy group, a thio group, a sulfinyl group, a sulfonyl group or a - $NR_1$'- group (wherein $R_1$' is as defined above), or a C3-C10 alkenylene group optionally substituted with an oxy group, a thio group, a sulfinyl group, a sulfonyl group or a - $NR_1$'- group (wherein $R_1$' is as defined above), and $R_1$ is as defined above;
(3) the C group: a C2-C10 alkenyl group substituted with a halogen atom, a $R_2$-$B_1$- group (wherein $R_2$ and $B_1$ are as defined above), a $D_4$-$R_4$- group [wherein $D_4$ represents a hydroxyl group or an $A_1$-O- group (wherein $A_1$ is as defined above), and $R_4$ is as defined above], a $D_5$- group [wherein $D_5$ represents an O=C($R_3$)- group (wherein $R_3$ is as defined above), an $A_1$-$(O)_n$-N=C($R_3$)- group (wherein $A_1$, n and $R_3$ are as defined above), a $R_1$-$B_0$-CO-$R_4$-$(O)_n$-N=C($R_3$)- group {wherein $R_1$, $R_4$, n and $R_3$ are as defined above, and $B_0$ represents an oxy group, a thio group or a -N($(O)_m R_1$')-group (wherein $R_1$' and m are as defined above)}, a $D_2$-$R_4$-$(O)_n$-N=C($R_3$)- group (wherein $D_2$, $R_4$, n and $R_3$ are as defined above) or a $R_1A_1$N-N=C($R_3$)- group (wherein $R_1$, $A_1$ and $R_3$ are as defined above)], a $R_1A_1$N-O-$R_4$- group (wherein $R_1$, $A_1$ and $R_4$ are as defined above), a $R_1$ ($A_1$-(O)n-)N- group (wherein $R_1$, $A_1$ and n are as defined above), a $D_2$- group (wherein $D_2$ is as defined above) or a $D_3$- group (wherein $D_3$ is as defined above);
(4) the D group: a C2-C10 alkynyl group substituted with a (b)-$R_4$- group [wherein in (b)

(b) 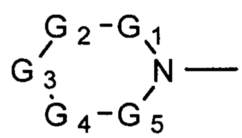,

$G_1$, $G_2$, $G_4$ and $G_5$ represent a methylene group which is connected with the adjacent atom via a single bond and which may be optionally substituted with a methyl group, or a methine group which is connected with the adjacent atom via a double bond and which may be optionally substituted with a methyl group, and $G_3$ represents a single bond, or a double bond, or a C1-C10 alkylene group optionally substituted with a methyl group, an oxy group, a thio group, a sulfinyl group, a sulfonyl group or a -$NR_1$- group (wherein $R_1$ is as defined above), or a C2-C10 alkenylene group optionally substituted with a methyl group, an oxy group, a thio group, a sulfinyl group, a sulfonyl group or a -$NR_1$- group (wherein $R_1$ is as defined above); and $R_4$ is as defined above], a (c)-$R_4$- group [wherein, in (c)

(c) 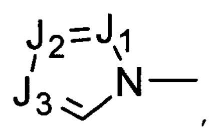,

$J_1$, $J_2$ and $J_3$ are the same or different, and represent a methine group optionally substituted with a methyl group, or a nitrogen atom; and $R_4$ is as defined above), a halogen atom, a $R_2$-$B_1$-$R_4$- group (wherein $R_2$, $B_1$ and $R_4$ are as defined above), a $D_4$-$R_4$- group (wherein $D_4$ and $R_4$ are as defined above), a $D_5$- group (wherein $D_5$ is as defined above), a $D_1$-$R_4$- group (wherein $D_1$ and $R_4$ are as defined above), a $D_2$- group (wherein $D_2$ is as defined above) or a $D_3$-$R_4$- group (wherein $D_3$ and $R_4$ are as defined above);
(5) the E group: an $A_2$-CO-$R_5$- group, provided that $R_5$ is not a vinylene group when $A_2$ is a hydroxyl group,

[wherein $A_2$ represents

(i) an $A_3$-$B_4$- group
wherein $A_3$ represents a hydrogen atom, or a C1-C10 alkyl group, or a C2-C10 haloalkyl group, or a C2-C10 alkenyl group optionally substituted with a halogen atom, or a C3-C10 alkynyl group optionally substituted with a halogen atom, or $R_a$-$(R_4)_m$- group (wherein $R_a$ represents a phenyl group, a pyridyl group, a furyl group or a thienyl group, which may be optionally substituted with a halogen atom, a C1-C10 alkyl group, a C1-C10 alkoxy group or a nitro group, and $R_4$ and m are as defined above), or a C1-C10 alkyl group substituted with a (b)-$R_4$- group (wherein (b) and $R_4$ are as defined above), a (c)-$R_4$- group (wherein (c) and $R_4$ are as defined above), a $R_2$-$B_1$-$R_4$- group (wherein $R_2$, $B_1$ and $R_4$ are as defined above), a $D_4$-$R_4$- group (wherein $D_4$ and $R_4$ are as defined above), a $D_5$- group (wherein $D_5$ is as defined above), a $D_1$-$R_4$- group (wherein $D_1$ and $R_4$ are as defined above), a $D_2$- group (wherein $D_2$ is as defined above), a $D_3$-$R_4$- group (wherein $D_3$ and $R_4$ are as defined above) or an $A_4$-$SO_2$-$R_4$- group {wherein $A_4$ represents a (b)- group (wherein (b) is as defined above), a (c)- group (wherein (c) is as defined above) or a $R_1R_1$'N- group (wherein $R_1$ and $R_1$' are as defined above), and $R_4$ is as defined above}, and $B_4$ represents an oxy group, a thio group or a -N($(O)_mR_1$)-group (wherein $R_1$ and m are as defined above), provided that $A_3$ is not a hydrogen atom when $B_4$ is a thio group;
(ii) a $R_1$-$B_4$-CO-$R_4$-$B_4$'- group, wherein $R_1$, $B_4$ and $R_4$ are as defined above, $B_4$' is the same as or different from $B_4$ and has the same meaning as $B_4$ has, provided that $R_2$ is not a hydrogen atom when $B_4$ is a thio group, or
a $D_2$-$R_4$-$B_4$- group, wherein $D_2$, $R_4$ and $B_4$ are as defined above;
(iii) a $R_2$-$SO_2$-$NR_1$- group, wherein $R_2$ is as defined above, provided that a hydrogen atom is excluded, and $R_1$ is as defined above;
(iv) a (b)- group, wherein (b) is as defined above,
(v) a (c)- group, wherein (c) is as defined above, or
(vi) a $R_1A_1$N-$NR_1$'- group, wherein $R_1$, $A_1$ and $R_1$' are as defined above; and
$R_5$ represents a C2-C10 alkenylene group optionally substituted with a halogen atom or a C2-C10 alkynylene group];

(6) the F group: an $A_5$-$B_5$-$R_6$- group
[wherein $A_5$ represents a C2-C10 alkyl group substituted with a $D_4$- group (wherein $D_4$ is as defined above), a $D_1$-group (wherein $D_1$ is as defined above), a $D_3$- group (wherein $D_3$ is as defined above) or an $A_4$-$SO_2$ group (wherein $A_4$ is as defined above), or a C1-C10 alkyl group substituted with a $R_2$-$B_1$- group (wherein $R_2$ and $B_1$ are as defined above), a $D_2$- group (wherein $D_2$ is as defined above), a $D_5$- group (wherein $D_5$ is as defined above) or an $A_2$-CO- group (wherein $A_2$ is as defined above),
$B_5$ represents a $B_1$- group (wherein $B_1$ is as defined above) or a -$NA_1$- group (wherein $A_1$ is as defined above), and
$R_6$ represents a single bond or a C1-C10 alkylene group];
(7) the G group: an $A_6$-$B_5$-$R_6$- group
[wherein $A_6$ represents an (a)-$R_4$- group (wherein (a) and $R_4$ are as defined above), or a C2-C10 alkenyl group, or a C2-C10 alkynyl group, or a C2-C10 alkenyl group substituted with a halogen atom, a $R_2$-$B_1$-group (wherein $R_2$ and $B_1$ are as defined above), a $D_5$- group (wherein $D_5$ is as defined above), a $D_2$- group (wherein $D_2$ is as defined above) or an $A_2$-CO- group (wherein $A_2$ is as define above), or a C2-C10 alkynyl group substituted with a halogen atom, a $R_2$-$B_1$-group (wherein $R_2$ and $B_1$ are as defined above), a $D_5$-group (wherein $D_5$ is as defined above), a $D_2$- group (wherein $D_2$ is as defined above) or an $A_2$-CO- group (wherein $A_2$ is as defined above), or a C3-C10 alkenyl group substituted with a (b)- group (wherein (b) is as defined above), a (c)-group (wherein (c) is as defined above), a $D_4$- group (wherein $D_4$ is as defined above), a $D_1$- group (wherein $D_1$ is as defined above) or a $D_3$- group (wherein $D_3$ is as defined above), or a C3-C10 alkynyl group substituted with a $D_4$-group (wherein $D_4$ is as defined above), a $D_1$- group (wherein $D_1$ is as defined above) or a $D_3$- group (wherein $D_3$ is as defined above), and $B_5$ and $R_6$ are as defined above];
(8) the H group:

a $D_2$-N(-$(O)_n$-$A_1$)-$R_6$- group, wherein $D_2$, n, $A_1$ and $R_6$ are as defined above,
a $D_2$- group, wherein $D_2$ is as defined above, provided that a cyano group is excluded,
a $R_1$($R_1$'$(O)_n$)N-$CR_1$"=N-$R_6$- group, wherein $R_1$, $R_1$', n and $R_6$ are as defined above, $R_1$" is the same as or different from $R_1$ and has the same meaning as $R_1$ has,
a $R_1$-$(O)_n$-N=$CR_1$'-$NR_2$-$R_6$- group, wherein $R_1$, n, $R_1$', $R_2$ and $R_6$ are as defined above,
a $R_2$-$B_3$-$NR_1$-CO-$NR_1$'-$R_6$- group, wherein $R_2$, $B_3$, $R_1$, $R_1$' and $R_6$ are as defined above,

a $D_2$-CO-$NR_1$-$R_6$- group, wherein $D_2$, $R_1$ and $R_6$ are as defined above, or
an $A_2$-COCO-$NR_1$-$R_6$- group, wherein $A_2$, $R_1$ and $R_6$ are as defined above;

(9) the I group:

an $A_7$-$B_6$-$N((O)_nR_1)$-$R_6$- group [wherein $A_7$ represents a C2-C10 alkenyl group optionally substituted with a halogen atom, or a C2-C10 alkynyl group, or a C3-C10 haloalkynyl group, or a $R_2$-$B_1$-$R_4$- group (wherein $R_2$, $B_1$ and $R_4$ are as defined above), or a $D_4$-$R_4$- group (wherein $D_4$ and $R_4$ are as defined above), or a $D_5$-$R_4$- group (wherein $D_5$ and $R_4$ are as defined above), or a $D_1$-$R_4$- group (wherein $D_1$ and $R_4$ are as defined above), or a (b)-$R_4$- group (wherein (b) and $R_4$ are as defined above), or a (c)-$R_4$- group (wherein (c) and $R_4$ are as defined above), or a $D_2$-$R_4$- group (wherein $D_2$ and $R_4$ are as defined above), or a $D_3$-$R_4$- group (wherein $D_3$ and $R_4$ are as defined above), or an $A_4$-$SO_2$-$R_4$- group (wherein $A_4$ and $R_4$ are as defined above), or an $A_2$-CO-$R_4$- group (wherein $A_2$ and $R_4$ are as defined above), $B_6$ represents a carbonyl group or a thiocarbonyl group, and n, $R_1$ and $R_6$ are as defined above],
an $A_8$-CS-$N((O)_nR_1)$-$R_6$- group [wherein $A_8$ represents a hydrogen atom or a C1-C10 alkyl group optionally substituted with a halogen atom, and n, $R_1$ and $R_6$ are as defined above],
an $A_7'$-$B_2'$-$B_3$-$N((O)_nR_1)$-$R_6$- group [wherein $A_7'$ represents a C3-C10 alkenyl group optionally substituted with a halogen atom, or a C3-C10 alkynyl group optionally substituted with a halogen atom, or a $R_2$-$B_1$-$R_4'$- group (wherein $R_2$ and $B_1$ are as defined above, and $R_4'$ represents a C2-C10 alkylene group), or a $D_4$-$R_4'$- group (wherein $D_4$ and $R_4'$ are as defined above), or a $D_1$-$R_4'$ - group (wherein $D_1$ and $R_4'$ are as defined above), or a (b)-$R_4'$- group (wherein (b) and $R_4'$ are as defined above), or a (c)-$R_4'$- group (wherein (c) and $R_4'$ are as defined above), or a $D_2$-$R_4$-group (wherein $D_2$ and $R_4$ are as defined above), or a $D_3$-$R_4'$ - group (wherein $D_3$ and $R_4'$ are as defined above), or an $A_2$-CO-$R_4$- group (wherein $A_2$ and $R_4$ are as defined above), $B_2'$ represents an oxy group, a thio group or a -$N((O)_{n'}R_1')$-group (wherein n' is the same as or different from n and has the same meaning as n has, and $R_1'$ is as defined above), and $B_3$, n, $R_1$ and $R_6$ are as defined above],
an $A_8'$-$B_2'$-CS-$N((O)_nR_1)$-$R_4$- group [wherein $A_8'$ represents a C1-C10 alkyl group or a C2-C10 haloalkyl group, $B_2'$ is as defined above, and n, $R_1$ and $R_6$ are as defined above],
an $A_8'$-S-$B_3'$-$N((O)_nR_1)$-$R_6$- group [wherein $A_8'$, n, $R_1$ and $R_6$ are as defined above, and $B_3'$ represents a carbonyl group or a sulfonyl group] or
an $A_7''$-$SO_2$-$N((O)_nR_1)$-$R_6$- group [wherein $A_7''$ represents a C2-C10 alkenyl group, or a C3-C10 alkenyl group substituted with a halogen atom, or a C3-C10 alkynyl group optionally substituted with a halogen atom, or a $R_2$-$B_1$-$R_4'$- group (wherein $R_2$, $B_1$ and $R_4'$ are as defined above), or a $D_4$-$R_4'$-group (wherein $D_4$ and $R_4'$ are as defined above), or a $D_5$-$R_4$-group (wherein $D_5$ and $R_4$ are as defined above), or a $D_1$-$R_4'$ - group (wherein $D_1$ and $R_4'$ are as defined above), or a (b)-$R_4'$- group (wherein (b) and $R_4'$ are as defined above), or a (c)-$R_4'$- group (wherein (c) and $R_4'$ are as defined above), or a $D_2$-$R_4$- group (wherein $D_2$ and $R_4$ are as defined above), or a $NO_2$-$R_4$- group (wherein $R_4$ is as defined above), or an $A_2$-CO-$R_4$- group (wherein $A_2$ and $R_4$ are as defined above), and n, $R_1$ and $R_4$ are as defined above];

(10) the J group:

an $A_7$-CO- group (wherein $A_7$ is as defined above),
an $A_9$-CS- group (wherein $A_9$ represents $A_7$ or $A_8$),
an $A_9'(O)_mN$=$C(A_9)$- group (wherein $A_9'$ represents $A_7'$ or $A_8'$, and m and $A_9$ are as defined above),
a $D_2$-CO- group (wherein $D_2$ is as defined above),
an $A_2$-COCO- group (wherein $A_2$ is as defined above),
an $A_9$-CO-$B_1'$-$R_6$- group (wherein $A_9$ and $R_6$ are as defined above, and $B_1'$ represents an oxy group or a thio group, provided that $A_9$ is not $A_8$ when $B_1'$ is an oxy group),
an $A_9$-CS-$B_1'$-$R_6$- group (wherein $A_9$, $B_1'$ and $R_6$ are as defined above),
an $A_7''$-$SO_2$-$B_1'$-$R_6$- group (wherein $A_7''$, $B_1'$ and $R_6$ are as defined above),
an $A_8$-$SO_2$-$B_1'$-$R_6$- group (wherein $A_8$, $B_1'$ and $R_6$ are as defined above, provided that $A_8$ is not a hydrogen atom),
an $A_9'$-$B_2'$-$B_3$-$B_1'$-$R_6$- group (wherein $A_9'$, $B_2'$, $B_3$, $B_1'$ and $R_6$ are as defined above), and
a C2-C10 alkenyl group substituted with a (b)- group (wherein (b) is as defined above) or a (c)- group (wherein (c) is as defined above);

(11) the K group: an $A_{10}$-$N((O)_nR_1)$-CO-$R_6$- group [wherein $A_{10}$ represents a hydrogen atom (provided that n is not 0), an $A_7''$-$SO_2$- group (wherein $A_7''$ is as defined above), an $A_8$-$SO_2$- group (wherein $A_8$ is as

defined above, provided that $A_8$ is not a hydrogen atom), an $A_9$'O- group (wherein $A_9$' is as defined above, provided that n is not 1), an $A_9$'-group (wherein $A_9$' is as defined above, provided that $A_8$' is excluded when n is 0), a $R_2OCH_2$- group (wherein $R_2$ is as defined above), an $A_2$-CO-$R_4$- group (wherein $A_2$ and $R_4$ are as defined above) or an $A_2$-CO-CH($CH_2$CO-$A_2$)- group (wherein $A_2$ is as defined above), and n, $R_1$ and $R_6$ are as defined above];

(12) the L group:

an $A_{10}$'-N((O)$_n$$R_1$)-$SO_2$-$R_6$- group [wherein $A_{10}$' represents a hydrogen atom (provided that n is not 0), an $A_9$'O- group (wherein $A_9$' is as defined above, provided that n is not 1), an $A_9$'- group (wherein $A_9$' is as defined above, provided that $A_8$' is excluded when n is 0), a $R_2$-CO- group (wherein $R_2$ is as defined above), an $A_2$-CO-$R_4$- group (wherein $A_2$ and $R_4$ are as defined above) or an $A_2$-CO-CH ($CH_2$CO-$A_2$)- group (wherein $A_2$ is as defined above), and n, $R_1$ and $R_6$ are as defined above],
an $A_9$"$R_1$N-$SO_2$-N((O)$_n$$R_1$')-$R_6$- group [wherein $A_9$" represents a hydrogen atom or an $A_9$'- group (wherein $A_9$' is as defined above), and $R_1$, n, $R_1$' and $R_6$ are as defined above], and
a (b)-$SO_2$-N((O)$_n$$R_1$')-$R_6$- group [wherein (b), n, $R_1$' and $R_6$ are as defined above];

(13) the M group:

a $R_1$($R_2$S)C=N-$R_6$- group (wherein $R_1$, $R_2$ and $R_6$ are as defined above),
a $R_2$B($R_2$'B')C=N-$R_6$- group (wherein $R_2$ and $R_6$ are as defined above, $R_2$' is the same as or different from $R_2$ and has the same meaning as $R_2$ has, and B and B' are the same or different and represent an oxy group or a thio group),
a $R_1R_1$'N-($R_2$S)C=N-$R_6$- group (wherein $R_1$, $R_1$', $R_2$ and $R_6$ are as defined above),
a $R_1$N=C($SR_2$)-$NR_2$'-$R_6$- group (wherein $R_1$, $R_2$, $R_2$' and $R_6$ are as defined above), and
a $R_1$($R_1$'O)N-$R_6$- group (wherein $R_1$, $R_1$' and $R_6$ are as defined above);

(14) the N group: an $A_{11}$-P(=O) ($OR_1$')-$R_4$- group
[wherein $A_{11}$ represents a $R_1$- group (wherein $R_1$ is as defined above), a $R_1$O-$R_6$- group (wherein $R_1$ and $R_6$ are as defined above) or a $R_1$OCO-$CHR_0$- group (wherein $R_1$ and $R_0$ are as defined above), and $R_1$' and $R_4$ are as defined above];

III. in ($Y_A$)q, $Y_A$ is a substituent on a carbon atom and represents a group included in the following X group or Y group, q represents 0, 1, 2, 3, 4 or 5, and the sum of p (wherein p is as defined above) and q is not more than 5, and when q is not less than 2, $Y_A$s are the same or different, and when q is not less than 2, adjacent two same or different $Y_A$s may together form a group included in the Z group to be fused to the A ring;

(1) the X group: a $M_a$-group, wherein $M_a$ represents a $R_b$- group (wherein $R_b$ represents a C1-C10 alkyl group optionally substituted with a halogen atom), a halogen atom, a nitro group, a cyano group, a $R_c$-$B_a$-$R_d$- group (wherein $R_c$ represents a C1-C10 alkyl group optionally substituted with a halogen atom, $B_a$ represents an oxy group, a thio group, a sulfinyl group or a sulfonyl group, and $R_d$ represents a single bond or a C1-C10 alkylene group), an $HOR_d$- group (wherein $R_d$ is as defined above), a $R_e$-CO-$R_d$- group (wherein $R_e$ represents a hydrogen atom, or a C1-C10 alkyl group optionally substituted with a halogen atom, and $R_d$ is as defined above), a $R_e$-CO-O-$R_d$- group (wherein $R_e$ and $R_d$ are as defined above), a $R_e$O-CO-$R_d$- group (wherein $R_e$ and $R_d$ are as defined above), an HO-CO-CH=CH- group, a $R_eR_e$'N-$R_d$- group (wherein $R_e$ and $R_e$' are the same or different, $R_e$ is as defined above, $R_e$' has the same meaning as $R_e$ has, and $R_d$ is as defined above), a $R_e$-CO-$NR_e$'-$R_d$- group (wherein $R_e$, $R_e$' and $R_d$ are as defined above), a $R_b$O-CO-N($R_e$)-$R_d$- group (wherein $R_b$, $R_e$ and $R_d$ are as defined above), a $R_eR_e$'N-CO-$R_d$-group (wherein $R_e$, $R_e$' and $R_d$ are as defined above), a $R_eR_e$'N-CO-$NR_e$"-$R_d$- group (wherein $R_e$, $R_e$' and $R_e$" are the same or different, $R_e$ and $R_e$' are as defined above, $R_e$" has the same meaning as $R_e$ has, and $R_d$ is as defined above), a $R_eR_e$'N-C(=$NR_e$")-$NR_e$"'-$R_d$- group (wherein $R_e$, $R_e$', $R_e$" and $R_e$"' are the same or different. $R_e$, $R_e$' and $R_e$" are as defined above, $R_e$"' has the same meaning as $R_e$ has, and $R_d$ is as defined above), a $R_b$-$SO_2$-$NR_e$-$R_d$- group (wherein $R_b$, $R_e$ and $R_d$ are as defined above), a $R_eR_e$'N-$SO_2$-$R_d$- group (wherein $R_e$, $R_e$' and $R_d$ are as defined above), a C2-C10 alkenyl group or a C2-C10 alkynyl group;
(2) the Y group: a $M_b$-$R_d$- group, wherein $M_b$ represents a $M_c$-group [wherein $M_c$ represents a $M_d$-$R_d$'- group {wherein $M_d$ represents a phenyl group optionally substituted with a $M_a$- group (wherein $M_a$ is as defined above), a pyridyl group optionally substituted with a $M_a$- group (wherein $M_a$ is as defined above), a naphthyl group optionally substituted with a $M_a$- group (wherein $M_a$ is as defined above), a (b)-group (wherein (b) is as defined above), a (c)- group (wherein (c) is as defined above), a (d)- group

(d) 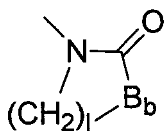

(wherein 1 is 2, 3 or 4, $B_b$ represents an oxy group or a thio group) or an (e)- group

(e) 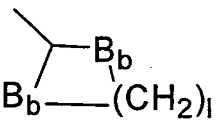

(wherein 1 and $B_b$ are as defined above)}, and $R_d$' is the same as or different from $R_d$ and has the same meaning as $R_d$ has], a $M_c$-$B_a$- group (wherein $M_c$ and $B_a$ are as defined above), a $M_c$-CO- group (wherein $M_c$ is as defined above), a $M_c$-CO-O- group (wherein $M_c$ is as defined above), a $M_c$O-CO-group (wherein $M_c$ is as defined above), a $M_cR_e$N- group (wherein $M_c$ and $R_e$ are as defined above), a $M_c$-CO-N$R_e$- group (wherein $M_c$ and $R_e$ are as defined above), a $M_c$O-CO-N$R_e$-group (wherein $M_c$ and $R_e$ are as defined above), a $M_cR_e$N-CO-group (wherein $M_c$ and $R_e$ are as defined above), a $M_cR_e$N-CO-N$R_e$'- group (wherein $M_c$, $R_e$ and $R_e$' are as defined above), a $M_cR_e$N-C(=N$R_e$')-N$R_e$"- group (wherein $M_c$, $R_e$, $R_e$' and $R_e$" are as defined above), a $M_c$-$SO_2$-N$R_e$- group (wherein $M_c$ and $R_e$ are as defined above) or a $M_cR_e$N-$SO_2$-group (wherein $M_c$ and $R_e$ are as defined above), and

$R_d$ is as defined above;

(3) the Z group: a -N=C($Y_a$)-$Y_a$'- group (wherein $Y_a$ represents a hydrogen atom, or a C1-C10 alkyl group optionally substituted with a halogen atom, or a C1-C10 alkoxy group, and $Y_a$' represents an oxy group, or a thio group, or an imino group optionally substituted with a Cl-C10 alkyl group),

a -$Y_b$-$Y_b$'-$Y_b$"- group (wherein $Y_b$ and $Y_b$" are the same or different, and represent a methylene group, or an oxy group, or a thio group, or a sulfinyl group, or an imino group optionally substituted with a C1-C10 alkyl group, and $Y_b$' represents a C1-C4 alkylene group optionally substituted with a halogen atom, or a C1-C4 alkylene group optionally substituted with an oxo group), or

a -$Y_c$-O-$Y_c$'-O- group (wherein $Y_c$ and $Y_c$' are the same or different, and represent a C1-C10 alkylene group);

IV. B is

a group represented by formula (IV-1):

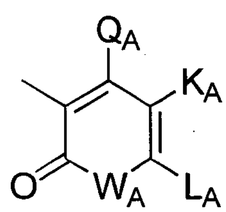 (IV-1)

wherein,

(1) $Q_A$ represents a hydroxyl group, a (b)- group (wherein (b) is as defined above), an $A_9$-$B_6$-$B_c$- group [wherein $A_9$ and $B_6$ are as defined above, and $B_c$ represents an oxy group or a -N((O)$_m$$R_1$)- group (wherein m and $R_1$ are as defined above), provided that $B_c$ is not a sulfonyl group when $A_9$ is a hydrogen atom], an $A_7$"-$SO_2$-$B_c$- group (wherein $A_7$" and $B_c$ are as defined above), an $A_8$-$SO_2$-$B_c$- group (wherein $A_8$ and $B_c$ are as defined above, provided that $A_B$ is not a hydrogen atom), a $R_1R_1$'N-$SO_2$-$B_c$- group (wherein $R_1$, $R_1$' and $B_c$ are as defined above), a ($b_0$)-$SO_2$-O- group (wherein (b) and $B_c$ are as defined above), an $A_9$'-$B_c$-group (wherein $A_9$' and $B_c$ are as defined above), a $D_5$-$R_4$-$B_c$- group (wherein $D_5$, $R_4$ and $B_c$ are as defined above), a $M_c$-$B_3$-$B_c$-group (wherein $M_c$, $B_3$ and $B_c$ are as defined above) or a $M_c$-$B_c$- group (wherein $M_c$ and $B_c$ are as defined above),

(2) $W_A$ represents an oxygen atom or a -N$T_A$- group [wherein $T_A$ represents a hydrogen atom, an $A_9$'- group

(wherein $A_9$' is as defined above), a $D_5$-$R_4$- group (wherein $D_5$ and $R_4$ are as defined above) or a $M_c$- group (wherein $M_c$ is as defined above)], and

(3) $K_A$ and $L_A$ form a $-V_A=V_A'-V_A''=V_A'''-$ group {wherein $V_A$, $V_A'$, $V_A''$ and $V_A'''$ are the same or different, and represent a methine group optionally substituted with a $M_a$-group (wherein $M_a$ is as defined above), or a -N= group, and at least one of $V_A$, $V_A'$, $V_A''$ and $V_A'''$ represents a -N= group};

a group represented by formula (IV-2):

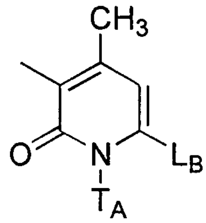

(IV-2)

wherein $T_A$ is as defined above, and $L_B$ represents a hydroxyl group or a methyl group;

a group represented by formula (IV-3):

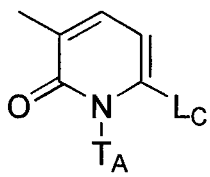

(IV-3)

wherein $T_A$ is as defined above, and $L_C$ represents a C1-C10 alkyl group;

a group represented by formula (IV-4):

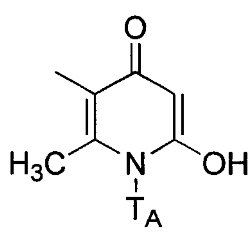

(IV-4)

wherein $T_A$ is as defined above;

a group represented by formula (IV-5):

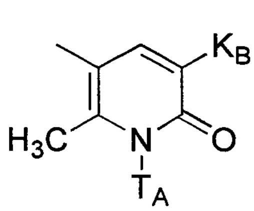

(IV-5)

wherein $T_A$ is as defined above, and $K_B$ represents a cyano group or a UOCO- group (wherein U represents a hydrogen atom or C1-C10 alkyl group);

a group represented by formula (IV-6):

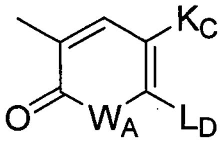

(IV-6)

wherein $W_A$ is as defined above, and $K_C$ and $L_D$ form a C3-C10 alkylene group, or a C4-C10 alkenylene group optionally substituted with 1 or more same or different $M_a$- groups (wherein $M_a$ is as defined above);
a group represented by formula (IV-7):

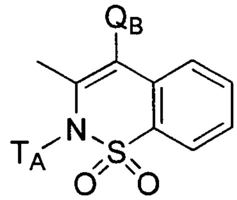

(IV-7)

wherein $T_A$ is as defined above, $Q_B$ represents a hydroxyl group, an $A_9$-$B_6$-O- group [wherein $A_9$ and $B_6$ are as defined above], an $A_7$"-$SO_2$-O- group (wherein $A_7$" is as defined above), an $A_8$-$SO_2$-O- group (wherein $A_8$ is as defined above, provided that $A_8$ is not a hydrogen atom), a $R_1R_1$' N-$SO_2$-O-group (wherein $R_1$ and $R_1$' are as defined above), a (b)-$SO_2$-O- group (wherein (b) is as defined above), an $A_9$'-O- group (wherein $A_9$' is as defined above), a $D_5$-$R_4$-O- group (wherein $D_5$ and $R_4$ are as defined above), a $M_c$-$B_3$-O- group (wherein $M_c$ and $B_3$ are as defined above), or a $M_c$-O- group (wherein $M_c$ is as defined above); or
a group represented by formula (IV-8)

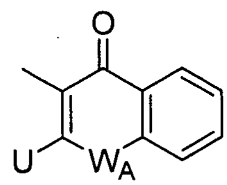

(IV-8)

wherein U and $W_A$ are as defined above; and
the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range].

**5.** A cinnamoyl compound represented by the formula (V):

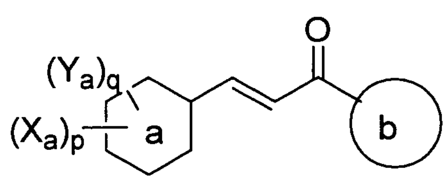

(V)

wherein,

I. a represents a benzene ring or a pyridine ring, $X_a$ is a substituent on a carbon atom and represents a Cl-C10 alkyl group substituted with a cyano group, or a C1-C10 alkyl group substituted with a tetrahydropyran-4-ylidene group, or a C2-C10 alkenyl group substituted with a halogen atom or a cyano group, or a C2-C10 alkenyl group substituted with a C1-C10 alkoxycarbonyl group, or a C3-C10 alkynyl group substituted with a hydroxyl group,

or an $a_0$-$r_1$-b-$r_1$'- group {wherein $a_0$ represents a methyl group substituted with a C1-C10 alkylthio group, a methyl group substituted with a C1-C10 alkylsulfinyl group, a methyl group substituted with a C1-C10 alkylsulfonyl group, a C2-C10 alkenyl group, a C2-C10 alkynyl group, a $r_2$O-CO- group (wherein $r_2$ represents a C1-C10 alkyl group or a C2-C10 alkyl group substituted with a hydroxyl group), a carboxy group, a rr'N-CO- group (wherein r and r' are the same or different, and respresent a hydrogen atom or a C1-C10 alkyl group), $a_1$-NH-CO- group (wherein $a_1$ represents a C2-C10 alkyl group substituted with a C1-C10 alkoxy group), an $a_1$'-CO-group (wherein $a_1$' represents a morpholino group), a rr'N-$CH_2$- group (wherein r and r' are as defined above), a $r_0$-$(O)_1$-CONH-$CH_2$- group (wherein $r_0$ represents a C1-C10 alkyl group, and 1 represents 0 or 1), a r-$OCH_2$-group (wherein r is as defined above), a $r_0$-CO- group (wherein $r_0$ is as defined above), a cyano group or a sulfomethyl group, $r_1$ represents a C1-C10 alkylene group, $r_1$' represents a single bond or a C1-C10 alkylene group, and b represents an oxy group, a thio group, a sulfinyl group, a sulfonyl group, or an imino group}, or an $a_2$-y-CO-NH- group (wherein $a_2$ represents a C2-C10 alkyl group substituted with a C1-C10 alkoxy group, and y represents an oxy group or an imino group), or a rO-COCO-NH- group (wherein r is as defined above), or an $a_3$-z-NH- group (wherein $a_3$ represents a C2-C10 alkenyl group, or a C1-C10 alkyl group substituted with a C1-C10 alkoxy group, a C1-C10 alkoxycarbonyl group, a carboxy group or a cyano group, and z represents a carbonyl group or a sulfonyl group), or an $a_4$-NHCO- group {wherein $a_4$ represents a C1-C10 alkoxy group, or a C3-C10 alkenyloxy group, or a $r_0$-$SO_2$- group (wherein $r_0$ is as defined above), or a C2-C10 alkyl group substituted with a hydroxyl group or a C1-C10 alkoxy group, or a C2-C10 alkyl group substituted with a $r_0r_0$'N-group (wherein $r_0$ is as defined above, and $r_0$' is the same as or different from $r_0$, and represents the same meaning as $r_0$ has), or a C1-C10 alkyl group substituted with a rO-CO- group (wherein r is as defined above), a cyano group or an aminocarbonyl group, or a rO-CO-(rO-$COCH_2$)CH- group (wherein r is as defined above)}, or an $a_5$-$NHSO_2$- group (wherein $a_5$ represents a C2-C10 alkyl group substituted with a C1-C10 alkoxyl group), or a $r_0$ON=CH- group (wherein $r_0$ is as defined above), or a $r_0$NHCSNH- group (wherein $r_0$ is as defined above), or a $r_0$NHC(-S$r_0$')=N- group (wherein $r_0$ and $r_0$' are as defined above), or a $(rO)_2P(=O)CH_2$- group (wherein r is as defined above), p represents 0, 1, 2 or 3 and, and when p is not less than 2, $X_a$s are same or different,

$Y_a$ represents are halogen atom, or a C1-C10 alkyl group optionally substituted with a halogen atom, or a C1-C10 alkyl group optionally substituted with a C1-C10 alkoxy group, or a C2-C10 alkenyl group, or a C2-C10 alkynyl group, or a 2-oxo-oxazolidin-3-yl group, or a [1,3]dioxolan-2-yl group, or a C1-C10 alkoxy group substituted with a morpholino group, or an $a_0$'-b'- group (wherein $a_0$' represents a C1-C10 alkyl group optionally substituted with a halogen atom, and b' represents an oxy group, a thio group, a sulfinyl group or a sulfonyl group), or a nitro group, or a cyano group, or a rO-CO- group (wherein r is as defined above), or a $r_0r_0$'N- group (wherein $r_0$ and $r_0$' are as defined above), or a $r_0$CO-NH- group (wherein $r_0$ is as defined above), or a $r_0r_0$'NCONH-group (wherein $r_0$ and $r_0$' are as defined above), or a rr'NCO- group (wherein r and r' are as defined above), or a hydroxyl group, q represents 0, 1, 2 or 3, and when q is not less than 2, $Y_a$s are the same or different, and when q is not less than 2, adjacent $Y_a$s may be fused to the a ring to form a 4,5,6,7-tetrahydrobenzo[b]thiophene ring;

II. b is a group represented by formula (V-1):

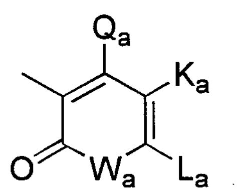
(V-1)

wherein $Q_a$ represents a $r_a$-O- group {$r_a$ represents a hydrogen atom, or a C1-C10 alkyl group, or a C3-C10 alkenyl group, or a C3-C10 alkynyl, or a C1-C10 alkyl group substituted with a $r_0r_0$'N-$CH_2$- group (wherein $r_0$ and $r_0$' are the same or different), a $rOCH_2$ group (wherein r is as defined above), a $r_0$-CO- group (wherein $r_0$ is as defined above), a C1-C10 alkoxycarbonyl group, a carboxy group, an aminocarbonyl group or a cyano group, or a $r_3$-$r_1$- group (wherein $r_3$ represents a phenyl group or a pyridyl group, and $r_1$ is as defined above)}, or a piperidino group, or a morpholino group, or a $r_4r_4$'N-group (wherein $r_4$ and $r_4$' are the same of different, and represent a hydrogen atom, or a C1-C10 alkyl group, or C3-C10 alkenyl group, or a C3-C10 alkynyl group, or a C2-C10 alkyl group substituted with a C1-C10 alkoxy group, provided that they are not a hydrogen atom at the same time); $W_a$ represents an oxygen atom or a - $NT_a$- group [wherein $T_a$ represents a $r_b$- group (wherein $r_b$ is the same as or different from $r_a$, and represents the same meaning as $r_a$ has) or a $r_3$'- group (wherein $r_3$' is the same as or different from $r_3$, and represents the same meaning as $r_3$ has)], and $K_a$ and $L_a$ form a -$V_a$=$V_a$'-$V_a$''=$V_a$'''- group (wherein $V_a$, $V_a$', $V_a$'' and $V_a$''' are the same or different, and represent a methine group or a

-N= group, and at least one of $V_a$, $V_a$'$V_a$" and $V_a$"' represents a -N= group);
a group represented by formula (V-2):

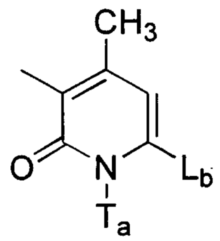

(V-2)

wherein $T_a$ is as defined above, and $L_b$ represents a hydroxyl group or a methyl group;
a group represented by formula (V-3):

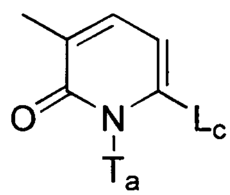

(V-3)

wherein $T_a$ is as defined above, and $L_c$ represents a C1-C10 alkyl group;
a group represented by formula (V-4):

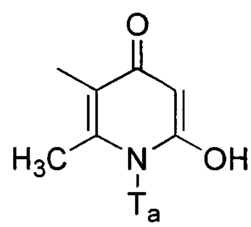

(V-4)

wherein $T_a$ is as defined above;
a group represented by formula (V-5):

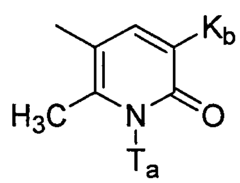

(V-5)

wherein $T_a$ is as defined above, and $K_b$ represents a cyano group or a UOCO- group (wherein U represents a hydrogen atom or C1-C10 alkyl group);
a group represented by formula (V-6):

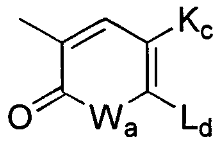

(V-6)

wherein $W_a$ is as defined above, and $K_c$ and $L_d$ form a C3-C10 alkylene group or a C4-C10 alkenylene group; a group represented by formula (V-7):

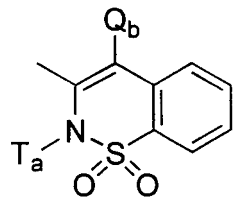

(V-7)

wherein $T_a$ is as defined above, and $Q_b$ represents a $r_a$-O-group (wherein $r_a$ is as defined above); or a group represented by formula (V-8):

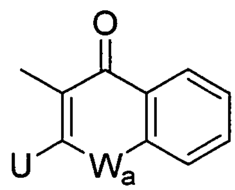

(V-8)

wherein U and $W_a$ are as defined above; and
the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range.

**6.** A process for producing a cinnamoyl compound represented by formula (VI'):

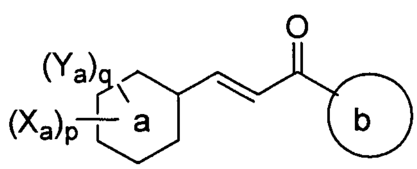

(VI')

wherein a, $X_a$, $Y_a$, p and q are defined below, and
b represents a group represented by formula (VI'-1):

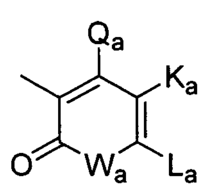

(VI'-1)

wherein $Q_a$, $W_a$, $K_a$ and $L_a$ are as defined below, a group represented by formula (VI'-2):

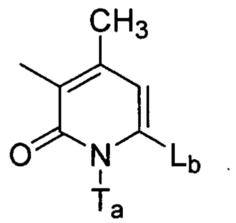

(VI'-2)

wherein $T_a$ and $L_b$ are as defined below, a group represented by formula (VI'-3):

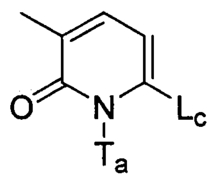

(VI'-3)

wherein $T_a$ and $L_c$ are as defined below, a group represented by formula (VI'-4):

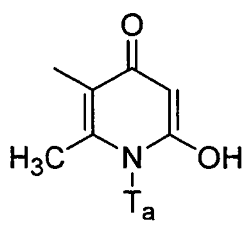

(VI'-4)

wherein $T_a$ is as defined below, a group represented by formula (VI'-5) :

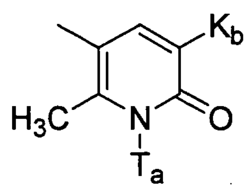

(VI'-5)

wherein $T_a$ and $K_b$ are as defined below, a group represented by formula (VI'-6):

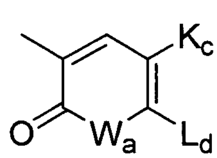

(VI'-6)

wherein $W_a$, $K_c$ and $L_d$ are as defined below, a group represented by formula (VI'-7):

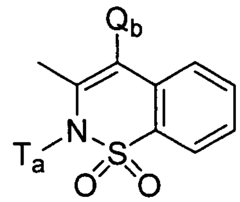

(VI'-7)

wherein $T_a$ and $Q_b$ are as defined below, or a group represented by formula (VI'-8):

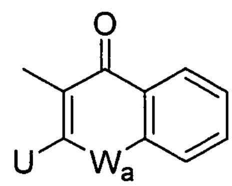

(VI'-8)

wherein U and $W_a$ are as defined below; which comprises reacting a benzaldehyde derivative represented by the formula (VI):

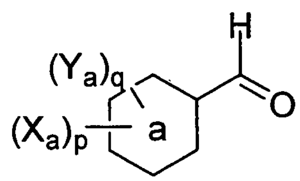

(VI)

[wherein,

I. a represents a benzene ring or a pyridine ring, $X_a$ is a substituent on a carbon atom and represents a C1-C10 alkyl group substituted with a cyano group, or a C1-C10 alkyl group substituted with a tetrahydropyran-4-ylidene group, or a C2-C10 alkenyl group substituted with a halogen atom or a cyano group, or a C2-C10 alkenyl group substituted with a C1-C10 alkoxycarbonyl group, or a C3-C10 alkynyl group substituted with a hydroxyl group, or an $a_0$-$r_1$-b-$r_1$'- group {wherein $a_0$ represents a methyl group substituted with a C1-C10 alkylthio group, a methyl group substituted with a C1-C10 alkylsulfinyl group, a methyl group substituted with a C1-C10 alkylsulfonyl group, a C2-C10 alkenyl group, a C2-C10 alkynyl group, a $r_2$O-CO- group (wherein $r_2$ represents a C1-C10 alkyl group or a C2-C10 alkyl group substituted with a hydroxyl group), a carboxy group, a rr'N-CO- group (wherein r and r' are the same or different, and respresent a hydrogen atom or a C1-C10 alkyl group), $a_1$-NH-CO- group (wherein $a_1$ represents a C2-C10 alkyl group substituted with a C1-C10 alkoxy group), an $a_1$'-CO- group (wherein $a_1$' represents a morpholino group), a rr'N-$CH_2$- group (wherein r and r' are as defined above), a $r_0$-$(O)_1$-CONH-$CH_2$- group (wherein $r_0$ represents a C1-C10 alkyl group, and 1 represents 0 or 1), a r-$OCH_2$-group (wherein r is as defined above), a $r_0$-CO- group (wherein $r_0$ is as defined above), a cyano group or a sulfomethyl group, $r_1$ represents a C1-C10 alkylene group, $r_1$' represents a single bond or a C1-C10 alkylene group, and b represents an oxy group, a thio group, a sulfinyl group, a sulfonyl group, or an imino group}, or an $a_2$-y-CO-NH- group (wherein $a_2$ represents a C2-C10 alkyl group substituted with a C1-C10 alkoxy group, and y represents an oxy group or an imino group), or a rO-COCO-NH- group (wherein r is as defined above), or an $a_3$-z-NH- group (wherein $a_3$ represents a C2-C10 alkenyl group, or a C1-C10 alkyl group substituted with a C1-C10 alkoxy group, a C1-C10 alkoxycarbonyl group, a carboxy group or a cyano group, and z represents a carbonyl group or a sulfonyl group), or an $a_4$-NHCO- group {wherein $a_4$ represents a C1-C10 alkoxy group, or a C3-C10 alkenyloxy group, or a $r_0$-$SO_2$- group (wherein $r_0$ is as defined above), or a C2-C10 alkyl group substituted with a hydroxyl group or a C1-C10 alkoxy group, or a C2-C10 alkyl group substituted with a $r_0r_0$'N-group (wherein $r_0$ is as defined above, and $r_0$' is the same as or different from $r_0$, and represents the same meaning as $r_0$ has), or a C1-C10 alkyl group substituted with a rO-CO- group (wherein r is as defined above), a cyano group or an aminocarbonyl group, or a rO-CO-(rO-$COCH_2$)CH- group (wherein r is as defined above)},

or an $a_5$-$NHSO_2$- group (wherein $a_5$ represents a C2-C10 alkyl group substituted with a C1-C10 alkoxyl group), or a $r_0$ON=CH- group (wherein $r_0$ is as defined above), or a $r_0$NHCSNH- group (wherein $r_0$ is as defined above), or a $r_0$NHC(-S$r_0$')=N- group (wherein $r_0$ and $r_0$' are as defined above), or a $(rO)_2P(=O)CH_2$- group (wherein r is as defined above), p represents 0, 1, 2 or 3 and, and when p is not less than 2, $X_a$s are same or different, $Y_a$ represents are halogen atom, or a C1-C10 alkyl group optionally substituted with a halogen atom, or a C1-C10 alkyl group optionally substituted with a C1-C10 alkoxy group, or a C2-C10 alkenyl group, or a C2-C10 alkynyl group, or a 2-oxo-oxazolidin-3-yl group, or a [1,3]dioxolan-2-yl group, or a C1-C10 alkoxy group substituted with a morpholino group, or an $a_0$'-b'- group (wherein $a_0$' represents a C1-C10 alkyl group optionally substituted with a halogen atom, and b' represents an oxy group, a thio group, a sulfinyl group or a sulfonyl group), or a nitro group, or a cyano group, or a rO-CO- group (wherein r is as defined above), or a $r_0r_0$'N- group (wherein $r_0$ and $r_0$' are as defined above), or a $r_0$CO-NH- group (wherein $r_0$ is as defined above), or a $r_0r_0$'NCONH- group (wherein $r_0$ and $r_0$' are as defined above), or a rr'NCO- group (wherein r and r' are as defined above), or a hydroxyl group, q represents 0, 1, 2 or 3, and when q is not less than 2, $Y_a$s are the same or different, and when q is not less than 2, adjacent $Y_a$s may be fused to the a ring to form a 4,5,6,7-tetrahydrobenzo[b]thiophene ring] with a compound represented by formula (VI-1):

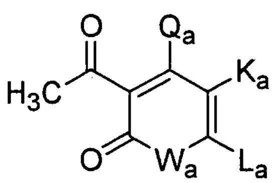

(VI-1)

[wherein

$Q_a$ represents a $r_a$-O- group {$r_a$ represents a hydrogen atom, or a C1-C10 alkyl group, or a C3-C10 alkenyl group, or a C3-C10 alkynyl, or a C1-C10 alkyl group substituted with a $r_0r_0$'N-$CH_2$- group (wherein $r_0$ and $r_0$' are the same or different), a $rOCH_2$ group (wherein r is as defined above), a $r_0$-CO- group (wherein $r_0$ is as defined above), a C1-C10 alkoxycarbonyl group, a carboxy group, an aminocarbonyl group or a cyano group, or a $r_3$-$r_1$- group (wherein $r_3$ represents a phenyl group or a pyridyl group, and $r_1$ is as defined above)}, or a piperidino group, or a morpholino group, or a $r_4r_4$'N-group (wherein $r_4$ and $r_4$' are the same of different, and represent a hydrogen atom, or a C1-C10 alkyl group, or C3-C10 alkenyl group, or a C3-C10 alkynyl group, or a C2-C10 alkyl group substituted with a C1-C10 alkoxy group, provided that they are not a hydrogen atom at the same time); $W_a$ represents an oxygen atom or a -$NT_a$- group [wherein $T_a$ represents a $r_b$- group (wherein $r_b$ is the same as or different from $r_a$, and represents the same meaning as $r_a$ has) or a $r_3$'- group (wherein $r_3$' is the same as or different from $r_3$, and represents the same meaning as $r_3$ has)], and $K_a$ and $L_a$ form a -$V_a$=$V_a$'-$V_a$''=$V_a$'''- group (wherein $V_a$, $V_a$', $V_a$'' and $V_a$''' are the same or different, and represent a methine group or a -N= group, and at least one of $V_a$, $V_a$'$V_a$'' and $V_a$''' represents a -N= group)], a compound represented by formula (VI-2):

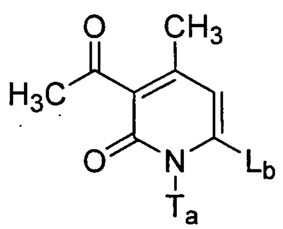

(VI-2)

[wherein $T_a$ is as defined above, and $L_b$ represents a hydroxyl group or a methyl group], a compound represented by formula (VI-3):

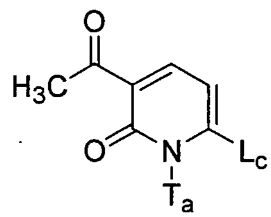

(VI-3)

[wherein $T_a$ is as defined above, and $L_c$ represents a C1-C10 alkyl group], a compound represented by formula (VI-4):

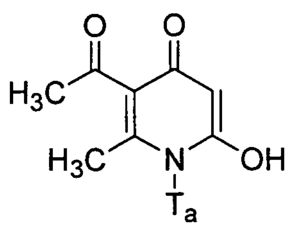

(VI-4)

[wherein $T_a$ is as defined above], a compound represented by formula (VI-5):

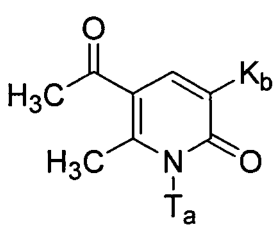

(VI-5)

[wherein $T_a$ is as defined above, and $K_b$ represents a cyano group or a UOCO- group (wherein U represents a hydrogen atom or C1-C10 alkyl group)], a compound represented by formula (VI-6):

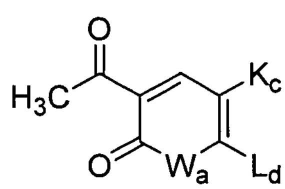

(VI-6)

[wherein $W_a$ is as defined above, and $K_c$ and $L_d$ form a C3-C10 alkylene group or a C4-C10 alkenylene group], a compound represented by formula (VI-7):

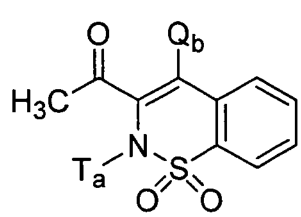

(VI-7)

[wherein $T_a$ is as defined above, and $Q_b$ represents a $r_a$-O-group (wherein $r_a$ is as defined above)], or a compound represented by formula (VI-8):

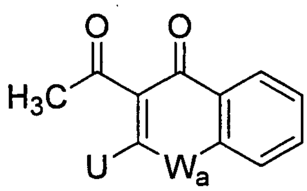

(VI-8)

[wherein U and $W_a$ are as defined above]; and
the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range.

**7.** A process for producing a compound represented by formula (VII-3):

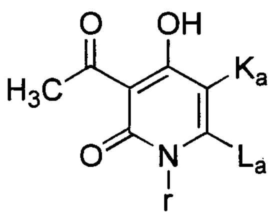

(VII-3)

[wherein r, $K_a$ and $L_a$ are as defined below] which comprises 3-oxobutyrylamidating a compound represented by formula (VII-1):

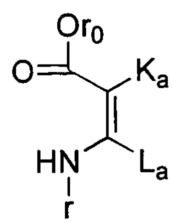

(VII-1)

[wherein r represents a hydrogen atom or a C1-C10 alkyl group, $r_0$ represents a C1-10 alkyl group, $K_a$ and $L_a$ form a - $V_a$=$V_a$'-$V_a$"=$V_a$'''- group (wherein $V_a$, $V_a$', $V_a$" and $V_a$''' are the same or different, represent a methine group or a -N= group, and at least one of $V_a$, $V_a$', $V_a$" and $V_a$''' represents a -N= group)] to produce a compound represented by formula (VII-2):

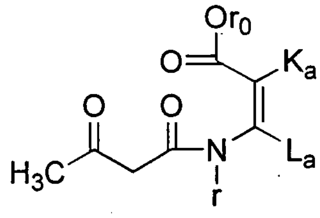

(VII-2)

[wherein r, $r_0$, $K_a$ and $L_a$ are as defined above], and then cyclizing the compound represented by the formula (VII-2).

**8.** A process for producing a compound represented by formula (VIII-2):

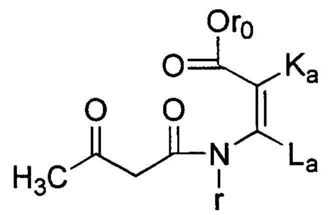

(VIII-2)

[wherein r, $r_0$, $K_a$ and $L_a$ are as defined below] which comprises 3-oxobutyrylamidating a compound represented by formula (VIII-1):

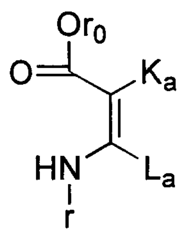

(VIII-1)

[wherein r represents a hydrogen atom or a C1-C10 alkyl group, $r_0$ represents a C1-10 alkyl group, $K_a$ and $L_a$ form a - $V_a$=$V_a$'-$V_a$"=$V_a$"'- group (wherein $V_a$, $V_a$', $V_a$" and $V_a$"' are the same or different, represent a methine group or a -N= group, and at least one of $V_a$, $V_a$', $V_a$" and $V_a$"' , represents a -N= group)].

**9.** A compound represented by the formula (IX):

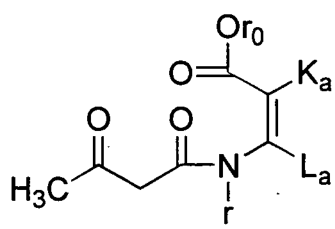

(IX)

wherein r represents a hydrogen atom or a C1-C10 alkyl group, $r_0$ represents a C1-10 alkyl group, $K_a$ and $L_a$ form a - $V_a$=$V_a$'-$V_a$"=$V_a$"'- group (wherein $V_a$, $V_a$', $V_a$" and $V_a$"' are the same or different, represent a methine group or a -N= group, and at least one of $V_a$, $V_a$', $V_a$" and $V_a$"' represents a -N= group).

**10.** A compound represented by the formula (X):

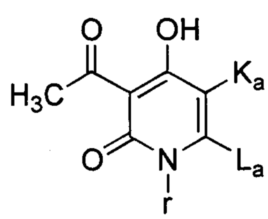

(X)

wherein r represents a hydrogen atom or a C1-C10 alkyl group, $K_a$ and $L_a$ form a -$V_a$=$V_a$'-$V_a$"=$V_a$"'- group (wherein $V_a$, $V_a$', $V_a$" and $V_a$"' are the same or different and represent a methine group or a -N= group, and at least one of $V_a$, $V_a$', $V_a$" and $V_a$"' represents a -N= group).

**11.** A process for producing a compound represented by formula (XI-2):

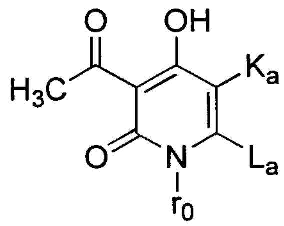

(XI-2)

[wherein $r_0$ represents a C1-C10 alkyl group, and $K_a$ and $L_a$ are as defined below] which comprises alkylating a compound representd by formula (XI-1):

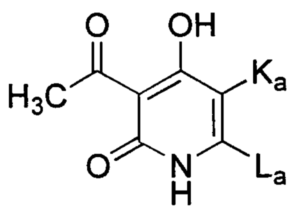

(XI-1)

[wherein $K_a$ and $L_a$ form a $-V_a=V_a'-V_a''=V_a'''-$ group (wherein $V_a$, $V_a'$, $V_a''$ and $V_a'''$ are the same or different, and represent a methine group or a -N= group, and at least one of $V_a$, $V_a'$, $V_a''$ and $V_a'''$ represents a -N= group)].

**12.** A composition for suppressing transcription of an extracellular matrix gene, which comprises an inert carrier and a cinnamoyl compound represented by formula (I'):

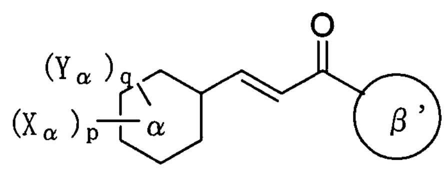

(I')

wherein,

I. $\alpha$ represents a benzene ring or a pyridine ring; in $(Y_\alpha)_q$, $Y_\alpha$ is a substituent on a carbon atom and represents a group included in the following $X_0$ group or the $Y_0$ group, q represents 0, 1, 2, 3, 4 or 5, and when q is not less than 2, $Y_\alpha$s are the same or different, and when q is not less than 2, adjacent two same or different $Y_\alpha$s may together form a group included in the $Z_0$ group to be fused to the $\alpha$ ring; and in $(X_\alpha)_p$, $X_\alpha$ is a substituent on a carbon atom and represents a group which does not belong to the following $X_0$ group, $Y_0$ group and $Z_0$ group, p represents 0, 1, 2, 3, 4 or 5, and when p is not less than 2, $X_\alpha$s are the same or different and the sum of p and q is not more than 5;

(1) the $X_0$ group: a $M_a$-group, wherein $M_a$ represents a $R_b$- group (wherein $R_b$ represents a C1-C10 alkyl group optionally substituted with a halogen atom), a halogen atom, a nitro group, a cyano group, a hydroxyl group, a $R_c$-$B_a$-$R_d$-group (wherein $R_c$ represents a C1-C10 alkyl group optionally substituted with a halogen atom, $B_a$ represents an oxy group, a thio group, a sulfinyl group or a sulfonyl group, and $R_d$ represents a single bond or a C1-C10 alkylene group), an $HOR_d$- group (wherein $R_d$ is as defined above), a $R_e$-CO-$R_d$- group (wherein $R_e$ represents a hydrogen atom, or a C1-C10 alkyl group optionally substituted with a halogen atom, and $R_d$ is as defined above), a $R_e$-CO-O-$R_d$- group (wherein $R_e$ and $R_d$ are as defined above), a $R_e$O-CO-$R_d$- group (wherein $R_e$ and $R_d$ are as defined above), an HO-CO-CH=CH-group, a $R_eR_e'$N-$R_d$- group (wherein $R_e$ and $R_e'$ are the same or different, $R_e$ is as defined above, $R_e'$ has the same meaning as $R_e$ has, and $R_d$ is as defined above), a $R_e$-CO-N$R_e'$-$R_d$- group (wherein $R_e$, $R_e'$ and $R_d$ are as defined above), a $R_b$O-CO-N($R_e$)-$R_d$- group (wherein $R_b$, $R_e$ and $R_d$ are as defined above), a $R_eR_e'$N-CO-$R_d$- group (wherein $R_e$, $R_e'$ and $R_d$ are as defined above), a $R_eR_e'$N-CO-N$R_e''$-$R_d$- group (wherein $R_e$, $R_e'$ and $R_e''$ are the same or different, $R_e$ and $R_e'$ are as defined above, $R_e''$ has the same meaning as $R_e$ has,

and $R_d$ is as defined above), a $R_eR_e'N-C(=NR_e'')-NR_e'''-R_d$- group (wherein $R_e$, $R_e'$, $R_e''$ and $R_e'''$ are the same or different, $R_e$, $R_e'$ and $R_e''$ are as defined above, $R_e'''$ has the same meaning as $R_e$ has, and $R_d$ is as defined above), a $R_b-SO_2-NR_e-R_d$- group (wherein $R_b$, $R_e$ and $R_d$ are as defined above), a $R_eR_e'N-SO_2-R_d$- group (wherein $R_e$, $R_e'$ and $R_d$ are as defined above), a C2-C10 alkenyl group or a C2-C10 alkynyl group;

(2) the $Y_0$ group: a $M_{b0}-R_d$- group, wherein $M_{b0}$ represents a $M_{c0}$- group

[wherein $M_{co}$ represents a $M_{do}-R_d'$- group {wherein $M_{do}$ represents a 6 to 10-membered aryl group optionally substituted with a $M_a$- group (wherein $M_a$ is as defined above), a 5 to 10-membered heteroaryl group optionally substituted with a $M_a$-group (wherein $M_a$ is as defined above), a 3 to 10-membered cyclic hydrocarbon or heterocyclic group optionally substituted with a $M_a$- group (wherein $M_a$ is as defined above) and optionally containing an unsaturated bond, a ($b_0$)- group represented by

($b_0$) 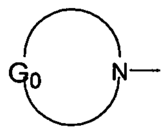

(wherein Go forms an optionally substituted, saturated or unsaturated, nonaromatic 5 to 14-membered hydrocarbon ring or heterocyclic ring), a ($c_0$)- group represented by

($c_0$) 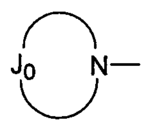

(wherein, $J_0$ forms a 5 to 7-membered aromatic ring optionally containing a nitrogen atom), a (do)- group represented by

($d_0$) 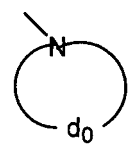

[wherein do forms a 5 to 12-membered hydrocarbon ring which is substituted with a carbonyl group or a thiocarbonyl group and further which may be optionally substituted with an oxy group, a thio group, a $-NR_1$- group {wherein $R_1$ represents a hydrogen atom, or a C1-C10 alkyl group, or a C2-C10 alkyl group substituted with a halogen atom or a $R_2-B_1$- group (wherein $R_2$ represents a C1-C10 alkyl group, a C3-C10 alkenyl group or a C3-C10 alkynyl group, and $B_1$ represents an oxy group, a thio group, a sulfinyl group or a sulfonyl group), a C3-C10 alkenyl group, or a C3-C10 alkynyl group}, a sulfinyl group, or a sulfonyl group], or a ($e_0$)- group represented by

($e_0$) 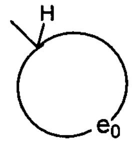

{wherein $e_0$ forms a 5 to 12-membered hydrocarbon ring optionally substituted with a carbonyl group, a thiocarbonyl group, an oxy group, a thio group, a $-NR_1$-group (wherein $R_1$ is as defined above), a sulfinyl group or a sulfonyl group}; and $R_d'$ is the same as or different from $R_d$ and has the same meaning as $R_d$ has],

a $M_{c0}-B_a$- group (wherein $M_{c0}$ and $B_a$ are as defined above), a $M_{c0}$-CO- group (wherein $M_{c0}$ is as defined above), a $M_{c0}$-CO-O-group (wherein $M_{c0}$ is as defined above), a $M_{c0}$O-CO- group (wherein $M_{c0}$ is as defined

above), a $M_{c0}R_eN$- group (wherein $M_{c0}$ and $R_e$ are as defined above), a $M_{c0}$-CO-$NR_e$- group (wherein $M_{c0}$ and $R_e$ are as defined above), a $M_{c0}$O-CO-$NR_e$-group (wherein $M_{c0}$ and $R_e$ are as defined above), a $M_{c0}R_eN$-CO-group (wherein $M_{c0}$ and $R_e$ are as defined above), a $M_{c0}R_eN$-CO-$NR_e$'- group (wherein $M_{c0}$, $R_e$ and $R_e$' are as defined above), a $M_{c0}R_eN$-C(=$NR_e$')-$NR_e$"- group (wherein $M_{c0}$, $R_e$, $R_e$' and $R_e$" are as defined above), a $M_{c0}$-$SO_2$-$NR_e$- group (wherein $M_{c0}$ and $R_e$ are as defined above) or a $M_{c0}R_eN$-$SO_2$- group (wherein $M_{c0}$ and $R_e$ are as defined above), and $R_d$ is as defined above;

(3) the $Z_0$ group: a 5- to 12-membered hydrocarbon ring or heterocyclic ring which may be substituted with a halogen atom, a C1-C10 alkoxy group, C3-C10 alkenyloxy group, a C3-C10 alkynyloxy group, a carbonyl group, a thiocarbonyl group, an oxy group, a thio group, a sulfinyl group or a sulfonyl group, and which is aromatic or nonaromatic and monocyclic or fused ring and is fused to the α ring; and

II. β' represents
a group represented by formula (I'-1):

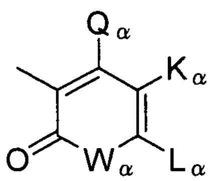
(I'-1)

wherein,

(1)$Q_\alpha$ represents an optionally substituted hydroxyl group, or an optionally substituted amino group,
(2)$W_\alpha$ represents an oxygen atom or a -$NT_\alpha$- group (wherein $T_\alpha$ represents a hydrogen atom, or a substituent on the nitrogen atom),
(3)$K_\alpha$ and $L_\alpha$ form a -$V_\alpha$=$V_\alpha$'-$V_\alpha$"=$V_\alpha$"'- group (wherein $V_\alpha$, $V_\alpha$', $V_\alpha$" and $V_\alpha$"' are the same or different, and represent an optionally substituted methine group or a -N= group, and at least one of $V_\alpha$, $V_\alpha$', $V_\alpha$" and $V_\alpha$"' represents a -N= group);
a group represented by formula (I'-2):

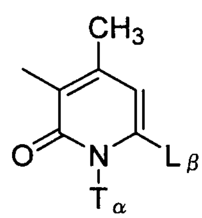
(I'-2)

wherein $T_\alpha$ is as defined above, and $L_\beta$ represents a hydroxyl group or a methyl group;
a group represented by formula (I'-3):

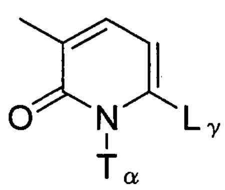
(I'-3)

wherein $T_\alpha$ is as defined above, and $L_\gamma$ represents a C1-C10 alkyl group;
a group represented by formula (I'-4):

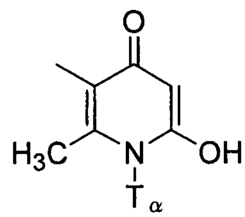

(I'-4)

wherein $T_\alpha$ is as defined above;
a group represented by formula (I'-5):

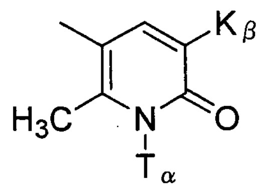

(I'-5)

wherein $T_\alpha$ is as defined above, and $K_\beta$ represents a cyano group or a UOCO- group (wherein U represents a hydrogen atom or a C1-C10 alkyl group);
a group represented by formula (I'-6):

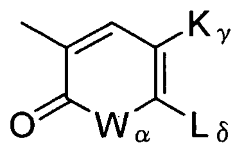

(I'-6)

wherein $W_\alpha$ is as defined above, and $K_r$ and $L_\delta$ form an optionally substituted C3-C10 alkylene group or an optionally substituted C4-C10 alkenylene group;
a group represented by formula (I'-7):

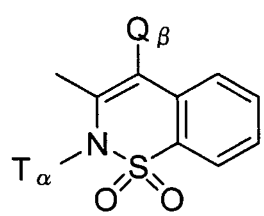

(I'-7)

wherein $T_\alpha$ is as defined above, and $Q_\beta$ represents an optionally substituted hydroxyl group; or
a group represented by formula (I'-8):

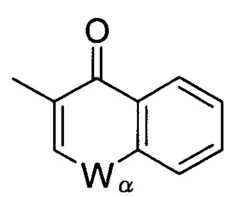

(I'-8)

wherein $W_\alpha$ is as defined above;
the term "as defined above" used for the same symbols among plural substituents means that the plural

substituents independently represent the same meaning as that described above and, among the plural substituents, although the selection range of substituents to be selected is the same, selected substituents may be the same or different as long as they are selected within the range.

**13.** A cinnamoyl compound represented by formula (II'):

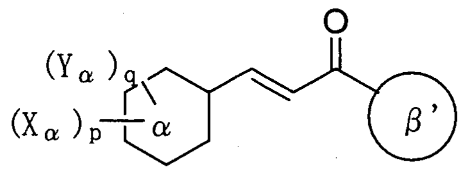

(II')

wherein,

I. $\alpha$ represents a benzene ring or a pyridine ring; in $(Y_\alpha)_q$, $Y_\alpha$ is a substituent on a carbon atom and represents a group included in the following $X_0$ group or the $Y_0$ group, q represents 0, 1, 2, 3, 4 or 5, and when q is not less than 2, $Y_\alpha$s are the same or different, and when q is not less than 2, adjacent two same or different $Y_\alpha$s may together form a group included in the $Z_0$ group to be fused to the $\alpha$ ring; and in $(X_\alpha)_p$, $X_\alpha$ is a substituent on a carbon atom and represents a group which does not belong to the following $X_0$ group, $Y_0$ group and $Z_0$ group, p represents 0, 1, 2, 3, 4 or 5, and when p is not less than 2, $X_\alpha$s are the same or different and the sum of p and q is not more than 5;

(1) the $X_0$ group: a $M_a$-group, wherein $M_a$ represents a $R_b$- group (wherein $R_b$ represents a C1-C10 alkyl group optionally substituted with a halogen atom), a halogen atom, a nitro group, a cyano group, a hydroxyl group, a $R_c$-$B_a$-$R_d$-group (wherein $R_c$ represents a C1-C10 alkyl group optionally substituted with a halogen atom, $B_a$ represents an oxy group, a thio group, a sulfinyl group or a sulfonyl group, and $R_d$ represents a single bond or a C1-C10 alkylene group), an $HOR_d$- group (wherein $R_d$ is as defined above), a $R_e$-CO-$R_d$- group (wherein $R_e$ represents a hydrogen atom, or a C1-C10 alkyl group optionally substituted with a halogen atom, and $R_d$ is as defined above), a $R_e$-CO-O-$R_d$- group (wherein $R_e$ and $R_d$ are as defined above), a $R_e$O-CO-$R_d$- group (wherein $R_e$ and $R_d$ are as defined above), an HO-CO-CH=CH-group, a $R_eR_e$'N-$R_d$- group (wherein $R_e$ and $R_e$' are the same or different, $R_e$ is as defined above, $R_e$' has the same meaning as $R_e$ has, and $R_d$ is as defined above), a $R_e$-CO-N$R_e$'-$R_d$- group (wherein $R_e$, $R_e$' and $R_d$ are as defined above), a $R_b$O-CO-N($R_e$)-$R_d$- group (wherein $R_b$, $R_e$ and $R_d$ are as defined above), a $R_eR_e$'N-CO-$R_d$- group (wherein $R_e$, $R_e$' and $R_d$ are as defined above), a $R_eR_e$'N-CO-N$R_e$''-$R_d$- group (wherein $R_e$, $R_e$' and $R_e$'' are the same or different, $R_e$ and $R_e$' are as defined above, $R_e$'' has the same meaning as $R_e$ has, and $R_d$ is as defined above), a $R_eR_e$'N-C(=N$R_e$'')-N$R_e$'''-$R_d$- group (wherein $R_e$, $R_e$', $R_e$'' and $R_e$''' are the same or different, $R_e$, $R_e$' and $R_e$'' are as defined above, $R_e$''' has the same meaning as $R_e$ has, and $R_d$ is as defined above), a $R_b$-$SO_2$-N$R_e$-$R_d$- group (wherein $R_b$, $R_e$ and $R_d$ are as defined above), a $R_eR_e$'N-$SO_2$-$R_d$- group (wherein $R_e$, $R_e$' and $R_d$ are as defined above), a C2-C10 alkenyl group or a C2-C10 alkynyl group;

(2) the $Y_0$ group: a $M_{b0}$-$R_d$- group, wherein $M_{b0}$ represents a $M_{c0}$- group [wherein $M_{co}$ represents a $M_{do}$-$R_d$'- group {wherein $M_{do}$ represents a 6 to 10-membered aryl group optionally substituted with a $M_a$- group (wherein $M_a$ is as defined above), a 5 to 10-membered heteroaryl group optionally substituted with a $M_a$-group (wherein $M_a$ is as defined above), a 3 to 10-membered cyclic hydrocarbon or heterocyclic group optionally substituted with a $M_a$- group (wherein $M_a$ is as defined above) and optionally containing an unsaturated bond, a ($b_0$)- group represented by

($b_0$) 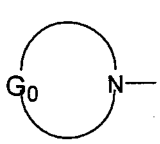

(wherein Go forms an optionally substituted, saturated or unsaturated, nonaromatic 5 to 14-membered hydrocarbon ring or heterocyclic ring), a ($c_0$)- group represented by

($c_0$) 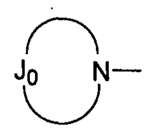

(wherein, $J_0$ forms a 5 to 7-membered aromatic ring optionally containing a nitrogen atom), a (do)- group represented by

($d_0$) 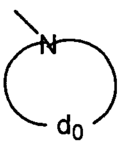

[wherein do forms a 5 to 12-membered hydrocarbon ring which is substituted with a carbonyl group or a thiocarbonyl group and further which may be optionally substituted with an oxy group, a thio group, a -$NR_1$- group {wherein $R_1$ represents a hydrogen atom, or a C1-C10 alkyl group, or a C2-C10 alkyl group substituted with a halogen atom or a $R_2$-$B_1$- group (wherein $R_2$ represents a C1-C10 alkyl group, a C3-C10 alkenyl group or a C3-C10 alkynyl group, and $B_1$ represents an oxy group, a thio group, a sulfinyl group or a sulfonyl group), a C3-C10 alkenyl group, or a C3-C10 alkynyl group}, a sulfinyl group, or a sulfonyl group], or a ($e_0$)- group represented by

($e_0$) 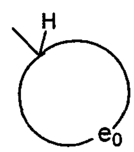

{wherein $e_0$ forms a 5 to 12-membered hydrocarbon ring optionally substituted with a carbonyl group, a thiocarbonyl group, an oxy group, a thio group, a -$NR_1$-group (wherein $R_1$ is as defined above), a sulfinyl group or a sulfonyl group}; and $R_d$' is the same as or different from $R_d$ and has the same meaning as $R_d$ has],
a $M_{c0}$-$B_a$- group (wherein $M_{c0}$ and $B_a$ are as defined above) ; a $M_{c0}$-CO- group (wherein $M_{c0}$ is as defined above), a $M_{c0}$-CO-O-group (wherein $M_{c0}$ is as defined above), a $M_{c0}$O-CO- group (wherein $M_{c0}$ is as defined above), a $M_{c0}R_eN$- group (wherein $M_{c0}$ and $R_e$ are as defined above), a $M_{c0}$-CO-$NR_e$- group (wherein $M_{c0}$ and $R_e$ are as defined above), a $M_{c0}$O-CO-$NR_e$-group (wherein $M_{c0}$ and $R_e$ are as defined above), a $M_{c0}R_eN$-CO-group (wherein $M_{c0}$ and $R_e$ are as defined above), a $M_{c0}R_eN$-CO-$NR_e$'- group (wherein $M_{c0}$, $R_e$ and $R_e$' are as defined above), a $M_{c0}R_eN$-C(=$NR_e$')-$NR_e$"- group (wherein $M_{c0}$, $R_e$, $R_e$' and Re" are as defined above), a $M_{c0}$-$SO_2$-$NR_e$- group (wherein $M_{c0}$ and $R_e$ are as defined above) or a $M_{c0}R_eN$-$SO_2$- group (wherein $M_{c0}$ and $R_e$ are as defined above), and $R_d$ is as defined above;
(3) the $Z_0$ group: a 5- to 12-membered hydrocarbon ring or heterocyclic ring which may be substituted with a halogen atom, a C1-C10 alkoxy group, C3-C10 alkenyloxy group, a C3-C10 alkynyloxy group, a carbonyl group, a thiocarbonyl group, an oxy group, a thio group, a sulfinyl group or a sulfonyl group, and which is aromatic or nonaromatic and monocyclic or fused ring and is fused to the α ring; and

II. β' represents
a group represented by formula (II'-1):

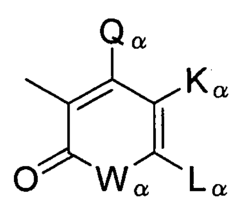

(II'-1)

wherein,

(1)$Q_\alpha$ represents an optionally substituted hydroxyl group, or an optionally substituted amino group,
(2)Wα represents an oxygen atom or a -NTα- group (wherein $T_\alpha$ represents a hydrogen atom, or a substituent on the nitrogen atom),
(3)$K_\alpha$ and $L_\alpha$ form a $-V_\alpha=V_\alpha'-V_\alpha''=V_\alpha'''-$ group (wherein $V_\alpha$, $V_\alpha'$, $V_\alpha''$ and $V_\alpha'''$ are the same or different, and represent an optionally substituted methine group or a -N= group, and at least one of $V_\alpha$, $V_\alpha'$, $V_\alpha''$ and $V_\alpha'''$ represents a -N= group);
a group represented by formula (II'-2):

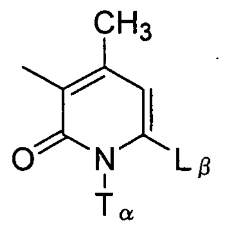

(II'-2)

wherein $T_\alpha$ is as defined above, and $L_\beta$ represents a hydroxyl group or a methyl group;
a group represented by formula (II'-3):

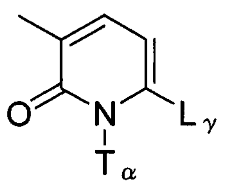

(II'-3)

wherein $T_\alpha$ is as defined above, and $L_\gamma$ represents a C1-C10 alkyl group;
a group represented by formula (II'-4):

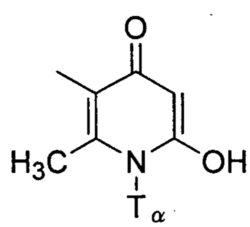

(II'-4)

wherein $T_\alpha$ is as defined above;
a group represented by formula (II'-5):

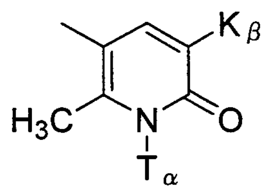

(II'-5)

wherein $T_\alpha$ is as defined above, and $K_\beta$ represents a cyano group or a UOCO- group (wherein U represents a hydrogen atom or a C1-C10 alkyl group);

a group represented by formula (II'-6):

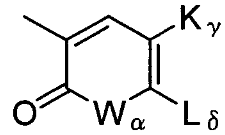

(II'-6)

wherein $W_{\alpha}$ is as defined above, and $K_{\gamma}$ and $L_{\delta}$ form an optionally substituted C3-C10 alkylene group or an optionally substituted C4-C10 alkenylene group;
a group represented by formula (II'-7):

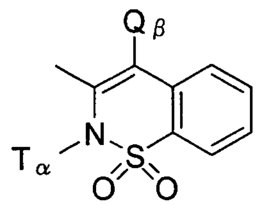

(II'-7)

wherein $T_{\alpha}$ is as defined above, and $Q_{\beta}$ represents an optionally substituted hydroxyl group; or
a group represented by formula (II'-8):

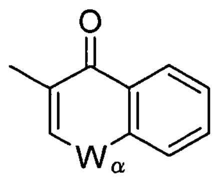

(II'-8)

wherein $W_{\alpha}$ is as defined above;
the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range.

**14.** A cinnamoyl compound represented by formula (V'):

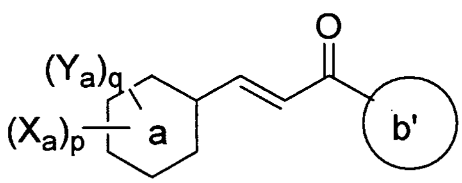

(V')

[wherein,

I. a represents a benzene ring or a pyridine ring, $X_a$ is a substituent on a carbon atom and represents a C1-C10 alkyl group substituted with a cyano group, or a C1-C10 alkyl group substituted with a tetrahydropyran-4-ylidene group, or a C2-C10 alkenyl group substituted with a halogen atom or a cyano group, or a C2-C10 alkenyl group substituted with a C1-C10 alkoxycarbonyl group, or a C3-C10 alkynyl group substituted with a hydroxyl group, or an $a_0$-$r_1$-b-$r_1$'- group {wherein $a_0$ represents a methyl group substituted with a C1-C10 alkylthio group, a methyl group substituted with a C1-C10 alkylsulfinyl group, a methyl group substituted with a C1-C10 alkylsulfonyl group, a C2-C10 alkenyl group, a C2-C10 alkynyl group, a $r_2$O-CO- group (wherein $r_2$ represents a C1-

C10 alkyl group or a C2-C10 alkyl group substituted with a hydroxyl group), a carboxy group, a rr'N-CO- group (wherein r and r' are the same or different, and respresent a hydrogen atom or a C1-C10 alkyl group), $a_1$-NH-CO- group (wherein $a_1$ represents a C2-C10 alkyl group substituted with a C1-C10 alkoxy group), an $a_1$'-CO- group (wherein $a_1$' represents a morpholino group), a rr'N-$CH_2$- group (wherein r and r' are as defined above), a $r_0$-$(O)_1$-CONH-$CH_2$- group (wherein $r_0$ represents a C1-C10 alkyl group, and 1 represents 0 or 1), a r-$OCH_2$-group (wherein r is as defined above), a $r_0$-CO- group (wherein $r_0$ is as defined above), a cyano group or a sulfomethyl group, $r_1$ represents a C1-C10 alkylene group, $r_1$' represents a single bond or a C1-C10 alkylene group, and b represents an oxy group, a thio group, a sulfinyl group, a sulfonyl group, or an imino group}, or an $a_2$-y-CO-NH- group (wherein $a_2$ represents a C2-C10 alkyl group substituted with a C1-C10 alkoxy group, and y represents an oxy group or an imino group), or a rO-COCO-NH- group (wherein r is as defined above), or an $a_3$-z-NH- group (wherein $a_3$ represents a C2-C10 alkenyl group, or a C1-C10 alkyl group substituted with a C1-C10 alkoxy group, a C1-C10 alkoxycarbonyl group, a carboxy group or a cyano group, and z represents a carbonyl group or a sulfonyl group), or an $a_4$-NHCO- group {wherein $a_4$ represents a C1-C10 alkoxy group, or a C3-C10 alkenyloxy group, or a $r_0$-$SO_2$- group (wherein $r_0$ is as defined above), or a C2-C10 alkyl group substituted with a hydroxyl group or a C1-C10 alkoxy group, or a C2-C10 alkyl group substituted with a $r_0r_0$'N-group (wherein $r_0$ is as defined above, and $r_0$' is the same as or different from $r_0$, and represents the same meaning as $r_0$ has), a C1-C10 alkyl group substituted with or a rO-CO- group (wherein r is as defined above), a cyano group or an aminocarbonyl group, or a rO-CO-(rO-$COCH_2$)CH- group (wherein r is as defined above)}, or an $a_5$-$NHSO_2$- group (wherein $a_5$ represents a C2-C10 alkyl group substituted with a C1-C10 alkoxyl group), or a $r_0$ON=CH- group (wherein $r_0$ is as defined above), or a $r_0$NHCSNH- group (wherein $r_0$ is as defined above), or a $r_0$NHC(-S$r_0$')=N- group (wherein $r_0$ and $r_0$' are as defined above), or a $(rO)_2P(=O)CH_2$- group (wherein r is as defined above), p represents 0, 1, 2 or 3 and, and when p is not less than 2, $X_a$s are same or different,

$Y_a$ represents are halogen atom, or a C1-C10 alkyl group optionally substituted with a halogen atom, or a C1-C10 alkyl group optionally substituted with a C1-C10 alkoxy group, or a C2-C10 alkenyl group, or a C2-C10 alkynyl group, or a 2-oxo-oxazolidin-3-yl group, or a [1,3]dioxolan-2-yl group, or a C1-C10 alkoxy group substituted with a morpholino group, or an $a_0$'-b'- group (wherein $a_0$' represents a C1-C10 alkyl group optionally substituted with a halogen atom, and b' represents an oxy group, a thio group, a sulfinyl group or a sulfonyl group), or a nitro group, or a cyano group, or a rO-CO- group (wherein r is as defined above), or a $r_0r_0$'N- group (wherein $r_0$ and $r_0$' are as defined above), or a $r_0$CO-NH- group (wherein $r_0$ is as defined above), or a $r_0r_0$'NCONH-group (wherein $r_0$ and $r_0$' are as defined above), or a rr'NCO- group (wherein r and r' are as defined above), or a hydroxyl group, q represents 0, 1, 2 or 3, and when q is not less than 2, $Y_a$s are the same or different, and when q is not less than 2, adjacent $Y_a$s may be fused to the a ring to form a 4,5,6,7-tetrahydrobenzo[b]thiophene ring;

II. b is

a group represented by formula (V'-1):

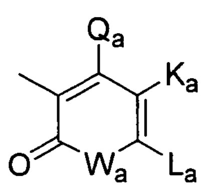

(V' -1)

wherein $Q_a$ represents a $r_a$-O- group {$r_a$ represents a hydrogen atom, or a C1-C10 alkyl group, or a C3-C10 alkenyl group, or a C3-C10 alkynyl, or a C1-C10 alkyl group substituted with a $r_0r_0$'N-$CH_2$- group (wherein $r_0$ and $r_0$' are the same or different), a $rOCH_2$ group (wherein r is as defined above), a $r_0$-CO- group (wherein $r_0$ is as defined above), a C1-C10 alkoxycarbonyl group, a carboxy group, an aminocarbonyl group or a cyano group, or a $r_3$-$r_1$-group (wherein $r_3$ represents a phenyl group or a pyridyl group, and $r_1$ is as defined above)}, or a piperidino group, or a morpholino group, or a $r_4r_4$'N-group (wherein $r_4$ and $r_4$' are the same of different, and represent a hydrogen atom, or a C1-C10 alkyl group, or C3-C10 alkenyl group, or a C3-C10 alkynyl group, or a C2-C10 alkyl group substituted with a C1-C10 alkoxy group, provided that they are not a hydrogen atom at the same time); $W_a$ represents an oxygen atom or a - $NT_a$- group [wherein $T_a$ represents a $r_b$- group (wherein $r_b$ is the same as or different from $r_a$, and represents the same meaning as $r_a$ has) or a $r_3$' - group (wherein $r_3$' is the same as or different from $r_3$, and represents the same meaning as $r_3$ has)], and $K_a$ and $L_a$ form a -$V_a$=$V_a$' -$V_a$''=$V_a$'''- group (wherein $V_a$, $V_a$', $V_a$'' and $V_a$''' are the same or different, and represent a methine group or a -N= group, and at least one of $V_a$, $V_a$' $V_a$'' and $V_a$''' represents a -N= group);

a group represented by formula (V'-2):

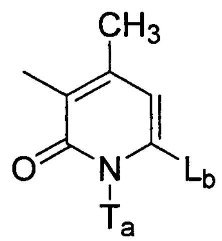

(V'-2)

wherein $T_a$ is as defined above, and $L_b$ represents a hydroxyl group or a methyl group;
a group represented by formula (V'-3):

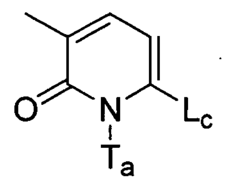

(V'-3)

wherein $T_a$ is as defined above, and $L_c$ represents a C1-C10 alkyl group;
a group represented by formula (V'-4):

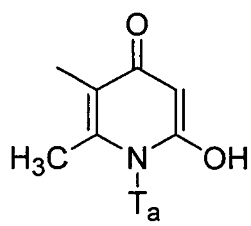

(V'-4)

wherein $T_a$ is as defined above;
a group represented by formula (V'-5):

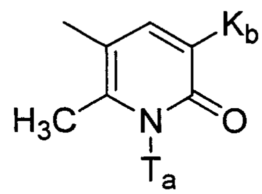

(V'-5)

wherein $T_a$ is as defined above, and $K_b$ represents a cyano group or a UOCO- group (wherein U represents a hydrogen atom or C1-C10 alkyl group);
a group represented by formula (V'-6):

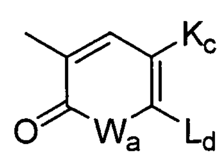

(V'-6)

wherein $W_a$ is as defined above, and $K_c$ and $L_d$ form a C3-C10 alkylene group or a C4-C10 alkenylene group; a group represented by formula (V'-7):

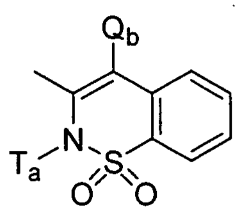

(V' -7)

wherein $T_a$ is as defined above, and $Q_b$ represents a $r_a$-O-group (wherein $r_a$ is as defined above); or a group represented by formula (V'-8):

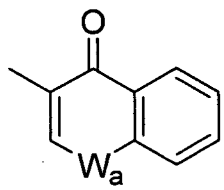

(V' -8)

wherein $W_a$ is as defined above; and
the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range.

**15.** A composition for suppressing transcription of an extracellular matrix gene, which comprises the compound according to any one of claims 2 to 5, 13 and 14, and an inert carrier.

**16.** A composition for suppressing transcription of an extracellular matrix gene, which comprises the compound according to claim 5 or 14, and an inert carrier.

**17.** Use of a cinnamoyl compound contained in the composition according to claim 1 or 12 as an active ingredient, as an active ingredient for suppressing transcription of an extracellular matrix gene.

**18.** Use of the compound according to any one of claims 2 to 5, 13 and 14, as an active ingredient for suppressing transcription of an extracellular matrix gene.

**19.** Use of the compound according to claim 5 or 14, as an active ingredient for suppressing transcription of an extracellular matrix gene.

**20.** Use of a cinnamoyl compound contained in the composition according to claim 1 or 12 as an active ingredient, as an active ingredient for decreasing expression of an extracellular matrix gene to induce a reduction in accumulation of an extracellular matrix and thereby improving tissue fibrosis.

**21.** Use of the compound according to any one of claims 2 to 5, 13 and 14, as an active ingredient for decreasing expression of an extracellular matrix gene to induce a reduction in accumulation of an extracellular matrix and thereby improving tissue fibrosis.

**22.** A method for improving tissue fibrosis, which comprises administering an effective amount of a cinnamoyl compound contained in the composition according to claim 1 or 12 as an active ingredient to a mammalian patient in need thereof.

**23.** A method for improving tissue fibrosis, which comprises administering an effective amount of the compound according to any one of claims 2 to 5, 13 and 14 to a mammalian patient in need thereof.

**24.** An agent for treating chronic renal failure, which comprises the compound according to any one of claims 2 to 5, 13 and 14 and an inert carrier.

**25.** Use of a cinnamoyl compound contained in the composition according to claim 1 or 12 as an active ingredient, or the compound according to any one of claims 2 to 5, 13 and 14, as an active ingredient for treating chronic renal failure.

**26.** A method for treating chronic renal failure, which comprises administering an effective amount of a cinnamoyl compound contained in the composition according to claim 1 or 12 as an active ingredient, or the compound according to any one of claims 2 to 5, 13 and 14 to a mammalian patient in need thereof.

**27.** An agent for treating heart failure, which comprises a cinnamoyl compound contained in the composition according to claim 1 or 12 as an active ingredient, or the compound according to any one of claims 2 to 5, 13 and 14, and an inert carrier.

**28.** Use of a cinnamoyl compound contained in the composition according to claim 1 or 12 as an active ingredient, or the compound according to any one of claims 2 to 5, 13 and 14, as an active ingredient for treating heart failure.

**29.** A method for treating heart failure, which comprises administering an effective amount of a cinnamoyl compound contained in the composition according to claim 1 or 12 as an active ingredient, or the composition according to any one of claims 2 to 5, 13 and 14 to a mammalian patient in need thereof.

**30.** A composition for suppressing the activity of TGF-β, which comprises a cinnamoyl compound contained in the composition according to claim 1 or 12 as an active ingredient, and an inert carrier.

**31.** A composition for suppressing the activity of TGF-β, which comprises the compound according to any one of claims 2 to 5, 13 and 14, and an inert carrier.

**32.** Use of a cinnamoyl compound contained in the composition according to claim 1 or 12 as an active ingredient, as an active ingredient for suppressing the activity of TGF-β.

**33.** Use of the compound according to any one of claims 2 to 5, 13 and 14, as an active ingredient for suppressing the activity of TGF-β.

**34.** A composition for hair growth which comprises a cinnamoyl compound contained in the composition according to claim 1 or 12 as an active ingredient, or the compound according to any one of claims 2 to 5, 13 and 14, and an inert carrier.

**35.** Use of a cinnamoyl compound contained in the composition according to claim 1 or 12 as an active ingredient, or the compound according to any one of claims 2 to 5, 13 and 14, as an active ingredient for inhibiting a promoting effect of TGF-β on transition to a hair regression phase to induce extension of a hair growth phase and thereby providing a hair-growing effect.

**36.** A method for growing hair, which comprises administering an effective amount of a cinnamoyl compound contained in the composition according to claim 1 or 12 as an active ingredient, or the compound according to any one of claims 2 to 5, 13 and 14, to a mammalian patient in need thereof.

**INTERNATIONAL SEARCH REPORT**

International application No.
PCT/JP2006/304539

A. CLASSIFICATION OF SUBJECT MATTER
***A61K31/352***(2006.01), ***A61K31/4375***(2006.01), ***A61K31/4412***(2006.01), ***A61K31/47***(2006.01), ***A61K31/5415***(2006.01), ***A61P9/04***(2006.01), ***A61P13/12***(2006.01), ***A61P17/14***(2006.01), ***A61P43/00***(2006.01), ***C07D213/64***(2006.01),
According to International Patent Classification (IPC) or to both national classification and IPC

B. FIELDS SEARCHED
Minimum documentation searched (classification system followed by classification symbols)
A61K31/352, A61K31/4375, A61K31/4412, A61K31/47, A61K31/5415, A61P9/04, A61P13/12, A61P17/14, A61P43/00, C07D213/64, C07D213/69, C07D213/80, C07D213/803, C07D215/22, C07D279/02, C07D311/12, C07D311/22, C07D401/06,

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAPLUS(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | LEVAI, A. et al., "Dimethyldioxirane epoxidation of 3-aryl-1-(3-coumarinyl)propen-1-ones", Journal of Heterocyclic Chemistry, 2004, Vol.41, No.5, pages 707 to 709, particularly, compounds 1 to 10 | 2-5,13,14 |
| X | HAGGARTY, S.J. et al., "Small Molecule Modulation of the Human Chromatid Decatenation Checkpoint", Chemistry & Biology, 2003, Vol.10, No.12, pages 1267 to 1279, particularly, page 1270, 48NO2 | 2-5,13,14 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 31 May, 2006 (31.05.06) | 13 June, 2006 (13.06.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2006/304539

C (Continuation). DOCUMENTS CONSIDERED TO BE RELEVANT

| Category* | Citation of document, with indication, where appropriate, of the relevant passages | Relevant to claim No. |
|---|---|---|
| X | OGANESIAN, E.T. et al., "Electronic structure-activity relationships (ESAR) in propene derivatives. 1,4-Carboxyvinylenechalcones and 3-cinnamoylcoumarins", Khimiko-Farmatsevticheskii Zhurnal, 1994, Vol.28, No.11, pp.36-3, particularly, compound II | 2-5,13,14 |
| X | HASSAN, M.A. et al., "Synthesis and reactions of polysubstituted 2(1H)pyridones and pyridines", Revue Roumaine de Chimie, 1982, Vol.27, No.8, pp.963-7, particularly, compound III | 2-6,13,14 |
| X | POLYAKOV, V.K. et al., "Analogs of chalcones from substituted 3-formyland 3-acetylchromones", Ukrainskii Khimicheskii Zhurnal (Russian Edition) 1982, Vol.48, No.7, pp.772-5, particularly, compounds XXI to XXVIII | 2-6,13,14 |
| X | DIMROTH, P. et al., "Reaction of diketene with acetoacetamides", Liebigs Annalen der Chemie, 1979, Vol.D, No.6, pp.769-75, particularly, compound 13 | 2-6,13,14 |
| X<br>A | JP 2003-21901 A (Fuji Photo Film Co., Ltd.), 24 January, 2003 (24.01.03), Pages 10, 11 & EP 1273971 A2 & US 2003/162130 A1 | 2-4,13<br>5,14 |
| X<br>A | JP 2-279702 A (NOF Corp.), 15 November, 1990 (15.11.90), Page 7 (Family: none) | 2,3,13<br>4,5,14 |
| X<br>A | JP 61-114234 A (The Mead Corp.), 31 May, 1986 (31.05.86), Page 10, table 2 & CA 1293407 A1 & US 4713312 A | 2-4,13<br>5,14 |
| X<br>A | JP 56-4604 A (Eastman Kodak Co.), 19 January, 1981 (19.01.81), Page 7, table 1; page 8, table 2 & EP 22188 A2 & US 4278751 A & CA 1137696 A1 | 2-4,13<br>5,14 |
| A | JP 2004-175780 A (Sumitomo Chemical Co., Ltd.), 24 June, 2004 (24.06.04), Full text & WO 2003/80592 A1 | 1-6,12-16, 24,27,30,31, 34 |
| A | JP 2004-123620 A (Sumitomo Chemical Co., Ltd.), 22 April, 2004 (22.04.04), Full text (Family: none) | 1-6,12-16, 24,27,30,31, 34 |

Form PCT/ISA/210 (continuation of second sheet) (April 2005)

**INTERNATIONAL SEARCH REPORT**

International application No.
PCT/JP2006/304539

C (Continuation). DOCUMENTS CONSIDERED TO BE RELEVANT

| Category* | Citation of document, with indication, where appropriate, of the relevant passages | Relevant to claim No. |
|---|---|---|
| A | JP 2004-123621 A (Sumitomo Chemical Co., Ltd.), 22 April, 2004 (22.04.04), Full text (Family: none) | 1-6,12-16, 24,27,30,31, 34 |
| P,A | WO 2005/028439 A1 (Sumitomo Chemical Co., Ltd.), 31 March, 2005 (31.03.05), Full text & JP 2006-104062 A | 1-6,12-16, 24,27,30,31, 34 |
| P,A | WO 2005/028441 A1 (Sumitomo Chemical Co., Ltd.), 31 March, 2005 (31.03.05), Full text & JP 2006-104061 A | 1-6,12-16, 24,27,30,31, 34 |
| P,A | WO 2005/028441 A1 (Sumitomo Chemical Co., Ltd.), 31 March, 2005 (31.03.05), Full text & JP 2006-104061 A | 1-6,12-16, 24,27,30,31, 34 |
| P,A | WO 2005/028463 A1 (Sumitomo Chemical Co., Ltd.), 31 March, 2005 (31.03.05), Full text & JP 2006-104063 A | 1-6,12-16, 24,27,30,31, 34 |

Form PCT/ISA/210 (continuation of second sheet) (April 2005)

**INTERNATIONAL SEARCH REPORT**

International application No.
PCT/JP2006/304539

Continuation of A. CLASSIFICATION OF SUBJECT MATTER
(International Patent Classification (IPC))

***C07D213/69***(2006.01), ***C07D213/80***(2006.01), ***C07D213/803***(2006.01), ***C07D215/22***(2006.01), ***C07D279/02***(2006.01), ***C07D311/12***(2006.01), ***C07D311/22***(2006.01), ***C07D401/06***(2006.01), ***C07D405/06***(2006.01), ***C07D413/08***(2006.01), ***C07D417/06***(2006.01), ***C07D471/04***(2006.01)

(According to International Patent Classification (IPC) or to both national classification and IPC)

Continuation of B. FIELDS SEARCHED
Minimum documentation searched (International Patent Classification (IPC))

C07D405/06, C07D413/08, C07D417/06, C07D471/04

Minimum documentation searched (classification system followed by classification symbols)

Form PCT/ISA/210 (extra sheet) (April 2005)

**INTERNATIONAL SEARCH REPORT**

International application No.
PCT/JP2006/304539

**Box No. II Observations where certain claims were found unsearchable (Continuation of item 2 of first sheet)**

This international search report has not been established in respect of certain claims under Article 17(2)(a) for the following reasons:

1. [×] Claims Nos.: (see below)
because they relate to subject matter not required to be searched by this Authority, namely:
Claims 17 to 23, 25, 26, 28, 29, 32, 33, 35 and 36 pertain to methods for treatment of the human body by therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
This international application includes at least the following three inventions:
1st invention: an invention pertaining to a composition for inhibiting the extracellular matrix gene transcription of claims 1, 12, 15 and 16, an invention pertaining to a cinnamoyl compound of claims 2 to 5, 13 and 14, an invention pertaining to a process for producing a cinnamoyl compound of claim 6, an invention pertaining to a therapeutic agent of claims 24 and 27, an invention pertaining to a composition for inhibiting a TGF-β activity of claims 30 and 31, and an invention pertaining to a mediated tonic composition of claim 34;

(continued to extra sheet)

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [×] No required additional search fees were timely paid by the applicant. Consequently, this international search report is restricted to the invention first mentioned in the claims; it is covered by claims Nos.:
1 to 6, 12 to 16, 24, 27, 30, 31 and 34.

**Remark on Protest** the

[ ] The additional search fees were accompanied by the applicant's protest and, where applicable, payment of a protest fee..

[ ] The additional search fees were accompanied by the applicant's protest but the applicable protest fee was not paid within the time limit specified in the invitation.

**INTERNATIONAL SEARCH REPORT**

International application No.
PCT/JP2006/304539

Continuation of Box No.III of continuation of first sheet(2)

2nd invention: an invention pertaining to an intermediate of claims 7, 10 and 11, and an invention pertaining to a process for producing the intermediate; and
3rd invention: an invention pertaining to an intermediate of claims 8 and 9, and an invention pertaining to a process for producing the intermediate.

The inventions of the 1st invention include a specific cinnamoyl compound; the inventions of the 2nd invention pertain to an intermediate in the production of the cinnamoyl compound. The common structure between the cinnamoyl compound of the 1st invention and the intermediate of the 2nd invention is publicly known, as disclosed in, for example, JP 2002-371078 A and WO 1992/081483 A1.
The inventions of the 3rd invention pertain to an intermediate in the production of the intermediate of the 2nd invention. However, it is obvious that there is no common structure between the intermediate of the 1st invention and the intermediate of the 2nd invention and there is also no common structure between the intermediate of the 3rd invention and the cinnamoyl compound of the 1st invention.
Since it does not appear that there is a technical relationship among the three inventions mentioned above involving special technical features beyond the prior art, these inventions are not considered as relating to a group of inventions so linked as to form a single general inventive concept.
In addition, as a result of the search, it was clearly found that the inventions pertaining to the compounds of claims 2 to 5, 13 and 14 include a plurality of known compounds. Therefore, the 1st invention includes multiple inventions.

<Supplemental remarks on related documents>

As for the compounds corresponding to those of claims 2 to 5, 13 and 14, compounds disclosed in the documents of the following registry numbers are also publicly known, in addition to those disclosed in the documents cited as citations. However, since the number of the citations becomes excessively large if these documents are also added as citations, these documents are not presented as citations. (Each of these known compounds has any one of the structures shown as "○-2", "○-3", "○-6" or "○-8" in the claims.)

| | | | | |
|---|---|---|---|---|
| 79072-79-0 | 79984-74-0 | 80467-51-2 | 91527-64-9 | 91527-66-1 |
| 103974-22-7 | 107126-89-6 | 119233-95-3 | 119233-96-4 | 120040-19-9 |
| 120996-74-9 | 130029-48-0 | 130029-49-1 | 130029-50-4 | 130029-51-5 |
| 130029-52-6 | 130029-53-7 | 136261-21-7 | 136261-22-8 | 136261-23-9 |
| 140399-58-2 | 142835-09-4 | 143150-66-7 | 143150-67-8 | 152208-79-2 |
| 152208-80-5 | 152208-85-0 | 152208-87-2 | 159760-51-7 | 169826-68-0 |
| 169826-70-4 | 172369-25-4 | 174842-31-0 | 174842-32-1 | 188570-54-9 |
| 193619-91-9 | 193619-94-2 | 193619-95-3 | 193619-96-4 | 193619-97-5 |
| 198014-95-8 | 225367-38-4 | 225367-39-5 | 225367-40-8 | 225367-41-9 |
| 225367-42-0 | 401515-49-9 | 401515-50-2 | 401515-52-4 | 401516-56-3 |
| 402932-83-6 | 402932-84-7 | 402932-87-0 | 416890-22-7 | 676618-09-0 |
| 855160-81-5 | 856078-65-4 | | | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9735565 A
- JP 09227547 B
- WO 0020371 A
- JP 2002371078 B
- WO 0179187 A
- WO 9218483 A
- EP 330645 A
- WO 2005028439 A


### Non-patent literature cited in the description


- *J. Invest. Dermatol.,* 1990, vol. 94, 365
- *Proc. Natl. Acad. Sci. USA,* 1991, vol. 88, 6642
- *J. Am. Soc. Nephrol.,* 2004, vol. 15, 2637
- *Cardiovasc. Pathol.,* 2004, vol. 13, 119
- *Clin. Nephrol.,* 1995, vol. 44, 211
- *J. Hepatol.,* 1998, vol. 29, 263
- *Lab. Invest.,* 1990, vol. 63, 171
- *Diabetes,* 1996, vol. 45, 522-530
- *Proc. Natl. Acad. Sci. USA,* 1999, vol. 96, 12719-12724
- *Proc. Natl. Acad. Sci. USA,* 2000, vol. 97, 8015-8020
- *Autoimmunity,* 2002, vol. 35, 277-282
- *J. Hepatol.,* 2002, vol. 37, 331-339
- *Life Sci.,* 2002, vol. 71, 1559-1606
- *Liebigs Annalen der Chemie,* 1979, vol. D (6), 769
- *J. Heterocyclic Chem.,* 1981, vol. 18 (3), 603
- *Polish J. Chem.,* 1982, vol. 56 (2), 419
- *Synthetic Communication,* 1986, vol. 16 (10), 1195
- *Ukrainskii Khimicheskii Zhurnal,* 1982, vol. 48 (7), 772
- *J. Organic Chem.,* 1965, vol. 30, 2241
- *Tetrahederon,* 1997, vol. 53 (47), 16061
- *Clin. Liver Dis.,* 2003, vol. 7, 195-210
- *Am. J. Physiol. Renal Phsiol.,* 2000, vol. 278, F830-F838
- *Kidney Int.,* 2003, vol. 64, 149-159
- *Am. J. Pathol.,* 2001, vol. 158, 1653-1663
- *Kidney Int.,* 2001, vol. 60, 1745-1755
- *J. Invest. Dermatol.,* 2001, vol. 118, 461-470